# EUROPEAN PATENT SPECIFICATION

(11) **EP 1 889 838 B1**
(45) Date of publication and mention of the grant of the patent: **14.10.2009**
(21) Application number: 07112106.5
(22) Date of filing: 24.05.2001
(51) Int. Cl.: C07D 231/48, C07D 401/04, C07D 401/12, C07D 401/14, C07D 403/04, C07D 403/12, C07D 403/14, C07D 405/04, C07D 405/14, C07D 409/04, C07D 417/04, C07D 417/12, A61K 31/4439, A61K 31/415, A61K 31/506, A61P 7/00

(54) **Thrombopoietin mimetics**
Thrombopoietin-Mimetika
Substance mimétique à thrombopoïétine

(30) Priority: 25.05.2000 US 207084 P; 30.08.2000 US 228929 P
(43) Date of publication of application: 20.02.2008
(62) Divisional of application: 01941599.1
(73) Proprietor: SmithKline Beecham Corporation, Philadelphia, PA 19101 (US)
(72) Inventor: Duffy, Kevin, J., Collegeville, PA 19426 (US); Eppley, Daniel, F., Abilene, TX 79605 (US); Erickson-Miller, Connie, L., Collegeville, PA 19426 (US); Jenkins, Julian, Greenford, Middlesex UB6 0HE (GB); Liu, Nannan, Collegeville, PA 19426 (US); Luengo, Juan, I., Collegeville, PA 19426 (US); Price, Alan, T., Collegeville, PA 19426 (US); Shaw, Antony, N., Collegeville, PA 19426 (US); Visonneau, Sophie, Collegeville, PA 19426 (US); Wiggall, Kenneth, Collegeville, PA 19426 (US)
(74) Representative: Connell, Anthony Christopher

(56) References cited:
- WO-A-00/35446
- WO-A-01/07423
- WO-A-01/17349
- WO-A-01/21180
- WO-A-01/77080
- US-A- 5 622 818
- US-B1- 6 214 813
- OLSZEWSKI ET AL: "Potential photoaffinity labels for tubulin. Synthesis and evaluation of diazocyclohexadienone and azide analogs of cholchicine, combretastatin and 3,4,5-trimethoxybiphenyl" J. ORG. CHEM., vol. 59, no. 15, 1994, pages 4285-4296, XP002458479

## Description

### FIELD OF THE INVENTION

This invention relates to thrombopoietin (TPO) mimetics and their use as promoters of thrombopoiesis and megakaryocytopoiesis.

### BACKGROUND OF THE INVENTION

Megakaryocytes are bone marrow-derived cells, which are responsible for producing circulating blood platelets. Although comprising <0.25% of the bone marrow cells in most species, they have > 10 times the volume of typical marrow cells. See Kuter et al. Proc. Natl. Acad. Aci. USA 91: 11104-11108 (1994). Megakaryocytes undergo a process known as endomitosis whereby they replicate their nuclei but fail to undergo cell division and thereby give rise to polypoid cells. In response to a decreased platelet count, the endomitotic rate increases, higher ploidy megakaryocytes are formed, and the number of megakaryocytes may increase up to 3-fold. See Harker J. Clin. Invest. 47: 458-465 (1968). In contrast, in response to an elevated platelet count, the endomitotic rate decreases, lower ploidy megakaryocytes are formed, and the number of megakaryocytes may decrease by 50%.

The exact physiological feedback mechanism by which the mass of circulating platelets regulates the endomitrotic rate and number of bone marrow megakaryocytes is not known. The circulating thrombopoietic factor involved in mediating this feedback loop is now thought to be thrombopoietin (TPO). More specifically, TPO has been shown to be the main humoral regulator in situations involving thrombocytopenia. See, e.g., Metcalf Nature 369:519-520 (1994). TPO has been shown in several studies to increase platelet counts, increase platelet size, and increase isotope incorporation into platelets of recipient animals. Specifically, TPO is thought to affect megakaryocytopoiesis in several ways: (1) it produces increases in megakaryocyte size and number; (2) it produces an increase in DNA content, in the form of polyploidy, in megakaryocytes; (3) it increases megakaryocyte endomitosis; (4) it produces increased maturation of megakaryocytes; and (5) it produces an increase in the percentage of precursor cells, in the form of small acetylcholinesterase-positive cells, in the bone marrow. Because platelets (thrombocytes) are necessary for blood clotting and when their numbers are very low a patient is at risk of death from catastrophic hemorrhage, TPO has potential useful application in both the diagnosis and the treatment of various hematological disorders, for example, diseases primarily due to platelet defects (see Harker et al. Blood 91: 4427-4433 (1998)). Ongoing clinical trials with TPO have indicated that TPO can be administered safely to patients (see Basser et al. Blood 89: 3118-3128 (1997); Fanucchi et al. New Engl. J. Med. 336: 404-409 (1997)). In addition, recent studies have provided a basis for the projection of efficacy of TPO therapy in the treatment of thrombocytopenia, and particularly thrombocytopenia resulting from chemotherapy, radiation therapy, or bone marrow transplantation as treatment for cancer or lymphoma. (See Harker, Curr. Opin. Hematol. 6: 127-134 (1999)).

The gene encoding TPO has been cloned and characterized. See Kuter et al., Proc. Natl. Acad. Sci. USA 91: 11104-11108 (1994); Barley et al., Cell 77: 1117-1124 (1994); Kaushansky et al., Nature 369:568-571 (1994); Wendling et al., Nature 369: 571-574 (1994); and Sauvage et al., Nature 369: 533-538 (1994). Thrombopoietin is a glycoprotein with at least two forms, with apparent molecular masses of 25 kDa and 31 kDa, with a common N-terminal amino acid; sequence. See, Baatout, Haemostasis 27: 1-8 (1997); Kaushansky, New Engl. J. Med. 339: 746-754 (1998). Thrombopoietin appears to have two distinct regions separated by a potential Arg-Arg cleavage site. The amino-terminal region is highly conserved in man and mouse, and has some homology with erythropoietin and interferon-a and interferon-b. The carboxy-terminal region shows wide species divergence.

The DNA sequences and encoded peptide sequences for human TPO receptor (TPO-R; also known as c-mpl) have been described. (See, Vigon et al. Proc. Natl. Acad. Sci. USA 89: 5640-5644 (1992)). TPO-R is a member of the haematopoietin growth factor receptor family, a family characterized by a common structural design of the extracellular domain, including for conserved C residues in the N-terminal portion and a WSXWS motif close to the transmembrane region. (See Bazan Proc. Natl. Acad. Sci. USA 87: 6934-6938 (1990)). Evidence that this receptor plays a functional role in hematopoiesis includes observations that its expression if restricted to spleen, bone marrow, or fetal liver in mice (see Souyri et al. Cell 63: 1137-1147 (1990)) and to megakaryocytes, platelets, and CD34⁺ cells in humans (see Methia et al. Blood 82: 1395-1401 (1993)). Further evidence for TPO-R as a key regulator of megakaryopoiesis is the fact that exposure of CD34⁺ cells to synthetic oligonucleotides antisense to TPO-R RNA significantly inhibits the appearance of megakaryocyte colonies without affecting erythroid or myeloid colony formation. Some workers postulate that the receptor functions as a homodimer, similar to the situation with the receptors for G-CSF and erythropoietin. (see Alexander et al. EMBO J. 14: 5569-5578 (1995)).

The slow recovery of platelet levels in patients suffering from thrombocytopenia is a serious problem, and has lent urgency to the search for a blood growth factor agonist able to accelerate platelet regeneration (see Kuter, Seminars in Hematology, 37: Supp 4: 41-49 (2000)).

It would be desirable to provide compounds which allow for the treatment of thrombocytopenia by acting as a TPO mimetic.

As disclosed herein it has unexpectedly been discovered that certain hydroxy-I-azobenzene derivatives are effective as agonists of the TPO receptor, they are potent TPO mimetics.

### SUMMARY OF THE INVENTION

This invention relates to compounds of Formula (VI): 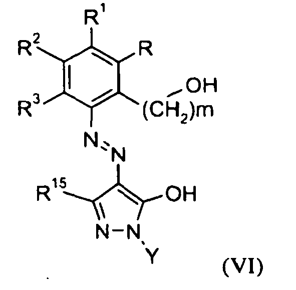 wherein:
R is a substituted aryl; and R¹ is hydrogen;
or:
R is hydrogen; and R¹ is a substituted aryl;
and in either case:
R² and R³ are each independently selected from hydrogen, C₁₋₆alkyl, C₁₋₆alkoxy, nitro, cyano, halogen, aryl, substituted aryl, substituted alkyl, cycloalkyl, phosphonic acid, phosphinic acid and sulfonic acid;
R¹⁵ is selected from the group consisting of alkyl, substituted alkyl, C₁-C₁₂aryl, alkoxy and halogen;
m is 0-4; and
Y is selected from:
   phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, and solvates thereof.

These compounds fall within the scope of Reference Formula (I): 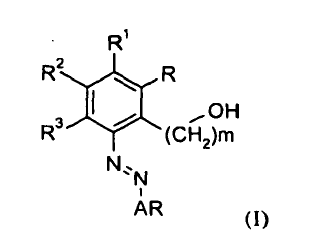 wherein:
R, R¹, R² and R³ are each independently selected from hydrogen, C₁₋₆alkyl, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid, -SO₂NR⁵R⁶, and a heterocyclic methylene substituent as represented by Formula (III), 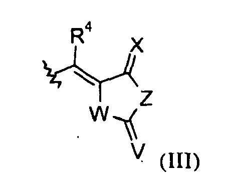 where,
   p is 0-6,
   n is 0-2,
   V, W, X and Z are each independently selected from O, S and NR¹⁶, where R¹⁶ is selected from: hydrogen, alkyl, cycloalkyl, C₁-C₁₂aryl, substituted alkyl, substituted cycloalkyl and substituted C₁-C₁₂aryl,
   R⁴ is selected from: hydrogen, alkyl, cycloalkyl, C₁-C₁₂aryl, substituted alkyl, substituted cycloalkyl and substituted C₁-C₁₂aryl, and
   R⁵ and R⁶ are each independently selected from hydrogen, alkyl, substituted alkyl, C₃₋₆cycloalkyl, and aryl,
   or R⁵ and R⁶ taken together with the nitrogen to which they are attached represent a 5 to 6 member saturated ring containing up to one other heteroatom selected from oxygen and nitrogen;
m is 0-6; and
AR is a cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, aryl, substituted cycloalkyl, substituted aryl, aryloxy, oxo, hydroxy, alkoxy, cycloalkyl, acyloxy, amino, N-acylamino, nitro, cyano, halogen, -C(O)OR⁴, -C(O)NR¹⁰R¹¹, S(O)₂NR¹⁰R¹¹, -S(O)ₙR⁴ and protected -OH,
where n is 0-2,
R⁴ is hydrogen, alkyl, cycloalkyl, C₁-C₁₂aryl, substituted alkyl, substituted cycloalkyl and substituted C₁-C₁₂aryl, and
R¹⁰ and R¹¹ are independently hydrogen, cycloalkyl, C₁-C₁₂aryl, substituted cycloalkyl, substituted C₁-C₁₂aryl, alkyl or alkyl substituted with one or more substituents selected from the group consisting of: alkoxy, acyloxy, aryloxy, amino, N-acylamino, oxo, hydroxy, -C(O)OR⁴, -S(O)ₙR⁴, -C(O)NR⁴R⁴, - S(O)₂NR⁴R⁴, nitro, cyano, cycloalkyl, substituted cycloalkyl, halogen, aryl, substituted aryl and protected -OH,
or R¹⁰ and R¹¹ taken together with the nitrogen to which they are attached represent a 5 to 6 member saturated ring containing up to one other heteroatom selected from oxygen and nitrogen,
where R⁴ is as described above and n is 0-2;
and pharmaceutically acceptable salts, hydrates, solvates and esters thereof;
provided that at least one of R, R¹, R² and R³ is a substituted aryl group or a heterocyclic methylene substituent as represented in Formula (III).

This invention relates to the use of a compound of Formula (VI) in the manufacture of a medicament for treating thrombocytopenia.

The present invention also relates to the discovery that the compounds of Formula (VI) are active as agonists of the TPO receptor.

In a further aspect of the invention there is provided novel processes and novel intermediates useful in preparing the presently invented TPO mimetic compounds.

Included in the present invention are pharmaceutical compositions comprising a pharmaceutical carrier and compounds useful in the methods of the invention.

Also included in the present invention are methods of co-administering the presently invented TPO mimetic compounds with further active ingredients.

### DETAILED DESCRIPTION OF THE INVENTION

This invention relates to compounds of Formula (VI) as described above.

The presently invented compounds of Formula.(VI) fall within the scope of reference Formula (II): 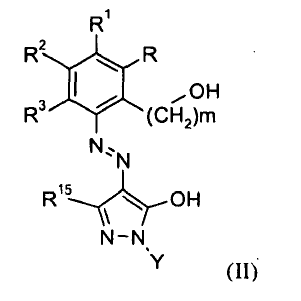 wherein:
R, R¹, R² and R³ are each independently selected from hydrogen, C₁₋₆alkyl, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid, -SO₂NR⁵R⁶, and a heterocyclic methylene substituent as represented by Formula (III), 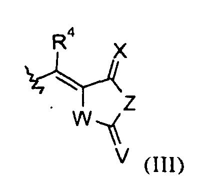 where
   p is 0-6,
   n is 0-2
   V, W, X and Z are each independently selected from O, S, and NR¹⁶, where R¹⁶ is selected from: hydrogen, alkyl, cycloalkyl, C₁-C₁₂aryl, substituted alkyl, substituted cycloalkyl and substituted C₁-C₁₂aryl,
   R⁴ is hydrogen, alkyl, cycloalkyl, C₁-C₁₂aryl, substituted alkyl, substituted cycloalkyl and substituted C₁-C₁₂aryl, and
   R⁵ and R⁶ are each independently selected from hydrogen, alkyl, substituted alkyl, C₃₋₆cycloalkyl, and aryl,
   or R⁵ and R⁶ taken together with the nitrogen to which they are attached represent a 5 to 6 member saturated ring containing up to one other heteroatom selected from oxygen and nitrogen;
R¹⁵ is selected from the group consisting of alkyl, C₁-C₁₂aryl, hydroxy, alkoxy, substituted alkyl, substituted C₁-C₁₂aryl and halogen;
m is 0-6; and
Y is selected from alkyl, substituted alkyl and a cyclic or polycyclic aromatic ring containing from 3 to 14 carbon atoms and optionally containing from one to three heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, C₁-C₁₂aryl, substituted cycloalkyl, substituted C₁-C₁₂aryl, hydroxy, aryloxy, alkoxy, cycloalkyl, nitro, cyano, halogen and protected -OH;
and pharmaceutically acceptable salts, hydrates, solvates and esters thereof;
provided that at least one of R, R¹, R² and R³ is a substituted aryl group or a heterocyclic methylene substituent as represented in Formula (III).

Included among the presently invented compounds of Formula Reference (II) are compounds in which R¹⁵ is not alkoxy.

Included among the presently invented compounds of Formula Reference (II) are those having Formula (VI): 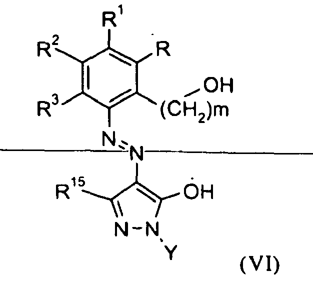 wherein:
R is a substituted aryl; and R¹ is hydrogen;
or:
R is hydrogen; and R¹ is a substituted aryl; and in either case:
   R² and R³ are each independently selected from hydrogen, C₁₋₆alkyl, C₁₋₆alkoxy, nitro, cyano, halogen, aryl, substituted aryl, substituted alkyl, cycloalkyl, phosphonic acid, phosphinic acid and sulfonic acid;
   R¹⁵ is selected from the group consisting of alkyl, substituted alkyl, C₁₋C₁₂aryl, alkoxy and halogen;
   m is 0-4; and
   Y is selected from,
      phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, solvates and esters thereof.

Particularly preferred among the presently invented Formula VI compounds are those in which,
R is a substituted C₁-C₁₂aryl;
   and
R¹ is hydrogen;
R² and R³ are each independently selected from hydrogen, C₁₋₆alkyl, C₁₋₆alkoxy, nitro, cyano, halogen, substituted alkyl and cycloalkyl;
R¹⁵ is selected from the group consisting of alkyl, substituted alkyl, C₁-C₁₂aryl, alkoxy and halogen;
m is 0-2; and
Y is selected from, phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, solvates and esters thereof.

The most preferred among the presently invented Formula VI compounds are those in which,
R is a substituted phenyl or pyridinyl ring; and
R¹ is hydrogen;
R² and R³ are each independently selected from hydrogen, C₁₋₆alkyl, substituted alkyl and halogen;
R¹⁵ is selected from the group consisting of C₁₋₄alkyl, C₁₋₄alkoxy, C₁-C₁₂aryl and halogen;
m is 0; and
Y is selected from,
phenyl, pyridinyl and pyrimidinyl, where the phenyl, pyridinyl and pyrimidinyl is optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, solvates and esters thereof.

Preferred among the presently invented compounds are:
4'-{N'-[1-(3,4-Dimethylphenyl)-3-methyl-5-oxo-1,5-dihydropyrazol-4-ylidene]hydrazino}-3'-hydroxybiphenyl-4-carboxylic acid;
4'-{N'-[1-(3,4-Dimethylphenyl)-3-methyl-5-oxo-1,5-dihydropyrazol-4-ylidene]hydrazino}-3'-hydroxybiphenyl-3-carboxylic acid;
3'-{N'-[1-(3,4-Dimethylphenyl)-3-methyl-5-oxo-1,5-dihydropyrazol-4-ylidene]hydrazino}-2'-hydroxybiphenyl-3-carboxylic acid;
3'-{N'-[1-(4-tert-Butylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino} -5'-chloro-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxybiphenyl-3-carboxylic acid;
3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid;
2-Aza-5'-chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxy-5'-methylbiphenyl-3-carboxylic acid;
2-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-3-carboxylic acid;
3'-{N'-[1-(4-tert-Butylphenyl)-3-methyl-5-oxo-1,5-dihydropyrazol-4-ylidene]hydrazino}-2'-hydroxy-5'-methylbiphenyl-3-carboxylic acid;
3-{N'-[1-(3,4-dimethylphenyl)-3-methyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-3-methyl-5-oxo-1,5-dihydropyrazol-4-ylidene]hydrazino }-5'-fluoro-2'-hydroxybiphenyl-3-carboxylic acid;
7-({N'-[1-(3,4-dimethylphenyl)-3-methyl-5-oxo-1,5-dihydropyrazol-4-ylidene]hydrazino}-2-hydroxyphenyl)quinolin-4[1H]-one-3-carboxylic acid;
7-({N'-[1-(4-tert-butylphenyl)-3-methyl-5-oxo-1,5-dihydropyrazol-4-ylidene]hydrazino}-2-hydroxyphenyl)quinolin-4[1H]-one-3-carboxylic acid;
3-Aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3-Aza-3'-(N'-[1-{3-methyl-[4-(1-methylethyl)phenyl]-5-oxo-1,5-dihydropyrazol-4-ylidene}hydrazino)-2'-hydroxybiphenyl-5-carboxylic acid;
3-Aza-3'-{N'-[1-(4-tertbutylphenyl-3-methyl-5-oxo-1,5-dihydropyrazol-4-ylidene]hydrazino}-2'-hydroxybiphenyl-5-carboxylic acid;
5'-Chloro-3'-{N'-[1'-(3,4-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-Dimethylphenyl)-3,5-dioxo-1,5-dihydropyrazol-4-ylidene]hydrazino}-2'-hydroxybiphenyl-3-carboxylic acid;
3'-{N'-[1-(2-Ethoxy-2-oxoethyl)-3-methyl-5-oxo-1,5-dihydropyrazol-4-ylidene]hydrazino}-2'-hydroxybiphenyl-3-carboxylic acid;
3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-(N'-{1-[2-(N-tert-butyl)amino-2-oxoethyl]-3-methyl-5-oxo-1,5-dihydropyrazol-4-ylidene}hydrazino)-2'-hydroxybiphenyl-3-carboxylic acid;
3'-{N'-[3-Chloro-1-(3,4-dimethylphenyl)-5-oxo-1,5-dihydropyrazol-4-ylidene]hydrazino}-2'-hydroxybiphenyl-3-carboxylic acid;
5-chloro-3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-{N'-[1-(3,4-Dimethylphenyl)-3-methyl-5-oxo-1,5-dihydropyrazol-4-ylidene]hydrazino}-2'-hydroxybiphenyl-3,5-dicarboxylic acid;
3-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-5-carboxylic acid;
3'-{N'-[1-(3,4-Dimethylphenyl)-3-methyl-5-oxo-1,5-dihydropyrazol-4-ylidene]hydrazino}-2'-hydroxybiphenyl-4-carboxylic acid;
3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;
3'-{N'-[1-(4-methoxyphenyl)-3-methyl-5-oxo-1,5-dihydropyrazol-4-ylidene]hydrazino}-2'-hydroxybiphenyl-3-carboxylic acid;
(3-{N'-[1-(3,4-dimethylphenyl)-3-methyl-5-oxo-1,5-dihydropyrazol-4-ylidene]hydrazino}-2-hydroxy-3'-biphenyl)-1,1,1,-trifluoromethanesulfonamide;
3'-{N'-[1-(3,4-Dichlorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(3-trifluoromethylphenyl)-1,5-dihydropyrazol-4-ylidene]hydrazino}-2'-hydroxybiphenyl-3-carboxylic acid;
8-{N'-[1-(3,4-dimethylphenyl)-3-methyl-5-oxo-1,5-dihydropyrazol-4-ylidene]hydrazino}quinolin-4[1H]-one-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-carboxylic acid;
3'-{N'-[3-methyl,5-oxo-1-(4-N-methylcarboxamidolphenyl)-1,5-dihydropyrazol-4-ylidene]hydrazino}-2'-hydroxybiphenyl-3-carboxylic acid;
N-[1-(3'-{N'-[1-(3,4-dimethylphenyl)-3-methyl-5-oxo-1,5-dihydropyrazol-4-ylidene]hydrazino}-2'-hydroxybiphenyl-3-yl)methanoyl]methanesulfonamide;
3'-{N'-[3-methyl-5-oxo-1-phenyl-1,5-dihydropyrazol-4-ylidene]hydrazino}-2'-hydroxybiphenyl-3-carboxylic acid;
3'-{N'-[3-methyl-1-(4-methylphenyl)-5-oxo-1,5-dihydropyrazol-4-ylidene]hydrazino}-2'-hydroxybiphenyl-3-carboxylic acid;
3'-{N'-[1-(4-chlorophenyl)-3-methyl-5-oxo-1,5-dihydropyrazol-4-ylidene]hydrazino}-2'-hydroxybiphenyl-3-carboxylic acid;
3'-{N'-[1-(4-fluorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethoxyphenyl)-1,5-dihydropyrazol-4-ylidene]hydrazino}-2'-hydroxybiphenyl-3-carboxylic acid;
3'-{N'-[1-(3,4-dimethylphenyl)-3-ethoxy-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethoxy)-5-oxo-1,5-dihydropyrazol-4-ylidene]hydrazino}-2'-hydroxybiphenyl-3-carboxylic acid;
3'-{N'-[3-tert-butyl-1-(3,4-dimethylphenyl)-5-oxo-1,5-dihydropyrazol-4-ylidene]hydrazino}-2'-hydroxybiphenyl-3-carboxylic acid;
3'-{N'-[3-methyl-1-(4-methyl-2,3,5,6-tetrafluorophenyl)-5-oxo-1,5-dihydropyrazol-4-ylidene]hydrazino}-2'-hydroxybiphenyl-3-carboxylic acid;
3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[1-(3.4-dimethylphenyl)-3-phenyl-5-oxo-1,5-dihydropyrazol-4-ylidene]hydrazino}-2'-hydroxybiphenyl-3-carboxylic acid;
3-{N'-[1-(3,4-dimethylphenyl)-5-oxo-3-phenyl-1,5-dihydropyrazol-4-ylidene]hydrazino}-2-hydroxy-3'-tetrazol-5-ylbiphenyl;
3-{N'-[1-(3,4-dimethylphenyl)-3-methoxy-5-oxo-1,5-dihydropyrazol-4-ylidene]hydrazino -2-hydroxy-3'-tetrazol-5-ylbiphenyl;
3-{N'-[1-(3,4-dimethylphenyl)-3-ethoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-(1-methylethoxy)-5-oxo-1,5-dihydropyrazol-4-ylidene]hydrazino}-2-hydroxy-3'-tetrazol-5-ylbiphenyl;
3-{N'-[1-(4-fluorophenyl)-3-methyl-5-oxo-1,5-dihydropyrazol-4-ylidene]hydrazino}-2-hydroxy-3'-tetrazol-5-ylbiphenyl;
3-{N'-[1-(4-fluoro-3-methylphenyl)-3-methyl-5-oxo-1,5-dihydropyrazol-4-ylidene]hydrazino}-2-hydroxy-3'-tetrazol-5-ylbiphenyl;
3-{N'-[3-methyl-5-oxo-1-(4-trifluoromethylphenyl)-1,5-dihydropyrazol-4-ylidene]hydrazino} -2-hydroxy-3'-tetrazol-5-ylbiphenyl;
3'- {N'-[1-(3,4-dimethylphenyl)-3-(pyridin-4-yl-5-oxo-1,5-dihydropyrazol-4-ylidene]hydrazino}-2'-hydroxybiphenyl-3-carboxylic acid;
3-{N'-[1-(3,4-dimethylphenyl)-3-pyridin-4-yl-5-oxo-1,5-dihydropyrazol-4-ylidene]hydrazino}-2-hydroxy-3'-tetrazol-5-ylbiphenyl;
3-{N'-[1-(3,4-dimethylphenyl)-3-pyridin-2-yl-5-oxo-1,5-dihydropyrazol-4-ylidene]hydrazino}-2-hydroxy-3'-tetrazol-5-ylbiphenyl;
3'-{N'-[1-(3.4-dimethylphenyl)-3-(pyridin-2-yl-5-oxo-1,5-dihydropyrazol-4-ylidene]hydrazino}-2'-hydroxybiphenyl-3-carboxylic acid;
3-{N'-[1-(3-fluoro-4methylphenyl)-3-methyl-5-oxo-1,5-dihydropyrazol-4-ylidene]hydrazino}-2-hydroxy-3'-tetrazol-5-ylbiphenyl;
3'- {N'-[1-(3-fluoro-4-methylphenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethylpyrimidin-2-yl)-1,5-dihydropyrazol-4-ylidene]hydrazino}-2'-hydroxybiphenyl-3-carboxylic acid;
3'-N-tert-butoxycarbonylamino-3- {N'-[1-(3,4-Dimethylphenyl)-3-methyl-5-oxo-1,5-dihydropyrazol-4-ylidene]hydrazino}-2-hydroxybiphenyl;
3'-amino-3-{N'-[1-(3,4-dimethylphenyl)-3-methyl-5-oxo-1,5-dihydropyrazol-4-ylidene]hydrazino}-2-hydroxybiphenyl;
3-{N'-[1-(3-fluorophenyl)-3-methyl-5-oxo-1,5-dihydropyrazol-4-ylidene]hydrazino}-2-hydroxy-3'-tetrazol-5-ylbiphenyl;
3'-{N'-[1-(3-fluorophenyl)-3-methyl-5-oxo-1,5-dihydropyrazol-4-ylidene]hydrazino}-2'-hydroxybiphenyl-3-carboxylic acid;
3-{N'-[3-methyl-5-oxo-1-(2,3.4,5,6-pentafluorophenyl)-1,5-dihydropyrazol-4-ylidene]hydrazino}-2-hydroxy-3'-tetrazol-5-ylbiphenyl;
3'-{N'-[3-methyl-5-oxo-1-(2,3,4,5,6-pentafluorophenyl)-1,5-dihydropyrazol-4-ylidene]hydrazino}-2'-hydroxybiphenyl-3-carboxylic acid;
3'-{N'-[1-(3,4-difluorophenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methoxymethyl-5-oxo-1,5-dihydropyrazol-4-ylidene]hydrazino}-2'-hydroxybiphenyl-3-carboxylic acid;
3-{N'-[1-(3,4-dimethylphenyl)-3-methoxymethyl-5-oxo-1,5-dihydropyrazol-4-ylidene]hydrazino}-2-hydroxy-3'-tetrazol-5-ylbiphenyl;
3-{N'-[1-(3,4-difluorophenyl)-3-methyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-trifluoromethyl-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-6-fluoro-2'-hydroxybiphenyl-3-carboxylic acid;
3'-{N'-[1-(3,4-dimethylphenyl)-5-oxo-3-propyl-1,5-dihydropyrazol-4-ylidene]hydrazino}-2'-hydroxybiphenyl-3-carboxylic acid;
3-{N'-[1-(3,4-dimethylphenyl)-5-oxo-3-propyl-1,5-dihydropyrazol-4-ylidene]hydrazino}-2-hydroxy-3'-tetrazol-5-ylbiphenyl;
3'-{N'-[1-(3,4-dimethylphenyl)-3-(1-methyl-1H-pyrrol-3-yl)-5-oxo-1,5-dihydropyrazol-4-ylidene]hydrazino}-2'-hydroxybiphenyl-3-carboxylic acid;
3- {N'-[1-(3,4-dimethylphenyl)-3-(1-methyl-1H-pyrrol-3-yl)-5-oxo-1,5-dihydropyrazol-4-ylidene]hydrazino}-2-hydroxy-3'-tetrazol-5-ylbiphenyl;
3'-{N'-[1-(3,4-dimethylphenyl)-3-furan-2-yl-5-oxo-1,5-dihydropyrazol-4-ylidene]hydrazino}-2'-hydroxybiphenyl-3-carboxylic acid;
3-{N'-[1-(3,4-dimethylphenyl)-3-furan-2-yl-5-oxo-1,5-dihydropyrazol-4-ylidene]hydrazino}-2-hydroxy-3'-tetrazol-5-ylbiphenyl;
N-(2'-hydroxy-3'-{N'-1'3-methyl-5-oxo-1-(4-trifluoromethyl-phenyl)-1,5-dihydro-pyrazol-4-ylidene]hydrazino}biphenyl-3-yl)-1,1,1-trifluoromethanesulfonamide;
N-(2'-hydroxy-3'-{N'-[1-(3-fluoro-4-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'-{N'-[1-(4-fluoro-3-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'- {N'-[1-(3,4-difluorophenyl)-3-methyl-5-oxo-1,5-dihydro-pyrazol-4-ylidene]hydrazino} biphenyl-3-yl)-1,1,1-trifluoromethanesulfonamide;
N-(3'-{N'-[1-(3,4-dimethylphenyl)-3-methyl-5-oxo-1,5-dihydropyrazol-4-ylidene]hydrazino}-2'-hydroxybiphenyl-3-yl)guanidine;
3'-{N'-[1-(3,4-dimethylphenyl)-3-ethyl-5-oxo-1,5-dihydropyrazol-4-ylidene]hydrazino}-2'-hydroxybiphenyl-3-carboxylic acid;
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-thien-2-yl-1,5-dihydropyrazol-4-ylidene]hydrazino}-2'-hydroxybiphenyl-3-carboxylic acid;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-5-oxo-3-thiazol-2-yl-1,5-dihydropyrazol-4-ylidene]hydrazino}-2'-hydroxybiphenyl-3-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethyl)-5-oxo-1,5-dihydropyrazol-4-ylidene]hydrazino}-2'-hydroxybiphenyl-3-carboxylic acid;
3'-{N'-[3-(benzyloxymethyl)-1-(3,4-dimethylphenyl)-5-oxo-1,5-dihydropyrazol-4-ylidenelhydrazinol-2'-hydroxybiphenyl-3-carboxylic acid;
3'-{N'-[3-ethyl-5-oxo-1-(4-trifluoromethylphenyl)-1,5-dihydropyrazol-4-ylidene]hydrazino}-2'-hydroxybiphenyl-3-carboxylic acid;
3'-{N'-[5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-hydroxymethyl-5-oxo-1,5-dihydropyrazol-4-ylidene]hydrazino}-2'-hydroxybiphenyl-3-carboxylic acid;
3'-{N'-[3-benzyloxymethyl-5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-methylsulfanylmethyl-5-oxo-1,5-dihydropyrazol-4-ylidene]hydrazino}-2'-hydroxybiphenyl-3-carboxylic acid;
3'-{N'-[-1-(3,4-dimethylphenyl)-5-oxo-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-methylsulfanylmethyl-1,5-dihydropyrazol-4-ylidene]hydrazino}-2'-hydroxybiphenyl-3-carboxylic acid;
N-(3'-{N'-[1-(3,4-dimethylphenyl)-3-methyl-5-oxo- 1,5-dihydro-pyrazol-4-ylidene]hydrazino}-2'-hydroxybiphenyl-3-yl)methanesulfonamide;
3'-[N'-(1-benzo[1,3]dioxol-5-yl-3-methyl-5-oxo-1,5-dihydropyrazol-4-ylidene)hydrazino]-2'-hydroxybiphenyl-3-carboxylic acid;
3'-{N'-[1-(3,5-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-4'-hydroxybiphenyl-4-carboxylic acid;
3'-{N'-[1-(3-chloro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-4'-hydroxybiphenyl-3-carboxylic acid;
3'-{N'-[1-(3,4-di methylphenyl)-3-methyl-5-oxo-1,5-dihydropyrazol-4-ylidene]hydrazino}-2'-hydroxybiphenyl-3-phosphonic acid;
3'-{N'-[1-(3,4-dimethylphenyl)-3-methyl-5-oxo-1,5-dihydropyrazol-4-ylidene]hydrazino}-2'-hydroxybiphenyl-3,4-dicarboxylic acid;
2',6-dihydroxy-3'-{N'[1-(3,4-dimethylphenyl)-3-methyl-5-oxo-1,5-dihydropyrazol-4-ylidene]hydrazino}biphenyl-3-carboxylic acid;
4-aza-3'-{N'-[1-(3,4-dimethylphenyl)-3-methyl-5-oxo-1,5-dihydropyrazo1-4-ylidene]hydrazino}-2'-hydroxybiphenyl-5-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-sulfonic acid; and
5-(3'-{N'-[1-(3,4-Dimethylphenyl)-3-methyl-5-oxo-1,5-dihydropyrazol-4-ylidene]hydrazino}-2'-hydroxybiphenyl-3-ylmethylene)thiazolidine-2,4-dione;
and pharmaceutically acceptable salts, hydrates, solvates and esters thereof.

Compounds of Formula (VI) are included in the pharmaceutical compositions of the invention and used in the methods of the invention.

By the term "protected hydroxy" or "protected -OH" as used herein, is meant the alcoholic or carboxylic-OH groups which can be protected by conventional blocking groups in the art such as described in "Protective Groups In Organic Synthesis" by Theodora W. Greene, Wiley-Interscience, 1981, New York. Compounds containing protected hydroxy groups may also be useful as intermediates in the preparation of the pharmaceutically active compounds of the invention.

By the term "aryl" as used herein, is meant a cyclic or polycyclic aromatic ring containing from 1 to 14 carbon atoms and optionally containing from one to five heteroatoms, provided that when the number of carbon atoms is 1 the aromatic ring contains at least four heteroatoms, when the number of carbon atoms is 2 the aromatic ring contains at least three heteroatoms, when the number of carbons is 3 the aromatic ring contains at least two heteroatoms and when the number of carbon atoms is 4 the aromatic ring contains at least one heteroatom.

By the term "C₁-C₁₂aryl" as used herein, is meant phenyl, naphthalene, 3,4-methylenedioxyphenyl, pyridine, biphenyl, quinoline, pyrimidine, quinazoline, thiophene, furan, pyrrole, pyrazole, imidazole and tetrazole.

When referring to compounds of Reference Formula (I) and (II), the term "substituted" as used herein, is meant that the subject chemical moiety has one or more substituents selected from the group consisting of: -CO₂R²⁰, aryl, - C(O)NHS(O)₂R²⁰, -NHS(O)₂R²⁰, hydroxyalkyl, alkoxy, -C(O)NR²¹R²², acyloxy, alkyl, amino, N-acylamino, hydroxy, -(CH₂)_{g}C(O)OR⁸, -S(O)ₙR⁸, nitro, tetrazole, cyano, oxo, halogen, trifluoromethyl, protected -OH and a heterocyclic methylene substituent as represented by Formula (III), 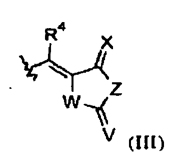 where g is 0-6; R⁸ is hydrogen or alkyl; R²⁰ is selected form hydrogen, C₁-C₄alkyl, aryl and trifluoromethyl; R²¹ and R²² are independently selected form hydrogen, C₁-C₄alkyl, aryl and trifluoromethyl; V, W, X and Z are each independently selected from O, S, and NR¹⁶, where R¹⁶ is selected from: hydrogen, alkyl, cycloalkyl, C₁-C₁₂aryl, substituted alkyl, substituted cycloalkyl and substituted C₁-C₁₂aryl: and n is 0-2.

When referring to compounds of Formula (VI), the term "substituted" as used herein, is meant that the subject chemical moiety has one or more substituents selected from the group consisting of: -CO₂R²⁰, aryl, -C(O)NHS(O)₂R²⁰, -NHS(O)₂R²⁰, hydroxyalkyl, alkoxy, -C(O)NR²¹R²², acyloxy, alkyl, amino, N-acylamino, hydroxy, -(CH₂)_{g}C(O)OR⁸, -S(O)ₙR⁸, nitro, tetrazole, cyano, oxo, halogen, trifluoromethyl and protected -OH, where g is 0-6, R⁸ is hydrogen or alkyl, R²⁰ is selected form hydrogen, C₁-C₄alkyl, aryl and trifluoromethyl, and R²¹ and R²² are independently selected form hydrogen, C₁-C₄alkyl, aryl and trifluoromethyl, and n is 0-2.

By the term "alkoxy" as used herein is meant -Oalkyl where alkyl is as described herein including -OCH₃ and -OC(CH₃)₂CH₃.

The term "cycloalkyl" as used herein is meant a nonaromatic, unsaturated or saturated, cyclic or polycyclic C₃-C₁₂.

Examples of cycloalkyl and substituted cycloalkyl substituents as used herein include: cyclohexyl, 4-hydroxy-cyclohexyl, 2-ethylcyclohexyl, propyl 4-methoxycyclohexyl, 4-methoxycyclohexyl, 4-carboxycyclohexyl, cyclopropyl and cyclopentyl.

By the term "acyloxy" as used herein is meant -OC(O)alkyl where alkyl is as described herein. Examples of acyloxy substituents as used herein include: -OC(O)CH₃, - OC(O)CH(CH₃)₂ and -OC(O)(CH₂)₃CH₃.

By the term "N-acylamino" as used herein is meant -N(H)C(O)alkyl, where alkyl is as described herein. Examples of N-acylamino substituents as used herein include: - N(H)C(O)CH₃, -N(H)C(O)CH(CH₃)₂ and -N(H)C(O)(CH₂)₃CH₃.

By the term "aryloxy" as used herein is meant -Oaryl where aryl is phenyl, naphthyl, 3,4-methylenedioxyphenyl, pyridyl or biphenyl optionally substituted with one or more substituents selected from the group consisting of: alkyl, hydroxyalkyl, alkoxy, trifuloromethyl, acyloxy, amino, N-acylamino, hydroxy, -(CH₂)_{g}C(O)OR⁸, -S(O)ₙR⁸, nitro, cyano, halogen and protected -OH, where g is 0-6, R⁸ is hydrogen or alkyl, and n is 0-2. Examples of aryloxy substituents as used herein include: phenoxy, 4-fluorophenyloxy and biphenyloxy.

By the term "heteroatom" as used herein is meant oxygen, nitrogen or sulfur.

By the term "halogen" as used herein is meant a substituent selected from bromide, iodide, chloride and fluoride.

By the term "alkyl" and derivatives thereof and in all carbon chains as used herein is meant a linear or branched, saturated or unsaturated hydrocarbon chain, and the carbon chain will contain from 1 to 12 carbon atoms. Examples of alkyl substituents as used herein include: -CH₃, -CH₂-CH₃, -CH₂-CH₂-CH₃, -CH(CH₃)₂, - C(CH₃)₃, -(CH₂)₃-CH₃, -CH₂-CH(CH₃)₂, -CH(CH₃)-CH₂-CH₃, -CH=CH₂, and -CDC-CH₃.

By the term "treating" and derivatives thereof as used herein, is meant prophylatic and therapeutic therapy.

Compounds of Formula (VI) are included in the pharmaceutical compositions of the invention and used in the methods of the invention. Where a -COOH or -OH group is present, pharmaceutically acceptable esters can be employed, for example methyl, ethyl, pivaloyloxymethyl, and the like for -COOH, and acetate maleate and the like for -OH, and those esters known in the art for modifying solubility or hydrolysis characteristics, for use as sustained release or prodrug formulations.

The novel compounds of Reference Formulas I and II are prepared as shown in Schemes I to IV below, or by analogous methods, wherein the 'R' substituents, AR, Y and m are as defined in Reference Formulas I and II respectively and provided that the 'R' and m substituents, AR
and Y do not include any such substituents that render inoperative the processes of Schemes I to IV. All of the starting materials are commercially available or are readily made from commercially available starting materials by those of skill in the art. 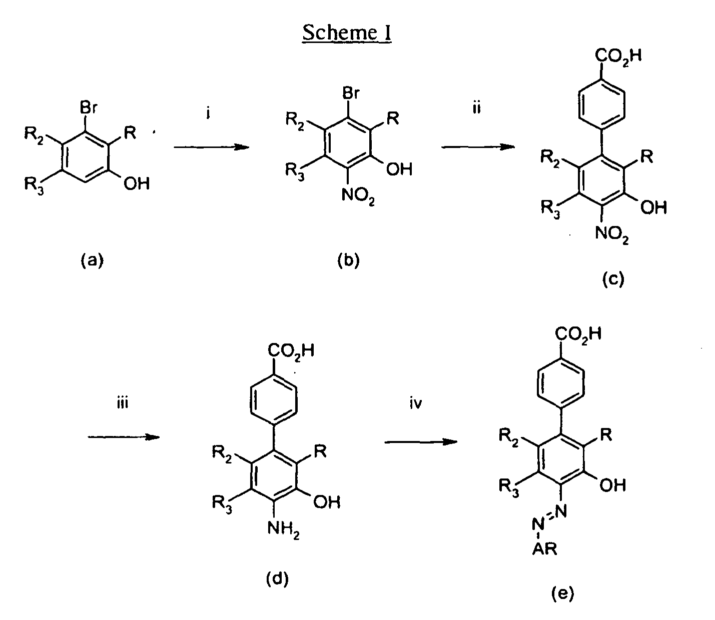 i), nitric acid;, sulfuric acid;; ii) 4-carboxyphenylboronic acid;, Pd(PPh₃)_{4,} Na2CO3 dioxane, water; iii) H_{2,} Pd-C; iv) NaNO₂, AR, NaHCO₃, water, EtOH

Scheme I outlines the formation of Reference Formula I compounds. As used in scheme I, a 3-bromophenol (a) is nitrated with nitric acid or sodium nitrate and sulfuric acid to give nitro phenol (b). Coupling of (b) with a substituted arylboronic acid, such as 3-carboxyphenylboronic acid or 4-carboxyphenylboronic acid in the presence of a catalyst, preferably tetrakistriphenylphosphino palladium and a base such as sodium carbonate ot triethylamine in a suitable solvent such as aqueous 1,4-dioxane or dimethylformamide afforded substituted aryl compound (c). Reduction of the nitro group by catalytic hydrogenation or mediated by a reducing metal such as iron of tin dichloride in a suitable solvent such as ethanol, acetic acid or water gives the aniline (d). Compound (d) is diazotized by reaction with sodium nitrite and an appropriate acid, such as nitric acid, sulfuric acid or, preferably, hydrochloric acid, in an appropriate aqueous solvent, such as water or, preferably an ethanol-water mixture to produce a diazonium species which is directly converted to compound (e) in a coupling reaction with an appropriate aryl species in the presence of a base, preferably sodium hydrogen carbonate, or an acid, preferably hydrochloric acid. 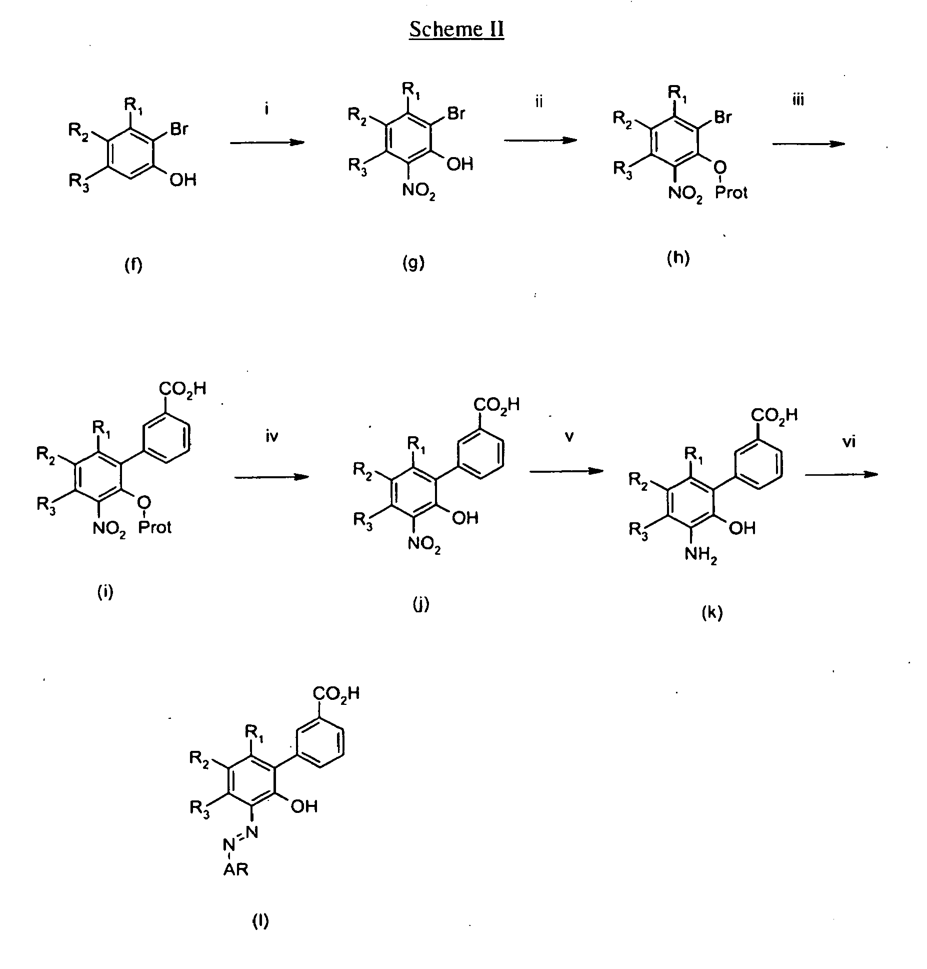 i), NaNO₂, sulfuric acid;; ii), MeI, K₂CO₃, acetone; iii) 3-carboxyphenylboronic acid;, Pd(PPh₃)₄, Na2CO3, dioxane, water; iv) 48% aqu. HBr, AcOH; v) H₂, Pd-C; vi) NaNO₂, AR, NaHCO₃, water, EtOH

Scheme II outlines an alternative synthesis of Reference Formula I compounds. A 2-bromophenol (f) (such as 2-bromophenol or 2-bromo-5-methylphenol is nitrated with nitric acid or sodium nitrate and sulfuric acid, to give nitro compound (g). The phenol (g) is then protected by reaction with an alkylating agent such as benzyl bromide or preferably methyl iodide in the presence of a base such as sodium hydride or potassium carbonate in a suitable solvent such as dimethylformamide, tetrahydrofuran or acetone to give protected nitrophenol (h) (Prot = alkyl or substituted alkyl, e.g. methyl, benzyl). Coupling of (h) with a substituted arylboronic acid, such as 3-carboxyphenylboronic acid or 4-carboxyphenylboronic acid, in the presence of a catalyst, preferably tetrakistriphenylphosphino palladium and a base such as sodium carbonate ot triethylamine in a suitable solvent such as aqueous 1,4-dioxane or dimethylformamide afforded substituted aryl compound (i). Removal of the protecting group (Prot) is accomplished using an protic or Lewis acid; such as concentrated hydrobromic acid, boron tribromide or trimethylsilyl iodide to affored the phenol (j). Reduction of the nitro group by catalytic hydrogenation or mediated by a reducing metal such as iron of tin dichloride in a suitable solvent such as ethanol, acetic acid; or water gives the aniline (k). Compound (k) is diazotized by reaction with sodium nitrite and an appropriate acid, such as nitric acid, sulfuric acid or, preferably, hydrochloric acid, in an appropriate aqueous solvent, such as water or, preferably, an ethanol-water mixture to produce a diazonium species which is directly converted to compoud (1) in a coupling reaction with an appropriate aryl species in the presence of a base, preferably sodium hydrogen carbonate, or an acid, preferably hydrochloric acid. 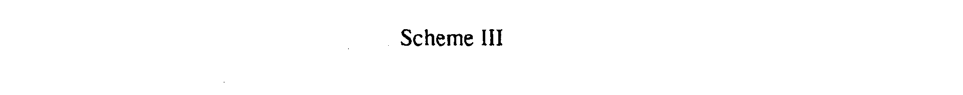 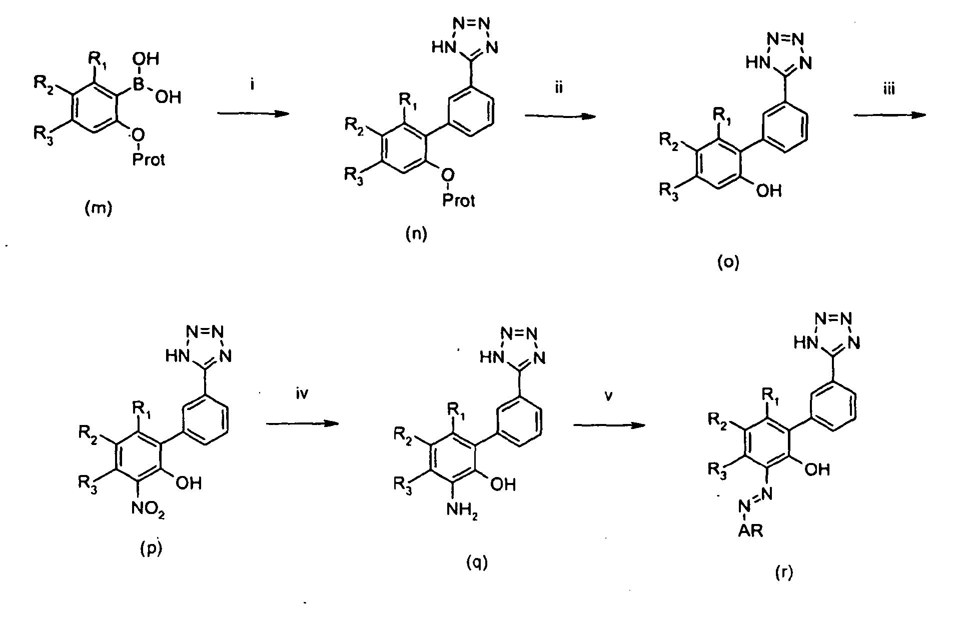 i) 5-(3-bromophenyl)tetrazole, Pd(PPh₃)₄, Na2CO3, dioxane, water; ii) 48% aqu. HBr, AcOH; iii) HNO₂, AcOH; iv) H₂, Pd-C; v) NaNO₂, AR, NaHCO₃, water, EtOH

Scheme III outline a further procedure for the synthesis of Reference Formula I compounds. A protected hydroxyphenylboronic acid; (m) (Prot = alkyl or substituted alkyl, e.g. methyl, benzyl) such as 5-chloro-2-methoxyphenylboronic acid, 5-fluoro-2-methoxyphenyl, boronic acid or 2-methoxy-5-formylphenylboronic acid, is coupled with a substituted halogenoaryl species, such as 5-(3-bromophenyl)tetrazole or 5-bromonicotinic acid, in the presence of a catalyst, preferably tetrakistriphenylphosphino palladium, and a base, such as sodium carbonate or triethylamine in a suitable solvent such as aqueous 1,4-dioxane or dimethylformamide afforded substituted aryl compound (n). Removal of the protecting group Prot is accomplished using an protic or Lewis acid, such as concentrated hydrobromic acid, boron tribromide or trimethylsilyl iodide to affored the phenol (o). Nitration of (o) with nitric acid, or sodium nitrate in the presence of an acid, such as acetic or hydrochloric acid, affords the nitro compound (p). Reduction of the nitro group by catalytic hydrogenation or mediated by a reducing metal such as iron of tin dichloride in a suitable solvent such as ethanol, acetic acid or water gives the aniline (q). Compound (q) is diazotized by reaction with sodium nitrite and an appropriate acid, such as nitric acid, sulfuric acid or, preferably, hydrochloric acid, in an appropriate aqueous solvent, such as water or, preferably, an ethanol-water mixture to produce a diazonium species which is directly converted to compound (r) in a coupling reaction with an appropriate aryl species in the presence of a base, preferably sodium hydrogen carbonate, or an acid, preferably hydrochloric acid. 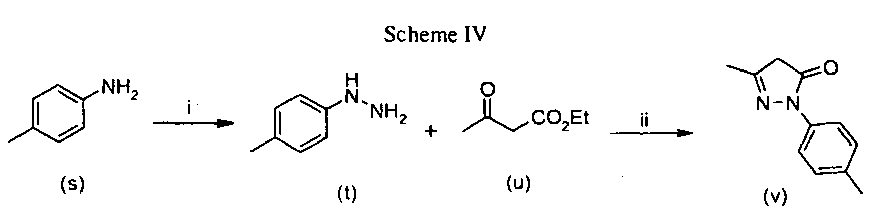 i) NaNO₂, HCl, water then SnCl₂, water; ii) AcOH, heat

Scheme IV outlines the formation of pyrazoles for use in scheme I-III. An amine such as 4-methylaniline, compound (s), is diazotized by the action of sodium nitrite and an appropriate acid, such as hydrochloric acid, nitric acid or sulfuric acid, in an appropriate aqueous solvent system, such as water or ethanol-water mixtures, then reduced *in situ* by tin chloride to afford hydrazine, compound (t). The hydrazine is then condensed with a electrophilic carbonyl species such as ethyl acetoacetate (u), ethyl cyanoacetate or diethyl malonate, in an appropriate solvent such as acetic acid or ethanol at an appropriate temperature typically 0-100° to give the corresponding pyrazole, compound (v) as described herein.

There is provided a process for preparing a compound of Formula (VI),
the process comprising reaction of a compound of Formula (VII) 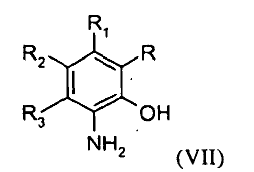 or a protected form thereof with a compound of Formula (VIII) or tautomeric equivalent (IX) 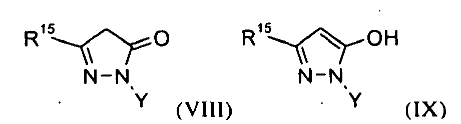 wherein
either:
R is a phenyl or pyridinyl ring substituted by one or more of the following:
   -C(O)OH, tetrazole, sulfonic acid, hydroxy, methyl, fluoro, NHSO₂CF₃, and C(O)NHSO₂CH₃; and R¹ is hydrogen,
or:
R is hydrogen; and R¹ is a phenyl or pyridinyl ring substituted by one or more of the following: -C(O)OH, tetrazole, sulfonic acid, hydroxy, methyl, fluoro, NHSO₂CF₃, and C(O)NHSO₂CH₃;
and in either case:
R² and R³ are independently selected from hydrogen, C₁₋₆alkyl and halogen;
R¹⁵ is selected from the group consisting: of C₁₋₆alkyl, C₁₋₆alkoxy, trifluoromethyl and halogen; and
Y is selected from,
   phenyl that is optionally substituted with from one to three substituents selected from the group consisting of: alkyl, substituted alkyl, trifluoromethyl and halogen; and
followed if necessary or desired by salt formation.

In preparing the presently invented compounds of Reference Formula (I), the following novel intermediates are prepared:
4'-Amino-3'-hydroxybiphenyl-4-carboxylic acid;
4'-Amino-3'-hydroxybiphenyl-3-carboxylic acid;
3'-Amino-2'-hydroxybiphenyl-3-carboxylic acid;
3'-Amino-2'-hydroxybiphenyl-4-carboxylic acid;
3-Amino-2-hydroxy-3'-(1H-tetrazol-5-yl)biphenyl;
3-Amino-2-hydroxy-4'-(1H-tetrazol-5-yl)biphenyl;
3-Amino-5-chloro-2-hydroxy-4'-(1H-tetrazol-5-yl)-biphenyl;
6-(3-Amino-2-hydroxyphenyl)pyridine-2-carboxylic acid;
6-(3-Amino-5-chloro-2-hydroxyphenyl)pyridine-2-carboxylic acid;
6-(3-Amino -2-hydroxy-5-methylphenyl)pyridine-2-carboxylic acid;
5-(3-Amino-2-hydroxyphenyl)nicotinic acid;
5-(3-Amino-2-hydroxy-5-methylphenyl)nicotinic acid;
2-(3-Amino-2-hydroxyphenyl)isonicotinic acid;
3'-Amino-2'-hydroxy-5'-methylbiphenyl-3-carboxylic acid;
3'-Amino-5'-fluoro-2'-hydroxybiphenyl-3-carboxylic acid;
3'-Amino-5'-chloro-2'-hydroxybiphenyl-3-carboxylic acid;
3'-Amino-2'-hydroxybiphenyl-3,5-dicarboxylic acid;
N-[1-(3'-Amino-2'-hydroxybiphenyl-3-yl)methanoyl]methanesulfonamide;
N-(3'-Amino-2'-hydroxybiphenyl-3-yl)-1,1,1-trifluoro-methanesulfonamide;
(3'-Amino-2'-hydroxybiphenyl-3-yl)phosphonic acid;
3'-Amino-2'-hydroxybiphenyl-3,4-dicarboxylic acid;
3'-Amino-4,2'-dihydroxybiphenyl-3-carboxylic acid;
3'-Amino-2'-hydroxybiphenyl-3-sulfonic acid;
3'-Hydroxy-4'-nitrobiphenyl-4-carboxylic acid;
3'-Hydroxy-4'-nitrobiphenyl-3-carboxylic acid;
2'-Hydroxy-3'-nitrobiphenyl-3-carboxylic acid;
2'-Hydroxy-3'-nitrobiphenyl-4-carboxylic acid;
5-Chloro-2-hydroxy-3-nitro-3'-(1H-tetrazol-5-yl)biphenyl;
5-Chloro-2-hydroxy-3-nitro-4'-(1H-tetrazol-5-yl)biphenyl;
6-(5-Chloro-2-hydroxy-3-nitrophenyl)pyridine-2-carboxylic acid;
6-(2-Hydroxy-5-methyl-3-nitrophenyl)pyridine-2-carboxylic acid;
5-(5-Chloro-2-hydroxy-3-nitrophenyl)nicotinic acid;
5-(5-Chloro-2-hydroxy-5-methyl-3-nitrophenyl)nicotinic acid;
2-(5-Chloro-2-hydroxy-3-nitrophenyl)isonicotinic acid;
5'-Chloro-2'-hydroxy-3'-nitrobiphenyl-3-carboxylic acid;
5'-Chloro-2'-hydroxy-3'-nitrobiphenyl-3,5-dicarboxylic acid;
N-[1-(5'-Chloro-2'-hydroxy-3'-nitrobiphenyl-3-yl)methanoyl]methanesulfonamide;
1,1,1-Trifluoro-N-(2'-hydroxy-3'-nitrobiphenyl-3-yl)methanesulfonamide;
(5'-Chloro-2'-hydroxy-3'-nitrobiphenyl-3-yl)phosphonic acid;
5'-Chloro-2'-hydroxy-3'-nitrobiphenyl-3,4-dicarboxylic acid;
5'-Chloro-4,2'-dihydroxy-3'-nitrobiphenyl-3-carboxylic acid;
5'-Chloro-2'-hydroxy-3'-nitrobiphenyl-3-sulfonic acid;
2'-Methoxy-3'-nitrobiphenyl-3-carboxylic acid;
2'-Methoxy-3'-nitrobiphenyl-4-carboxylic acid;
5-Chloro-2-hydroxy-3'-(1H-tetrazol-5-yl)biphenyl;
5-Chloro-2-hydroxy-4'-(1H-tetrazol-5-yl)biphenyl;
6-(5-Chloro-2-hydroxyphenyl)pyridine-2-carboxylic acid;
6-(2-Hydroxy-5-methylphenyl)pyridine-2-carboxylic acid;
6-(2-Hydroxy-5-methylphenyl)pyridine-2-carboxylic acid;
5-(5-Chloro-2-hydroxy-5-methylphenyl)nicotinic acid;
2-(5-Chloro-2-hydroxyphenyl)isonicotinic acid;
5'-Chloro-2'-hydroxybiphenyl-3-carboxylic acid;
5'-Chloro-2'-hydroxybiphenyl-3,5-dicarboxylic acid;
N-[1-(5'-Chloro-2'-hydroxybiphenyl-3-yl)methanoyl]methanesulfonamide;
3'-Amino-3-nitrobiphenyl-2-ol;
(5'-Chloro-2'-hydroxybiphenyl-3-yl)phosphonic acid;
5'-Chloro-2'-hydroxybiphenyl-3,4-dicarboxylic acid;
5'-Chloro-4,2'-dihydroxybiphenyl-3-carboxylic acid;
5'-Chloro-2'-hydroxybiphenyl-3-sulfonic acid;
5-Chloro-2-methoxy-3'-(1H-tetrazol-5-yl)biphenyl;
5-Chloro-2-methoxy-4'-(1H-tetrazol-5-yl)biphenyl;
6-(5-Chloro-2-methoxyphenyl)pyridine-2-carboxylic acid;
6-(2-Methoxy-5-methylphenyl)pyridine-2-carboxylic acid;
6-(2-Methoxy-5-methylphenyl)pyridine-2-carboxylic acid;
5-(5-Chloro-2-methoxy-5-methylphenyl)nicotinic acid;
2-(5-Chloro-2-methoxyphenyl)isonicotinic acid;
5'-Chloro-2'-methoxybiphenyl-3-carboxylic acid;
5'-Chloro-2'-methoxybiphenyl-3,5-dicarboxylic acid;
N-[1-(5'-Chloro-2'-methoxybiphenyl-3-yl)methanoyl]methanesulfonamide;
N-(2'-Methoxy-3'-nitrobiphenyl-3-yl)-acetamide;
(5'-Chloro-2'-methoxybiphenyl-3-yl)phosphonic acid;
5'-Chloro-2'-methoxybiphenyl-3,4-dicarboxylic acid;
5'-Chloro-4-hydroxy-2'-methoxybiphenyl-3-carboxylic acid; and
5'-Chloro-2'-methoxybiphenyl-3-sulfonic acid.

The treatment of thrombocytopenia, as described herein, is accomplished by increasing the production of platelets.

By the term "co-administering" and derivatives thereof as used herein is meant either simultaneous administration or any manner of separate sequential administration of a TPO mimetic compound, as described herein, and a further active ingredient or ingredients, known to treat thrombocytopenia, including chemotherapy-induced thrombocytopenia and bone marrow transplantation and other conditions with depressed platelet production. The term further active ingredient or ingredients, as used herein, includes any compound or therapeutic agent known to or that demonstrates advantageous properties when administered with TPO or a TPO mimetic. Preferably, if the administration is not simultaneous, the compounds are administered in a close time proximity to each other. Furthermore, it does not matter if the compounds are administered in the same dosage form, e.g. one compound may be administered topically and another compound may be administered orally.

Examples of a further active ingredient or ingredients for use in combination with the presently invented TPO mimetic compounds include but are not limited to:
chemoprotective or myeloprotective agents such as G-CSF, BB 10010 (Clemons et al., Breast Cancer Res. Treatment, 1999, 57, 127), amifostine (Ethyol) (Fetscher et al., Current Opinion in Hemat., 2000, 7, 255-60), SCF, IL-11, MCP-4, IL-1-beta, AcSDKP (Gaudron et al., Stem Cells, 1999, 17, 100-6), TNF-a, TGF-b, MIP-1a (Egger et al., Bone Marrow Transpl., 1998, 22 (Suppl. 2), 34-35), and other molecules identified as having anti-apoptotic, survival or proliferative properties.

TPO has been demonstrated to act as a mobilizer of stem cells into the peripheral blood (Neumann T. A. et al., Cytokines, Cell. & Mol. Ther., 2000, 6, 47-56). This activity can synergize with stem cell mobilizers such as G-CSF (Somolo et al., Blood, 1999, 93, 2798-2806). The TPO mimetic compounds of the present invention are thus useful in increasing the numbers of stem cells in circulation in donors prior to leukapheresis for hematopoietic stem-cell transplantation in patients receiving myelo-ablative chemotherapy.

Likewise, TPO stimulates growth of myeloid cells, particularly those of granulocyte/macrophage lineage (Holly et al., US-5989537). Granulocyte/macrophage progenitors are cells of the myeloid lineage that mature as neutrophils, monocytes, basophils and eosinophils. The compounds described in the present invention have thus therapeutic utility in stimulating the poliferation of neutrophils in patients with neutropenic conditions.

Additional examples of a further active ingredient or ingredients for use in combination with the presently invented TPO mimetic compounds include but are not limited to: stem cell, megakaryocyte, neutrophil mobilizers such as chemotherapeutic agents (i.e., cytoxan, etoposide, cisplatin, Ballestrero A. et al., Oncology, 2000, 59, 7-13), chemokines, IL-8, Gro-beta (King, A. G. et al. J. Immun., 2000, 164, 3774-82), receptor agonist or antagonist antibodies, small molecule cytokine or receptor agonists or antagonists, SCF, Flt3 ligand, adhesion molecule inhibitors or antibodies such as: anti-VLA-4 (Kikuta T. et al., Exp. Hemat., 2000, 28, 311-7) or anti-CD44 (Vermeulen M. et al., Blood, 1998, 92, 894-900), cytokine/chemokine/interleukin or receptor agonist or antagonist antibodies, MCP-4 (Berkhout TA., et al., J. Biol. Chem., 1997, 272, 16404-16413; Uguccioni M. et al., J. Exp. Med., 1996, 183, 2379-2384).

Because the pharmaceutically active compounds of the present invention are active as TPO mimetics they exhibit therapeutic utility in treating thrombocytopenia and other conditions with depressed platelet production.

By the term "thrombocytopenia" and derivatives thereof as used herein is to be broadly interpreted as any decrease in the number of blood platelets below what is considered normal or desired for a healthy individual. Thrombocytopenia is known to have many causative factors, including but not limited to, radiation therapy, chemotherapy, immune therapy, immune thrombocytopenic purpura (ITP, Bussel J. B., Seminars in Hematology, 2000, 37, Suppl 1,1-49), myelodysplastic syndrom (MDS), aplastic anemia, AML, CML, viral infections (including, but not limited to; HIV, hepatitis C, parvovirus) liver disease, myeloablation, bone marrow transplant, stem cell transplant, peripheral blood stem cell transplant, progenitor cell defect, polymorphisms in stem cells and progenitor cells, defects in TPO, neutropenia (Sawai, N. J. Leukocyte Biol., 2000, 68, 137-43), dendritic cell mobilization (Kuter D. J. Seminars in Hematology. 2000, 37, Suppl 4, 41-49), proliferation, activation or differentiation. The pharmaceutically active compounds of this invention are useful in treating thrombocytopenia regardless of the factor or factors causing the condition. The pharmaceutically active compounds of this invention are also useful in treating thrombocytopenia when the causative factor or factors of the condition are unknown or have yet to be identified.

Prophylactic use of the compounds of this invention is contemplated whenever a decrease in blood or blood platelets is anticipated. Prophylactic use of the compounds of this invention results in a build up of platelets or a commencement of platelet production prior to an anticipated loss of blood or blood platelets. Prophylactic uses of the compounds of this invention includes but is not limited to transplant surgery, surgery, anesthesia prior to child birth and gut protection.

Human dendritic cells have been shown to express the TPO receptor (Kumamoto et al., Br. J. Haem, 1999, 105, 1025-1033) and TPO is a potent mobilizer of dendritic cells. The TPO mimetic compounds of the current invention are also useful as a vaccine adjuvant in that they increase the activity and mobility of dendritic cells. The pharmaceutically active compounds of this invention are useful as an immunological adjuvant, given in combination with an orally, transdermally or subcutaneously delivered vaccine and/or immunomodulator, by increasing the activity and mobility of dendritic cells.

TPO is known to have various effects including anti-apototic/survival effects on megakaryocytes, platelets and stem cells, and proliferative effects on stem cells and megakaryocytic cells (Kuter D. J. Seminars in Hematology, 2000, 37, 41-9). These TPO activities effectively increase the number of stem and progenitor cells so that there is synergistic effects when Tpo is used in conjunction with other cytokines that induce differentiation.

The TPO mimetic compounds of the current invention are also useful in acting on cells for survival or proliferation in conjunction with other agents known to act on cells for survival or proliferation. Such other agents include but are not limited to: G-CSF, GM-CSF, TPO, M-CSF, EPO, Gro-beta, IL-11, SCF, FLT3 ligand, LIF, IL-3, IL-6, IL-1, Progenipoietin, NESP, SD-01, or IL-5 or a biologically active derivative of any of the aforementioned agents, KT6352 (Shiotsu Y. et al., Exp. Hemat. 1998, 26, 1195-1201), uteroferrin (Laurenz JC., et al. Comp. Biochem. & Phys., Part A. Physiology., 1997, 116, 369-77), FK23 (Hasegawa T., et al. Int. J. Immunopharm., 1996, 18 103-112) and other molecules identified as having anti-apoptotic, survival or proliferative properties for stem cells, progenitor cells, or other cells expressing Tpo Receptors.

In determining potency as TPO mimetics, the following assays were employed:

### Luciferase Assay

Compounds of the present invention were tested for potency as mimetics of the TPO receptor in a Luciferase assay such as described in Lamb, et al., Nucleic Acids Research 23: 3283-3289 (1995) and Seidel, et al., Proc. Natl. Acad. Sci., USA 92: 3041 - 3045 (1995) by substituting a TPO-responsive BaF3 cell line (Vigon et al. Proc. Natl. Acad. Sci. USA 1992, 89, 5640-5644) for the HepG2 cells utilized therein. The murine BaF3 cells express TPO receptors and closely match the pattern of STAT (signal transducers and activators of transcription) activation observed in primary murine and human bone marrow cells.

### Proliferation Assay

Some of the more preferred compounds of this invention were active in an in vitro proliferation assay using the human UT7TPO cell line. UT7TPO cells are a human megakaryoblastic cell line that express TPO-R, whose survival and growth is dependent on the presence of TPO (Komatsu et al. Blood 1996, 87,4552).

### Differentiation Assay

Likewise, some of the most preferred compounds of this invention were also positive in stimulating the maturation of megakaryocytes from human bone marrow cells. In this assay, purified human CD34+ progenitor cells were incubated in liquid culture with test compounds for 10 days and the number of cells expressing the transmembrane glycoprotein CD41 (gpIIb), a megakaryocytic marker, was then measured by flow cytometry (see Cwirla, S. E. et al Science, 1997, 276, 1696).

The pharmaceutically active compounds within the scope of this invention are useful as TPO mimetics in mammals, particularly humans, in need thereof.

Some of the preferred compounds within the scope of the invention showed activation from about 4% to 100% control at a concentration of 0.001-10 uM in the luciferase assay. The preferred compounds of the invention also promoted the proliferation of UT7TPO and 32D-mpl cells at a concentration of 0.003 to 30 uM. The preferred compounds of the invention also showed activity in the CD41 megakaryocytic assay at a concentration of 0.003 to 30 uM.

The drug may be administered to a patient in need thereof by any conventional route of administration, including, but not limited to, intravenous, intramuscular, oral, subcutaneous, intradermal, and parenteral.

The pharmaceutically active compounds of the present invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid;. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid such as an ampoule, or an aqueous or nonaqueous liquid suspension.

The pharmaceutical preparations are made following conventional techniques of a pharmaceutical chemist involving mixing, granulating, and compressing, when necessary, for tablet forms, or mixing, filling and dissolving the ingredients, as appropriate, to give the desired oral or parenteral products.

Doses of the presently invented pharmaceutically active compounds in a pharmaceutical dosage unit as described above will be an efficacious, nontoxic quantity preferably selected from the range of 0.001 - 100 mg/kg of active compound, preferably 0.001 - 50 mg/kg. When treating a human patient in need of a TPO mimetic, the selected dose is administered preferably from 1-6 times daily, orally or parenterally. Preferred forms of parenteral administration include topically, rectally, transdermally, by injection and continuously by infusion. Oral dosage units for human administration preferably contain from 0.05 to 3500 mg of active compound. Oral administration, which uses lower dosages is preferred. Parenteral administration, at high dosages, however, also can be used when safe and convenient for the patient.

Optimal dosages to be administered may be readily determined by those skilled in the art, and will vary with the particular TPO mimetic in use, the strength of the preparation, the mode of administration, and the advancement of the disease condition. Additional factors depending on the particular patient being treated will result in a need to adjust dosages, including patient age, weight, diet, and time of administration.

The method of this invention of inducing TPO mimetic activity in mammals, including humans, comprises administering to a subject in need of such activity an effective TPO mimetic amount of a pharmaceutically active compound of the present invention.

The invention also provides for the use of a compound of Formula (VI) in the manufacture of a medicament for use as a TPO mimetic.

The invention also provides for the use of a compound of Formula (VI) in the manufacture of a medicament for use in therapy.

The invention also provides for the use of a compound of Formula (VI) in the manufacture of a medicament for use in enhancing platelet production.

The invention also provides for the use of a compound of Formula (VI) in the manufacture of a medicament for use in treating thrombocytopenia.

The invention also provides for a pharmaceutical composition for use as a TPO mimetic which comprises a compound of Formula (VI) and a pharmaceutically acceptable carrier.

The invention also provides for a pharmaceutical composition for use in the treatment of thrombocytopenia which comprises a compound of Formula (VI) and a pharmaceutically acceptable carrier.

The invention also provides for a pharmaceutical composition for use in enhancing platelet production which comprises a compound of Formula (VI) and a pharmaceutically acceptable carrier.

No unacceptable toxicological effects are expected when compounds of the invention are administered in accordance with the present invention.

In addition, the pharmaceutically active compounds of the present invention can be co-administered with further active ingredients, such as other compounds known to treat thrombocytopenia, including chemotherapy-induced thrombocytopenia and bone marrow transplantation and other conditions with depressed platelet production, or compounds known to have utility when used in combination with a TPO mimetic.

Contemplated Equivalents - It will be appreciated by the person of ordinary skill in the art that the compounds of Reference Formulas I and II may also exist in tautomeric forms. For example, in Formula I, the double bond that is drawn between the two nitrogen atoms exists between the lower nitrogen atom and the AR substituent. Tautomeric forms of the compounds of Reference Formulas I and II are exemplified by the following Formula (IV): 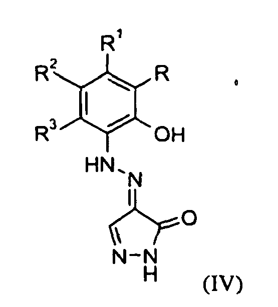 where the 'R' groups are as defined above. All such compounds are included in the scope of the invention and inherently included in the definition of the compounds of Reference Formulas I and II.

Without further elaboration, it is believed that one skilled in the art can, using the preceding description, utilize the present invention to its fullest extent.

### Experimental Details

### Example 1

### Preparation of 4'-{N'-[1-(3,4-dimethylphenyl)-3-methyl-5-oxo-1,5-dihydropyrazol-4-ylidenelhydrazinol-3'-hydroxybiphenyl-4-carboxylic acid;

### a) 5-bromo-2-nitrophenol

3-Bromophenol (32.9 g, 0.19 mol) was added slowly to a cold (10°C) solution of sodium nitrate (29.0 g, 0.34 mol) in conc. sulfuric acid; (40.0 g) and water (70.0 mL) and the resulting mixture was allowed to stir at room temperature for 2h. Water (200 mL) was added and the resulting mixture was extracted with diethyl ether and the extract was dried (MgSO₄), filtered and concentrated. The residue was purified by flash chromatography (silica gel, 10% ethyl acetate/hexanes) to afford first the title compound (8.1 g, 20%), mp 40-42°C, then the undesired isomer, 3-bromo-4-nitrophenol, as a yellow solid (12.7 g, 31%). mp 125-127°C.

### b) 3'-hydroxy-4'-nitrobiphenyl-4-carboxylic acid;

A solution of the compound from Example 1a) (2.18 g, 0.01 mol.), 4-carboxyphenylboronic acid; (1.74 g, 0.0105 mol.), 2M aqu. sodium carbonate (10.0 mL; 0.02 mol.) and tetrakistriphenylphosphino palladium(0) (0.5 g) in 1,4-dioxane (60.0 mL) was stirred and heated under reflux under a nitrogen atmosphere for 24h.

The reaction mixture was cooled and evaporated and the residue treated with 6M aqu. hydrochloric acid; (100 mL). The grey precipitate was filtered and washed well with water then diethyl ether to afford the title compound (2.3 g; 88%) as a colorless solid. ¹H NMR (300 MHz, d₆-DMSO) δ 13.5-10.5 (br s, 2H), 8.06 (d, J = 8.4 Hz, 2H), 8.03 (d, J = 8.6 Hz, 1H), 7.83 (d, J = 8.4 Hz, 1H), 7.45 (d, J = 1.8 Hz, 1H), 7.35 (dd, J = 8.6, 1.8 Hz, 1H).

### c) 4'-amino-3'-hydroxybiphenyl-4-carboxylic acid;, hydrochloride salt

A solution of the compound from Example 1b) (1.6 g, 0.0062 mol.) in ethanol (75.0 mL), water (50.0 mL) and 3M aqu. sodium hydroxide (2.0 mL, 0.0062 mol.) was hydrogenated over 10% palladium on carbon (0.2 g) at room temperature and 50 psi for 2h.

The reaction mixture was filtered, treated with 3M aqu. hydrochloric acid; (25.0 mL) then evaporated and the residue triturated with a little water to afford the title compound (1.18 g; 72%) as a brown solid. ¹H NMR (300 MHz, d₆-DMSO) δ 10.90 (s, 1H), 10.5-8.5 (br s, 3H), 8.03 (d, J = 8.2 Hz, 2H), 7.71 (d, J = 8.2 Hz, 2H), 7.41 (d, J = 8.2 Hz, 1H), 7.36 (d, J = 1.6 Hz, 1H), 7.22 (dd, J = 8.2, 1.6 Hz,1H).

### d) 1-(3,4-Dimethylphenyl)-3-methyl-3-pyrazolin-5-one

A solution of 3,4-dimethylphenylhydrazine hydrochloride (17.7 g; 0.1 mol.), ethyl acetoacetate (13.0 g; 0.1 mol.) and sodium acetate (8.2 g; 0.1 mol.) in glacial acetic acid; (250 mL) was stirred and heated under reflux for 24h.
The mixture was cooled and evaporated and the residue dissolved in diethyl ether (1L) and carefully washed with sat. aqu. sodium hydrogen carbonate (5 x 200 mL). The ethereal layer was evaporated to afford the title compound (15.4 g; 76%). ¹H NMR (300 MHz, d₆-DMSO) δ 11.30 (br s, 1H), 7.49 (d, J = 1.4 Hz, I H), 7.43 (dd, J = 8.2 Hz, I H), 7.14 (d, J = 8.2 Hz, I H), 5.31 (s, 1H), 2.20 (s, 3H), 2.22 (s, 3H), 2.08 (s, 3H); MS(ES) m/z 203 [M+H]. e) 4'-{N'-[1-(3,4-Dimethylphenyl)-3-methyl-5-oxo-1,5-dihydropyrazol-4-ylidene]hydrazino}-3'-hydroxybiphenyl-4-carboxylic acid;, hemihydrate A suspension of the compound from Example 1c) (1.0 g; 0.0044 mol.) in 1M aqu. hydrochloric acid; (15.0 mL) was cooled to 5°C then treated dropwise with a solution of sodium nitrite (0.32 g; 0.0046 mol.) in water (5.0 mL). The yellow mixture was stirred at 5°C for a further 10 min. then treated in one portion with the compound from Example 1d) (0.882 g, 0.0044 mol.) followed by the portion-wise addition of sodium hydrogen carbonate (1.8 g; 0.022 mol.) and ethanol (20.0 mL) ensuring the final pH of the reaction mixture is approximately 7-8. The red solution was then stirred at room temperature for 24h.

The mixture was filtered to give a red solid which was slurried in water (50.0 mL) and then acid;ified with concentrated hydrochloric acid;. Filtration afforded the title compound (0.68 g; 35%) as an orange powder, mp = 280°C (dec.).. ¹H NMR (300 MHz, d₆-DMSO) δ 13.62 (s, I H), 13.2-12.2 (br s, I H), 10.92 (s, I H), 8.02 (d, J = 8.2 Hz, 2H), 7.73-7.69 (m, 5H), 7.63 (d, 8.2 Hz, 1H), 7.31 (d, J = 8.4 Hz, 1H), 7.29 (s, 1H), 7.19 (d, J = 8.4 Hz, 1H), 2.30 (s, 3H), 2.26 (s, 3H), 2.2 (s, 3H); Anal. (C₂₅H₂₂N₄O₄.0.5 H₂O) calcd: C, 66.51; H, 5.13; N, 12.41. found: C, 66.74; H, 5.08; N, 12.36.

### Example 2

### Preparation of 4'-{N'-[1-(3,4-dimethylphenyl)-3-methyl-5-oxo-1,5-dihydropyrazol-4-ylidene]hydrazino}-3'-hydroxybiphenyl-3-carboxylic acid;

### a) 3'-hydroxy-4'-nitrobiphenyl-3-carboxylic acid;

Following the procedure of Example 1b), except substituting 4-carboxyphenylboronic acid; for 3-carboxyphenylboronic acid;, the title compound was prepared (1.75 g; 68%) as a brown powder. ¹H NMR (300 MHz, d₆-DMSO) δ 13.16 (br s, 1H), 11.18 (s, 1H), 8.20 (d, J = 0.9 Hz, 1H), 8.03 (d, J = 8.6 Hz, 2H), 7.96 (dd, J = 7.8, 0.9 Hz, 1H), 7.66 (t, J = 7.8 Hz, 1H), 7.45 (s, 1H), 7.34 (d, J = 8.6Hz, 1H).

### b) 4-amino-3'-hydroxybiphenyl-3-carboxylic acid;, hydrochloride salt

Following the procedure of Example 1c), except substituting 3'-hydroxy-4'-nitrobiphenyl-3-carboxylic acid; for 3'-hydroxy-4'-nitrobiphenyl-4-carboxylic acid;, the title compound was prepared (1.32 g; 83%) as a tan solid. ¹H NMR (300 MHz, d₆-DMSO) δ 10.96 (s, 1H), 10.5-9.5 (br s, 3H), 8.12 (t, J = 2.6 Hz, 1H), 7.96 (dt, J = 7.9, 1.3 Hz, 1H), 7.86 (dt, J = 8.2, 1.3 Hz, 1H), 7.62 (t, J = 7.9 Hz, 1H), 7.44 (d, J = 8.2 Hz, 1H), 7.35 (d, J = 1.9 Hz, 1H), 7.21 (dd, J = 8.2, 1.9 Hz, 1H).

### c) 4'-{N'-[1-(3,4-dimethylphenyl)-3-methyl-5-oxo-1,5-dihydropyrazol-4-ylidene]hydrazino}-3'-hydroxybiphenyl-3-carboxylic acid;

Following the procedure of Example 1e), except substituting 4-amino-3'-hydroxybiphenyl-3-carboxylic acid;, hydrochloride salt for 4-amino-3'-hydroxybiphenyl-4-carboxylic acid;, hydrochloride salt, the title compound was prepared 1.04 g; 62%) as an orange solid. mp 282°C (dec.). ¹H NMR (300 MHz, d₆-DMSO) δ 13.66 (s, 1H), 13.15 (s, 1H), 10.89 (s, 1H), 8.16 (s, 1H), 7.94 (d, J = 7.7 Hz, I H), 7.88 (d, J = 7.9 Hz, I H), 7.73-7.71 (m, 2H), 7.65-7.57 (m, 2H), 7.32-7.30 (m, 2H), 7.20 (d, J = 8.3 Hz, 1H), 2.31 (s, 3H), 2.27 (s, 3H), 2.22 (s, 3H); Anal. (C₂₅H₂₂N₄O₄.0.25 H₂O) calcd: C, 67.18; H, 4.97; N, 12.53. found: C, 67.26; H, 4.96; N, 12.46.

### Example 3

### Preparation of 3'-{N'-[1-(3,4-dimethylphenyl)-3-methyl-5-oxo-1,5-dihydropyrazol-4-ylidene]hydrazino}-2'-hydroxybiphenyl-3-carboxylic acid;

### a) 2-bromo-6-nitrophenol

Following the procedure of Example 1a) except substituting 2-bromophenol for 3-bromophenol, the title compound was prepared (10.9 g; 25%) as a bright, yellow solid. ¹H NMR (300 MHz, CDCl₃) δ 11.10 (S, 1h), 8.13 (d, J = 7.9 Hz, 1H), 7.89 (d, J = 7.9 Hz, 1H), 6.90 (t, J = 7.9 Hz, I H).

### b) 2-bromo-6-nitroanisole

a mixture of the compound from Example 2a) (10.8 g; 0.0495 mol.), methyl iodide (3.4 mL; 0.00545 mol.) and potassium carbonate (8.2 g; 0.0592 mol.) in acetone (250 mL) was stirred and heated under reflux for 24h.

The mixture was evaporated and the residue triturated with water to afford the title compound (8.7 g; 76%). mp 55-56°C. ¹H NMR (300 MHz, CDCl₃ δ 7.81-7.74 (m, 2H), 7.13 (t, J = 8.1 Hz, 1H), 4.02 (s, 3H); Anal. (C₇H₆NO₃Br) calcd: C, 36.24; H, 2.61; N, 6.04. found: C, 36.30; H, 2.59; N, 5.73.

### c) 2'-methoxy-3'-nitrobiphenyl-3-carboxylic acid;

Following the procedure of Example 1b), except substituting the compound from Example 3b) for 5-bromo-2-nitrophenol, and substituting 3-carboxy phenylboronic acid for 4-carboxy boronic acid the title compound was prepared (2.13 g; 47%) as a tan powder. ¹H NMR (300 MHz, d₆-DMSO) δ 8.12 (s, 1H), 8.03 (d, J = 7.9 Hz, 1H), 7.94 (dd, J = 7.9 Hz, 1.5 Hz, 1H), 7.85 (d, J = 7.9 Hz, 1H), 7.76 (dd, J = 7.5, 1.5 Hz, 1H), 7.66 (t, J = 7.5 Hz, 1H), 7.46 (t, j = 7.9 HzI H), 3.46 (s, 3H).

### d) 2'-hydroxy-3'nitrobiphenyl-3-carboxylic acid

A solution of the compound from Example 3c) (2.13 g; 0.0077 mol.) in glacial acetic acid; (25.0 mL) and 48% aqu/ hydrobromic acid; (25.0 mL) was stirred and heated under reflux for 5h.

The mixture was cooled and filtered to afford the title compound (1.57 g; 79%) as a tan powder. ¹H NMR (300 MHz, d₆-DMSO) δ 13.90 (s, 1H), 10.66 (s, 1H), 8.12 (t, J = 1.7 Hz, 1H), 8.07 (dd, J = 8.4, 1.7 Hz, 1H), 7.98 (dt, 7.8, 1.5 Hz, 1H), 7.79 (dt, J = 8.1, 1.7 Hz, I H), 7.74 (dd, J = 7.5, 1.7 Hz, I H), 7.62 (t, J = 7.8 Hz, I H), 7.17 (dd, J = 8.4, 7.5 Hz, 1H).

### e) 3-amino-2'-hydroxybiphenyl-3-carboxylic acid; hydrochloride salt.

Following the procedure of Example 1c), except substituting the compound from Example 3d) for 3'-hydroxy-4'-nitrobiphenyl-4-carboxylic acid;, the title compound was prepared (1.51 g; 100%) as a brown solid.11.3-8.7 (br s, 4H), 8.08 (s, 1H), 7.95 (d, J = 7.8 Hz, 1H), 7.74 (d, J = 7.8 Hz, 1H), 7.61 (t, J = 7.8 Hz, 1H), 7.34 (dd, J = 7.8, 1.4 Hz, 1 H), 7.24 (dd, J = 7.8, 1.3 Hz, 1H), 7.04 (t, J = 7.8 Hz, 1 H).

### f) 3'-{N'-[1-(3,4-dimethylphenyl)-3-methyl-5-oxo-1,5-dihydropyrazol-4-ylidene]hydrazino}-2'-hydroxybiphenyl-3-carboxylic acid;, hydrate

Following the procedure of Example 1e), except substituting the compound from Example 3e) for 4'-amino-3'-hydroxybiphenyl-4-carboxylic acid;, hydrochloride salt, the title compound was prepared (0.055 g; 32%) as an orange solid. mp 228°C (dec.). ¹H NMR (300 MHz, d₆-DMSO) δ 13.76 (s, 1H), 13.12 (s, 1H), 9.70 (s, 1H), 8.14 (s, 1H), 7.97 (dd, J = 7.7 Hz, 1H), 7.81 (dd, J = 7.7 Hz, 1H), 7.74-7.60 (m, 5H), 7.22-7.13 (m, 3H), 2.34 (s, 3H), 2.27 (s, 3H), 2.23 (s, 3H); Anal. (C₂₅H₂₂N₄O₄.1.0 H₂O) calcd: C, 65.21; H, 5.25; N, 12.17. found: C, 65.60; H, 4.96; N, 12.04.

### Example 4

### Preparation of 3'-{N'-[1-(4-tert-butylphenyl)-3-methyl-5-oxo-1,5-dihydropyrazol-4-ylidene]hydrazino}-2'-hydroxybiphenyl-3-carboxylic acid:, hemihydrate

### a) 1-(4-tert-Butyl)-3-methyl-3-pyrazolin-5-one

Following the procedure of example 1d), except substituting 4-*tert*-butylphenylhydrazine hydrochloride for 3,4-dimethylphenylhydrazine hydrochloride, the title compound was prepared (13.8 g; 60%). ¹H NMR (300 MHz, d₆-DMSO) δ 11.32 (s, 1H), 7.68 (d, J = 7.8 Hz, 2H), 7.40 (d, J = 7.8 Hz, 2H), 5.32 (s, 1H), 2.09 (s, 3H), 1.33 (s, 9H).

### b) 3'-{N'-[1-(4-tert-Butylphenyl)-3-methyl-5-oxo-1,5-dihydropyrazol-4-ylidene]hydrazino}-2'-hydroxybiphenyl-3-carboxylic acid;, hemihydrate

Following the procedure of Example 1e), except substituting the compound from Example 3e) for 4-amino-3'-hydroxybiphenyl-3-carboxylic acid;, hydrochloride salt and the compound from Example 4a) for 1-(3,4-dimethylphenyl)-3-methyl-3-pyrazolin-5-one, the title compound was prepared (0.391 g; 42%) as an orange solid, mp 145°C (dec.). ¹H NMR (300 MHz, d₆-DMSO) δ 13.76 (s, 1H), 13.07 (s,I H), 9.72 (s, 1H), 8.14 (s, 1H), 7.98 (dd, J = 7.8, 1.2 Hz, 1H), 7.83 (t, J = 8.7 Hz, 1H), 7.73 (dd, J = 6.4, 3.1 Hz, 1H), 7.63 (t, J = 7.7 Hz, 1H), 7.49 (d, J = 7.8 Hz, 2H), 7.20-7.16 (m, 2H), 2.35 (s, 3H), 1.31 (s, 9H). Anal. (C₂₇H₂₆N₄O₄.0.5 H₂O) calcd: C, 67.63; H, 5.67; N, 11.68. found: C, 67.53; H, 5.46; N, 11.66.

### Example 5

### 2-Aza-3'-{N'-[1-(4-tert-butylphenyl)-3-methyl-5-oxo-1,5-dihydropyrazol-4-ylidene]hydrazino}-5'-chloro-2'-hydroxybiphenyl-3-carboxylic acid;

Following the procedure of Example 8, except substituting the compound from Example 4a for the compound from Example 1d, the title compound was prepared as red powder (0.08 g, 44%). MS(ES) m/z 506 [M+H].

### Example 6

### 2-Aza-3'-{N'-[1-(4-tert-butylphenyl)-3-methyl-5-oxo-1,5-dihydropyrazol-4-ylidene]hydrazino}-2'-hydroxybiphenyl-3-carboxylic acid;

### a) 6-(5-chloro-2-methoxyphenyl)-pyridine-2-carboxylic acid;:

Following the procedure of Example 1b), except substituting 2-methoxy-5-chlorophenylboronic acid; for 4-carboxyphenylboronic acid; , and substituting 6-bromopyridine-2-carboxylic acid; for the compound of 1a), the title compound was prepared (6.7 g; 100%) as a white powder. MS(ES) m/z 264 [M+H].

### b) 6-(5-chloro-2-hydroxyphenyl)-pyridine-2-carboxylic acid;:

Following the procedure of Example 3d), except substituting the compound of 6a) for the compounds of 3c), the title compound was prepared (3.5 g; 74%) as a grey powder. MS(ES) m/z 250 [M+H].

### c) 6-(5-chloro-2-hydroxy-3-nitrophenyl)-pyridine-2-carboxylic acid;:

Following the procedure of Example 7c), except substituting the compound from example 6b) for the compound from example 7b), the title compound was prepared (1.3 g; 73%) as a powder. ¹H NMR (300 MHz, d₆-DMSO) δ 8.71 (d, J = 9Hz, 1H), 8.6 (d, J = 3.4Hz, 1H), 8.33 (t, J = 8.6Hz, 1H), 8.19 (d, J = 8.6Hz, 1H), 8.14 (d, J=3.4Hz, 1H)

### d)3-Aza-3'- {N'-[1-(4-tert-butylphenyl)-3-methyl-5-oxo-1,5-dihydropyrazol-4-ylidene]hydrazino}-2'-hydroxybiphenyl-3-carboxylic acid;

Following the procedure of Example 1c), except substituting the compound of 6c) for the compound of 1b), the crude product was isolated. A suspension of the crude product ( 0.0033mol.) in 1M aqu. hydrochloric acid; (25.0 mL) was cooled to 5°C then treated dropwise with a solution of sodium nitrite (0.22 g; 0.0033 mol.) in water (5.0 mL). The yellow mixture was stirred at 5°C for a further 10 min. then treated in one portion with the compound from Example 4a) (0.68 g, 0.003 mol.) followed by the portion-wise addition of sodium hydrogen carbonate and ethanol ensuring the final pH of the reaction mixture is approximately 7-8. The red solution was then stirred at room temperature for 24h.

The mixture was filtered to give a red solid which was slurried in water (50.0 mL) and then acid;ified with concentrated hydrochloric acid;. Filtration afforded the title compound (0.95g; 67%) as a powder.MS(ES) m/z 472 (M+H)⁺.

### Example 7

### 3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid;:

### a) 5-(5-chloro-2-methoxyphenyl)-nicotinic acid;:

Following the procedure of Example 1b), except substituting 2-methoxy-5-chlorophenylboronic acid; for 4-carboxyphenylboronic acid; , and substituting 5-bromonicotinic acid; for the compound of 1a), the title compound was prepared. MS(ES) m/z 264 [M+H].

### b) 6-(5-chloro-2-hydroxyphenyl)-pyridine-2-carboxylic acid;:

Following the procedure of Example 3d), except substituting the compound of 7a) for the compounds of 3c), the title compound was prepared. MS(ES) m/z 250 [M+H].

### c) 6-(5-chloro-2-hydroxy-3-nitrophenyl)-pyridine-2-carboxylic acid;:

To the solution of 6-(5-chloro-2-hydroxyphenyl)-pyridine-2-carboxylic acid; (2.3 g, 10.1 mmol) in 100ml acetic acid; was added 1ml fuming nitric acid; and stirred at 35°C to 40°C for half an hour. The reaction mixture was diluted with water and adjusted pH to 2.5. The resulting preciptation was collected, washed and dried to give solid (2.74g; 78%, three steps). MS(ES) m/z 295 [M+H].

### d)2-Aza-3'-{N'-[1-(4-tert-butylphenyl)-3-methyl-5-oxo-1,5-dihydropyrazol-4-ylidene]hydrazino}-2'-hydroxybiphenyl-3-carboxylic acid;

Following the procedure of Example 1c), except substituting the compound of 7c) for the compound of 1b), the crude product was isolated. A suspension of the crude product ( 0.0015mol.) in 1M aqu. hydrochloric acid; (25.0 mL) was cooled to 5°C then treated dropwise with a solution of sodium nitrite (0.11 g; 0.0015 mol.) in water (5.0 mL). The yellow mixture was stirred at 5°C for a further 10 min. then treated in one portion with the compound from Example 4a) (0.34 g, 0.0015 mol.) followed by the portion-wise addition of sodium hydrogen carbonate and ethanol ensuring the final pH of the reaction mixture is approximately 7-8. The red solution was then stirred at room temperature for 24h. The mixture was filtered to give a red solid which was slurried in water (50.0 mL) and then acid;ified with concentrated hydrochloric acid;. Filtration afforded the title compound (0.2 g; 29%) as a powder. ¹H NMR (300 MHz, d₆-DMSO) δ13.8 (br, 2H), 9.9 (s, 1H), 9.08 (s, 1H), 8.9 (s,1H), 8.4 (s, 1H), 7.82 (d, J=7.7Hz, 2H), 7.75 (d, J = 7.9Hz, 1H), 7.50 (d, J = 7.0Hz, 2H), 7.20 (m, 2H), 2.34 (s, 3H), 1.32(s, 9H) MS(ES) m/z 472 (M+H)⁺.

### Example 8

### 2-Aza-5'-chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;:

The compound from Example 6c) (1g, 3.39 mmol) in ethanol was treated with tin chloride (3.2 g, 17 mmol) and refluxed for 3 hours. After quenched with 3N hydrochloride, the precipitate was collected and washed with ether. 1.1g crude product was isolated. Following the procedure of Example 1e), except substituting the above crude product for the compound from Example 1c), the title compound was prepared as powder (75%). ¹H NMR (300 MHz, d₆-DMSO) δ 8.42 (d, J = 9.2Hz, 1H), 8.2 (t, J=8.0Hz, 1H), 8.0 (m, 1H), 7.8(s, 1H), 7.62 (m, 2H), 7.2 (d, J = 9.2Hz, 1H), 2.34 (s, 3H), 2.28(s, 3H), 2.22 (s, 3H).

### Example 9

### 2-Aza-3'-{N'[1-(4-tert-butylphenyl)-3-methyl-5-oxo-1,5-dihydropyrazol-4-ylidene]hydrazino}-2'-hydroxy-5'-methylbiphenyl-3-carboxylic acid;:

### a) 6-(2-hydroxy-5-methylphenyl)-pyridine-2-carboxylic acid;:

Following the procedure of Example 6a) and 6b), except substituting 5-methyl-2-methoxy boronic acid; for 2-methoxy boronic acid;, the title compound was prepared as solid (0.26 g, 31 %, two steps). ¹H NMR (300 MHz, d₆-DMSO) δ 8.42 (d, J = 8.5Hz, 1H), 8.2 (t, J=8.2Hz, 1H), 8.04 (d, J = 8.5Hz, 1H), 7.9(s, 1H), 7.17 (d, J = 8.2Hz, 1H), 6.88 (d, J = 8.2Hz, 1H), 2.22 (s, 3H).

### b) 6-(2-hydroxy-3-amino-5-methylphenyl)-pyridine-2-carboxylic acid;:

Following the procedure of Example 7c), except substituting the compound from example 9a) for the compound from example 7b). The crude product was isolated , redissolved in ethanol and water, and treated with 10%Pd/C. The reaction mixture was shaked under 50psi hydrogen for 3 hours before filtered and concentrated down to give green power (80%). MS(ES) m/z 245 (M+H)⁺.

### c) 2-Aza-3'-{N'-[1-(4-tert-butylphenyl)-3-methyl-5-oxo-1,5-dihydropyrazol-4-ylidene]hydrazino}-2'-hydroxy-5'-methylbiphenyl-3-carboxylic acid;:

Following the procedure of Example 4b), except substituting the compound from example 9b) for the compound from example 3e), the title compound was prepared as solid (26%), ¹H NMR (300 MHz, d₆-DMSO) δ 8.25 (d, J = 7.7Hz, 1H), 8.15 (t, J = 7.7Hz, 1H), 7.9 (m, 3H), 7.78(s, 1H), 7.58 (s, 1H), 7.5 (d, J = 7.7Hz, 2H), 2.36 (s, 3H), 2.35(s, 3H), 1.23 (s, 9H).

### Example 10

### 2-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-3-carboxylic acid;:

Following the procedure of Example 9), except substituting 1-(3,4-dimethylphenyl)-3-methyl-3-pyrazolin-5-one for 1-(4-tert-Butyl)-3-methyl-3-pyrazolin-5-one, the title compound was prepared as solid (40%). ¹H NMR (300 MHz, d₆-DMSO) δ 8.45 (d, J = 8.0Hz, 1H), 8.25 (t, J = 8.0Hz, 1H), 8.05 (d, J = 8.0, 1H), 7.85(s, 1H), 7.78 (s, 1H), 7.68 (d, J = 7.7Hz, 1H), 7.62 (s, 1H), 7.22 (d, J = 8.0Hz, 1H), 2.36 (s, 3H), 2.35(s, 3H), 2.28 (s, 3H), 2.25 (s, 3H).

### Example 11

### 3'-{N'-[1-(4-tert-Butylphenyl)-3-methyl-5-oxo-1,5-dihydropyrazol-4-ylidene]hydrazino}-2'-hydroxy-5'-methylbiphenyl-3-carboxylic acid;:

### a) 2'-Hydroxy-5'-methylbiphenyl-3-carboxylic acid;:

Following the procedure of Example 1b), except substituting 2-methoxy-5-methylphenylboronic acid; for 4-carboxyphenylboronic acid; and 3-bromophenylcarboxylic acid; for the compound from example 1a), the crude compound was isolated and treated with 1:1 of hydrobromide and acetic acid;. The reaction mixture was refluxed for 4 hours. Water was added and the resulting mixture was extracted with ethyl acetate. The exact was dried (MgSO₄), filtered and concentrated to the title product (50%, two steps). ¹H NMR (300 MHz, d₆-DMSO) δ 8.23 (s, 1H), 8.1 (d, J = 8.1Hz,I H), 7.79 (d, J = 8.1Hz, 1H), 7.58 (t, J = 7.8Hz, 1H), 7.1 (s, J = 8.2Hz, 1H), 7.05 (s,I H), 6.85 (d, J = 7.8Hz, 1H), 2.15 (s, 3H).

### b) 2'-Hydroxy-3-nitro-5'-methylbiphenyl-3-carboxylic acid;:

Following the procedure of example 7c), except substituting the compound of example 11 a) for the compound of example 7b), the title compound was prepared as solid (0.4 g, 77%). ¹H NMR (300 MHz, d₆-DMSO) δ 8.13 (s, I H), 8.0 (d, J = 6.9Hz, 1H), 7.84 (s, 1H), 7.8 (d, J = 6.9Hz, 1H), 7.6 (m, 2H), 2.3 (s, 3H).

### c) 3'-{N'-[1-(4-tert-Butylphenyl)-3-methyl-5-oxo-1,5-dihydropyrazol-4-ylidene]hydrazino}-2'-hydroxy-5'-methylbiphenyl-3-carboxylic acid;:

Following the procedure of example 7d), except substituting the compound of example 11b) for the compound of example 7c), the title compound was prepared as solid (0.003 g, <10%).¹H NMR (300 MHz, d₆-DMSO) δ 8.13 (s, 1H), 7.95 (d, J = 8.0Hz, 1H), 7.82 (t, J = 7.7Hz, 2H), 7.72 (m, 2H), 7.61 (t, J = 7.7Hz, 2H), 7.53 (s, 1H), 7.5 (d, 8.0Hz, 2H), 2.33 (s, 6H), 1.22 (s, 9H). MS(ES) m/z 485 (M+H)⁺.

### Example 12

### 3-{N'-[1-(3,4-dimethylphenyl)-3-methyl-5-oxo-1,5-dihydropyrazol-4-ylidene]hydrazino}-2-hydroxy-3'-(tetrazol-5-yl)biphenyl

### a) 5-(5'-chloro-2-methoxybiphenyl-3-yl)-1H-tetrazole:

Following the procedure of Example 1b), except substituting 2-methoxy-5-chlorophenylboronic acid; for 4-carboxyphenylboronic acid; , and substituting 5-(3-bromophenyl)-1H-tetrazole for the compound of I a), the title compound was prepared (1.36 g; 100%) as a white solid. ¹H NMR (300 MHz, d₆-DMSO) δ 8.16(s, 1H), 8.05(d, J =7.6Hz, 1H), 7.7(d, J = 6.6Hz, 1H), 7.67(t, J = 7.7Hz, 1H), 7.48(m, 2H), 7.2(d, J = 9.1Hz, 1H), 3.8(s, 3H), MS(ES) m/z 287 [M+H].

### b) 5-(5'-chloro-2-hydroxybiphenyl-3-yl)-1H-tetrazole:

Following the procedure of Example 3d), except substituting the compound of 12a) for the compounds of 3c), the title compound was prepared. MS(ES) m/z 250 [M+H].

### c) 5-(5'-chloro-2'-hydroxybiphenyl-3'-nitro-3-yl)-1H-tetrazole:

Following the procedure of Example 7c), except substituting the compound of 12b) for the compound of 7c), the title compound was prepared as yellow solid (0.5 g; 84%,). ¹H NMR (300 MHz, d₆-DMSO) δ 8.2(s, 1H), 8.1(d, J = 5.9Hz, 1H), 8.09(d, J = 7.4Hz, 1H), 7.8(d, J = 2.7Hz, 1H), 7.75(m, 2H), MS(ES) m/z 295 [M+H].

### d)3-{N'-[1-(3,4-dimethylphenyl)-3-methyl-5-oxo-1,5-dihydropyrazol-4-ylidene]hydrazino}-2-hydroxy-3'-(tetrazol-5-yl)biphenyl

Following the procedure of Example 1c), except substituting the compound of 12c) for the compound of I b), the crude product was isolated. A suspension of the crude product ( 0.0015mol.) in 1M aqu. hydrochloric acid; (25.0 mL) was cooled to 5°C then treated dropwise with a solution of sodium nitrite (0.11 g; 0.0015 mol.) in water (5.0 mL). The yellow mixture was stirred at 5°C for a further 10 min. then treated in one portion with the compound from Example 1d) (0.34 g, 0.0015 mol.) followed by the portion-wise addition of sodium hydrogen carbonate and ethanol ensuring the final pH of the reaction mixture is approximately 7-8. The red solution was then stirred at room temperature for 24h. The mixture was filtered to give a red solid which was slurried in water (50.0 mL) and then acid;ified with concentrated hydrochloric acid;. Filtration afforded the title compound (0.14 g; 20%) as a powder. ¹H NMR (300 MHz, d₆-DMSO) δ 13.7 (s, 1H), 9.8 (s, 1H), 8.26 (s, 1H), 8.1(d, J = 1.5Hz,I H), 7.75(m, 3H), 7.6(d, J = 2.2Hz, 1H), 7.2(m, 3H), 2.35(s,3H), 2.25(d, J = 2.2Hz, 6H).

### Example 13

### 3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;:

### a) 5'-Fluoro-2'-methoxybiphenyl-3-carboxylic acid;:

Following the procedure of Example 1b), except substituting 5-fluoro-2-methoxyphenylboronic acid; for 4-carboxyphenylboronic acid; , and substituting 3-bromophenyl carboxylic acid; for the compound of 1a), the title compound was prepared (1.59 g; 100%) as a white solid. ¹H NMR (300 MHz, d₆-DMSO) δ 8.26(s, 1H), 8.1(d, J = 8.1 Hz, 1H), 7.79(d, J = 8.1Hz, 1H), 7.10(dd, J =9.0, 3.4Hz, 1H), 7.05 (dd, J = 9.0, 3.4Hz 1H), 6.95 (dd, J = 9.0, 4.6Hz, 1H), 3.8(s, 3H).

### b) 5'-Fluoro-2'-hydroxybiphenyl-3-carboxylic acid;:

Following the procedure of Example 1b), except substituting the compound from Example 13a) for the compound from Example 1a), the title compound was prepared as white solid (1.0 g, 71%).¹H NMR (300 MHz, d₆-DMSO) δ 9.7(s, 1H), 8.19(s,1H), 7.91 (d, J = 6.9Hz, 1H), 7.81 (d, J = 6.9Hz, 1H), 7.59(t, J =8.0Hz, 1H), 7.19(dd, J = 9.2, 3.4Hz 1H), 6.9 (dd, J = 9.2, 4.6Hz, 1H).

### c) 5'-Fluoro-2'-hydroxy-3'-aminobiphenyl-3-carboxylic acid;:

Following the procedure of Example 7c), except substituting the compound from Example 13b) for the compound from Example 7b). The crude product was isolated, then dissolved in 10ml ethanol, treated with 5% Pd/C and shaked under 50 psi hydrogen for 1.5 hours. After concentrated, the title compound was prepared as dark gum (0.047 g, 36%, two steps). MS(ES) m/z 248 [M+H].

### d) 3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;:

Following the procedure of Example 1e), except substituting the compound from Example 13c) for the compound from Example 1c), the title compound was prepared as solid (0.03 g, 41%). ¹H NMR (300 MHz, d₆-DMSO) δ) 8.28(s, 1H), 7.91(m, 4H), 7.59(t, J = 8.0, 1H), 7.19(m, 2H), 6.9 (dd, J = 9.2, 3.4Hz, 1H), 2.35 (s, 1H), 2.2 (s, 3H), 2.18 (s, 3H). MS(ES) m/z 461 [M+H].

### Example 14

### 7-({N'-[1-(3,4-dimethylphenyl)-3-methyl-5-oxo-1,5-dihydropyrazol-4-ylidene]hydrazino}-2-hydroxyphenyl)quinolin-4[1H]-one-3-carboxylic acid;:

### a) 7-(5-chloro-2-hydroxyphenyl)-4-oxo-1,4-dihydroquinoline-3-carboxylic acid;:

Following the procedure of Example 1b), except substituting 2-methoxy-5-chlorophenylboronic acid; for 4-carboxyphenylboronic acid; , and substituting 7-bromo-4-oxo-1,4-dihydroquinoline-3-carboxylic acid; for the compound of 1a), the crude product was isolated. The above crude product was treated glacial acetic acid; (25.0 mL) and 48% aqu/ hydrobromic acid; (25.0 mL) was stirred and heated under reflux for 5h.

The mixture was cooled and filtered to afford the title compound (1.85 g; 73%). ¹H NMR (300 MHz, d₆-DMSO) δ 13.4(d. J = 6.5Hz, 1H), 10.2 (s, I H), 8.9 (d, J = 6.8Hz, 1H), 8.3 (d, J = 8.6Hz, 1H), 8.06 (s, 1H), 7.8 (dd, J = 8.5Hz, 1.5Hz, 1H), 7.45(d, J = 2.7Hz, 1H), 7.35 (dd, J = 7.7., 2.6 Hz, 1H), 7.05 (d, J = 8.7Hz, 1H).

### b) 7-(5-chloro-2-hydroxy-3-nitrophenyl)-4-oxo-1,4-dihydroquinoline-3-carboxylic acid;:

Following the procedure of Example 7c), except substituting the compound of 14a) for the compound of 7b), the title compound was prepared (0.84 g; 90%) as a powder. MS(ES) m/z 361 [M+H].

### c) 7-({N'-[1-(3,4-dimethylphenyl)-3-methyl-5-oxo-1,5-dihydropyrazol-4-ylidene]hydrazino}-2-hydroxyphenyl)quinolin-4[1H]-one-3-carboxylic acid;:

Following the procedure of Example 1c), except substituting the compound of 14b) for the compound of 1b), the crude product was isolated. Then follow the procedure of example 1e), except substituting the above crude product for the compound of 1c) and substituting 1-(3,4-dimethylphenyl)-3-methyl-3-pyrazolin-5-one for the compound of 1d), the title compound was prepared as solid (0.04 g; 8%,).¹H NMR (300 MHz, d₆-DMSO) δ 15.3 (s, 1H), 13.8(s, 1H), 13.4(s, 1H), 9.98(s, 1H), 8.92(d, J = 6.6Hz, 1H), 8.38(d, J = 8.5Hz, 1H), 7.99(s, 1H), 7.77(m, 4H), 7.15(m, 3H), 2.35(s, 3H), 2.23(s, 3H), 2.09(s, 3H). MS(ES) m/z 510 [M+H].

### Example 15

### 7-({N'-[1-(4-tert-butyl-phenyl)-3-methyl-5-oxo-1,5-dihydropyrazol-4-ylidene]hydrazino}-2-hydroxyphenyl)quinolin-4[1H]-one-3-carboxylic acid;:

Following the procedure of Example 14, except substituting the compounds of 4a) for the compound of 1d), the title compound was prepared (0.24 g, 40%) as solid. ¹H NMR (300 MHz, d₆-DMSO) δ 8.68(s, 1H), 8.43(d, J = 8.4Hz, 1H), 7.68(m, 5H), 7.48(d, J = 8.7Hz, 1H), 7.10(m, 2H), 2.4(s, 3H), 1.34(s, 9H).

### Example 16

### 3-Aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;:

Following the procedure of Example 7, except substituting the compounds of 1d) for the compound of 4a), the title compound was prepared (0.51 g, 78%) as solid. ¹H NMR (300 MHz, CDCl3) δ 9.16 (s, I H), 8.9 (s, 1H), 8.5 (s, I H), 7.7 (t, J = 4.8 Hz, 1H), 7.6 (s, 1H), 7.58 (d, J = 8.6 Hz, 1 H), 7.18 (d, J = 8. 1Hz, 1 H), 7.13 (d, J = 4.8Hz, 1H), 2.4 (s, 3H), 2.31 (s, 3H), 2.27 (s, 3H), MS(ES) m/z 444 [M+H].

### Example17

### 3-Aza-3'-(N'-[1{3-methyl-[4-(1-methylethyl)phenyl]-5-oxo-1,5-dihydropyrazol-4-ylidene}hydrazino)-2'-hydroxybiphenyl-5-carboxylic acid;:

### a) 1-(4-isopropylphenyl)-3-methyl-3-pyrazolin-5-one

Following the procedure of example 1d), except substituting 4-isopropylphenylhydrazine hydrochloride for 3,4-dimethylphenylhydrazine hydrochloride, the title compound was prepared (3.2 g; 89%). ¹H NMR (300 MHz, CDC13) δ 7.7 (d, J = 7.8 Hz, 2H), 7.20 (d, J = 7.8 Hz, 2H), 3.45 (s, 2H), 2.2 (s, 3H), 2.1 (s, 3H), MS(ES) m/z 217 [M+H] .

### b) 3-Aza-3'-(N'-[1-{3-methyl-[4-(1-methylethyl)phenyl]-5-oxo-1,5-dihydropyrazol-4-ylidene}hydrazino)-2'-hydroxybiphenyl-5-carboxylic acid;

Following the procedure of Example 7, except substituting the compounds of 17a) for the compound of 4a), the title compound was prepared (0.16 g, 23%) as solid. ¹H NMR (300 MHz, CDCl3) δ 13.7(br s, 1H), 9.9 (s, 1H), 9.0 (s, 1H), 8.9 (s, 1H), 8.4 (s, 1H), 7.83 (d, J = 7.6 Hz, 2H), 7.78 (d, J = 7.8Hz, 1H), 7.33 (d, J = 8.6Hz, 1H), 7.2 (m. 2H),2.35 (s, 3H), 1.2 (d, J = 6.7Hz, 6H), MS(ES) m/z 458 [M+H].

### Example 18

### 3-Aza-3'-{N'-[1-(3-tertbutylphenyl-3-methyl-5-oxo-1,5-dihydropyrazol-4-ylidene]hydrazino}-2'-hydroxybiphenyl-5-carboxylic acid;:

### a) 1-(3-tert-Butylphenyl)-3-methyl-3-pyrazolin-5-one

Following the procedure of example 1d), except substituting 3-*tert*-butylphenylhydrazine hydrochloride for 3,4-dimethylphenylhydrazine hydrochloride, the title compound was prepared (0.8 g; 70%). ¹H NMR (300 MHz, CDC13) δ 7.9 (s, 1H), 7.68 (d, J = 2.6Hz, 1H), 7.24 (m, 3H), 3.4 (s, 2H), 2.2 (s, 3H), 1.33 (s, 9H).

### b)3-Aza-3'-{N'-[1-(3-tert-butylphenyl-3-methyl-5-oxo-1,5-dihydropyrazol-4-ylidene]hydrazino}-2'-hydroxybiphenyl-5-carboxylic acid;:

Following the procedure of Example 7, except substituting the compounds of 18a) for the compound of 4a), the title compound was prepared (0.2 g, 25%) as solid. ¹H NMR (300 MHz, CDCl3) δ 13.7(brs, 1H), 9.9 (s,1H), 9.0 (s,1H), 8.9 (s,1H), 8.4 (s,1H), 8.0 (s,1H), 7.79 (dd, J = 8.5Hz, 1.9Hz, 2H), 7.4 (t, J = 7.9Hz, 1H), 7.28 (m, 3H), 2.35 (s, 3H), 1.3 (s, 9H), MS(ES) m/z 472 [M+H].

### Example 19

### 5'-Chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;

### a) 5'-chloro-2'-methoxybiphenyl)-3-carboxylic acid;:

Following the procedure of Example 1b), except substituting 2-methoxy-5-chlorophenylboronic acid; for 4-carboxyphenylboronic acid; , and substituting 3-bromocarboxylic acid; for the compound of 1a), the title compound was prepared (2.82 g; 75%) as a white powder.¹H NMR (300 MHz, CDC13) δ 8.21 (s, 1H), 8.09 (d, J = 8.0Hz, 1H), 7.78 (d, J = 8.0Hz, 1H), 7.53 (t, J = 8.0Hz, 1H), 7.34 (s, 1H), 7.32(dd, J = 9.0Hz, 4.0Hz, 1H), 6.93(d, J = 9.0Hz, 1H), 3.87 (s, 3H).

### b) 5'-chloro-2'-hydroxyphenyl-3-carboxylic acid;:

Following the procedure of Example 3d), except substituting the compound of 20a) for the compounds of 3c), the title compound was prepared (2.33g; 90%) as solid. ¹H NMR (300 MHz, d₆-DMSO) δ 10.19 (s, 1H), 8.15 (s, 1H), 7.92 (d, J = 8.0Hz, 1H), 7.79 (d, J = 8.0Hz, 1H), 7.55 (t, J = 8.0Hz, 1H), 7.34 (s, 1H), 7.25(dd, J = 9.0Hz, 3.4Hz, 1H), 6.93(d, J =9.0Hz,1H).

### c) 5'-chloro-2'-hydroxy-3'-nitrophenyl-3-carboxylic acid;:

Following the procedure of example 7c), except substituting the compound of 20b) for the compound of 7b), the title compound was prepared as yellow powder (0.4 g, 68%). ¹H NMR (300 MHz, d₆-DMSO) δ 10.8 (s, I H), 8.14 (s, I H), 8.1 (d, J = 3.4Hz, 1H), 7.99 (d, J = 7.5Hz, 1H), 7.79 (m, 2H), 7.63 (t, J = 8.1Hz, 1H).

### d) 5'-Chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;:

Following the procedure of example 8, except substituting the compound of example 20c) for the compound of example 6c), the title compound was prepared as solid. ¹H NMR (300 MHz, d₆-DMSO) δ 10.0(s, 1H), 8.14 (s, 1H), 8.0 (d, J = 8.0Hz, 1H), 7.84 (d, J = 8.0Hz, I H), 7.64 (m, 4H), 7.22 (d, J = 8.1Hz, 1H), 7.2 (s, 1H), 2.33 (s,1H), 2.28 (s,1H), 2.25 (s, 1H), MS(ES) m/z 477 [M+H].

### Example 20

### Preparation of 3'-{N'-[1-(3,4-Dimethylphenyl)-3,5-dioxo-1,5-dihydropyrazol-4-ylidene]hydrazino}-2'-hydroxybiphenyl-3-carboxylic acid;

A suspension of 3-amino-2-hydroxybiphenyl-3'-carboxylic acid;, hydrochloride salt (0.057 g; 0.0002 mol.) in 1M aqu. hydrochloric acid; (3.0 mL) was cooled to 5°C then treated dropwise with a solution of sodium nitrite (0.017 g; 0.00024 mol.) in water (2.0 mL). The yellow mixture was stirred at 5°C for a further 10 min. then treated with 1-(3,4-dimethylphenyl) pyrazolidine-3,5-dione (44 mg; 0.0002 mol.), followed by the portionwise addition of sodium hydrogen carbonate solution ensuring the final pH of the reaction mixture is approximately 7-8, and ethanol (3.0 mL) to improve solubility. The red solution was then stirred at room temperature for 24h.

The mixture was diluted with water, acid;ified with 1M aq. hydrochloric acid;, stirred for a further 30 minutes and filtered to give a brown solid which was washed with water to afford the title compound (0.04 g; 42%) as a brown powder, MS m/e 445 [M+H]⁺.

### Example 21

### Preparation of 3'-{N'-[1-(2-Ethoxy-2-oxoethyl)-3-methyl-5-oxo-1,5-dihydropyrazol-4-ylidene]hydrazino}-2'-hydroxybiphenyl-3-carboxylic acid;

### a) Ethyl 3-methyl-3-pyrazolin-5-one-1-acetate

Following the procedure of Example 1d), except using ethyl hydrazino-acetate hydrochloride in place of 3,4-dimethylphenylhydrazine, the title compound was prepared (1.12 g; 20%) as a pale yellow solid, ¹H NMR (300 MHz, d₆-DMSO) δ 10.90 (s, 1H), 5.15 (s, 1H), 4.58 (s, 2H), 4.13 (q, J = 7.1 Hz, 2H), 2.01 (s, 3H), 1.20 (t, J = 7.1 Hz, 3H).

### b) 3'-{N'-[1-(2-Ethoxy-2-oxoethyl)-3-methyl-5-oxo-1,5-dihydropyrazol-4-ylidene]hydrazino}-2'-hydroxybiphenyl-3-carboxylic acid;

0.23M aq. sodium nitrite (1 mL, 0.23 mmol) was added dropwise to a stirred suspension of 3'-amino-2'-hydroxybiphenyl-3-carboxylic acid;, hydrochloride salt (0.050 g, 0.188 mmol) in 1M aq. hydrochloric acid; (2.5 mL) at 5°C. The mixture was stirred 10 min. and a solution of ethyl 3-methyl-3-pyrazolin-5-one-1-acetate (0.035 g, 0.188 mmol) in ethanol (3 mL) added. Sodium hydrogen carbonate was added until the pH was 8, and the reaction was allowed to warm to room temperature, then stirred for 18h, and poured into aq. acetic acid; (0.5 M, 50 mL). The mixture was extracted (2 x 10 mL ethyl acetate), and the extracts washed with water (2 x 5 mL), brine (5 mL) and dried (MgSO₄). The solvent was removed under reduced pressure and the residue purified by preparative hplc (ODS-silica, 10-90% acetonitrile/water-0. 1%TFA) to afford the title compound (0.024 g; 30%) as an orange solid. MS(ES) m/z 425 (M+H)⁺.

### Example 22

### 3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl

Following the procedure of Example 25d), the title compound was prepared as solid (0.08g, 10%) after purified by preparative hplc (ODS-silica, 10-90% acetonitrile/water-0.1 %TFA) ¹H NMR (300 MHz, d₆-DMSO) δ 13.7(br s, 1H), 9.7 (s, 1H), 8.15(d, J = 8.3Hz, 2H), 7.84(d, J = 8.3Hz, 2H), 7.73(m, 2H), 7.64(dd, J = 2.3Hz, 8.2Hz, 1H), 7.24(m, 3H), 2.35(s, 3H), 2.27(s, 3H), 2.23(s, 3H). MS(ES) m/z 467 [M+H].

### Example 23

### Preparation of 3'-(N'-{1-[2-(N-tert-butyl)amino-2-oxoethyl]-3-methyl-5-oxo-1,5-dihydropyrazol-4-ylidene}hydrazino)-2'-hydroxybiphenyl-3-carboxylic acid;

### a) 3-Methyl-N-tert-butyl-3-pyrazolin-5-one-1-acetamide

A solution of trimethylaluminium in toluene (2M, 1 mL, 2.00 mmol) was injected into an ice-cooled, stirred solution of tert-butylamine (0.210 mL, 2.00 mmol) in dichloromethane (3 mL) under argon. After 5 min., ethyl 3-methyl-3-pyrazolin-5-one-1-acetate (0.092 g, 0.500 mmol) was added in one portion and the mixture stirred at room temperature for 24h. Water (0.67 mL, 37.2 mmol) was added dropwise and stirring continued until amorphous solid had separated. Ethyl acetate (5 mL) was added, followed by excess sodium hydrogen carbonate, and the mixture stirred for 15 min., then filtered through a plug of Celite®. The cake was washed repeatedly with ethyl acetate and the solvent removed from the filtrate under reduced pressure to give the title compound (0.106 g; 100%) as an amorphous solid suitable for use in the next step. ¹H NMR (300 MHz, d₆-DMSO) δ 7.52 (s, 1H), 4.95 (s, 1H), 4.21 (s, 2H), 1.98 (s, 3H), 1.24 (s, 9H).

### b) 3'-(N'-{1-[2-(N-tert-butyl)amino-2-oxoethyl]-3-methyl-5-oxo-1,5-dihydropyrazol-4-ylidene}hydrazino)-2'-hydroxybiphenyl-3-carboxylic acid;

Following the procedure of Example 21b), except using 3-methyl-N-tert-butyl-3-pyrazolin-5-one-1-acetamide in place of ethyl 3-methyl-3-pyrazolin-5-one-1-acetate, the title compound was prepared (0.05 g; 29%) as an orange solid. MS(ES) m/z 452 (M+H)⁺.

### Example 24

### Preparation of 3'-{N'-[3-Chloro-1-(3,4-dimethylphenyl)-5-oxo-1,5-dihydropyrazol-4-ylidene]hydrazino}-2'-hydroxybiphenyl-3-carboxylic acid;

### a) 2-Acetyl(3,4-dimethylphenyl)hydrazine

Prepared according to the method of E.C. Taylor and J. S. Hinkle, J. Org. Chem., 1987, 52(18), 4107.

### b) 1-(3,4-Dimethylphenyl) pyrazolidine-3,5-dione

Prepared according to Chemical Abstracts 1964, 60, 15880 (M Yokoyama, T Kurihara and T Takahashi, Japan 2872 (1964).

### c) 1-(3,4-Dimethylphenyl)-5-chloro-3-oxo-pyrazolidine

Prepared according to Chemical Abstracts 1964, 60, 15880 (M Yokoyama, T Kurihara and T Takahashi, Japan 2872 (1964).

The two products, 1-(3,4-dimethylphenyl)-5-chloro-3-oxo-pyrazolidine and 1-(3,4-dimethylphenyl)-3,5-dichloropyrazolidine were separated by flash chromatography (silica gel, dichloromethane-hexane to 1% methanol in dichloromethane). MS m/e 241 [M+H]⁺ (dichloro derivative) and MS m/e 223 [M+H]⁺ (monochloro derivative).

### d)3'-{N'-[3-Chloro-1-(3,4-dimethylphenyl)-5-oxo-1,5-dihydropyrazol-4-ylidene]hydrazino}-2'-hydroxybiphenyl-3-carboxylic acid;

A suspension of 3-amino-2-hydroxybiphenyl-3'-carboxylic acid;, hydrochloride salt (0.2 g; 0.00076 mol.) in 1M aqu. hydrochloric acid; (3.0 mL) and tetrahydrofuran (3.0 mL) was cooled to 5°C then treated dropwise with a solution of sodium nitrite (0.057 g; 0.00083 mol.) in water (1.0 mL). The yellow mixture was stirred at 5°C for a further 10 min. then treated with a solution of 1-(3,4-dimethylphenyl)-5-chloro-3-oxo-pyrazolidine (0.168 g; 0.00076 mol.) in tetrahydrofuran (3.0 mL), followed by the portionwise addition of sodium hydrogen carbonate solution ensuring the final pH of the reaction mixture is approximately 7-8. The red solution was then stirred at room temperature for 24h.

The mixture was diluted with water and acid;ified with 1M aq. hydrochloric acid;, stirred for a further 30 minutes and filtered to give a brown solid which was washed with water to afford the title compound (0.3 g; 85 %) as a brown powder, mp = 210°C (dec.). MS m/e 463 [M+H]⁺.

### Example 25

### 5-chloro-3-{N'-[{1-(3,4-dimethyphenyl)-3-methyl-5-oxo-1,5-dihydropyrazol-4-ylidene]hydrazino}-2-hydroxy-4'-(tetrazol-5-yl)biphenyl:

### a) 5-(5'-chloro-2-methoxybiphenyl-4-yl)-1H-tetrazole:

Following the procedure of Example 1b), except substituting 2-methoxy-5-chlorophenylboronic acid; for 4-carboxyphenylboronic acid; , and substituting 5-(4-bromophenyl)-1H-tetrazole for the compound of I a), the title compound was prepared (1.4 g; 100%) as a solid. ¹H NMR (300 MHz, d₆-DMSO) δ 8.1(d, J = 8.5Hz, 2H), 7.72(d, J = 8.5Hz, 2H), 7.4(m, 2H), 7.15 (d, J = 9.0Hz, 1H), 3.8(s, 3H).

### b) 5-(5'-chloro-2-hydroxybiphenyl-4-yl)-1H-tetrazole:

Following the procedure of Example 3d), except substituting the compound of 29a) for the compounds of 3c), the title compound was prepared. ¹H NMR (300 MHz, d₆-DMSO) δ 10.1(s,1H), 8.1(d, J = 6.6Hz, 2H), 7.8(d, J = 6.6Hz, 2H), 7.38(d, J = 2.7Hz, 1H), 7.2 (dd, J = 2.7Hz, 8.7Hz, 1H).

### c) 5-(5'-chloro-2'-hydroxybiphenyl-3'-nitro-3-yl)-1 H-tetrazole:

Following the procedure of Example 1a), except substituting the compound of 29b) for 3-bromophenol, the title compound was prepared as solid (0.95 g; 86%,).¹H NMR (300 MHz, d₆-DMSO) δ 8.12(m, 3H), 7.8(m, 3H).

### d) 5-chloro-3-{N'-[1-(3,4-dimethylphenyl)-3-methyl-5-oxo-1,5-dihydropyrazol-4-ylidene]hydrazino[-2-hydroxy-4'-(tetrazol-5-yl)biphenyl:

Following the procedure of example 1c), except substituting the compound of 29c) for the compound of example 1b). A mixture product (1:1) of 5-(5'-chloro-2'-hydroxybiphenyl-3'-amino-3-yl)-1H-tetrazole and 5-(2'-hydroxybiphenyl-3'-amino-3-yl)-1H-tetrazole was abtained. MS m/e 254 and 288 [M+H]⁺

Following the procedure of Example 1e), except substituting the above mixture compounds for the compound of 1c), the title compound was prepared as solid (0.12 g, 14%) after purified by preparative hplc (ODS-silica, 10-90% acetonitrile/water-0.1%TFA) ¹H NMR (300 MHz, d₆-DMSO) δ 13.6(s, 1H), 8.15(d, J = 8.2Hz, 2H), 7.84(d, J = 8.2Hz, 2H), 7.69(s, 1H), 7.64(d, J = 8.2Hz, 1H), 7.24(d, J = 2.4Hz, 1H), 7.23(d, J = 8.3Hz, 1H), 2.35(s, 3H), 2.27(s, 3H), 2.23(s, 3H). MS(ES) m/z 501 [M+H].

### Example 26

### 3'-{N'-[1-(3,4-Dimethylphenyl)-3-methyl-5-oxo-1,5-dihydropyrazol-4-ylidene]hydrazino}-2'-hydroxybiphenyl-3,5-dicarboxylic acid;:

### a) 5'-Chloro-2'-hydroxy-3'-nitrobiphenyl-3,5-dicarboxylic acid;:

Following the procedure of Example 1b), except substituting 2-methoxy-5-chlorophenylboronic acid; for 4-carboxyphenylboronic acid; , and substituting 5-bromo-isophthalic acid; for the compound of 1a), the crude product was isolated and treated with glacial acetic acid; (25.0 mL) and 48% aqueous hydrobromic acid; (25.0 mL) was stirred and heated under reflux for 5h. The mixture was cooled and filtered to afford the crude compound as a powder. Then, following the procedure of Example 1a), except substituting the above crude compound for 3-bromophenol, the title compound was prepared as solid (0.58 g; 33%, three steps) as a solid. ¹H NMR (300 MHz, d₆-DMSO) δ 8.5(t, J = 1.6Hz, 1H), 8.33(d, J = 1.6Hz,1H), 8.2(d, J = 1.6Hz, 2H), 8.1(d, J = 2.7Hz, 1H), 7.8(d, J = 2.7Hz, 1H).

### b) 3'-{N'-[1-(3,4-Dimethylphenyl)-3-methyl-5-oxo-1,5-dihydropyrazol-4-ylidene]hydrazino}-2'-hydroxybiphenyl-3,5-dicarboxylic acid;:

Following the procedure of Example 12d), except substituting the compound of 30c) for the compound of 12c), the title compound was prepared as solid. ¹H NMR (300 MHz, d₆-DMSO) δ 8.5(s, 1H), 8.33(s, 2H), 7.76(m, 2H), 7.63(d, J = 8.0Hz, 1H), 7.24(m, 3H), 2.35(s, 3H), 2.27(s, 3H), 2.23(s, 3H). MS(ES) m/z 487 [M+H].

### Example 27

### 3-Aza-3'-{N'-{1-(3,4-dimethylphenyl)-3-methyl-5-oxo-1,5-dihydropyrazol-4-ylidene]hydrazino}-2'-hydroxy-5'-methylbiphenyl-5-carboxylic acid;:

### a) 5-methyl-2-methoxyphenylboronic acid;:

To the THF solution of 2-bromo-1-methoxy-4-methybenzene(1.39 g, 15mmol) at - 78°C under nitrogen was added n-butyllithium (15mmol) dropwise. After stiring at -78°C for one hour, the solution was added to trimethylborate (15mmol) in THF (100 mL). The reaction mixture was warmed to room temperature and continuously stirred for four hours before poured into 3N hydrochloride (125 mL), then extracted with ethyl acetate three time. The organics were combined, dried over magnesium and concentrated to give yellow-white solid(2.2 g, 89%). ¹H NMR (300 MHz, d₆-DMSO) δ 7.6(s, 1H), 7.38(d, J = 2.3Hz, 1H), 7.19(dd, J = 8.4Hz, 2.3Hz, 1H), 6.87(d, J = 8.4Hz, 1H), 3.78(s, 3H), 2.2(s, 3H).

### b) 5-(2-Methoxy-5-methylphenyl)-nicotinic acid;:

Following the procedure of Example 7a), except substituting the compound of 31a) for 4-carboxyphenylboronic acid;, the title compound was prepared as solid (2.88 g, 90%). ¹H NMR (300 MHz, d₆-DMSO) δ 9.0(s, 1H), 8.8(s, I H), 8.3(t, J = 2.0Hz, 1H), 7.2(m,, 2H), 7.08(d, J = 9.0Hz, 1H), 3.77(s, 3H), 2.31(s, 3H).

### c) 5-(2-Hydroxy-5-methylphenyl)-nicotinic acid;:

Following the procedure of Example 3d), except substituting the compound of 31b) for the compounds of 3c), the title compound was prepared. ¹H NMR (300 MHz, d₆-DMSO) δ 8.98(s, I H), 8.93(s, 1H), 8.4(t, J = 2.1Hz, 1H), 7.2(s,, 1H), 7.0(d, J = 8.5Hz, 1H), 6.9(d, J = 8.9Hz, 1H), 2.31(s, 3H).

### d) 5-(2-Methoxy-5-methyl-3-nitrophenyl)-nicotinic acid;:

Following the procedure of Example 7c), except substituting the compound of 31c) for the compound of 7c), the title compound was prepared (0.7 g; 31 %). ¹H NMR (300 MHz, d₆-DMSO) δ 9.0(s, 1H), 8.94(s, 1H), 8.4(t, J = 2.1Hz, 1H), 7.9(s,, 1H), 7.68(s, 1H), 2.36(s, 3H).

### e) 3-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-5-carboxylic acid;:

Following the procedure of Example 7d), except substituting the compound of 31d) for 6-(5-chloro-2-hydroxy-3-nitrophenyl)-pyridine-2-carboxylic acid; and the compound of 1d) for the compound of 4a), the title compound was prepared as solid(0.66 g, 90%). ¹H NMR (300 MHz, CDCl3) δ 9.14(s, 1H), 8.9(s, I H), 8.6(s, I H), 7.61(d, J = 9.2Hz, 1H), 7,58(d, J = 7.3Hz, I H), 7.17(d, J = 8.1 Hz, 1H), 6.9(s, I H), 2.31(s, 3H), 2.28(s, 3H), 2.20(s, 3H).

### Example 28

### 3'-{N'-[1-(3,4-Dimethylphenyl)-3-methyl-5-oxo-1,5-dihydropyrazol-4-ylidenelhydrazino}-2'-hydroxybiphenyl-4-carboxylic acid;:

### a) 5'-Chloro-2'-hydroxy-3'-nitrobiphenyl-4-carboxylic acid;:

Following the procedure of Example 7a), 7b), and 7c), except substituting 4-bromophenylcarboxylic acid; for 5-bromonicotinic acid;, the title compound was prepared as solid (1.0 g, 77%, three steps). ¹H NMR (300 MHz, d₆-DMSO) δ 8.1(d, J = 2.7Hz, 1H), 8.0(d, J = 6.6Hz, 1H), 7.78(d, J = 2.7Hz, 1H), 7.67(d, J = 6.6Hz, 1H).

### b) 3'-{N'-[1-(3,4-Dimethylphenyl)-3-methyl-5-oxo-1,5-dihydropyrazol-4-ylidene]hydrazino}-2'-hydroxybiphenyl-4-carboxylic acid;:

Following the procedure of Example 7d), except substituting the compound of 32a) for 6-(5-chloro-2-hydroxy-3-nitrophenyl)-pyridine-2-carboxylic acid; and the compound of 1d) for the compound of 4a), the title compound was prepared as orange solid(0.23 g, 24%). ¹H NMR (300 MHz, d₆-DMSO) δ 13.7(s, 1H), 12.9(s, 1H), 9.7(s, 1H), 8.06(d, J = 8.4Hz, 1H), 7.75(d, J = 3.0Hz, 1H), 7.69(m, 3H), 7.65(d, J = 8.1Hz, 1H), 7.18(m, 3H), 2.33(s, 3H), 2.27(s, 3H), 2.23(s, 3H). MS(ES) m/z 442 [M+H].

### Example 29

### Preparation of 3'-{N'-[1-3,4-Dimethylphenyl)-3-methoxy-5-oxo-1,5-dihydropyrazol-4-ylidene]hydrazino}-2'-hydroxybiphenyl-3-carboxylic acid;:

### a) 1-(3,4-Dimethylphenyl)-3-methoxy-3-pyrazolin-5-one

A solution of 1-(3,4-dimethylphenyl)pyrazolidine-3,5-dione (0.675 g; 0.0033 mol.) in methanol (40.0 mL) was treated with conc. sulfuric acid; (10 drops) and stirred and heated under reflux for 24h.

Water (20.0 mL) was added and the mixture evaporated. The resudue was triturated with water to afford the title compound (0.428 g; 60%) as an orange powder. ¹H NMR (300 MHz, d₆-DMSO) revealed an approximate 1:1 mixture of hydroxypyrazole:pyrazolone tautomers. MS(ES) m/z 219 [M+H].

### b) 3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;

Following the procedure of Example 1e), except substituting the compound from Example 3e) for the compound from Example 1c) and the compound from Example 29a) for the compound from Example 1d), the title compound was prepared (0.142 g; 41 %) as an orange solid. ¹H NMR (300 MHz, d₆-DMSO) δ 13.7 (s, I H), 13.1(br s, I H), 9.71 (s, 1H), 8.14 (s, 1H), 7.97 (dd, J = 7.8, 1.4 Hz, 1H), 7.81 (d, J = 8.1 Hz, 1H), 7.72 (s, 1H), 7.65-7.60 (m, 3H), 7.22-7.11 (m, 3H), 4.06 (s, 3H), 2.27 (s, 3H), 2.23 (s, 3H); Anal. (C₂₅H₂₂N₄O₅.0.66 H₂O) calcd: C, 63.82; H, 5.00; N, 11.91. found: C, 63.53; H, 4.95; N, 11.49.

### Example 30

### Preparation of 3'-{N'-[1-(4-methoxyphenyl)-3-methyl-5-oxo-1,5-dihydropyrazol-4-ylidene]hydrazino}-2'-hydroxybiphenyl-3-carboxylic acid;

### a) 1-(4-Methoxyphenyl)-3-methyl-3-pyrazolin-5-one

Following the procedure of Example 1d), except substituting 4-methoxyphenylhydrazine hydrochloride for 3,4-dimethylphenylhydrazine hydrochloride, the title compound was prepared (2.2 g; 27%) as a cream powder. ¹H NMR (300 MHz, d₆-DMSO) δ 11.3 (s, 1H), 7.57 (d, J = 9.0 Hz, 2H), 6.98 (d, J = 9.0 Hz, 2H), 5.32 (br s, 1H), 3.77 (s, 3H), 2.10 (s, 3H).

### b) 3'-{N'-[1-(4-methoxyphenyl)-3-methyl-5-oxo-1,5-dihydropyrazol-4-ylidene]hydrazino}-2'-hydroxybiphenyl-3-carboxylic acid;

Following the procedure of Example 1e), except substituting the compound from Example 3e) for the compound from Example 1c) and the compound from Example 30a) for the compound from Example 1d) and washing the crude product with chloroform, the title compound was prepared (0.146 g; 49%) as an orange solid. ¹H NMR (300 MHz, d₆-DMSO) δ 13.7 (s, 1H), 13.1 (br s, 1H), 9.70 (s, 1H), 8.14 4 (t, J = 1.4 Hz, 1H), 7.97 (dt, J = 7.9, 1.4 Hz, 1H), 7.84-7.60 (m, 5H), 7.19-7.15 (m, 2H), 7.04 (d, J = 9.1 Hz, 2H), 3.79 (s, 3H), 2.34 (s, 3H); Anal. (C₂₄H₂₀N₄O₅.0.5 H₂O; 0.5 CHCl₃) calcd: C, 57.35; H, 4.22; N, 10.91. found: C, 57.69; H, 4.35; N, 10.80.

### Example 31

### 3-{N'-[1-(3,4-Dimethylphenyl)-3-methyl-5-oxo-1,5-dihydropyrazol-4-ylidene]hydrazino}-2-hydroxy-3'-trifluoromethanesulfonamidobiphenyl

### a) N-(2'-methoxy-3'-nitrobiphenyl-3-yl)-acetamide:

Following the procedure of example 1b), except substituting 3-acetamidobenzeneboronic acid; for the compound of 4-carboxyphenylboronic acid; and 1-bromo-2-methoxyl-3-nitrobenzene for the compound of example 1a), the title compound was prepared as solid. (1.16 g, 98%).¹H NMR (300 MHz, d₆-DMSO) δ7.73 (dd, J = 1.7 Hz, 7.5 Hz, 1H), 7.68 (s, 1H), 7.61 (d, J = 8.6 Hz, 1H), 7.55 (dd, J = 1.7Hz, 7.5Hz, 1H), 7.41 (t, J = 8.0Hz, 1H), 7.32(d, J = 8.6Hz, I H), 7.25(t, J = 8.0Hz, 1H), 3.52 (s, 3H), 2.10 (s, 3H).

### b) 3'-amino-3-nitrobiphenyl-2-ol hydrobromide salt:

Following the procedure of example 3d), except substituting the compound of 31a) for the compound of 3c), the title compound was prepared as solid (0.7 g, 56%). MS(ES) m/z 231 [M+H].

### c) 1,1,1-trifluoro-N-(2'-hydroxy-3'-nitrobiphenyl-3-yl)-methanesulfonamide:

The compound of example 31a) in ethyl acetate was added 5% sodium hydroxy solution and stirred for 10 min., then neutralized the aqueous solution with 3N HCl to pH=7. and extracted with ethyl acetate. After concentration, 0.25g neutral compound was obtained.

The neutral compound (0.25 g) was dissolved in Chloroform (25 mL), treated with triflic anhydride at 0°C and stirred at room temperature for 1 hour. 3N hydrochloride was addded to adjust pH to 4 and the mixture was extrated with ethyl acetate. The organic layers were combined, dried (MgSO₄) and concentrated to give yellow solid (0.39 g, 100%). MS(ES) m/z 340 [M+H].

### d) 3-{N'-[1-(3,4-Dimethylphenyl)-3-methyl-5-oxo-1,5-dihydropyrazol-4-ylidene]hydrazino-2-hydroxy-3'-trifluoromethanesulfonamidobiphenyl

Following the procedure of example 7d), except substituting the compound of example 31b) for the compound of example 7c) and 1-(3,4-dimethylphenyl)-3-methyl-3-pyrazolin-5-one for 1-(4-*tert*-butylphenyl)-3-methyl-3-pyrazolin-5-one, the title compound was prepared as solid (34%). MS(ES) m/z 546 [M+H].

### Example 32

### Preparation of 3'-{N'-[1-(3,4-Dichlorophenyl)-3-methyl-5-oxo-1,5-dihydropyrazol-4-ylidene]hydrazino}-2'-hydroxybiphenyl-3-carboxylic acid;

### a) 1-(3,4-Dichlorophenyl)-3-methyl-3-pyrazolin-5-one

Following the procedure of Example 1d), except substituting 3,4-dichlorophenylhydrazine hydrochloride for 3,4-dimethylphenylhydrazine hydrochloride, the title compound was prepared (7.5 g; 65%) as a cream powder. ¹H NMR (300 MHz, d₆-DMSO) δ 8.02 (d, J = 2.4 Hz, 1H), 7.8 (dd, J = 8.9, 2.5 Hz, 1H), 7.7 (d, J = 8.9, 1H), 5.31 (s, 1H), 2.1 (s, 3H).

### b) 3'-{N'-[1-(3,4-Dichlorophenyl)-3-methyl-5-oxo-1,5-dihydropyrazol-4-ylidene]hydrazino}-2'-hydroxybiphenyl-3-carboxylic acid;

Following the procedure of Example 1e), except substituting the compound from Example 3e) for the compound from Example 1c) and the compound from Example 37a) for the compound from Example 1d) and washing the crude product with chloroform, the title compound was prepared (0.253 g; 78%) as an orange solid. ¹H NMR (300 MHz, d₆-DMSO) δ 13.6 (s, 1H), 13.1 (s, 1H), 9.76 (s, 1H), 8.13 (m, 2H), 7.97 (d, J = 7.5 Hz, 1H), 7.90 (d, J = 8.9 Hz, 1H), 7.80 (d, J = 7.5 Hz, 1H), 7.68-7.60 (m, 3H), 7.17-7.09 (m, 2H), 2.31 (s, 3H); Anal. (C₂₃H₁₆Cl₂N₄O₄.0.5 H₂O) calcd: C, 56.11; H, 3.48; N, 11.38. found: C, 56.14; H, 3.47; N, 11.24.

### Example 33

### Preparation of 3'-{N'-[3-methyl-5-oxo-1-(3-trifluoromethylphenyl)-1,5-dihydropyrazol-4-ylidene]hydrazinol-2'-hydroxybiphenyl-3-carboxylic acid;

### a) 1-(3-trifluoromethylphenyl)-3-methyl-3-pyrazolin-5-one:

Following the procedure of Example 1d), except substituting 3-trifuoromethylphenylhydrazine hydrochloride for 3,4-dimethylphenylhydrazine hydrochloride, the title compound was prepared (5.1 g; 91 %). ¹H NMR (300 MHz, CDCl₃) δ 8.17 (s, 1H), 8.1(d, J = 7.3 Hz, 1H), 7.5 (t, J = 7.7, 1H), 7.45 (d. J = 7.7, 1H), 3.47 (s, 2H), 2.2 (s, 3H).

### b) 3'-{N'-[3-methyl-5-oxo-1-(3-trifluoromethylphenyl)-1,5-dihydropyrazol-4-ylidene}hydrazino}-2'-hydroxybiphenyl-3-carboxylic acid;

Following the procedure of Example 1e), except substituting the compound from Example 3e) for the compound from Example 1c) and the compound from Example 38a) for the compound from Example 1d) and washing the crude product with chloroform, the title compound was prepared (0.241 g; 66%) as an orange solid. MS(ES) m/z 483 (M+H)⁺.

### Example 34

### 8-{N'-[1-(3,4-dimethylphenyl)-3-methyl-5-oxo-1,5-dihydropyrazol-4-ylidene]hydrazino}quinolin-4[1H]-one-3-carboxylic acid;:

### a) 8-Nitro-4-oxo-1,4-dihydroquinoline-3-carboxylic acid;:

To the suspension of 2-nitroaniline (41.4 g, 0.3 mol) in 200ml diphenyl ether was added 2-ethoxymethylenemalonic acid; diethyl ether (64.8 g, 0.3 mol). The reaction mixture was heated to reflux for half an hour, cooled down to 70°C and poured in hexanes (600 mL). The resulting solid was collected and washed with hexanes to give crude product. The crude compound (49 g, 0.19 mol) was dissolved in 10% NaOH (1 L) and refluxed for two hours. After cooling to 60°C, the reaction mixture was quenched with 6N hydrochloride, the resulting precipitation was collected and washed with water to give the title compound as tan solid (22.6 g, 47%). ¹H NMR (300 MHz, d₆-DMSO) δ 12.9 (s, 1H), 8.84 (dd, J = 8.0Hz, 1.5Hz, 1H), 8.83 (t, J = 7.9Hz, 1H), 7.79 (dd, J = 8.0Hz, 1.5Hz, 1H).

### b) 8-Amino-4-oxo-1,4-dihydroquinoline-3-carboxylic acid;:

Following the procedure of example 1c), except substituting the compound from example 34a) for the compound for example 1b), the title compound was prepared as solid (1g, 51%).¹H NMR (300 MHz, d₆-DMSO) δ 12.7 (s, 1H), 8.7 (s, 1H), 7.54 (d, J = 7.0 Hz, 1H), 7.33 (t. J = 7.9Hz, 1H), 7.12(d, J = 6.6Hz, 1H).

### c)8-{N'-[1-(3,4-dimethylphenyl)-3-methyl-5-oxo-1,5-dihydropyrazol-4-ylidene]hydrazino}quinolin-4[1H]-one-3-carboxylic acid;:

Following the procedure of example 1e), except substituting the compound from example 34b) for the compound from example 1c), the title compound was prepared as solid (0.4 g, 83%). ¹H NMR (300 MHz, d₆-DMSO) δ 8.31 (d, J = 8.2Hz, 1H), 8.2 (d, J = 7.4 Hz, 1H), 8.09 (d, J = 14.9 Hz, 1H), 7.82 (d, J = 14.9Hz, 1H), 7.6(d, J = 8.0Hz, 1H), 7.56 (s, 1H), 7.42(d, J = 8.2Hz, 1H), 7.25(d, J = 8.3Hz), 2.46(s, 3H), 2.28(s, 3H), 2.25(s, 2.25).

### Example 35

### Preparation of 3'-{N'-[3-methyl-5-oxo-1-(4-trifluoromethylphenyl)-1,5-dihydropyrazol-4-ylidene]hydrazino}-2'-hydroxybiphenyl-3-carboxylic acid;

### a) 1-(4-trifluoromethylphenyl)-3-methyl-3-pyrazolin-5-one

Following the procedure of Example 1d), except substituting 4-trifuoromethylphenylhydrazine hydrochloride for 3,4-dimethylphenylhydrazine hydrochloride, the title compound was prepared (2.6 g; 46%). ¹H NMR (300 MHz, d₆-DMSO) δ 8.0 (d, J = 8.2 Hz, 2H), 7.85 (d, J = 8.7 Hz, 2H), 5.4 (s, 1H), 2.1 (s, 3H).

### b) 3'-{N'-[3-methyl-5-oxo-1-(3-trifluoromethylphenyl)-1,5-dihydropyrazol-4-ylidene]hydrazino}-2'-hydroxybiphenyl-3-carboxylic acid;

Following the procedure of Example 1e), except substituting the compound from Example 3e) for the compound from Example 1c) and the compound from Example 36a) for the compound from Example 1d) and washing the crude product with chloroform, the title compound was prepared (0.21 g; 58%) as an orange solid. ¹H NMR (300 MHz, d₆-DMSO) δ 13.7 (s, 1H), 13.1 (s, 1H), 9.78 (s,1H), 8.18 (d, J = 8.5 Hz, 2H), 8.14 (s, 1H), 7.98 (d, J = 1.3 Hz, 1H), 7.85 (d, J = 8.7 Hz, 2H), 7.8-(s, 1H), 7.75 (dd, J=7.3, 2.4, 1H), 7.64 (t, J = 5.5, 1H), 7.2 (m, 2H), 2.37 (s, 3H).

### Example 36

### 3'-{N'-[3-methyl-5-oxo-1-(4-N-methylcarboxamidolphenyl)-1,5-dihydropyrazol-4-ylidene]hydrazino}-2'-hydroxybiphenyl-3-carboxylic acid;:

### a) 4-(3-Methyl-5-oxo-4,5-dihydropyrazol-1-yl)-benzoic acid;:

Following the procedure of Example 1d), except substituting 4-hydrazinobenoic acid; for 3,4-dimethylphenylhydrazine hydrchloride, the title compound was prepared (1.91 g; 90%) as solid. ¹H NMR (300 MHz, d₆-DMSO) δ 12.9 (s, 1H),11.1 (s, 1H), 8.07 (d, J = 4.2Hz, 2H), 7.95 (d, J = 4.2 Hz, 2H), 5.4 (s, 1H), 2.13(s, 3H).

### b) 3'- N'-[3-methyl-5-oxo-1-(4-N-methylcarboxamidolphenyl)-1,5-dihydropyrazol-4-ylidene]hydrazino}-2'-hydroxybiphenyl-3-carboxylic acid;:

To the DMF solution of 4-(3-methyl-5-oxo-4,5-dihydropyrazol-1-yl)-benzoic acid; (0.218 g, 1mmol), 1-hydroxybenzotriazole hydrate (0.149 g, 1.1mmol) and methylamine (1.1mmol), was added 1-ethyl-3-(3-dimethylaminopropyl)carbodiimide hydrochloride (0.211, 1.1mmol). The reaction mixture was stirred at room temperature for 18 hours. Water was added and the resulting mixture was extracted with ethyl acetate and the extract was dried (MgSO4), filtered and concentrated. The crude product was isolated as solid. Following the procedure of Example 1e). except substituting the compound from Example 3e) for 4-amino-3'-hydroxybiphenyl-4-carboxylic acid;, hydrochloride salt and the above crude product for the compound from Example 1d), the title compound was prepared as solid (0.056 g, 34%, two steps). ¹H NMR (300 MHz, d₆-DMSO) δ 13.7(brs, 1H), 9.77 (s, 1H), 8.46(d, J = 4.6Hz, 1H), 8.14 (s, 1H), 8.03 (d, J = 8.8Hz , 1H), 7.95 (t, J = 7.8 Hz, 2H), 7.85(d, J = 6.7Hz, 1H), 7.75 (dd, J=7.9, 2.6Hz ,1H), 7.64 (t, J = 7.8Hz ,1H), 7.2 (m, 2H), 2.78 (s, 3H), 2.28 (s, 3H). MS(ES) m/z 487 (M+H)⁺.

### Example 37

### N-[1-(3'-{N'-[1-(3,4-dimethylphenyl)-3-methyl-5-oxo-1,5-dihydropyrazol-4-ylidene]hydrazino}-2'-hydroxybiphenyl-3-yl)methanoyl]methanesulfonamide

### a) N-[1-(5'-Chloro-2'-methoxybiphenyl-3-yl)-methanoyl]-methanesulfonamide:

Methanesulfonamide(1.74 g, 18.3 mmol) in CH2C12 (20ml) was treated with pyridine (1.44 g, 18.2 mmol) and 3-bromobenzoyl chloride (4.0 g, 9.11 mmol). The reaction mixture was stirred at room temperature over 18hrs. Water was added, the organic solution was dried over MgSO4, filtered, and concentrated to crude product. A solution of the above crude compound (2.85 g, 10.2mmol), 2-methoxy-5-chlorophenylboronic acid; (1.91 g, 10.2 mmol), 2M aqu. sodium carbonate (5.1 mL; 10.2mmol.) and tetrakistriphenylphosphino palladium(0) (0.5 g) in 1,4-dioxane (60.0 mL) was stirred and heated under reflux under a nitrogen atmosphere for 24h. The reaction mixture was cooled and evaporated and the residue treated with 6M aqu. hydrochloric acid; (100 mL). The grey precipitate was filtered and washed well with water then diethyl ether to afford the title compound (2.3 g; 88%) as a colorless solid.¹H NMR (300 MHz, CDC13) δ 8.8 (s, 1H), 7.95(s, 1H), 7.82(d, J = 8.0Hz, 1H), 7.75 (d, J = 8.0Hz, 1H), 7.55 (t, J = 8.0Hz , 1H), 7.3 (m, 2H), 6.95(d, J = 9.0Hz, 1H), 3.8 (s, 3H), 3.5 (s, 3H).

### b) N-[1-(5'-Chloro-2'-hydroxybiphenyl-3-yl)-methanoyl]methanesulfonamide:

The compound of example 37a) was dissolved in iodotrimethylsilane (10mL), The reaction mixture was stirred at room temperature for 18 hrs, and then heated at 60°C for 18hrs. After cooling down to room temperature, ice water was poured in, causing precipitation. The solid was collected and further purified through SiO2 chromatography (3%MeOH/CH₂Cl₂). The title compound was prepared as yellow gum(0.12 g, 30%). ¹H NMR (300 MHz, CDCl₃) δ 8.0 (s, 1H), 7.85(d, J = 8.0Hz, 1H), 7.72 (d, J = 8.0Hz, 1H), 7.6 (t, J = 8.0Hz, 1H), 7.23 (m, 1H), 7.19 (d, J = 9.0Hz, 1H), 6.9 (d, J = 9.0Hz, 1H), 3.5 (s, 3H).

### c) N-[1-(5'-Chloro-3'-nitro-2'-hydroxybiphenyl-3-yl)-methanoyl]methanesulfonamide:

Following the procedure of example 7c), except substituting the compound of example 37b) for the compound of example 7b), the title compouind was prepared as yellow solid (0.1 g, 87%). MS(ES) m/z 371 (M+H)⁺.

### d) N-[1-(3'-{N'-[1-(3,4-dimethylphenyl)-3-methyl-5-oxo-1,5-dihydropyrazol-4-ylidene]hydrazino}-2'-hydroxybiphenyl-3-yl)methanoyl]methanesulfonamide:

Following the procedure of example 12d), except substituting the compound of example 37c) for the compound of example 12c) the title compound was prepared as dark powder (0.04 g, 48%). MS(ES) m/z 520 (M+H)⁺.

### Example 38

### 3'-{N'-[3-methyl-5-oxo-1-phenyl-1,5-dihydropyrazol-4-ylidene]hydrazino}-2'-hydroxybiphenyl-3-carboxylic acid;

Following the procedure of example 1e), except substituting 3'-amino-2'-hydroxybiphenyl-3-carboxylic acid; for 4'-amino-3'-hydroxybiphenyl-3-carboxylic acid; and 5-methyl-2-phenyl-2,4-dihydropyrazol-3-one for the compound of example 1d), the title compound was prepared as a solid (0.15 g, 65%).¹H NMR (300 MHz, d₆-DMSO) δ 14.9(s, 1H), 8.21 (s, 1H), 8.04 (d, J = 8.0Hz, 2H), 7.9(d, J = 8.0Hz, 1H), 7.78 (d, J = 8.0, 1H), 7.54(t, J = 8.0Hz, 1H), 7.34 (m, 3H), 7.0 (m, 3H), 2.34 (s, 3H).

### Example 39

### 3'-{N'-[3-methyl-1-(4-methylphenyl)-5-oxo-1,5-dihydropyrazol-4-ylidenelhydrazino}-2'-hydroxybiphenyl-3-carboxylic acid;

Following the procedure of example 1e), except substituting 3'-amino-2'-hydroxybiphenyl-3-carboxylic acid; for 4'-amino-3'-hydroxybiphenyl-3-carboxylic acid; and 5-methyl-2-p-tolyl-2,4-dihydropyrazol-3-one for the compound of example 1d), the title compound was prepared as a solid (0.11 g, 46%). ¹H NMR (300 MHz, d₆-DMSO) δ 8.19(s, 1H), 7.98 (d, J = 8.0Hz, 2H), 7.85(d, J = 8.0Hz , 1H), 7.72(d, J = 8.0, 1H), 7.42 (t, J = 8.0Hz, 1H), 7.34 (d, J = 8.0Hz, 2H), 7.05 (d, J = 8.0Hz, 1H), 6.9 (t, J = 8.0Hz, 1H), 2.34 (s, 3H), 2.23 (s, 3H). MS(ES) m/z 429(M+H)⁺.

### Example 40

### 3'-{N'-[1-(4-chlorophenyl)-3-methyl-5-oxo-1,5-dihydropyrazol-4-ylidene]hydrazino}-2'-hydroxybiphenyl-3-carboxylic acid:

Following the procedure of example 1e), except substituting 3'-amino-2'-hydroxybiphenyl-3-carboxylic acid; for 4'-amino-3'-hydroxybiphenyl-3-carboxylic acid; and 5-methyl-2-chlorophenyl-2,4-dihydropyrazol-3-one for the compound of example 1d), the title compound was prepared as a solid (0.22 g, 88%). ¹H NMR (300 MHz, d₆-DMSO) δ 8.21 (s, 1H), 8.12 (d, J = 8.0Hz, 2H), 7.9 (d, J = 8.0Hz,1H), 7.85(d, J = 8.0, 1H), 7.54 (t,J = 8.0Hz, 1H), 7.39 (m, 3H), 7.09 (m, 1H), 6.92 (t, J = 8.0Hz, 1H), 2.34 (s, 3H).

### Example 41

### 3'-{N'-[1-(4-fluorophenyl)-3-methyl-5-oxo-1,5-dihydropyrazol-4-ylidene]hydrazino}-2'-hydroxybiphenyl-3-carboxylic acid;:

### a) 2-(4-Fluorophenyl)-5-methyl-2,4-dihydropyrazol-3-one:

Following the procedure of example 1d), except substituting 4-Fluorophenylhydrazine for 3,4-dimethylphenylhydrazine, the title compound was prepared as a solid (3.0 g, 64%). MS(ES) m/z 193 (M+H)⁺.

### b) 3'-{N'-[1-(4-fluorophenyl)-3-methyl-5-oxo-1,5-dihydropyrazol-4-ylidene]hydrazino}-2'-hydroxybiphenyl-3-carboxylic acid;:

Following the procedure of example 1e), except substituting 5-(2'-hydroxybiphenyl-3'-amino-3-yl)-1H-tetrazole for 4-amino-3'-hydroxybiphenyl-3-carboxylic acid;, hydrochloridesalt and the compound of example 45a) for the compound of example 1d), the title compound was prepared (0.16 g, 59%). ¹H NMR (300 MHz, d₆-DMSO) δ 8.25(s, 1H), 8.05 (d, J = 7.5Hz, 1H), 7.75(dd, J = 2.1 Hz, 8.2Hz , 2H), 7.75 (m, 3H), 7.25(m, 4H), 2.34 (s, 3H). MS(ES) m/z 457(M+H)⁺.

### Example 42

### 3'-{N'-[3-methyl-5-oxo-1-(4-trifluoromethoxyphenyl)-1,5-dihydropyrazol-4-ylidene]hydrazino}-2'-hydroxybiphenyl-3-carboxylic acid;

### a) 5-methyl-2-(4-trifluoromethoxyphenyl)-2,4-dihydropyrazol-3-one:

Following the procedure of example 1d), except substituting 5-methyl-2-(4-trifluoromethoxyphenylhydrazine for 3,4-dimethylphenylhydrazine, the title compound was prepared as solid (2.0 g, 49%). ¹H NMR (300 MHz, ,d₆-DMSO) δ 7.85 (d, J = 9.1Hz, 2H), 7.45 (d, J = 8.8Hz, 2H), 5.39 (s, 1H), 2.12 (s, 3H). MS(ES) m/z 259 (M+H)⁺.

### b) 3'-{N'-[3-methyl-5-oxo-1-(4-trifluoromethoxyphenyl)-1,5-dihydropyrazol-4-ylidene]hydrazino}-2'-hydroxybiphenyl-3-carboxylic acid;:

Following the procedure of example 1e), except substituting 3'-amino-2'-hydrobiphenyl-3-carboxylic acid; for 4-amino-3'-hydroxybiphenyl-3-carboxylic acid;, hydrochloridesalt and the compound of example 49aa) for the compound of example 1d), the title compound was prepared (0.185 g, 60%). ¹H NMR (300 MHz, d₆-DMSO) δ 13.7 (br s, 1H), 13.1 (br s, 1H), 9.76 (br s, 1H), 8.1(s, 1H), 8.05 (d, J = 9. 1Hz, 2H), 7.97 (d, J = 7.7Hz, 1H), 7.8 (d, J = 7.8Hz, 1H), 7.75(d, J = 7.2Hz, 1H), 7.65 (t, J = 8.7Hz, 1H), 7.47(d, J = 9Hz, 2H), 7.18(m, 2H), 2.35 (s, 3H). MS(ES) m/z 499(M+H)⁺.

### Example 43

### 3'-{N'-[1-(3,4-dimethylphenyl)-3-ethoxy-5-oxo-1,5-dihydroyrazol-4-ylidene]hydrazino}-2'-hydroxybiphenyl-3-carboxylic acid;

### a) Acetic acid; N'-(3,4-dimethylphenyl)hydrazide:

3,4-dimethylphenyl hydrazine (5 g, 29 mmol) was partitioned in diluted sodium hydroxy and ether, the ether solution was dried and evaporated to a solid. The solid was redissolved in ether and treated with acetic anhydride(8 mL, 78 mmol). After stirred at room temperature for 18 hours, the solvent was evaporated to dryness and further washed either and hexane to give a solid (3 g, 60%). MS(ES) m/z 179 (M+H)⁺.

### b) 1-(3,4-dimethylphenyl)-pyrazolidine-3,5-dione:

The compound of example 47a (3.1 g, 17.7 mmol) in phosphorus trichloride (1.75ml) was treated with malonic acid; (1.84 g, 17.7 mmol) and heated to 100°C until the mixture stopped foaming. After cooling down to room temperature, the mixture was dissolved in sodium bicarbonate solution and extracted with ether. The aqueous solution was acid;ified with 60% Hydrochloride to pH 6 to 6.5, then extracted with ethyl acetate(4 times). The combined extract were dried and evaporated to a solid which recrystallized from ethanol to give the title compound.(0.9 g, 26%). ¹H NMR (300 MHz, CDC13) δ 7.29 (s, 1H), 7.20 (dd, J = 2.4 Hz, 8.2Hz, 1H), 7.10 (d, J = 8.2Hz, 1H), 3.33 (s, 1H), 2.23(s, 3H), 2.2(s, 3H).

### c) 2-(3,4-dimethylphenyl)-5-ethoxy-2,4-dihydropyrazole-3-one:

To the suspension of the compound of example 43b) (0.8 g, 4 mmol) in ethanol (15 mL) was added concentrated sulfuric acid; (0.1 mL, cat.). The reaction mixture was then heated to reflux for 48 hours. Potassium carbonate was added to the mixture and the solvent was removed under reduced pressure and the residue purified by column chromatography on SiO2 to afford the title compound (0.25 g; 27%) as a solid. ¹H NMR (300 MHz, CDCl3) δ 7.65 (s, 1H), 7.57 (dd, J = 2.4 Hz, 8.7Hz, 1H), 7.08 (d, J = 8.7Hz, 1H), 4.31(m, 2H), 3.4 (s, 2H), 2.23(s, 3H), 2.2(s, 3H), 1.4 (t, J = 8.6Hz, 3H).

### d) 3'-{N'-[1-(3,4-dimethylphenyl)-3-ethoxy-5-oxo-1,5-dihydropyrazol-4-ylidene]hydrazino}-2'-hydroxybiphenyl-3-carboxylic acid;:

Following the procedure of example 1e), except substituting 4-amino-3'-hydroxybiphenyl-3-carboxylic acid;, hydrochloridesalt for 3'-amino-2'-hydroxybiphenyl-3-carboxylic acid;, hydrochloridesalt and the compound of example 43c) for the compound of example 1d), the title compound was prepared (0.015 g, 8%). ¹H NMR (300 MHz, d₆-DMSO) δ 9.69 (s, 1H), 8.13(s, 1H), 7.98 (d, J = 7.6Hz, 1H), 7.8 (d, J = 8.4Hz , 1H), 7.71 (s, 1H), 7.65(m, 3H), 7.2 (m, 2H), 4.4(m, 2H), 2.28 (s, 3H), 2.23 (s, 3H), 1.35(t, J = 8.6Hz, 3H). MS(ES) m/z 473(M+H)⁺.

### Example 44

### 3'-{N'-[1-(3,4-dimethylphenyl)-3-(1-methylethoxy)-5-oxo-1,5-dihypyrazol-4-ylidene]hydrazino}-2'-hydroxybiphenyl-3-carboxylic acid;

### a) 2-(3,4-dimethylphenyl)-5-isopropoxy-2,4-dihydropyrazole-3-one:

Following the procedure of example 43c), except substituting isopropanol for ethanol, the title compound was prepared as a solid (0.28 g, 28%). ¹H NMR (300 MHz, CDCl3) δ 7.64 (s, 1H), 7.52 (dd, J = 2.4 Hz, 8.7Hz, 1H), 7.05 (d, J = 8.7Hz, 1H), 5.1(m, I H), 3.4 (s, 2H), 2.23(s, 3H), 2.2(s, 3H), 1.27 (s, 3H), 1.25 (s, 3H).

### b) 3'-{N'-[1-(3,4-dimethylphenyl)-3-(1-methylethoxy)-5-oxo-1,5-dihydropyrazol-4-ylidene]hydrazino}-2'-hydroxybiphenyl-3-carboxylic acid;:

Following the procedure of example 43d), except substituting the compound of example 44a) for the compound of example 43c), the title compound was prepared as a solid (0.005 g, 5%). ¹H NMR (300 MHz, d₆-DMSO) δ 9.65 (s, 1H), 8.10(s, 1H), 7.95 (d, J = 7.7Hz, 1H), 7.8 (d, J = 8.1 Hz , 1H), 7.6 (s, 1H), 7.6(m, 3H), 7.15 (m, 2H), 5.18(m, 1H), 2.23 (s, 3H), 2.08 (s, 3H), 1.46(s, 3H).1.44(s, 3H) MS(ES) m/z 487(M+H)⁺.

### Example 45

### 3'-{N'-[3-tert-butyl-1-(3,4-dimethylphenyl)-5-oxo-1,5-dihydropyrazol-4-ylidene]hydrazino}-2'-hydroxybiphenyl-3-carboxylic acid;:

### a) 5-tert-Butyl-2-(3,4-dimethylphenyl)-2,4-dihydropyrazol-3-one;

Following the procedure of example 1d), except substituting methyl pivaloylacetate for ethyl acetoacetate, the title compound was prepared as solid(4.1 g, 60%). ¹H NMR (300 MHz, ,d₆-DMSO) δ 7.53 (s, 1H), 7.45 (d, J = 8.2Hz, 1H), 7.15(d, J = 8.2Hz, 1H), 5.3 (s, 1H), 2.25(s, 3H), 2.22(s, 3H), 1.23 (s, 9H).

### b) 3'-{N'-[3-tert-butyl-1-(3,4-dimelhylphenyl)-5-oxo-1,5-dihydropyrazol-4-ylidene]hydrazino}-2'-hydroxybiphenyl-3-carboxylic acid;:

Following the procedure of example 1e), except substituting 3'-amino-2'-hydrobiphenyl-3-carboxylic acid; for 4-amino-3'-hydroxybiphenyl-3-carboxylic acid;, hydrochloridesalt and the compound of example 49aa) for the compound of example 1d), the title compound was prepared (0.06 g, 20%). ¹H NMR (300 MHz, CDCl₃) δ 8.22(s, 1H), 8.08 (d, J = 6.5Hz, 1H), 7.78 (d, J = 6.0Hz, 1H), 7.7 (s, 1H), 7.63(dd, J = 2.6Hz, 7.1Hz, 2H), 7.56 (t, J = 7.8, 1H), 7.1(m, 3H), 2.31 (s, 3H), 2.27 (s, 3H), 1.5(s, 9H). MS(ES) m/z 485(M+H)⁺.

### Example 46

### 3'-{N'-[3-methyl-1-(4-methyl-2,3,5,6-tetrafluorophenyl)-5-oxo-1,5-dihydropyrazol-4-ylidene]hydrazino}-2'-hydroxybiphenyl-3-carboxylic acid,

### a) 5-Methyl-2-(2,3,5,6-tetrafluoro-4-methylphenyl)-2,4-dihydropyrazol-3-one:

Following the procedure of example 1d), except substituting 2,3,5,6-tetrafluoro-4-methylphenyl hydrazine for 3,4-dimethylphenylhydrazine, the title compound was prepared as solid (1.68 g, 35%). MS(ES) m/z 261 (M+H)⁺.

### b) 3'-{N'-[3-methyl-1-(4-methyl-2,3,5,6-tetrafluorophenyl)-5-oxo-1,5-dihydropyrazol-4-ylidene]hydrazino}-2'-hydroxybiphenyl-3-carboxylic acid;:

Following the procedure of example 1e), except substituting 3'-amino-2'-hydroxybiphenyl-3-carboxylic acid; for 4-amino-3'-hydroxybiphenyl-3-carboxylic acid; and the compound of example 46a) for the compound of example 1d), the title compound was prepared (0.07 g, 18%). ¹H NMR (300 MHz, d₆-DMSO) δ 13.4 (br s, 1H), 9.78(s, 1H), 7.98 (d, J = 6.4Hz, 1H), 7.8 (d, J = 6.4Hz, 1H), 7.75 (dd, J = 2.2Hz, 7.4Hz, 1H), 7.61 (t, J = 7.7Hz, 1H), 7.19 (m, 2H), 2.33 (s, 6H).

### Example 47

### 3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid;

### a) 2-(4-Fluoro-3-methylphenyl)-5-methyl-2,4-dihydropyrazol-3-one:

Following the procedure of example 1d), except substituting 4-Fluoro-3-methylphenyl hydrazine for 3,4-dimethylphenylhydrazine, the title compound was prepared as solid (1.3 g, 73%). ¹H NMR (300 MHz, d₆-DMSO) δ 7.63 (m, 1H), 7.55 (m, 1H), 7.17 (t, J = 9.4Hz, I H), 5.29(br s, 1H), 2.26(s, 3H), 2.09(s, 3H).

### b) 3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid;:

Following the procedure of example 1e), except substituting 3'-amino-2'-hydroxybiphenyl-3-carboxylic acid; for 4-amino-3'-hydroxybiphenyl-3-carboxylic acid; and the compound of example 47a) for the compound of example 1d), the title compound was prepared (0.17 g, 60%).¹H NMR (300 MHz, d₆-DMSO) δ 13.7 (br s, 1H), 13.0 (s, 1H), 9.8(s,1H), 8.15 (s, 1H), 7.97(d, J = 7.7Hz, 1H), 7.75 (m, 4H), 7.63(t, J = 7.7Hz, 1H), 7.2 (m, 3H), 2.34 (s, 3H), 2.30 (s, 3H). MS(ES) m/z 445 (M+H)⁺.

### Example 48

### 3'-{N'-[1-(3,4-dimethylphenyl)-3-phenyl-5-oxo-1,5-dihydropyrazol-4-ylidene]hydrazino}-2'-hydroxybiphenyl-3-carboxylic acid;

Following the procedure of example 53, except substituting 3'-amino-2'-hydroxybiphenyl-3-carboxylic acid;, hydrochloridesalt for 5-(2'-hydroxybiphenyl-3'-amino-3-yl)-1H-tetrazole, the title compound was prepared as solid (0.186 g, 96%). ¹H NMR (300 MHz, d₆-DMSO) δ 9.80 (br s, 1H), 8.08(d, J = 8.4Hz, 2H), 8.16(s, 1H), 7.98 (d, J = 7.5Hz, 1H), 7.8 (m, 3H), 7.6 (m, 4H), 7.25(m,3H), 2.31 (s, 3H), 2.26 (s, 3H), MS(ES) m/z 505 (M+H)⁺.

### Example 49

### 3-{N'-1[1-(3,4-dimethylphenyl)-5-oxo-3-phenyl-1,5-dihydropyrazol-4-ylidenelhydrazino}-2-hydroxy-3'-tetrazol-5-ylbiphenyl

### a) 2-(3,4-dimethylphenyl)-5-phenyl-2,4-dihydropyrazole-3-one:

Following the procedure of example 1d), except substituting ethyl benzylacetoacetate for ethyl acetoacetate, the title compound was prepared as solid(2.5 g, 52%). ¹H NMR (300 MHz, CDC13) δ 7.84 (m, 2H), 7.75 (s, 1H), 7.47(dd, J = 3.2 Hz, 6.5Hz, 1H), 7.45 (m, 3H), 7.15(d, J = 6.5Hz, 1H), 2.28(s, 3H), 2.23(s, 3H).

### b) 3-{N'-[1-(3,4-dimethylphenyl)-5-oxo-3-phenyl-1,5-dihydropyrazol-4-ylidene]hydrazino}-2-hydroxy-3'-tetrazol-5-ylbiphenyl

Following the procedure of example 1e), except substituting 5-(2'-hydroxybiphenyl-3'-amino-3-yl)-1H-tetrazole for 4-amino-3'-hydroxybiphenyl-3-carboxylic acid;, hydrochloridesalt and the compound of example 49a) for the compound of example 1d), the title compound was prepared (0.008 g, 4%). MS(ES) m/z 529(M+H)⁺.

### Example 50

### 3- {N'-[1-(3,4-dimethylphenyl)-3-methoxy-5-oxo-1,5-dihydropyrazol-4-ylidene]hydrazino]-2-hydroxy-3'-tetrazol-5-ylbiphenyl

### a) 5-(2'-hydroxybiphenyl-3'-amino-3-yl)-1H-tetrazole:

Following the procedure of example 1c), except substituting the compound of example 12c) for the compound of example 1b), the title compound was prepared as a solid (1.2 g, 60%). ¹H NMR (300 MHz, d₆-DMSO) δ 8.23(s, 1H), 8.14(m, 1H), 7.7 (d, J = 5.6Hz, 2H), 7.46 (dd, J = 1.6Hz, 7.9Hz , 2H), 7.1 (t, J = 7.8Hz, 1H), MS(ES) m/z 254(M+H)⁺.

### b) 2-(3,4-dimethylphenyl)-5-methoxy-2,4-dihydropyrazole-3-one:

Following the procedure of example 43c), except substituting methanol for ethanol, the title compound was prepared as a solid (0.45 g, 56%). MS(ES) m/z 219(M+H)⁺.

### c) 3-{N'-[1-(3,4-dimethylphenyl)-3-methoxy-5-oxo-1,5-dihydropyrazol-4-ylidene]hydrazino[-2-hydroxy-3'-tetrazol-5-ylbiphenyl

Following the procedure of example 43d), except substituting the compound of example 50a) for 3'-amino-2'-hydroxybiphenyl-3-carboxylic acid;, hydrochloride salt, and the compound of example 50b) for the compound of example 47c), the title compound was prepared as a solid. MS(ES) m/z 483(M+H)⁺.

### Example 51

### 3-{N'-[1-(3,4-dimethylphenyl)-3-ethoxy-5-oxo-1,5-dihydropyrazol-4-ylidene]hydrazino}-2-hydroxy-3'-tetrazol-5-ylbiphenyl

Following the procedure of example 50c), except substituting the compound of example 43c) for the compound of example 50b), the title compound was prepared as solid (0.008 g, 4%). ¹H NMR (300 MHz, d₆-DMSO) δ 9.79 (br s, I H), 8.08(d, J = 7.4Hz, 1H), 7.7 (m, J = 7.7Hz, 5H), 7.2 (m, 3H), 4.4 (m, 2H), 2.27 (s, 3H), 2.23 (s, 3H), 1.47 (t, J = 7.0Hz. 3H), MS(ES) m/z 497(M+H)⁺

### Example 52

### 3-{N'-[1-(3,4-dimethylphenyl)-3-(1-methylethoxy)-5-oxo-1,5-dihydropyrazol-4-ylidene]hydrazino}-2-hydroxy-3'-tetrazol-5-ylbiphenyl

Following the procedure of example 50c), except substituting the compound of example 44a) for the compound of example 50b), the title compound was prepared as solid (0.008 g, 4%). MS(ES) m/z 511 (M+H)⁺.

### Example 53

### 3-{N'-[1-(4-fluorophenyl)-3-methyl-5-oxo-1,5-dihydropyrazol-4-ylidene]hydrazino]-2-hydroxy-3'-tetrazol-5-ylbiphenyl

### a) 2-(4-Fluorophenyl)-5-methyl-2,4-dihydropyrazol-3-one:

Following the procedure of example 1d), except substituting 4-fluorophenylhydrazine for 3,4-dimethylphenylhydrazine, the title compound was prepared as solid(3.0 g, 64 %). MS(ES) m/z 192(M+H)⁺.

### b) 3-{N'-[1-(4-fluorophenyl)-3-methyl-5-oxo-1,5-dihydropyrazol-4-ylidene]hydrazino}-2-hydroxy-3'-tetrazol-5-ylbiphenyl:

Following the procedure of example 1e), except substituting 5-(2'-hydroxybiphenyl-3'-amino-3-yl)-1H-tetrazole for 4-amino-3'-hydroxybiphenyl-3-carboxylic acid;, hydrochloridesalt and the compound of example 53a) for the compound of example 1d), the title compound was prepared (0.16 g, 60%). ¹H NMR (300 MHz, d₆-DMSO) δ 8.2 (s, 1H), 8.08 (d, J = 7.4Hz, 1H), 7.95 (dd, J = 2.1Hz, 9.2Hz, 2H), 7.75(m, 3H), 7.2 (m, 4H), 2.34(s, 3H). MS(ES) m/z 457(M+H)⁺.

### Example 54

### 3-{N'-[1-(4-fluoro-3-methylphenyl)-3-methyl-5-oxo-1,5-dihydropyrazol-4-ylidene]hydrazino}-2-hydroxy-3'-tetrazol-5-ylbiphenyl

Following the procedure of example 47b), except substituting 5-(2'-hydroxybiphenyl-3'-amino-3-yl)-1 H-tetrazole for 3'-amino-2'-hydroxybiphenyl-3-carboxylic acid;, the title compound was prepared as a solid (0.1 g, 37%). ¹H NMR (300 MHz, d₆-DMSO) δ 13.7 (br s, 1H), 9.9 (brs, 1H), 8.2 (s, 1H), 8.09 (d, J = 7.5Hz, 1H), 7.8 (m, 5H), 7.25(m, 3H), 2.35(s, 3H), 2.30 (s, 3H). MS(ES) m/z 471(M+H)⁺.

### Example 55

### 3-{N'-[3-methyl-5-5-oxo-1-(4-trifluoromethylphenyl)-1,5-dihydropyrazol-4-ylidene]hydrazino}-2-hydroxy-3'-tetrazol-5-ylbiphenyl

Following the procedure of example 54), except substituting the compound of 35a) for the compound of 47a), the title compound was prepared as a solid (0.115 g, 41 %). ¹H NMR (300 MHz, d₆-DMSO) δ 13.7 (br s, I H), 9.9 ( br s, I H), 8.25 (s, I H), 8.17 (d, J = 8.6Hz, 2H), 8.08 (s, J = 6.4Hz, 1H), 7.85(d, J = 8.8Hz, 2H), 7.76 (m, 3H), 7.25 (m, 2H), 2.38(s, 3H). MS(ES) m/z 505 (M+H)⁺.

### Example 56

### 3'-{N'-[1-(3,4-dimethylphenyl)-3-(pyridin-4-yl-5-oxo-1,5-dihydropyrazol-4-ylidene]hydrazino}-2'-hydroxybiphenyl-3-carboxylic acid;:

### a) 2-(3,4-Dimethylphenyl)-5-pyridin-4-yl-2,4-dihydropyrazol-3-one:

Following the procedure of example 1d), except substituting ethyl benzylacetoacetate for ethyl acetoacetate, the title compound was prepared as solid(1.0 g, 29 %). ¹H NMR (300 MHz, d₆-DMSO) δ 11.9(br s, 1H), 8.5(d, J = 4.6HZ, 2H), 7.78 (d, J = 4.6Hz, 2H), 7.58 (s, 1H), 7.50(dd, J = 2.1 Hz, 8.1Hz, 1H), 7.2 (d, J = 8.1Hz, 1H), 6.1(s, 1H), 2.29(s, 3H), 2.26(s, 3H).

### b) 3'-{N'-[1-(3.4-dimethylphenyl)-3-(pyridin-4-yl-5-oxo-1,5-dihydropyrazol-4-ylidene]hydrazino}-2'-hydroxybiphenyl-3-carboxylic acid;

Following the procedure of example 1e), except substituting the compound of example 60a) for the compound of example 1d) and substituting 3'-amino-2'-hyroxybiphenyl-3-carboxylic acid; for 4-amino-3'-hydrobiphenyl-3-carboxylic acid;, the title compound was prepared (0.13 g, 68 %). ¹H NMR (300 MHz, d₆-DMSO) δ 14.4(br s, 1H), 8.9(s, 2H), 8.5 (s, 2H), 8.19(s, 1H), 7.98 (d, J = 8.7Hz, 1H), 7.90(d, J = 7.3Hz, 1H), 7.8 (m, 2H), 7.75 (d, J = 7.3Hz, 1H), 7.65(t, J = 8.7Hz, 1H), 7.25 (m, 3H), 2.29(s, 3H), 2.26(s, 3H). MS(ES) m/z 506(M+H)⁺.

### Example 57

### 3-{N'-[1-(3,4-dimethylphenyridin-4-yl-5-oxo-1,5-dihydropyrazol-4-ylidene]hydrazino}-2-hydroxy-3'-tetrazol-5-ylbiphenyl

Following the procedure of example 56, except substituting 5-(2'-hydroxybiphenyl-3'-amino-3-yl)-1H-tetrazole for 3'-amino-2'-hyroxybiphenyl-3-carboxylic acid;, the title compound was preparaed as a solid (0.13 g, 61 %). ¹H NMR (300 MHz, d₆-DMSO) δ 14.4(br s, 1H), 10.1 (br s,I H), 8.9(s, 2H), 8.65 (s, 2H), 8.3(s, 1H), 8.13 (d, J = 7.4Hz, 1H), 7.8(m, 6H), 7.32(m, 3H), 2.28(s, 3H), 2.23(s, 3H). MS(ES) m/z 530(M+H)⁺.

### Example 58

### 3-{N'-[1-(3,4-dimethylphenyl)-3-pyridin-2-yl-5-oxo-1,5-dihydropyrazol-4-ylidene]hydrazino1-2-hydroxy-3'-tetrazol-5-ylbiphenyl

### a) 2-(3,4-dimethylphenyl)-5-pyridin-2-yl-2,4-dihydropyrazol-3-one:

Following the procedure of example 1d), except substituting ethyl picolinylacetate for ethyl acetoacetate, the title compound was prepared as solid(1.5 g, 44%).¹H NMR (300 MHz, d₆-DMSO) δ 11.7(s, 1H), 8.59(d, J = 4.7Hz, 1H), 7.98 (d, J = 7.0Hz, 1H), 7.85 (t, J = 7.6Hz, 1H), 7.60 (s,1H), 7.53 (d, J = 8.1Hz, 1H), 7.31 (d, J = 4.9Hz, 1H), 7.2(d, J = 8.2Hz, I H), 6.05 (s, I H), 2.29(s, 3H), 2.26(s, 3H).

### b) 3-{N'-[1-(3,4-dimethylphenyl)-3-pyridin-2-yl-5-oxo-1,5-dihydropyrazol-4-ylidene]hydrazino}-2-hydroxy-3'-tetrazol-5-ylbiphenyl

Following the procedure of example 1e), except substituting the compound of example 58a) for the compound of example 1d) and substituting 5-(2'-hydroxybiphenyl-3'-amino-3-yl)-1H-tetrazole for 4-amino-3'-hydrobiphenyl-3-carboxylic acid;, the title compound was prepared (0.17 g, 68%). ¹H NMR (300 MHz, d₆-DMSO) δ 8.9(br s, 1H), 8.43 ( br s, I H), 8.29(s, 1H), 8.15 (d, J = 8.1Hz, 2H), 7.80(m, 5H), 7.65 (m, 1H), 7.23 (m, 3H), 2.29(s, 3H), 2.26(s, 3H). MS(ES) m/z 530(M+H)⁺.

### Example 59

### 3'-{N'-[1-(3,4-dimethylphenyl)-3-(pyridin-2-yl-5--oxo-1,5-dihydropyrazol-4-ylidene]hydrazino}-2'-hydroxybiphenyl-3-carboxylic acid;

Following the procedure of example 58b), except substituting 3'-amino-2'-hyroxybiphenyl-3-carboxylic acid; for 5-(2'-hydroxybiphenyl-3'-amino-3-yl)-1H-tetrazole, the title compound was prepared as a solid (0.08 g, 83%). ¹H NMR (300 MHz, d₆-DMSO) δ 9.8(br s, 1H), 8.9(s, 1H), 8.49 (d, J = 8.1Hz, 1H), 8.19(s, 2H), 7.98 (d, J = 8.1Hz, 1H), 7.80(m, 4H), 7.75 (t, J= 7.0Hz, 2H), 7.1 (m, 3H), 2.29(s, 3H), 2.26(s, 3H). MS(ES) m/z 506(M+H)⁺.

### Example 60

### 3-{N'-[1-(3-Fluoro-4-methylphenyl)-3-methyl-5-oxo-1,5-dihydropyrazol-4-ylidene]hydrazino}-2-hydroxy-3'-tetrazol-5-ylbiphenyl

### a) 3-Fluoro-4-methylphenylhydrazine:

3-Fluoro-4-methylaniline (5.0 g, 40 mmol) was combined with a mixture of acetic acid; (125 mL), water (50 mL), and concentrated (50 mL)hydrochloric acid;.

The resulting solution was cooled in an ice bath and treated portion-wise with NaNO₂ (3.1 g, 45 mmol) in water (50 mL). After the reaction mixture had stirred at low temperature for 15 minutes, it was added to a solution of tin chloride (45 g, 200 mmol) in 100 ml concentrated hydrochloric acid; that was also cooled in an ice bath. The reaction mixture was then allowed to warm to room temperature and stirred for 30 minutes. The resulting slurry was extracted with ethyl acetate (400 mL), and the organic layer was washed with brine and dried over magnesium sulfate. The solution was then filtered and the solvent removed under reduced pressure. The yellow solid thus obtained was partitioned between 1 M sodium hydroxide (200 mL) and ethyl acetate (600mL)and the two-phase mixture filtered and separated. The organic layer was washed with brine, dried over magnesium sulfate, filtered, and the solvent removed under reduced pressure. The residue was taken up in diethyl ether and treated with hydrogen chloride gas. The resulting slurry was filtered and the precipitate dried under vacuum to give the hydrochloride salt of the desired product as a white solid (3.25 g, 50%).¹H NMR (300 MHz, d₆-DMSO) δ 10.3(br s, 1H), 8.4(br s, 1H), 7.17 (t, J = 8.6Hz, 1H), 6.83 (dd, J = 2.2Hz, 8.2Hz, 2H), 2.14(s, 3H).

### b) 2-(3-Fluoro-4-methylphenyl)-5-methyl-2,4-dihydropyrazol-3-on:

Following the procedure of example 1d), except substituting the compound of 60a) for 3,4-dimethylphenylhydrazine, the title compound was prepared as a solid (1.6g, 76%). ¹H NMR (300 MHz, d₆-DMSO) δ 11.6(br s, 1H), 7.5 (m, 2H), 7.3 (t, J = 8.8Hz, 1H), 5.3 (s, 1H), 2.23(s, 3H), 2.1(s, 3H). MS(ES) m/z 207(M+H)⁺.

### c) 3-{N'-[1-(3-fluoro-4-methylphenyl)-3-methyl-5-oxo-1,5-dihydropyrazol-4-ylidene]hydrazino}-2-hydroxy-3'-tetrazol-5-ylbiphenyl

Following the procedure of example 1e), except substituting the compound of example 64b) for the compound of example 1d) and substituting 5-(2'-hydroxybiphenyl-3'-amino-3-yl)-1H-tetrazole for 4-amino-3'-hydrobiphenyl-3-carboxylic acid;, the title compound was prepared (0.07 g, 44%). ¹H NMR (300 MHz, d₆-DMSO) δ 13.7 (br s, 1H), 9.9 (br s, 1H), 8.26 (s, 1H), 8.09 (d, J = 7.4Hz, 1H), 7.7(m, 5H), 7.38 (t, J = 8.3Hz, 1H), 7.23 (m, 2H), 2.35(s, 3H), 2.24(s, 3H). MS(ES) m/z 471(M+H)⁺.

### Example 61

### 3'-{N'-[1-(3-fluoro-4-methylphenyl)-3-methyl-5-oxo-1,5-dihydropyrazol-4-ylidene]hydrazino}-2'-hydroxybiphenyl-3-carboxylic acid;

Following the procedure of example 60c), except substituting 3'-amino-2'-hydrobiphenyl 3-carboxylic acid; for 5-(2'-hydroxybiphenyl-3'-amino-3-yl)-1H-tetrazole, the title compound was prepared as a solid (0.09 g, 33%). ¹H NMR (300 MHz, d₆-DMSO) δ 13.7 (br s, 1H), 13.1 (br s, 1H), 9.9 (br s,1H), 8.13 (s, 1H), 7.97 (d, J = 7.8Hz, 1H), 7.8 (d, J = 7.8Hz, 1H), 7.72(m, 3H), 7.38 (t, J = 8.3Hz, 1H), 7.18 (m, 2H), 2.35(s, 3H), 2.24(s, 3H). MS(ES) m/z 447(M+H)⁺.

### Example 62

### 3'-{N'-[3-methyl-5-oxo-1-(4-trifluoromethylpyrimidin-2-yl)-1,5-dihydropyrazol-4-ylidene]hydrazinol-2'-hydroxybiphenyl-3-carboxylic acid;

### a) 3-methyl-1-(4-trifluoromethyl-2-pyrimidyl)-3-pyrazolin-5-one

Ethyl 3-oxobutanoate (0.745 g, 5.73 mmol) was added to a stirred mixture of 2-hydrazino-4-trifluoromethylpyrimidine (1.02 g, 5.73 mmol), sodium acetate (0.47 g, 5.73 mmol) and acetic acid; (15 mL), then the mixture heated under reflux for 18h. After cooling, acetic acid; was removed under reduced pressure and the residue partitioned between water and ethyl acetate. The organic phase was washed with water, saturated aqueous NaCl, dried (MgSO₄) and evaporated under reduced pressure. The residue was chromatographed (silica gel, ethyl acetate) to give an impure product, which was partitioned between aqueous K₂CO₃ and ethyl acetate. The aqueous phase was washed with ethyl acetate, then acid;ified to pH 1 with aqueous HCl and extracted with ethyl acetate. The extracts were dried (MgSO₄) and evaporated under reduced pressure to give the title compound (0.403 g; 29%) as a pale yellow solid. MS(ES) m/z 245 (M+H)⁺.

### b) 3'-{N'-[3-methyl-5-oxo-1-(4-trifluoromethyl-2-pyrimidyl)-1,5-dihydropyrazol-4-ylidene]hydrazino}-2'-hydroxybiphenyl-3-carboxylic acid;:

1M aq. sodium nitrite (0.45 mL, 0.45 mmol) was added dropwise to a stirred solution of 3'-amino-2'-hydroxybiphenyl-3-carboxylic acid; hydrochloride (0.100 g, 0.376 mmol) in 1M aq. hydrochloric acid; (1 mL)/ethanol (5 mL) at 5°C. The mixture was stirred 10 min. at room temperature and 3-methyl-1-(4-trifluoromethyl-2-pyrimidyl)-3-pyrazolin-5-one (0.092 g, 0.376 mmol) added, followed by sodium hydrogencarbonate until the pH was 8. The reaction was stirred for 18h, then partitioned between aq. hydrochloric acid; and ethyl acetate. The extracts were washed with aq. Na₂CO₃. The aqueous phase was washed with ethyl acetate, then acid;ified to pH 1 with aq. hydrochloric acid;. The solid was filtered, then purified by preparative hplc (ODS-silica, 10-90% acetonitrile/water-0. 1%TFA) to afford the title compound (0.035 g; 19%) as an orange solid. MS(ES) m/z 485 (M+H)⁺.

### Example 63

### 3'-N-tert-butoxycarbonylamino-3-{N'-[1-(3,4-Dimethylphenyl)-3-methyl-5-oxo-1,5-dihydropyrazol-4-ylidene]hydrazino}-2-hydroxybiphenyl

### a) 2'-hydroxy-3'-nitrobiphenyl-3-yl-carbamic acid; tert-butyl ester:

The compound from example 31b) (0.75 g, 3.3mmol), 3'-amino-3-nitrobiphenyl-2-ol, in methylene chloride (10 mL) was treated with tert-butyl dicarbonate (0.78g, 3.6mmol) at 0°C. The reaction mixture was warmed up to room temperature and stirred for 48hrs. 10% sodium hydroxy was added, after agitated for 10 min., 3N hydrochloride was added to neutralize the solution to pH=7. The layers were separated, the organic layer was washed with water, dried over MgSO4 and concentrated to a brown gum (0.77 g, 72 %). ¹H NMR (300MHz, d₆-DMSO) δ 8.13 (d, J =8.7Hz, 1H), 7.65 (m, 2H), 7.3 (m, 3H), 7.08(J = 8.7Hz, 1H), 6.58 (br s, 1H), 1.32(s, 6H), 1.28 (s, 3H).

### b) 3'-N-tert-butoxycarbonylamino-3-{N'-[1-(3,4-Dimethylphenyl)-3-methyl-5-oxo-1,5-dihydropyrazol-4-ylidene]hydrazino}-2-hydroxybiphenyl:

Follow the procedure of example 1c), except substituing 2'-hydroxy-3'-nitrobiphenyl-3-yl-carbamic acid; tert-butyl ester for the compound from example 1b), the crude product was abtained. The aboved crude product (0.1 g, 0.33 mmol) in 1M aqu. hydrochloric acid; (0.35 mL) was cooled to 5°C then treated dropwise with a solution of sodium nitrite (0.035 g; 0.5 mmol.) in water (5.0 mL). The yellow mixture was stirred at 5°C for a further 10 min. then treated in one portion with the compound from Example 1d) (0.067 g, 0.3mol.) followed by the portion-wise addition of sodium hydrogen carbonate and ethanol ensuring the final pH of the reaction mixture is approximately 7-8. The red solution was then stirred at room temperature for 24h.The mixture was filtered to give a red solid which was slurried in water and then acid;ified with concentrated hydrochloric acid;. Filtration afforded the title compound (0.12 g; 71%) as a red powder. MS(ES) m/z 514 (M+H)⁺.

### Example 64

### 3'-amino-3-{N'-[1-(3,4-dimethylphenyl)-3-methyl-5-oxo-1,5-dihydropyrazol-4-ylidene]hydrazino}-2-hydroxybiphenyl

The compound from example 63b) (0.03 g , 0.06 mmol) was treated with 0.45ml trifluoroacetic acid; in methylene chloride (5 mL). After stirring at room temperature for 2 hrs, the reaction mixture was concentrated and washed with diethyl ether to give the title compound as a red powder(0.02 g, 65%). MS(ES) m/z 414 (M+H)⁺.

### Example 65

### 3-{N'-[1-(3-fluoropheny)-3-methyl-5-oxo-1,5-dihydropyrazol-4-ylidene]hydrazino}-2-hydroxy-3'-tetrazol-5-ylbiphenyl

### a) 2-(3-fluorophenyl)-5-methyl-2,4-dihydropyrazol-3-one:

Following the procedure of example 1d), except substituting 3-fluorophenylhydrazine hydrochloride for 3,4-dimethylphenylhydrazine hydrochloride, the title compound was prepared as solid (4.6 g, 73%). MS(ES) m/z 193 (M+H)⁺.

### b) 3-{N'-[1-(3-fluorophenyl)-3-methyl-5-oxo-1,5-dihydropyrazol-4-ylidene]hydrazino}-2-hydroxy-3'-tetrazol-5-ylbiphenyl:

Follow the procedure of example 1e), except 2-(3-fluorophenyl)-5-methyl-2,4-dihydropyrazol-3-one for the compound of 1d) and 5-(2'-hydroxybiphenyl-3'-amino-3-yl)-1H-tetrazole for the compound 1c), the title compound was obtained as a solid (0.03 g, 18%). ¹H NMR (300 MHz, d₆-DMSO) δ 13.7 (br s, 1H), 8.2 (s, 1H), 8.08 (d, J = 8.5Hz, 1H), 7.8 (m, 5H), 7.53 (m, 1H), 7.21 (m, 2H), 7.08(t, J = 7.8Hz, 1H), 2.37(s, 3H). MS(ES) m/z 457(M+H)⁺.

### Example 66

### 3'-{N'-[1-(3-fluorophenyl)-3-methyl-5-oxo-1,5-dihydropyrazol-4-ylidene]hydrazino}-2'-hydroxybiphenyl-3-carboxylic acid;

Follow the procedure of example 1e), except substituting 3'-amino-2'-hydroxybiphenyl-3-carboxylic acid; for 4'-amino-2'-hydroxybiphenyl-3-carboxylic acid; and 2-(3-fluorophenyl)-5-methyl-2,4-dihydropyrazol-3-one for the compound of example 1d), the title compound was obtained as a solid (0.07 g, 45%). ¹H NMR (300 MHz, d₆-DMSO) δ 13.7 (br s, 1H), 9.7 (s, 1H), 8.14 (s,I H), 7.97 (d, J = 7.7Hz, 1H), 7.8 (m, 4H), 7.64 (t, J = 7.7Hz, 1H), 7.52 (m, 1H), 7.2(m, 2H), 7.07 (t, J = 7.2, 1H), 2.35(s, 3H). MS(ES) m/z 433(M+H)⁺.

### Example 67

### 3{N'-[3-methyl-5-oxo-1-(2,3,4,5,6-pentafluorophenyl)-1,5-dihydropyrazol-4-ylidene]hydrazino}1-2-hydroxy-3'-tetrazol-5-ylbiphenyl

### a) methyl-pentafluorophenyl-2,4-dihydropyrazol-3-one:

Following the procedure of example 1d), except substituting pentafluorophenylhydrazine hydrochloride for 3,4-dimethylphenylhydrazine hydrochloride, the title compound was prepared as solid (1.7 g, 25%). MS(ES) m/z 265 (M+H)⁺.

### b) 3-{N'-[3-methyl-5-oxo-1-(2,3,4,5,6-pentafluorophenyl),1,5-dihydropyrazol-4-ylidene]hydrazino}-2-hydroxy-3'-tetrazol-5-ylbiphenyl:

Follow the procedure of example 1e), except substituting methyl pentafluorophenyl-2,4-dihydropyrazol-3-one for the compound of 1d) and 5-(2'-hydroxybiphenyl-3'-amino-3-yl)-1H-tetrazole for the compound 1c), the title compound was obtained as a solid (0.04 g, 23%). ¹H NMR (300 MHz, d₆-DMSO) δ 9.9(s, 1H), 8.2 (s, 1H), 8.07 (d, J = 7.3Hz, 1H), 7.76 (m, 3H), 7.23 (m, 2H), 2.34(s, 3H). MS(ES) m/z 529(M+H)⁺.

### Example 68

### 3'-{N'-[3-methyl-5-oxo-1-(2,3,4,5,6-pentafluorophenyl)-1,5-dihydropyrazol-4-ylidene]hydrazino}-2'-hydroxybiphenyl-3-carboxylic acid;

Follow the procedure of example 1e), except substituting methyl pentafluorophenyl-2,4-dihydropyrazol-3-one for the compound of 1d) and 3'-amino-2'-hydroxybiphenyl-3-carboxylic acid; for the compound 1c), the title compound was obtained as a solid (0.1 g, 51%). ¹H NMR (300 MHz, d₆-DMSO) δ 9.8(s, 1H), 8.12(s, 1H), 7.98 (d, J = 7.9Hz, 1H), 7.79 (d, J = 7.7Hz, 1H), 7.76 (d, J = 7.5Hz, 1H), 7.61 (t, J = 7.8Hz, 1H), 7.2(m, 2H), 2.34(s, 3H). MS(ES) m/z 505(M+H)⁺.

### Example 69

### 3'-{N'-[1-(3,4-difluorophenyl)-3-methyl-5-oxo-1,5-dihydropyrazol-4-ylidene]hydrazino}-2'-hydroxybiphenyl-3 -carboxylic acid;

### a) 2-(3,4-difluorophenyl-5-methyl-2,4-dihydropyrazol-3-one:

Following the procedure of example 1d), except substituting 3,4-difluorophenylhydrazine hydrochloride for 3,4-dimethylphenylhydrazine hydrochloride, the title compound was prepared as solid (0.82 g, 70%). MS(ES) m/z 211 (M+H)⁺.

### b) 3'-{N'-[1-(3,4-difluorophenyl)-3-methyl-5-oxo-1,5-dihydropyrazol-4-ylidene]hydrazino}-2'-hydroxybiphenyl-3-carboxylic acid;:

Follow the procedure of example 1e), except substituting 2-(3,4-difluorophenyl-5-methyl-2,4-dihydropyrazol-3-one for the compound of 1d) and 3'-amino-2'-hydroxybiphenyl-3-carboxylic acid; for the compound 1c), the title compound was obtained as a solid (0.11 g, 57%). ¹H NMR (300 MHz, d₆-DMSO) δ 13.6(br s, 1H), 13.1(br s, 1H), 9.9(br s, 1H), 8.1 (s, 1H), 7.96(d, J = 7.8Hz, I H), 7.94 (dd, J = 7.5Hz, 2.4Hz, 1H), 7.76 (m, 3H), 7.62 (t, J = 7.8 Hz, 1H), 7.55 (m, 1H), 7.19(m, 2H), 2.34(s, 3H). MS(ES) m/z 451 (M+H)⁺.

### Example 70

### 3'-{N'-[1-(3,4-dimethylphenyl)-3-methoxymethyl-5-oxo-1,5-dihydropyrazol-4-ylidene]hydrazino}-2'-hydroxybiphenyl-3-carboxylic acid;

### a) 2-(3,4-dimethylphenyl)-5-methoxymethyl-2,4-dihydropyrazol-3-one:

Following the procedure of example 1d), except substituting methyl 4-methoxyacetoacetate for ethyl acetoacetate, the title compound was prepared as a solid (2.7 g, 58%). MS(ES) m/z 233 (M+H)⁺.

### b) 3'-{N'-[1-(3,4-dimethylphenyl)-3-methoxymethyl-5-oxo-1,5-dihydropyrazol-4-ylidene]hydrazino}-2'-hydroxybiphenyl-3-carboxylic acid;:

Follow the procedure of example 1e), except substituting 2-(3,4-dimethylphenyl)-5-methoxymethyl-2,4-dihydropyrazol-3-one for the compound of 1d) and 3'-amino-2'-hydroxybiphenyl-3-carboxylic acid; for the compound 1c), the title compound was obtained as a solid (0.189 g, 64%).¹H NMR (300 MHz, d₆-DMSO)δ 13.9(br s, 1H), 13.1(br s,1H), 9.9(br s, 1H), 8.1 (s, 1 H), 7.96(d, J = 7.7Hz, 1H), 7.81 (d, J = 6.5Hz,, 1H), 7.71 (s, 1H), 7.65 (m, 3H), 7.2 (m, 3H), 4.5 (s, 2H), 3.4 (s, 3H), 2.28(s, 3H), 2.24(s, 3H). MS(ES) m/z 473(M+H)⁺.

### Example 71

### 3-{N'-[1-(3,4-dimethylphenyl)-3-methoxymethyl-5-oxo-1,5-dihydropyrazol-4-ylidene]hydrazinol-2-hydroxy-3'-tetrazol-5-ylbiphenyl

Follow the procedure of example 1e), except substituting 2-(3,4-dimethylphenyl)-5-methoxymethyl-2,4-dihydropyrazol-3-one for the compound of 1d) and 5-(2'-hydroxybiphenyl-3'-amino-3-yl)-1H-tetrazole for the compound 1c), the title compound was obtained as a solid (0.126 g, 67%). ¹H NMR (300 MHz, d₆-DMSO) δ 13.9(br s, 1H), 9.9(s,1H), 8.25 (s, 1H), 8.09(d, J = 7.4Hz, 1H), 7.78 (m, 3H), 7.71 (s, 1H), 7.65 (dd, J =2.1Hz and 7.2Hz, 1H), 7.25 (m, 3H), 4.5 (s, 2H), 3.4 (s, 3H), 2.28(s, 3H), 2.24(s, 3H). MS(ES) m/z 497(M+H)⁺.

### Example 72

### 3-{N'-[1-(3,4-difluorophenyl)-3-methyl-5-oxo-1,5-dihydropyrazol-4-ylidene]hydrazino}-2-hydroxy-3'-tetrazol-5-ylbiphenyl

Follow the procedure of example 1e), except 2-(3,4-difluorophenyl-5-methyl-2,4-dihydropyrazol-3-one for the compound of 1d) and 5-(2'-hydroxybiphenyl-3'-amino-3-yl)-1H-tetrazole for the compound 1c), the title compound was obtained as a solid (0.095 g, 56%). ¹H NMR (300 MHz, d₆-DMSO) δ 8.25 (s, I H), 8. 1(d, J = 7.3Hz, 1H), 7.95 (m, I H), 7.78 (m, 4H), 7.58 (m, 1H), 7.22(m, 2H), 2.37(s, 3H). MS(ES) m/z 475(M+H)⁺.

### Example 73

### 3'-{N'-[1-(3,4-dimethylphenyl)-5-oxo-3-tritluoromethyl-1,5-dihydropyrazol-4-ylidene]hydrazino}-2'-hydroxybiphenyl-3-carboxylic acid;

### a) 3,4-dimethylphenyltrifluoromethyl-2,4-dihydropyrazol-3-one:

Following the procedure of example 1d), except substituting 4,4,4-trifluoro-3-oxo-butyric acid; for 3,4-dimethylphenylhydrazine hydrochloride, the title compound was prepared as solid (4.0 g, 52%). MS(ES) m/z 257 (M+H)⁺.

### b) 3'-{N'-[1-(3,4-dimethylphenyl)-5-oxo-3-trifluoromethyl-1,5-dihydropyrazol-4-ylidene]hydrazino}-2'-hydroxybiphenyl-3-carboxylic acid;:

Follow the procedure of example 1e), except substituting 3,4-dimethylphenyltrifluoromethyl-2,4-dihydropyrazol-3-one for the compound of 1d) and 3'-amino-2'-hydroxybiphenyl-3-carboxylic acid; for the compound 1c), the title compound was obtained as a solid (0.012 g, 4%). ¹H NMR (300 MHz, d₆-DMSO) δ 13.1(br s, 1H), 10.1(br s,1H), 8.1 (s, 1H), 7.98(d, J = 7.6Hz, 1H), 7.82 (d, J = 7.7Hz, 1H), 7.6 (m, 3H), 7.27 (m, 2H), 7.55 (m, 1H), 7.2(t, J = 7.8Hz, 1H), 2.30(s, 3H), 2.26(s, 3H). MS(ES) m/z 497(M+H)⁺

### Example 74

### 3'-{N'-[1-(3,4-dimethylphenyl)-3-methyl-5-oxo-1,5-dihydropyrazol-4-ylidene]hydrazino}-6-fluoro-2'-hydroxybiphenyl-3-carboxylic acid;

### a) 5'-chloro-6-fluoro-2'-methoxybiphenyl-3-carboxylic acid;:

Follow the procedure of example 1b), except substituting 2-methoxy-5-chlorophenylboronic acid; for 4-carboxyphenylboronic acid; and 3-bromo-4-fluorobenzoic acid;, the title compound was prepared as a solid (0.2 g, 16%). MS(ES) m/z 281(M+H)⁺, 561(2M+H)⁺.

### b) 5'-chloro-6-fluoro-2'-hydroxybiphenyl-3-carboxylic acid;:

Follow the procedure of example 3d), except substituting 5'-chloro-6-fluoro-2'-methoxybiphenyl-3-carboxylic acid; for the compound from example 3c), the title compound was prepared as a solid (1.1 g, 59%). MS(ES) m/z 267(M+H)⁺.

### c) 5'-chloro-6-fluoro-2'-hydroxy-3'-nitrobiphenyl-3-carboxylic acid;:

Follow the procedure of example 7c), except substituting 5'-chloro-6-fluoro-2'-hydroxy-3'-nitrobiphenyl-3-carboxylic acid; for 6-(5-chloro-2-hydroxyphenyl)-pyridine-2-carboxylic acid;, the title compound was prepared as a solid (1 g, 86%). MS(ES) m/z 312(M+H)⁺, 623(2M+H)⁺. ¹H NMR (300 MHz, d₆-DMSO) δ 10.8(s, 1H), 8.13(d, J = 2.7Hz, 1H), 8.09(m, 1H), 8.0 (dd, J = 7.1Hz, 2.2Hz, 1H), 7.82(d, J = 2.7Hz, 1H), 7.48 (t, J = 9.0, 1H).

### d) 5'-chloro-6-fluoro-2'-hydroxy-3'-aminobiphenyl-3-carboxylic acid;:

Following the procedure of Example 1c), except substituting 5'-chloro-6-fluoro-2'-hydroxy-3'-nitrobiphenyl-3-carboxylic acid; for the compound of 1b), the crude product was isolated. MS(ES) m/z 248(M+H)⁺

### e) 3'-{N'-[1-(3,4-dimethylphenyl)-3-methyl-5-oxo-1,5-dihydropyrazol-4-ylidene]hydrazino}-6-fluoro-2'-hydroxybiphenyl-3-carboxylic acid;:

Follow the procedure of example 1e), except substituting 5'-chloro-6-fluoro-2'-hydroxy-3'-aminobiphenyl-3-carboxylic acid; for the compound from example 1c), the title compound was obtained as a solid (0.25 g, 42%). ¹H NMR (300 MHz, d₆-DMSO) δ 13.7(br s, 1H), 13.1(br s,1H), 9.8(s, 1H), 8.08 (m, 2H), 7.78(dd, J = 7.0Hz, 2.8Hz, 1H), 7.71 (s, 1H), 7.65 (dd, J = 2. 1Hz, 8.1Hz, 1H), 7.46 (t, J = 8.8Hz, 1H), 7.21 (d, J = 8.3Hz, 1H), 7.14(m, 2H), 2.34(s, 3H), 2.27(s, 3H), 2.23(s, 3H). MS(ES) m/z 461(M+H)⁺.

### Example 75

### 3'-{N'-[1-(3,4-dimethyphenyl)-5-oxo-3-propyl-1,5-dihydropyrazol-4-ylidene]hydrazino}-2'-hydroxybiphenyl-3-carboxylic acid:

### a) 2-(3,4-dimethylphenyl)-5-propyl-2,4-dihydropyrazol-3-one:

Following the procedure of example 1d), except substituting ethyl butyrylacetate for ethyl acetoacetate, the title compound was prepared as a solid (3.0 g, 64%). ¹H NMR (300 MHz, CDCl3) δ 7.61 (s, 1H), 7.56(dd, J = 2.3Hz and 8.2Hz, 1H), 7.13(d, J = 8.1Hz, 1H), 3.39(s, 2H), 2.47 (t, J = 7.5Hz, 2H), 2.28 (s, 3H), 2.24(s, 3H), 1.64(m, 2H), 0.99(t, J = 7.4Hz, 3H).

### b) 3'-{N'-[1-(3,4-dimethylphenyl)-5-oxo-3-propyl-1,5-dihydropyrazol-4-ylidene]hydrazino}-2'-hydroxybiphenyl-3-carboxylic acid;:

Follow the procedure of example 1e), except substituting 2-(3,4-dimethylphenyl)-5-propyl-2,4-dihydropyrazol-3-one for the compound from example 1d) and 3'-amino-2'-hydroxybiphenyl-3-carboxylic acid; for the compound from example 1c), the title compound was obtained as a solid (0.145 g, 93%). ¹H NMR (300 MHz, d₆-DMSO) δ 13.7 (br s, 1H), 9.7 (s, 1H), 8.14 (s, 1H), 7.97 (d, J = 7.8Hz, 1H), 7.8 (d, J = 7.8Hz, 1H), 7.62 (m, 4H), 7.62 (m, 3H), 2.7 (t, J = 7.5Hz, 2H), 2.28 (s, 3H), 2.24(s, 3H), 1.8 (m, 2H), 1.1 (t, J = 7.4Hz, 3H).

### Example 76

### 3-{N'-[1-(3,4-dimethylphenyl)-5-oxo-3-propyl-1,5-dihydropyrazol-4-ylidene]hydrazino}-2-hydroxy-3'-tetrazol-5-ylbiphenyl

Follow the procedure of example 1e), except substituting 2-(3,4-dimethylphenyl)-5-propyl-2,4-dihydropyrazol-3-one for the compound from example 1d) and 5-(2'-hydroxybiphenyl-3'-amino-3-yl)-1H-tetrazole for the compound from example 1c), the title compound was obtained as a solid (0.06 g, 36%). ¹H NMR (300 MHz, d₆-DMSO) δ 8.27 (s, 1H), 8.08 (d, J = 7.4Hz, 1H), 7.78 (m, 3H), 7.65 (d, J = 8.2Hz, 1H), 7.18 (m, 4H), 2.7 (t, J = 7.5Hz, 2H), 2.27 (s, 3H), 2.23(s, 3H), 1.8 (m, 2H), 1.03 (t, J = 7.4Hz, 3H). MS(ES) m/z 495(M+H)⁺.

### Example 77

### 3'-{N'-[1-(3,4-dimethyphenyl)-3-(1-methyl-1H-pyrrol-3-yl)-5-oxo-1,5-dihydropyrazol-4-ylidene]hydrazino}-2'-hydroxybiphenyl-3-carboxylic acid;

### a) 3-(1-methyl-1H-pyrrol-3-yl)-3-oxo-propionic acid; ethyl ester:

To a suspension of 60% sodium hydride (5.1 g, 128 mmol) and diethyl carbonate (10.3 mL, 85 mmol) in benzene was added a solution of 3-acetyl-1-methylpyrrole (5.25 g, 43 mmol) in benzene dropwise over 45 min. The mixture was then heated for 30 min., the mixture was thickened to a paste towards the end of reaction. The mixture was cooled, diluted with ethyl acetate and carefully acid;ified with acetic acid;. Water was added, the layers separated and the aqueous was extracted with ethyl acetate (2 times). Combined organic was washed with water, dried and concentrated under reduced precessure to give crude product. The crude was further purified through column chromatography on SiO2 (50% ethyl acetate/hexane) to give the title compound (7.4 g, 89%). ¹H NMR (300 MHz, CDC13) δ 7.26(s, 1H), 6.5(s, 2H), 4.1 (m, 2H), 3.68 (s, 2H), 3.64(s, 3H), 1.2 (t, 7.2Hz, 3H).

### b) 2-(3,4-dimethylphenyl)-5-(1-methyl-1H-pyrrol-3-yl)-2,4-dihydropyrazol-3-one:

Following the procedure of example 1d), except substituting 3-(1-methyl-1H-pyrrol-3-yl)-3-oxo-propionic acid; ethyl ester: for ethyl acetoacetate, the title compound was prepared as a solid (3.0 g, 62 %). MS(ES) m/z 268(M+H)⁺

### c) 3'-{N'-[1-(3,4-dimethylphenyl)-3-(1-methyl-1H-pyrrol-3-yl)-5-oxo-1,5-dihydropyrazol-4-ylidene]hydrazino}-2'-hydroxybiphenyl-3-carboxylic acid;:

Follow the procedure of example 1e), except substituting 2-(3,4-dimethylphenyl)-5-propyl-2,4-dihydropyrazol-3-one for the compound from example 1d) and 3'-amino-2'-hydroxybiphenyl-3-carboxylic acid; for the compound from example 1c), the title compound was obtained as a solid (0.099g, 43%). ¹H NMR (300 MHz, d₆-DMSO) δ 14.0 (br s, 1H), 9.6 (s, 1H), 8.15 (s, 1H), 7.95 (d, J = 8.4Hz, 1H), 7.82 (m, 3H), 7.72 (dd, J = 2.1Hz and 8.1Hz,1H), 7.63 (m, 2H), 7.23 (m, 3H), 6.89(t, J = 2.5Hz, 1H), 6.68(d, J = 2.8Hz, 1H), 3.89(s, 3H), 2.29 (s, 3H), 2.24(s, 3H). MS(ES) m/z508(M+H)⁺.

### Example 78

### 3-{N'-[1-(3,4-dimethylphenyl)-3-(1-methyl-1H-pyrrol-3-yl)-5-oxo-1,5-dihydropyrazol-4-ylidene]hydrazino}-2-hydroxy-3'-tetrazol-5-ylbiphenyl

Follow the procedure of example 1e), except substituting 2-(3,4-dimethylphenyl)-5-(1-methyl-1H-pyrrol-3-yl)-2,4-dihydropyrazol-3-one for the compound from example 1d) and 5-(2'-hydroxybiphenyl-3'-amino-3-yl)-1H-tetrazole for the compound from example 1c), the title compound was obtained as a solid (0.078 g, 35%). ¹H NMR (300 MHz, d₆-DMSO) δ 14.0 (br s, 1H), 9.7 (s, 1H), 8.26 (s, 1H), 8.1 (d, J = 7.5Hz, H), 7.85 (t, J = 5.0Hz, 1H), 7.82 (s, 1H), 7.75 (m, 3H), 7.62 (s, 1H), 7.24(s, 3H), 6.9(s, 1H), 6.7(s, 1H), 3.89(s, 3H), 2.29 (s, 3H), 2.24(s, 3H). MS(ES) m/z 532(M+H)⁺.

### Example 79

### 3'-{N'-[1-(3,4-dimethylphenyl)-3-furan-2-yl-5-oxo-1,5-dihydropyrazol-4-ylidene]hydrazino}-2'-hydroxybiphenyl-3-carboxylic acid;

### a) 2-(3,4-dimethylphenyl)-5-furan-2-yl-2,4-dihydropyrazol-3-one:

Following the procedure of example 1d), except substituting ethyl-β-oxo-3-furanpropionate for ethyl acetoacetate, the title compound was prepared as a solid (2.0 g, 48%).¹H NMR (300 MHz, CDCl3) δ 7.74(s, 1H), 7.7 (s, 1H), 7.6 (d, J = 7.8Hz, 1H), 7.45 (s, 1H), 7.2 (d, J = 7.8Hz, 1H), 6.8(s, 1H), 3.68 (s, 2H), 2.3(s, 3H), 2.2 (s, 1H).

### b) 3'- {N'-[1-(3,4-dimethylphenyl)-3-furan-2-yl-5-oxo-1,5-dihydropyrazol-4-ylidene]hydrazino}-2'-hydroxybiphenyl-3-carboxylic acid;

Follow the procedure of example 1e), except substituting 2-(3,4-dimethylphenyl)-5-furan-2-yl-2,4-dihydropyrazol-3-one for the compound from example 1d) and 3'-amino-2'-hydroxybiphenyl-3-carboxylic acid; for the compound from example 1c), the title compound was obtained as a solid (0.22 g, 11%). ¹H NMR (300 MHz, d₆-DMSO) δ 14.0 (s, 1H), 9.8 (s, I H), 8.55 (s, 1H), 8.15(s,1H), 7.95 (m, 2H), 7.82 (m, 2H), 7.71 (dd, J = 2.0Hz and 8.0Hz, 1H), 7.63 (t, J = 7.7Hz, 1H), 7.23 (m, 3H), 7.1(s, 1H), 2.3 (s, 3H), 2.2(s, 3H). MS(ES) m/z495(M+H)⁺.

### Example 80

### 3-{N'-[1-(3,4-dimethylphenyl)-3-furan-2-yl-5-oxo-1,5-dihydropyrazol-4-ylidene]hydrazino}-2-hydroxy-3'-tetrazol-5-ylbiphenyl

Follow the procedure of example 1e), except substituting 2-(3,4-dimethylphenyl)-5-furan-2-yl-2,4-dihydropyrazol-3-one for the compound from example 1d) and 5-(2'-hydroxybiphenyl-3'-amino-3-yl)-1H-tetrazole for the compound from example 1c), the title compound was obtained as a solid (0.022 g, 11%). ¹H NMR (300 MHz, d₆-DMSO) δ 14.0 (s, 1H), 9.8 (s, 1H), 8.56 (s, 1H), 8.26(s, 1H), 8.1 (d, J = 7.5Hz, 1H), 7.96 (dd, J = 2.0Hz and 7.7Hz, 1H), 7.9 (s, 1H), 7.75 (m, 4H), 7.25 (m, 3H), 7.1(s, 1H), 2.3 (s, 3H), 2.2(s, 3H). MS(ES) m/z495(M+H)⁺.

### Example 81

### N-(2'-hydroxy-3'-{N'-[3-methyl-5-oxo-1-(4-trifluoromethyl-phenyl)-1,5-dihydro-pyrazol-4-ylidene]hydrazino}biphenyl-3-yl)-1,1,1-trifluoromethanesulfonamide

### a) 1,1,1-trifluoro-N-(2'-hydroxy-3'-aminobiphenyl-3-yl)-methanesulfonamide:

Follow the procedure of example 1c) except substituting 1,1,1-trifluoro-N-(2'-hydroxy-3'-nitrobiphenyl-3-yl)-methanesulfonamide for the compound of example 1b), the title compound was prepared (100%). H NMR (300 MHz, CDC13) δ 7.4(m, 3H), 7.17 (d, J = 7.5Hz, 1H), 6.75 (d, J = 7.8Hz, 2H), 6.55 (t, J = 7.5Hz, 1H).

### b) N-(2'-hydroxy-3'-{N'-[3-methyl-5-oxo-1-(4-trifluoromethyl-phenyl)-1,5-dihydropyrazol-4-ylidene]hydrazino}biphenyl-3-yl)-1,1,1-trifluoromethanesulfonamide:

Follow the procedure of example 1e), except substituting 5-methyl-2-(4-trifluoromethylphenyl)-2,4-dihydropyrazol-3-one for the compound from example 1d) and 1,1,1-trifluoro-N-(2'-hydroxy-3'-aminobiphenyl-3-yl)-methanesulfonamide for the compound from example 1c), the title compound was obtained as a solid (0.041 g, 18%). ¹H NMR (300 MHz, d₆-DMSO) δ 13.7 (s, 1H), 9.8 (s, 1H), 8.19 (d, J = 8.6Hz, 2H), 7.8(d, J = 9.0Hz, 2H), 7.7 (d, J = 2.8Hz and 6.9Hz, 1H), 7.5 (m, 3H), 7.3 (d, J = 6.0Hz, 1H), 7.15 (m, 2H), 2.35 (s, 3H). MS(ES) m/z586(M+H)⁺.

### Example 82

### N-(2'-hydroxy-3'-{N'-[1-(3-fluoro-4-methylphenyl)-3-methyl-5-oxo-1,5-dihydro-pyrazol-4-ylidene]hydrazino}biphenyl-3-yl)-1,1,1-trifluoromethanesulfonamide

Follow the procedure of example 1e), except substituting 5-methyl-2-(4-trifluoromethylphenyl)-2,4-dihydropyrazol-3-one for the compound from example 1d) and 2-(3-fluoro-4-methylphenyl)-5-methyl-2,4-dihydropyrazol-3-one for the compound from example 1c), the title compound was obtained as a solid (0.038 g, 17%). ¹H NMR (300 MHz, d₆-DMSO) δ 13.7 (s, 1H), 9.8 (s, 1H), 7.7 (m, 3H), 7.54(t, d = 7.8Hz, 1H), 7.5 (d, J = 7.8Hz, 1H), 7.37 (t, J = 8.3Hz, 1H), 7.3 (d, J = 6.0Hz, 1H), 7.15 (m, 2H), 2.35 (s, 3H), 2.3 (s, 3H). MS(ES) m/z550(M+H)⁺.

### Example 83

### N-(2'-hydroxy-3'-{N'-[1-(4-fluoro-3-methylphenyl)-3-methyl-5-oxo-1,5-dihydro-pyrazol-4-ylidene]hydrazino}biphenyl-3-yl)-1,1,1-trifluoromethanesulfonamide

Follow the procedure of example 1e), except substituting 5-methyl-2-(4-trifluoromethylphenyl)-2,4-dihydropyrazol-3-one for the compound from example 1d) and 2-(4-fluoro-3-methylphenyl)-5-methyl-2,4-dihydropyrazol-3-one for the compound from example 1c), the title compound was obtained as a solid (0.042 g, 18%). ¹H NMR (300 MHz, d₆-DMSO) δ 13.7 (s. 1H), 9.8 (s, 1H), 7.83 (dd, J =1.8Hz and 7.5Hz, 1H), 7.75(m, 1H), 7.73(dd, J =2.0 and 7.8Hz, 1H), 7.53(t, d = 7.8Hz, 1H), 7.45 (m, 2H), 7.2 (m, 5H), 2.35 (s, 3H), 2.3 (s, 3H). MS(ES) m/z550(M+H)⁺.

### Example 84

### N-(2'-hydroxy-3'-{N'-[1-(3,4-difluorophenyl)-3-methyl-5-oxo- 1,5-dihydro-pyrazol-4-ylidene]hydrazino}biphenyl-3-yl)-1,1,1-trifluoromethanesulfonamide

Follow the procedure of example 1e), except substituting 5-methyl-2-(4-trifluoromethylphenyl)-2,4-dihydropyrazol-3-one for the compound from example 1d) and 2-(3,4-difluorophenyl)-5-methyl-2,4-dihydropyrazol-3-one for the compound from example 1c), the title compound was obtained as a solid (0.045 g, 20%). ¹H NMR (300 MHz, d₆-DMSO) δ 13.7 (s,1H), 9.8 (s. 1H). 7.98 (m, 1H), 7.78(m, I H), 7.73(dd, J =2.7 and 7.0Hz, 1H), 7.53(m, 4H), 7.3 (d, J = 5.6Hz, 1H), 7.18 (m, 2H), 2.35 (s, 3H). MS(ES) m/z554(M+H)⁺.

### Example 85

### N-(3'-{N'-[1-(3,4-dimethylphenyl)-3-methyl-5-oxo-1,5-dihydropyrazol-4-ylidene]hydrazino}-2'-hydroxybiphenyl-3-yl)guanidine

3'-amino-3-{N'-[1-(3,4-dimethylphenyl)-3-methyl-5-oxo-1,5-dihydropyrazol-4-ylidene]hydrazino}-2-hydroxybiphenyl (0.065 g, 0.16mmol) in methylene chloride (10 mL) was treated with 1,3-di-Boc-2-methyl isothiourea(0.055 g, 0.19mmol). The reaction mixture was stirred at room temperature for 18 hrs. Then, 1.5ml trifluoroacetic acid; in 20 ml methylene chloride was added, the reaction mixture was stirred for another 18hrs. After concentration, the resulting gum was washed with diethyl ether to give the title as a red powder (0.03 g, 58%).MS(ES) m/z456(M+H)⁺.

### Example 86

### 3'-{N'-[1-(3,4-dimethylphenyl)-3-ethyl-5-oxo-1,5-dihydropyrazol-4-ylidene]hydrazino}-2'-hydroxybiphenyl-3-carboxylic acid;

### a) 2-(3,4-dimethylphenyl)-5-ethyl-2,4-dihydropyrazol-3-one:

Following the procedure of example 1d), except substituting ethyl propionyl acetate for ethyl acetoacetate, the title compound was prepared as a solid (3.4 g, 46%). H NMR (300 MHz, CDCl3) δ 7.6(s, 1H), 7.56 (dd, J = 2.2Hz and 8.4Hz, 1H), 7.1 (d, J = 8.4Hz, 1H), 3.4 (s, 2H), 2.5(m, 2H), 2.28 (s, 3H), 2.24(s, 3H), 1.2(t, J = 7.5Hz, 3H).

### b) 3'-{N'-[1-(3,4-dimethylphenyl)-3-ethyl-5-oxo-1,5-dihydropyrazol-4-ylidene]hydrazino}-2'-hydroxybiphenyl-3-carboxylic acid;:

Follow the procedure of example 1e), except substituting 2-(3,4-dimethylphenyl)-5-ethyl-2,4-dihydropyrazol-3-one for the compound from example 1d) and 3'-amino-2'-hydroxybiphenyl-3-carboxylic acid; for the compound from example 1c), the title compound was obtained as a solid (0.035 g, 19%). ¹H NMR (300 MHz, d₆-DMSO) δ 13.8 (s, 1H), 9.7 (s,1H), 8.14 (s, 1H), 7.98 (d, J = 7.7Hz, 1H), 7.83 (d, J = 7.7Hz, 1H), 7.65 (m, 5H), 7.1 (m, 3H), 2.5(m, 2H), 2.27 (s, 3H), 2.23(s, 3H), 1.3 (t, J = 7.5Hz, 3H). MS(ES) m/z 457(M+H)⁺.

### Example 87

### 3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl

Follow the procedure of example 1e), except substituting 2-(3,4-dimethylphenyl)-5-ethyl-2,4-dihydropyrazol-3-one for the compound from example 1d) and 5-(2'-hydroxybiphenyl-3'-amino-3-yl)-1H-tetrazole for the compound from example 1c), the title compound was obtained as a solid (0.055 g, 29%). ¹H NMR (300 MHz, d₆-DMSO) δ 13.8 (s, 1H), 9.8 (s, 1H), 8.25 (s, 1H), 8.09(d, J = 7.5Hz, 1H), 7.76 (m, 4H), 7.65 (d, J = 7.2Hz, 1H), 7.22 (m, 3H), 2.5(m, 2H), 2.28 (s, 3H), 2.24(s, 3H), 1.3 (t, J = 7.5Hz, 3H).

### Example 88

### 3'-{N'-[1-(3,4-dimethylphenyl)-5-oxo-3-thien-2-yl-1,5-dihydropyrazol-4-ylidene]hydrazino}-2'-hydroxybiphenyl-3-carboxylic acid;

### a) 3-oxo-3-thiophen-2-yl-propionic acid; ethyl ester:

Follow the procedure of example 77a), except substituting 2-acetyl thiophene for 3-acetyl-1-methylpyrrole, the title compound was prepared as an oil (7 g, 64%). ¹H NMR (300 MHz, CDCl3) δ 7.75(d, J = 3.8Hz,I H), 7.69(d, J = 4.9Hz, 1H), 7.1(t, J = 4.9Hz, 1H), 4.2 (m , 2H), 3.9 (s, 2H), 1.2 (t, J = 7.2Hz, 3H).

### b) 2-(3,4-dimethylphenyl-5-thiophen-2-yl-2,4-dihydropyrazol-3-one:

Following the procedure of example 1d), except substituting 3-oxo-3-thiophen-2-yl-propionic acid; ethyl ester for ethyl acetoacetate, the title compound was prepared as a solid (3.4 g, 48%). ¹H NMR (300 MHz, CDCl3) δ 7.52 (s, 1H), 7.46 (m, 3H), 7.2(d, J = 8.1Hz, 1H), 7.1 (t, J = 3.8Hz, 1H), 5.9 (s. 1H), 2.28(s, 3H), 2.25(s, 3H).

### c) 3'-{N'-[1-(3,4-dimethylphenyl)-5-oxo-3-thien-2-yl-1,5-dihydropyrazol-4-ylidene]hydrazino}-2'-hydroxybiphenyl-3-carboxylic acid;:

Follow the procedure of example 1e), except substituting 2-(3,4-dimethylphenyl-5-thiophen-2-yl-2,4-dihydropyrazol-3-one for the compound from example 1d) and 3'-amino-2'-hydroxybiphenyl-3-carboxylic acid; for the compound from example 1c), the title compound was obtained as a solid (0.09 g, 44%). ¹H NMR (300 MHz, d₆-DMSO) δ 14.0 (s, 1H), 9.8 (s, 1H), 8.14 (s, 1H), 8.01 (d, J = 3.7Hz, 1H), 7.97(d, J = 7.8Hz, 1H), 7.82 (m, 4H), 7.65 (m, 2H), 7.25(m, 4H), 2.29 (s, 3H), 2.24(s, 3H). MS(ES) m/z 511(M+H)⁺

### Example 89

### 3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;

### a) 3-cyclopropyl-3-oxo-propionic acid; ethyl ester:

Follow the procedure of example 77a), except substituting cyclopropylmethylketone for 3-acetyl-1-methylpyrrole, the title compound was prepared as a solid (5.5 g, 90%). ¹H NMR (300 MHz, CDCl3) δ 4.2 (m , 2H), 3.55 (s, 2H), 2.1(m, 1H), 1.3 (t, J = 7.2Hz, 3H), 1.2 (m, 2H), 1.0 (m, 2H).

### b) 2-(3,4-dimethylphenyl-5-thiophen-2-yl-2,4-dihydropyrazol-3-one:

Following the procedure of example 1d), except substituting 3-cyclopropyl-3-oxo-propionic acid; ethyl ester for ethyl acetoacetate, the title compound was prepared as a solid (3.9 g, 64%). MS(ES) m/z 229(M+H)⁺.

### c) 3'- {N'-[3-cyclopropyl-1-(3,4-dimethylphenyl)-5-oxo-1,5-dihydropyrazol-4-ylidene]hydrazino}-2'-hydroxybiphenyl-3-carboxylic acid;:

Follow the procedure of example 1e), except substituting 2-(3,4-dimethylphenyl-5-thiophen-2-yl-2,4-dihydropyrazol-3-one for the compound from example 1d) and 3'-amino-2'-hydroxybiphenyl-3-carboxylic acid; for the compound from example 1c), the title compound was obtained as a solid (0.07 g, 29%). ¹H NMR (300 MHz, d₆-DMSO) δ 13.8 (s, 1 H), 9.7 (s, 1H), 8.14 (s, 1H), 7.99 (d, J = 7.9Hz, 1H), 7.8(d, J = 7.9Hz, 1H), 7.7 (m, 4H), 7.01 (m, 3H), 2.27 (s, 3H), 2.23(s, 3H), 2.16(m, I H), 1.19 (m, 2H), 1.18(m, 2H). MS(ES) m/z 469(M+H)⁺.

### Example 90

### 3'-{N'-[1-(3,4-dimethylphenyl)-5-oxo-3-thiazol-2-yl-1,5-dihydropyrazol-4-ylidene]hydrazino}-2'-hydroxybiphenyl-3-carboxylic acid;

### a) 3-oxo-3-thiazol-2-yl-propionic acid; ethyl ester:

Follow the procedure of example 77a), except substituting 2-acetylthiazone for 3-acetyl-1-methylpyrrole, the title compound was prepared as a solid (1.7g, 21%). MS(ES) m/z 200(M+H)⁺.

### b) 2-methyl-5-thiazol-2-yl-2,4-dihydropyrazol-3-one:

Following the procedure of example 1d), except 3-oxo-3-thiazol-2-yl-propionic acid; ethyl ester for ethyl acetoacetate, the title compound was prepared s a solid (0.35g, 15%). MS(ES) m/z 272(M+H)⁺.

### c) 3'-{N'-[1-(3,4-dimethylphenyl)-5-oxo-3-thiazol-2-yl-1,5-dihydropyrazol-4-ylidene]hydrazino}-2'-hydroxybiphenyl-3-carboxylic acid;:

Follow the procedure of example 1e), except substituting 2-methyl-5-thiazol-2-yl-2,4-dihydropyrazol-3-one for the compound from example 1d) and 3'-amino-2'-hydroxybiphenyl-3-carboxylic acid; for the compound from example 1c), the title compound was obtained as a solid (60mg, 32%). ¹H NMR (300 MHz, d₆-DMSO) δ 13.0 (br s, 1H), 9.7 (s, 1H), 8.16 (s, 1H), 7.99 (m, 2H), 7.7(m, 5H), 7.2 (m, 3H), 2.27 (s, 3H), 2.23(s, 3H). MS(ES) m/z 512(M+H)⁺.

### Example 91

### 3'-{N'-[1-(3,4-dimethylphenyl)-5-oxo-1,5-dihydropyrazol-4-ylidene]hydrazino}-2'-hydroxybiphenyl-3-carboxylic acid;

### a) 1-(3,4-dimethylphenyl)-5-oxo-4,5-dihydro-1H-pyrazole-4-carboxylic acid; ethyl ester:

Following the procedure of example 1d), except substituting diethyl(ethoxymethylene) malonate for ethyl acetoacetate, the title compound was prepared as a solid (2.68g, 89%). ¹H NMR (300 MHz, CDCl3) δ 7.7 (s , 1H), 7.55 (s, 1H), 7.5(d, J = 7.8Hz, 1H), 7.2 (d, J = 7.8Hz, 1H), 4.3 (m, 2H), 2.35(s, 3H), 2.33(s, 3H), 1.4 (t, J =7.5Hz, 3H). MS(ES) m/z 261(M+H)⁺.

### b) 2-(3,4-dimethylphenyl)-2,4-dihydropyrazol-3-one:

The compound from example 91 c) (1.34g, 5.15mmol) in 10ml methanol was treated with 10% sodium hydroxy solution. After stirring at room temperature for three hours, the reaction mixture was heated up to 100°C and agitated at this temperature for 48 hrs. 3N hydrochloride was added to acid;ified the mixture to pH=4. Ethyl acetate was added and the layers were separated. The organic layers was combined, dried over MgSO4 and concentrated to give the title compound as yellow powder (1.5 g, 87 %). ¹H NMR (300 MHz, CDC13) δ 7.6 (s , 1H), 7.4 (dd, J = 2.4Hz and 8.2Hz, 1H), 7.47(s, 1H), 7.15 (d, J = 8.2Hz, 1H), 3.5 (s, 2H), 2.39(s, 3H), 2.37(s, 3H). MS(ES) m/z 377 (2M+H)⁺. c) 3'-{N'-[1-(3,4-dimethylphenyl)-5-oxo-1,5-dihydropyrazol-4-ylidene]hydrazino}-2'-hydroxybiphenyl-3-carboxylic acid;:

Follow the procedure of example 1e), except substituting 2-(3,4-dimethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.927 g, 88 %). ¹H NMR (300 MHz, d₆-DMSO) δ 8.19 (s, 1H), 8.1 (s, 1H), 7.9 (d, J = 7.5Hz, 1H), 7.8(d, J = 7.5Hz, 1H), 7.65 (m, 3H), 7.2 (m, 3H), 2.27 (s, 3H), 2.23(s, 3H). MS(ES) m/z 429(M+H)⁺.

### Example 92

### 3'-{N'-[1-(3,4-dimethylphenyl)-3-(1-methylethyl)-5-oxo-1,5-dihydropyrazol-4-ylidene]hydrazino}-2'-hydroxybiphenyl-3-carboxylic acid;

### a) 2-(3,4-dimethylphenyl)-5-isopropyl-2,4-dihydropyrazole-3-one:

Following the procedure of example 1d), except substituting ethyl isobutyryl acetate for ethyl acetoacetate, the title compound was prepared as a solid (3.8g, 66%). ¹H NMR (300 MHz, CDCl3) δ 7.67 (s , 1H), 7.55 (d, J = 7.8Hz, 1H), 7.08 (d, J = 7.8Hz, 1H), 3.5 (s, 2H), 2.75 (m, 1H), 2.28 (s, 3H), 2.25 (s, 3H), 1.26 (s, 3H), 1.24 (s, 3H).

### b) 3'-{N'-[1-(3,4-dimethylphenyl)-3-(1-methylethyl)-5-oxo-1,5-dihydropyrazol-4-ylidene]hydrazino)-2'-hydroxybiphenyl-3-carboxylic acid;:

Follow the procedure of example 1e), except substituting 2-(3,4-dimethylphenyl)-5-isopropyl-2,4-dihydropyrazole-3-one for the compound from example 1d) and 3'-amino-2'-hydroxybiphenyl-3-carboxylic acid; for the compound from example 1c), the title compound was obtained as a solid (0.15 g, 71 %). ¹H NMR (300 MHz, d₆-DMSO) δ 13.8 (s, 1H), 9.6 (s,1H), 8.14 (s, 1H), 8.1 (s, 1H), 7.97 (d, J = 7.7Hz, 1H), 7.82 (d, J = 7.8Hz, 1H), 7.73 (s, 1H), 7.65 (m, 2H), 7.10 (m, 3H), 3.17 (m, 1H), 2.28 (s, 3H), 2.24 (s, 3H), 1.41 (s, 3H), 1.38 (s, 3H). MS(ES) m/z 471 (M+H)⁺.

### Example 93

### 3'-{N'-[3-(benzyloxymethyl)-1-(3,4-dimethylphenyl)-5-oxo-1,5-dihydropyrazol-4-ylidene]hydrazino}-2'-hydroxybiphenyl-3-carboxylic acid;

### a) 4-benzyloxy-3-oxo-butyric acid; ethyl ester:

A suspension of sodium hydride (60% in oil) in tolene (200 mL) was mechanically stirred at room temperature under argon and treated dropwise with benzyl alcohol in toluene (40 mL) over 40 minutes. The mixture was stirred for 1 hour, treated with ethyl 4-chloroacetate, and then stirred over 18 hours at room temperature.

At end of the reaction, citric acid; was added and layers were separated. The organic layers was washed water, dried over MgSO4 and concentrated to give the the title compound (7.2 g, 20%). MS(ES) m/z 237 (M+H)⁺.

### b) 5-benzyloxymethyl-2-(3,4-dimethylphenyl)-2,4-dihydropyrazol-3-one:

Following the procedure of example 1d), except substituting 4-benzyloxy-3-oxo-butyric acid; ethyl ester for ethyl acetoacetate, the title compound was prepared as a solid (1.7 g, 37 %). ¹H NMR (300 MHz, CDCl3) δ 7.58 (s , 1H), 7.55 (d, J = 8.1Hz,1H), 7.35 (m, 5H), 7.16 (d, J = 8.1Hz, 1H), 4.6 (s, 2H), 4.37 (s, 2H), 3.5 (s, 2H), 2.29 (s, 3H), 2.25 (s, 3H).

### c) 3'- {N'-[3-(benzyloxymethyl)-1-(3,4-dimethylphenyl)-5-oxo-1,5-dihydropyrazol-4-ylidene]hydrazino}-2'-hydroxybiphenyl-3-carboxylic acid;:

Follow the procedure of example 1e), except substituting 5-benzyloxymethyl-2-(3,4-dimethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.023 g, 12 %). ¹H NMR (300 MHz, d₆-DMSO) δ 13.1 (br s, 1H), 9.8 (br s, 1H), 8.15 (s, 1H), 7.97 (d, J = 7.8Hz, 1H), 7.82 (d, J = 7.7Hz, 1H), 7.56 (m, 4H), 7.40 (m, 5H), 7.18 (m, 3H), 4.69(s, 2H), 4.64(s, 2H), 2.28 (s, 3H), 2.26 (s, 3H). MS(ES) m/z 549(M+H)⁺.

### Example 94

### 3'-{N'-[3-ethyl-5-oxo-1-(4-trifluoromethylphenyl)-1,5-dihydropyrazol-4-ylidene]hydrazino}-2'-hydroxybiphenyl-3-carboxylic acid;

### a) 5-ethyl-2-(4-trifluoromethylphenyl)-2,4-dihydropyrazol-3-one:

Following the procedure of example 1d), except substituting ethyl propionyl acetate for ethyl acetoacetate and 4-(trifluoromethyl)phenyl hydrazine for 3,4-dimethylphenylhydrazine hydrochloride, the title compound was prepared as a solid (9.6 g, 99 %). ¹H NMR (300 MHz, CDCl3) δ 8.07 (d, J = 8.3Hz, 2H), 7.64 (d, J = 8.3 Hz, 2H), 3.45 (s, 2H), 2.55 (m, 2H), 1.27 (t, J = 7.6Hz, 3H).

### b) 3'-{N'-[3-ethyl-5-oxo-1-(4-trifluoromethylphenyl)-1,5-dihydropyrazol-4-ylidene]hydrazino}-2'-hydroxybiphenyl-3-carboxylic acid;:

Follow the procedure of example 1e), except substituting 5-ethyl-2-(4-trifluoromethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.1 g, 12 %). ¹H NMR (300MHz, d₆-DMSO) δ 13.6 (br s, 1H), 13.1 (br s. 1H), 9.8 (s, 1H), 8.19 (d, J = 8.6Hz, 1H), 8.14 (s,1H), 7.98 (d, J = 7.8Hz, 1H), 7.80 (m, 3H), 7.72 (d, J = 7.1 Hz,1H), 7.63 (t, J = 7.8Hz, 1H), 7.19 (m, 2H), 2.55 (m, 2H), 1.27 (t, J = 7.6Hz, 3H).

### Example 95

### 3'-{N'-[5-oxo-1-(4-trifluoromethylphenyl)-1,5-dihydropyrazol-4-ylidene]hydrazino}-2'-hydroxybiphenyl-3-carboxylic acid;

### a) 1-(4-trifluoromethylphenyl)-5-oxo-4,5-dihydro-1H-pyrazole-4-carboxylic acid; ethyl ester:

Following the procedure of example 1d), except substituting diethyl(ethoxymethylene) malonate for ethyl acetoacetate and trifluoromethylhydrazine for 3,4-dimethylphenylhydrazine, the title compound was prepared as a solid. ¹H NMR (300 MHz, CDC13) δ 8.0 (d, J = 8.2Hz, 2H), 7.64 (s, 1H), 7.53 (d, J = 8.5Hz, 2H), 4.18 (m, 2H), 1.25(m, 3H). MS(ES) m/z 301 (M+H)⁺.

### b) 2-(4-trifluoromethylphenyl)-2,4-dihydropyrazol-3-one:

Follow the procedure of example 91b), except substituting 1-(4-trifluoromethylphenyl)-5-oxo-4,5-dihydro-1H-pyrazole-4-carboxylic acid; ethyl ester for 1-(3,4-dimethylphenyl)-5-oxo-4,5-dihydro-1H-pyrazole-4-carboxylic acid; ethyl ester, the title compound was prepared as a solid. ¹H NMR (300 MHz, CDC13) δ 8.0 (d, J = 8.2Hz, 2H), 7.7 (d, J = 8.2Hz, 2H), 7.54 (s, 1H), 3.56 (s, 2H). MS(ES) m/z 229 (M+H)⁺.

### c) 3'-{N'-[5-oxo-1-(4-trifluoromethylphenyl)-1,5-dihydropyrazol-4-ylidene]hydrazino}-2'-hydroxybiphenyl-3-carboxylic acid;:

Follow the procedure of example 1e), except substituting 2-(4-trifluoromethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.25 g, 61 %). MS(ES) m/z 469 (M+H)⁺.

### Example 96

### 3'-{N'-[-1-(3,4-dimethylphenyl)-3-hydroxymethyl-5-oxo-1,5-dihydropyrazol-4-ylidene]hydrazino}-2'-hydroxybiphenyl-3-carboxylic acid;

5-benzyloxymethyl-2-(3,4-dimethylphenyl)-2,4-dihydropyrazol-3-one (0.5 g, 0.002 mol) in THF was treated with 10% Pd/C (cat.). The reaction mixture was shaked for 4 hours under hydrogen (50 psi). At end of the reaction, the mixture was concentrated and purified through column chromatography on SiO2 to give the crude product as a gum (0.15 g, 34 %).

Follow the procedure of example 1e), except substituting the above crude product for the compound from example 1d) and 3'-amino-2'-hydroxybiphenyl-3-carboxylic acid; for the compound from example 1c), the title compound was obtained as a solid (0.11 g, 65 %). ¹H NMR (300MHz, d₆-DMSO) δ 9.7 (s, 1H), 8.14 (s, 1H), 7.97 (d, J = 7.6Hz, 1H), 7.7 (m, 5H), 7.19 (m, 3H), 2.28 (s, 3H), 2.26 (s, 3H). MS(ES) m/z 459(M+H)⁺.

### Example 97

### 3'-{N'-[3-benzyloxymethyl-5-oxo-1-(4-trifluoromethylphenyl)-1,5-dihydropyrazol-4-ylidene]hydrazino}-2'-hydroxybiphenyl-3-carboxylic acid;

### a) 5-benzyloxymethyl-2-(4-trifluoromethylphenyl)-2,4-dihydropyrazol-3-one:

Following the procedure of example 1d), except substituting 4-benzyloxy-3-oxo-butyric acid; ethyl ester for ethyl acetoacetate and 4-trifluoromethylphenylhydrazine for 3,4-dimethylhydrazine, the title compound was prepared as a solid (1.6 g, 32 %). ¹H NMR (300 MHz, DMSO-d₆) δ 8.0 (d, J = 8.5Hz , 2H), 7.82 (d, J = 8.6Hz, 2H), 7.35 (m, 5H), 5.6 (s, 1H), 4.53 (s, 2H), 4.41 (s, 2H). MS(ES) m/z 349(M+H)⁺.

### b) 3'-{N'-[3-benzyloxymethyl-5-oxo-1-(4-trifluoromethylphenyl)-1,5-dihydropyrazol-4-ylidene]hydrazino}-2'-hydroxybiphenyl-3-carboxylic acid;:

Follow the procedure of example 1e), except substituting 5-benzyloxymethyl-2-(4-trifluoromethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.018 g, 8 %). ¹H NMR (300MHz, d₆-DMSO) δ 9.8 (s, 1H), 8.20(d, J = 7.6Hz, 2H), 8.14 (s, 1H), 7.99 (d, J = 7.7Hz, 1H), 7.84 (d, J = 7.2Hz, 2H), 7.72 (d, J = 7.1Hz, 1H), 7.63 (m, 2H), 7.38 (m, 5H), 7.21(m, 2H), 4.71(s, 2H), 4.68(s, 2H). MS(ES) m/z 589(M+H)⁺.

### Example 98

### 3'-{N'-[-1-(3,4-dimethylphenyl)-3-methylsulfanylmethyl-5-oxo-1,5-dihydropyrazol-4-ylidene]hydrazino}-2'-hydroxybiphenyl-3-carboxylic acid;

### a) 4-methylsulfanyl-3-oxo-butyric acid; ethyl ester:

To a solution of sodium thiomethoxide (5.0 g, 0.071 mol) in methanol (100 mL), stirred at 5°C under a slow nitrogen stream, was added a solution of ethyl 4-chloroacetoacetate (6.76 mL, 0.05 mol) in methanol. The reaction mixture was warmed up to room temperature and stirred for 18 hours. At the end reaction, methanol was evaporated and the aqueous was extracted with ether (3 times). The combined organics were dried and concentrated. The residue was further purified by distillation at 70-80°C (0.1mmHg) to give the title compound. MS(ES) m/z 163 (M+H)⁺, 177 (M+H)⁺. (mixture of methyl ester and ethyl ester).

### b) 2-(3,4-dimethylphenyl)-5-methylsulfanylmethyl-2,4-dihydropyrazol-3-one:

Following the procedure of example 1d), except substituting 4-methylsulfanyl-3-oxo-butyric acid; ethyl ester for ethyl acetoacetate, the title compound was prepared as a solid (0.6 g, 16%). ¹H NMR (300 MHz, DMSO-d₆) δ 7.52 (s, 1H), 7.45 (d, J = 8.2Hz, 1H), 7.18 (d, J = 8.2Hz, 1H), 5.48 (s1H), 3.5 (s, 2H), 2.27 (s, 3H), 2.25 (s, 3H), 2.22 (s, 3H).

### c) 3'- {N'-[-1-(3,4-dimethylphenyl)-3-methylsulfanylmethyl-5-oxo-1,5-dihydropyrazol-4-ylidene]hydrazino}-2'-hydroxybiphenyl-3-carboxylic acid;:

Follow the procedure of example 1e), except substituting 2-(3,4-dimethylphenyl)-5-methylsulfanylmethyl-2,4-dihydropyrazol-3-one for the compound from example 1d) and 3'-amino-2'-hydroxybiphenyl-3-carboxylic acid; for the compound from example 1c), the title compound was obtained as a solid (0.09 g, 49 %). ¹H NMR (300MHz, d₆-DMSO) δ 13.7 (br s, 1H), 9.8 (s, 1H), 8.15 (s, 1H), 7.98 (d, J = 7.8Hz, 1H), 7.82 (d, J = 7.8Hz, 1H), 7.74 (m, 2H), 7.65 (m, 2H), 7.19 (m, 3H), 3.79(s, 2H), 2.28 (s, 3H), 2.24 (s, 3H), 2.22 (s, 3H). MS(ES) m/z 489(M+H)⁺.

### Example 99

### 3'-{N'-[-1-(3,4-dimethylphenyl)-5-oxo-3-thiophen-3-yl-1,5-dihydropyrazol-4-ylidene]hydrazino}-2'-hydroxybiphenyl-3-carboxylic acid;

### a) 3-oxo-3-thiophen-3-yl-propionic acid; ethyl ester:

Follow the procedure of example 77a), except substituting 3-acetylthiophene for 3-acetyl-1-methyl-pyrazole, the title compound was prepared as solid (9.8 g, 74%). ¹H NMR (300 MHz, DMSO-d₆) δ 8.08 (d, J = 2.8Hz, I H), 7.52 (d, J = 2.8Hz, 1H), 7.30 (s, 1H), 4.19 (m, 2H), 3.86 (s, 2H), 1.28 (m, 3H).

### b) 2-(3,4-dimethylphenyl)-5-thiophen-3-yl-2.4-dihydropyrazol-3-one:

Following the procedure of example 1d), except substituting 3-oxo-3-thiophen-3-yl-propionic acid; ethyl ester for ethyl acetoacetate, the title compound was prepared as a solid (0.95 g, 27 %). ¹H NMR (300 MHz, DMSO-d₆) δ 7.8 (s, 1H), 7.57 (m, 4H), 7.18 (d, J = 8.3Hz, 1H), 5.89 (s, 1H), 2.27 (s, 3H), 2.24 (s, 3H).

### c) 3'-{N'-[-1-(3,4-dimethylphenyl)-5-oxo-3-thiophen-3-yl-1,5-dihydropyrazol-4-ylidene]hydrazino}-2'-hydroxybiphenyl-3-carboxylic acid;:

Follow the procedure of example 1e), except substituting 2-(3,4-dimethylphenyl)-5-thiophen-3-yl-2.4-dihydropyrazol-3-one for the compound from example 1d) and 3'-amino-2'-hydroxybiphenyl-3-carboxylic acid; for the compound from example 1c), the title compound was obtained as a solid (0.09 g, 46 %). ¹H NMR (300MHz, d₆-DMSO) δ 13.9 (br s, 1H), 13.1 (br s, 1H), 9.8 (s, 1H), 8.45 (d, J = 2.7 Hz, 1H), 8.16 (s, 1H), 7.97 (d, J = 7.7Hz, 1H), 7.84 (d, J = 7.7Hz, 1H), 7.67 (m, 5H), 7.19 (m, 3H), 2.27 (s, 3H), 2.24 (s, 3H).

### Example 100

### 3'-{N'-[5-oxo-1-(4-trifluoromethylphenyl)-3-thiophen-3-yl-1,5-dihydropyrazol-4-ylidene]hydrazino}-2'-hydroxybiphenyl-3-carboxylic acid;

### a) 5-thiophen-3-yl-2-(4-trifluoromethylphenyl)-2,4-dihydropyrazol-3-one:

Following the procedure of example 1d), except substituting 3-oxo-3-thiophen-3-yl-propionic acid; ethyl ester for ethyl acetoacetate and 4-trifluoromethylhydrazine for 3,4-dimethylhydrazine, the title compound was prepared as a solid (2.7 g, 67 %). ¹H NMR (300 MHz, DMSO-d₆) δ 8.08 (d, J = 8.5Hz, 2H), 7.88 (s, 1H), 7.83 (d, J = 8.5Hz, 2H), 7.60 (s, 1H), 7.54 (d, J = 5.0Hz,1H), 5.78 (s, 1H).

### b) 3'- (N'-[5-oxo-1-(4-trifluoromethylphenyl)-3-thiophen-3-yl-1,5-dihydropyrazol-4-ylidene]hydrazino}-2'-hydroxybiphenyl-3-carboxylic acid;:

Follow the procedure of example 1e), except substituting 5-thiophen-3-yl-2-(4-trifluoromethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.025 g, 13 %). ¹H NMR (300MHz, d₆-DMSO) δ 13.9 (br s, 1H), 9.8 (s, 1H), 8.53 (d, J = 2.8 Hz, 1H), 8.29 (d, J = 8.5Hz, 2H), 8.15 (s, 1H), 7.8 (m, 7H), 7.6 (t, J = 7.8Hz, 1H), 7.12 (m, 2H). MS(ES) m/z 551(M+H)⁺.

### Example 101

### 3'-{N'-[5-oxo-1-(4-trifluoromethylphenyl)-3-methylsulfanylmethyl-1,5-dihydropyrazol-4-ylidene]hydrazino}-2'-hydroxybiphenyl-3-carboxylic acid;

### a) 5-methylsulfanyl-2-(4-trifluoromethylphenyl)-2,4-dihydropyrazol-3-one:

Following the procedure of example 1d), except substituting 4-methylsulfanyl-3-oxo-butyric acid; ethyl ester for ethyl acetoacetate and 4-trifluoromethylphenylhydrazine for 3,4-dimethylphenylhydrazine, the title compound was prepared as a solid (0.9 g, 21%). ¹H NMR (300 MHz, DMSO-d₆) δ 8.02 (d, J = 8.6Hz, 2H), 7.8 (d, J = 8.6Hz, 2H), 5.49 (s, 1H), 3.5 (s, 2H), 2.05 (s, 3H).

### b) 3'-{N'-[5-oxo-1-(4-trifluoromethylphenyl)-3-methylsulfanylmethyl-1,5-dihydropyrazol-4-ylidene]hydrazino}-2'-hydroxybiphenyl-3-carboxylic acid;:

Follow the procedure of example 1e), except substituting 5-methylsulfanyl-2-(4-trifluoromethylphenyl)-2,4-dihydropyrazol-3-one for the compound from example 1d) and 3'-amino-2'-hydroxybiphenyl-3-carboxylic acid; for the compound from example 1c), the title compound was obtained as a solid (0.065 g, 35 %).¹H NMR (300MHz, d₆-DMSO) δ 13.7 (br s, 1H), 9.8 (s, 1H), 8.19 (s, 1H), 8.15 (d, J = 8.2Hz, 2H), 7.99 (d, J = 7.5Hz, 1H), 7.84 (m, 3H), 7.8 (d, J = 7.5Hz, 1H), 7.75 (t, J = 7.4 Hz, 1H), 7.22 (m, 2H), 3.9 (s, 2H), 2.23 (s, 3H). MS(ES) m/z 529 (M+H)⁺.

### Example 102

### N-(3'-{N'-[1-(3,4-dimethylphenyl)-3-methyl-5-oxo-1,5-dihydro-pyrazol-4-ylidene]hydrazino}-2'-hydroxybiphenyl-3-yl)methanesulfonamide

### a) N-(3'-nitro-2'-hydroxybiphenyl-3-yl)-methanesulfonamide:

3'-amino-3-nitrobiphenyl-2-ol (0.37 g, 1.62 mmol) in chloroform (10 mL) was treated with methylsulfonic chloride (2.4 mmol). After stirred at room temperature for 18 hours, the reaction mixture was added water. Layers were separated and the organic layer was dried and concentrated. The resulting gum was dissolved in THF, added 10% sodium hydroxy and heated to reflux for 18 hours. The precipitated was collected and washed with ether to give the the title compound as yellow solid (0.45 g, 90 %). MS(ES) m/z 309 (M+H)⁺.

### b) N-(3'-amino-2'-hydroxybiphenyl-3-yl)-methanesulfonamide:

Follow the procedure of example 3d), except substituting N-(3'-nitro-2'-hydroxybiphenyl-3-yl)-methanesulfonamide for 2'-methoxy-3'-nitrobiphenyl-3-carboxylic acid;, the title compound was prepared as a solid. MS(ES) m/z 279 (M+H)⁺.

### c) N-(3'-{N'-[1-(3,4-dimethylphenyl)-3-methyl-5-oxo-1,5-dihydro-pyrazol-4-ylidene]hydrazino}-2'-hydroxybiphenyl-3-yl)methanesulfonamide:

Follow the procedure of example 1e), except substituting N-(3'-amino-2'-hydroxybiphenyl-3-yl)-methanesulfonamide for the compound from example 1c), the title compound was obtained as a solid (0.053 g, 13 %). MS(ES) m/z 492 (M+H)⁺.

### Example 103

### 3'-[N'-(1-benzo[1,3]dioxol-5-yl-3-methyl-5-oxo-1,5-dihydropyrazol-4-ylidene)hydrazinol-2'-hydroxybiphenyl-3-carboxylic acid;

### a) 2-benzo[1,3]dioxol-5-yl-5-methyl-2,4-dihydropyrazole-3-one:

Aqueous sodium nitrite (0.69 g, 10 mmol) was added slowly to a stirred solution of [1,3]dioxol-5-phenylaniline (1.27 g, 10mmol) in aqueous HCl (0.5 mL) at-10°C to -20°C over 20 min. Then a solution of tin chloride in aqueous HCl was added rapidly. After 30 min. at -20°C, the solid was collected and washed with diethyl ether to afford a crude product.

Following the procedure of example 1d), except substituting the above crude product for 3,4-dimethylphenylhydrazine, the title compound was prepared as a solid (0.035 g, 8 %). ¹H NMR (300 MHz, DMSO-d₆) δ 7.38 (s, 1H), 7.28 (d, J = 8.5Hz, 1H), 6.8 (d, J = 8.4Hz, 1H), 5.97 (s, 2H), 3.46 (s, 2H), 2.2 (s, 3H).

### b) 3'-[N'-(1-benzo[1,3]dioxol-5-yl-3-methyl-5-oxo-1,5-dihydropyrazol-4-ylidene) hydrazino]-2'-hydroxybiphenyl-3-carboxylic acid;:

Follow the procedure of example 1e), except substituting 2-benzo[1,3]dioxol-5-yl-5-methyl-2,4-dihydropyrazole-3-one for the compound from example 1d) and 3'-amino-2'-hydroxybiphenyl-3-carboxylic acid; for the compound from example 1c), the title compound was obtained as a solid (0.051 g, 66 %). ¹H NMR (300MHz, d₆-DMSO) δ 13.9 (br s, 1H), 13.1 (br s, 1H), 9.8 (s, 1H), 8.14 (s, 1H), 7.98 (d, J = 7.7Hz, 1H), 7.80 (d, J = 7.7Hz, 1H), 7.73 (m, 1H), 7.62 (t, J = 7.7Hz, 1H), 7.47 (s, 1H), 7.39 (d, J = 8.5Hz, 1H), 7.17 (m, 2H), 7.0 (d, J = 8.5Hz, 1H), 6.07 (s, 2H), 2.33 (s, 3H). MS(ES) m/z 458 (M+H)⁺.

### Example 104

### 3'-{N'-[1-(3,5-dimethylphenyl)-3-methyl-5-oxo-1,5-dihydropyrazol-4-ylidenelhydrazino}-2'-hydroxybiphenyl-3-carboxylic acid;

### a) 2-(3,5-dimethylphenyl)-5-methyl-2,4-dihydropyrazole-3-one

Following the procedure of example 1d), except substituting 3,5-dimethylphenylhydrazine for 3,4-dimethylphenylhydrazine, the title compound was prepared as a solid (2.62 g, 52 %). ¹H NMR (300 MHz, CDCl₃) δ 7.46 (s, 2H), 6.8 (s, 1H), 3.4 (s, 2H), 2.33 (s, 6H), 2.18 (s, 3H).

### b) 3'-{N'-[1-(3,5-dimethylphenyl)-3-methyl-5-oxo-1,5-dihydropyrazol-4-ylidene]hydrazino}-2'-hydroxybiphenyl-3-carboxylic acid;:

Follow the procedure of example 1e), except substituting 2-(3,5-dimethylphenyl)-5-methyl-2,4-dihydropyrazole-3-one for the compound from example 1d) and 3'-amino-2'-hydroxybiphenyl-3-carboxylic acid; for the compound from example 1c), the title compound was obtained as a solid (0.22 g, 50 %). ¹H NMR (300MHz, d₆-DMSO) δ 13.7 (br s, 1H), 13.1 (br s,1H), 9.7 (s, 1H), 8.14 (s, 1H), 7.97 (d, J = 7.7Hz, 1H), 7.80 (d, J = 7.7Hz, 1H), 7.72 (dd, J = 1.9Hz and 6.8Hz, 1H), 7.62 (t, J = 7.7Hz, 1H), 7.58 (s, 2H), 7.17 7 (m, 2H), 7.0 (d, J = 8.5Hz, 1H), 6.87 (s, 1H), 2.33 (s, 9H). MS(ES) m/z 443 (M+H)⁺.

### Reference Example 105

### 3'-{N'-[1-(3,4-dimethylphenyl)-3-methyl-5-oxo-1,5-dihydropyrazol-4-ylidene]hydrazino}-4'-hydroxybiphenyl-4-carboxylic acid;

### a) 3'-nitro-4'-hydroxybiphenyl-4-carboxylic acid;:

Follow the procedure of example 7c), except substituting 4'-hydroxybiphenyl-4-carboxylic acid; for 6-(5-chloro-2-hydroxyphenyl)-pyridine-2-carboxylic acid;, the title compound was prepared as a solid (1g, 84 %). MS(ES) m/z 260 (M+H)⁺.

### b) 3'-amino-4'-hydroxybiphenyl-4-carboxylic acid;:

Follow the procedure of example 3e) except substituting 3'-nitro-4'-hydroxybiphenyl-4-carboxylic acid; for 2'-hydroxy-3'-nitrobiphenyl-3-carboxylic acid;, the title compound was prepared as a solid (0.88 g, 40 %). MS(ES) m/z 230 (M+H)⁺.

### c) 3'-{N'-[1-(3,4-dimethylphenyl)-3-methyl-5-oxo-1,5-dihydropyrazol-4-ylidene]hydrazino}-4'-hydroxybiphenyl-4-carboxylic acid;:

Follow the procedure of example 1e), except substituting 3'-amino-4'-hydroxybiphenyl-4-carboxylic acid; for the compound from example 1c), the title compound was obtained as a solid (0.067 g, 74 %). MS(ES) m/z 443 (M+H)⁺.

### Example 106

### 3'-{N'-[1-(3-chloro-4-methylphenyl)-3-methyl-5-oxo-1,5-dihydropyrazol-4-ylidenelhydrazino}-2'-hydroxybiphenyl-3-carboxylic acid;

### a) 2-(3-chloro-4-methylphenyl)-5-methyl-2,4-dihydropyrazol-3-one:

Following the procedure of example 1d), except substituting 3-chloro-4-methylphenylhydrazine for 3,4-dimethylphenylhydrazine, the title compound was prepared as a solid (3.5 g, 63 %). ¹H NMR (300 MHz, DMSO-d₆) δ 7.84 (s, 1H), 7.65 (d, J = 8.3Hz, 1H), 7.37 (d, J = 8.5Hz, 1H), 5.29 (br s, 2H), 2.33 (s, 3H), 2.16 (s, 3H).

### b) 3'-{N'-[1-(3-chloro-4-methylphenyl)-3-methyl-5-oxo-1,5-dihydropyrazol-4-ylidene]hydrazino}-2'-hydroxybiphenyl-3-carboxylic acid;:

Follow the procedure of example 1e), except substituting 2-(3,5-dimethylphenyl)-5-methyl-2,4-dihydropyrazole-3-one for the compound from example 1d) and 3'-amino-2'-hydroxybiphenyl-3-carboxylic acid; for the compound from example 1c), the title compound was obtained as a solid (0.22 g, 47 %). ¹H NMR (300MHz, d₆-DMSO) δ 13.7 (br s, 1H), 13.1 (br s,1H), 9.7 (s,1H), 8.14 (s, 1H), 7.98 (s,1H), 7.97 (d, J = 7.1Hz,1H), 7.79 (m, 2H), 7.7 (d, J = 7.1Hz, 1H), 7.62 (t, J = 7.6Hz, 1H), 7.42 (d, J = 8.5Hz, 1H), 7.19 (m, 2H), 2.23 (s, 6H). MS(ES) m/z 463 (M+H)⁺.

### Reference Example 107

### 3'-{N'-[1-(3,4-dimethylphenyl)-3-methyl-5-oxo-1,5-dihydropyrazol-4-ylidene]hydrazino}-4'-hydroxybiphenyl-3-carboxylic acid;

### a) 3'-amino-4'-hydroxybiphenyl-3-carboxylic acid;:

Follow the procedure of example 7c), except substituting 3'-nitro-4'-methoxybiphenyl-3-carboxylic acid; for 6-(5-chloro-2-hydroxyphenyl)-pyridine-2-carboxylic acid;, the crude product was isolated.

Follow the procedure of example 3d), except substituting the above crude product for 2'-methoxy-3'-nitrobiphenyl-3-carboxylic acid;, the title compound was prepared as a solid. ¹H NMR (300 MHz, DMSO-d₆) δ 8.1 (s, 1H), 7.9 (d, J = 7.7Hz, 1H), 7.8 (d, J = 7.7Hz, 1H), 7.56 (m, 3H), 7.06 (d, J = 7.8 Hz, 1H).

### b) 3'-{N'-[1-(3,4-dimethylphenyl)-3-methyl-5-oxo-1,5-dihydropyrazol-4-ylidene]hydrazino}-4'-hydroxybiphenyl-3-carboxylic acid;:

Follow the procedure of example 1e), except substituting 3'-amino-4'-hydroxybiphenyl-3-carboxylic acid; for the compound from example 1c), the title compound was obtained as a solid (0.05 g, 63 %). MS(ES) m/z 443 (M+H).

### Example 108

### 3'-{N'-[1-(3,4-dimethylphenyl)-3-methyl-5-oxo-1,5-dihydropyrazol-4-ylidene]hydrazino}-2'-hydroxybiphenyl-3-phosphonic acid;

### a) 3-bromobenzenediazonium tetrafluoroborate

3-Bromoaniline (17.2 g; 0.1 mol.) was added to a solution of lithium tetrafluoroborate (12.0 g; 0.128 mol.) in conc. hydrochloric acid; (20.0 mL) and water (80.0 mL) and the resulting suspension was cooled to 5 °C and treated dropwise with a solution of sodium nitrite (6.9 g; 0.1 mol.) in water (20.0 mL). The mixture was stirerd at 5 °C for 1h then filtered and the precipitate washed with cold water (20.0 mL), cold methanol (2 x 20.0 mL) and diethyl ether (3 x 20.0 mL) to give the title compound (25.1 g; 93%).

### b) (3-bromophenyl)phosphonic acid;

A suspension of the compound from Example 109a) (25.0 g; 0.093 mol.) in ethyl acetate (140 mL) was treated with phosphorous trichloride (8.2 mL; 0.094 mol.) followed by copper (I) bromide (2.07 g; 0.014 mol.). The mixture was warmed to initiate reaction (gas evolution) and then allowed to stir at room temperature (ice-bath cooling applied when necessary to avoid excessive exotherm), then when gas evolution has ceased the mixture is stirred and heated at 50 °C for a further 40 min. After cooling, water (30.0 mL) was slowly added and the reaction volume reduced to -50 mL by evaporation. The solid precipitate was removed then treated with 10% aqu. sodium hydroxide (100 mL) and filtered to remove insoluble material. The filtrate was acid;ified to pH 4 with conc. hydrochloric acid; then left at room temperature overnight and filtered and the solid crystallised from 6M aqu. hydrochloric acid; (60 mL) to afford the ttile compound (5.21 g; 24%) as a colorless solid. mp 144-149°C.

### c) (5'-chloro-2'-methoxybiphenyl-3-yl)phosphonic acid;:

Following the procedure of Example 1b), except substituting 2-methoxy-5-chlorophenylboronic acid; for 4-carboxyphenylboronic acid; , and substituting the compound from Example 108b) for the compound of 1a), the title compound was prepared (0.95 g; 76%) as a white powder. MS(ES) m/z 299 [M+H].

### d) (5'-chloro-2'-hydroxybiphenyl-3-yl)-phosphonic acid;:

Follow the procedure of example 3d), except substituting from Example 108c) for the compound from example 3c), the title compound was prepared as a solid (0.83 g; 90%). MS(ES) m/z 285 [M+H].

### e) (5'-chloro-2'-hydroxy-3'-nitrobiphenyl-3-yl)phosphonic acid;:

Following the procedure of Example 7c), except substituting the compound of 108d) for the compound of 7c), the title compound was prepared (0.76 g; 69%). MS(ES) m/z 330 [M+H].

### f) (3'-amino-2'-hydroxybiphenyl-3-yl)phosphonic acid;, hydrochloride salt:

Following the procedure of example 3e) except substituting the compound from Example 108e) for 2'-hydroxy-3'-nitrobiphenyl-3-carboxylic acid;, the title compound was prepared as a solid (0.33 g, 88 %). MS(ES) m/z 266 (M+H).

### g) 3'-{N'-[1-(3,4-dimethylphenyl)-3-methyl-5-oxo-1,5-dihydropyrazol-4-ylidene]hydrazino}-2'-hydroxybiphenyl-3-phosphonic acid;

Following the procedure of example 1e), except substituting the compound from Example 108f) for the compound from example 1c), the title compound was obtained as a red solid (0.092 g, 18 %). ¹H NMR (300 MHz, DMSO-d₆) δ 13.8 (br s, 1H), 10.5-9.5 (br s, 1H), 7.89-7.53 (m, 7H), 7.23-7.10 (m, 3H), 2.34 (s, 3H), 2.27 (s, 3H), 2.22 (s, 3H).

### Example 109

### 3'-{N'-[1-(3,4-dimethylphenyl)-3-methyl-5-oxo-1,5-dihydropyrazol-4-ylidene]hydrazino}-2'-hydroxybiphenyl-3,4-dicarboxylic acid;

### a) 5'-Chloro-2'-methoxy-biphenyl-3,4-dicarboxylic acid;:

Following the procedure of Example 1b), except substituting 2-methoxy-5-chlorophenylboronic acid; for 4-carboxyphenylboronic acid; , and substituting 4-bromophthalic acid; for the compound of 1a), the title compound was prepared (4.5 g; 82%) as a white powder. MS(ES) m/z 307 [M+H].

### b) 5'-Chloro-2'-hydroxy-biphenyl-3,4-dicarboxylic acid;:

Follow the procedure of example 3d), except substituting from Example 109a) for the compound from example 3c), the title compound was prepared as a solid (4.2 g; 98%). MS(ES) m/z 293 [M+H].

### c) 5'-Chloro-2'-hydroxy-3'-nitro-biphenyl-3,4-dicarboxylic acid;

Following the procedure of Example 7c), except substituting the compound of 109b) for the compound of 7c), the title compound was prepared (4.7 g; 98%). MS(ES) m/z 338 [M+H].

### d) 3'-Amino-2'-hydroxy-biphenyl-3,4-dicarboxylic acid;, hydrochloride salt:

Following the procedure of example 3e) except substituting the compound from Example 109c) for 2'-hydroxy-3'-nitrobiphenyl-3-carboxylic acid;, the title compound was prepared as a solid (4.1 g, 99 %). MS(ES) m/z 274 (M+H).

### e) 3'-{N'-[1-(3,4-dimethylphenyl)-3-methyl-5-oxo-1,5-dihydropyrazol-4-ylidene]hydrazino}-2'-hydroxybiphenyl-3,4-dicarboxylic acid;

Following the procedure of example 1e), except substituting the compound from Example 109d) for the compound from example 1c), the title compound was obtained as a red solid (0.05 g, 63 %). Anal. (C₂₆H₂₂N₄O₆.0.75 H₂O) calcd: C, 62.46; H, 4.74; N, 11.21. found: C, 62.63; H, 4.86; N, 10.84.

### Example 110

### 2',6-dihydroxy-3'-{N'-[1-(3,4-dimethylphenyl)-3-methyl-5-oxo-1,5-dihydropyrazol-4-ylidene]hydrazino}biphenyl-3-carboxylic acid;

### a) 5'-Chloro-4-hydroxy-2'-methoxy-biphenyl-3-carboxylic acid;:

Following the procedure of Example 1b), except substituting 2-methoxy-5-chlorophenylboronic acid; for 4-carboxyphenylboronic acid; , and substituting 5-bromo-2-hydroxybenzoic acid; for the compound of 1a), the title compound was prepared (4.5 g; 70%) as a white powder. MS(ES) m/z 279 [M+H].

### b) 5'-Chloro-4,2'-dihydroxy-biphenyl-3-carboxylic acid;:

Follow the procedure of example 3d), except substituting from Example 110a) for the compound from example 3c), the title compound was prepared as a solid (2.9 g; 69%). MS(ES) m/z 265 [M+H].

### c) 5'-Chloro-4,2'-dihydroxy-3'-nitrobiphenyl-3-carboxylic acid;

Following the procedure of Example 7c), except substituting the compound of 110b) for the compound of 7c), the title compound was prepared. MS(ES) m/z 310 [M+H].

### d) 3'-Amino-4,2'-dihydroxy-biphenyl-3-carboxylic acid;, hydrochloride salt:

Following the procedure of example 3e) except substituting the compound from Example 110c) for 2'-hydroxy-3'-nitrobiphenyl-3-carboxylic acid;, the title compound was prepared as a solid (0.9 g, 37 %). MS(ES) m/z 246 (M+H).

### e) 2',6-dihydroxy-3'- {N'-[1-(3,4-dimethylphenyl)-3-methyl-5-oxo-1,5-dihydropyrazol-4-ylidene]hydrazino}biphenyl-3-carboxylic acid;

Following the procedure of example 1e), except substituting the compound from Example 110d) for the compound from example 1c), the title compound was obtained as a red solid. Anal. (C₂₅H₂₂N₄O₅.0.5 H₂O) calcd: C, 64.23; H, 4.96; N, 11.98. found: C, 64.37; H, 4.97; N, 11.85.

### Example 111

### 4-aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;

### a) 2-(5-chloro-2-methoxyphenyl)isonicotinic acid;:

Following the procedure of Example 1b), except substituting 2-methoxy-5-chlorophenylboronic acid; for 4-carboxyphenylboronic acid; , and substituting 2-chloroisonicotinic acid; for the compound of 1a), the title compound was prepared as a grey powder (2.3 g; 87%) as a white powder. MS(ES) m/z 264 [M+H].

### b) 2-(5-chloro-2-hydroxyphenyl)isonicotinic acid;:

Follow the procedure of example 3d), except substituting from Example 111a) for the compound from example 3c), the title compound was prepared as a solid (1.55 g; 82%). MS(ES) m/z 250 [M+H].

### c) 2-(5-chloro-2-hydroxy-3-nitrophenyl)isonicotinic acid;:

Following the procedure of Example 7c), except substituting the compound of 111b) for the compound of 7c), the title compound was prepared as an orange powder (1.1 g; 65 %). MS(ES) m/z 296 [M+H].

### d) 2-(3-Amino-2-hydroxyphenyl)isonicotinic acid;, sodium salt:

Following the procedure of example 3e) except substituting the compound from Example 111c) for 2'-hydroxy-3'-nitrobiphenyl-3-carboxylic acid;, the title compound was prepared as a solid (0.81 g, 94 %). MS(ES) m/z 246 (M+H).

### e) 4-aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;

Following the procedure of example 1e), except substituting the compound from Example 111d) for the compound from example 1c), the title compound was obtained as a red solid (0.19 g; 43%). ¹H NMR (300 MHz, DMSO-d₆) δ 13.8 (br s, 1H), 8.74 (d, J = 4.9 Hz, 1H), 8.53 (s, 1H), 7.97 (m,1H), 7.85 (d, J = 7.3 Hz, 1H), 7.78-7.64 (m, 3H), 7.21 (d, J = 8.2 Hz, 1H), 7.09 (t, J = 7.9 Hz, 1H), 2.32 (s, 3H), 2.28 (s, 3H), 2.24 (s, 3H).

### Example 112

### 3'-{N'-[1-(3,4-dimethylphenyl)-5-oxo-1,5-dihydropyrazol-4-ylidene]hydrazino}-2'-hydroxybiphenyl-3-carboxylic acid;

### a) 1-(3,4-dimethylphenyl)-1,2-dihydropyrazol-3-one

A suspension of 3,4-dimethylphenylhydrazine hydrochloride (2.0 g; 0.012 mol.) and potassium carbonate (3.2 g; 0.023 mol.) in anhydrous ethanol (40.0 mL) was treated dropwise with diethyl(ethoxymethyleen)malonate (2.5 g; 0.012 mol.) and the mixture stirred and heated under reflux for 3 h. After cooling the mixture was evaporated and the residue suspended in water and acid;ified to pH 2 followed by extraction with ethyl acetate. After drying and evaporation the resulting intermediate 1-(3,4-dimethyl-phenyl)-3-oxo-2,3-dihydro-1H pyrazole-4-carboxylic acid; ethyl ester was dissolved in methanol (10.0 mL) and 10% aqueous sodium hydroxide (10.0 mL) and stirred at room temperature for 3 h and then heated under reflux to effect hydrolysis. The solution was cooled and acid;ified to pH 4 with 3M aqu. hydrochloric acid; and stirred at room temperature for 2h to effect decarboxylation. The mixture was extracted with ethyl acetate, dried and evaporated to give the title compound (87%) as a yellow solid. ¹H NMR (300 MHz, CDCl₃) δ 7.61 (d, J = 2.3 Hz, 1H), 7.55 (dd, J = 8.2 and 2.3 Hz, I H), 7.48 (t, J = 1.3 Hz, 1H), 1.16 (d, J = 8.2 Hz, 1H), 3.50 (d, J = 1.3 Hz, 2H).

### b) 3'-{N'-[1-(3,4-dimethylphenylj-5-oxo-1,5-dihydropyrazol-4-ylidene)hydrazino)-2'-hydroxybiphenyl-3-carboxylic acid;

Following the procedure of example 1e), except substituting the compound from Example 112d) for the compound from example 1c), the title compound was obtained as a red needles. mp 226-228 °C (ethanol). ¹H NMR (300 MHz, DMSO-d₆) δ 8.20 (s, 1H), 8.14 (s, 1H), 7.97 (m, 1H), 7.81 (m, 1H), 7.73-7.61 (m, 4H), 7.26-7.13 (m,3H), 2.29 (s, 3H), 2.25 (s, 3H). Anal. (C₂₄H₂₀N₄O₄.1.0 CH₃CH₂OH) calcd: C, 65.87; H, 5.52; N, 11.81. found: C, 65.17; H, 5.73; N, 11.58.

### Example 113

### 3'-{N'-[1-(3,4-dimethylphenyl)-3-methyl-5-oxo-1,5-dihydropyrazol-4-ylidene]hydrazino}-2'-hydroxybiphenyl-3-sulfonic acid;

### a) 5'-chloro-2'-methoxybiphenyl-3-sulfonic acid;:

Following the procedure of Example 1b), except substituting 2-methoxy-5-chlorophenylboronic acid; for 4-carboxyphenylboronic acid; , and substituting 3-bromobenzenesulfonic acid; for the compound of 1a), the title compound was prepared (0.41 g) as a white powder. MS(ES) m/z 299 [M+H].

### b) 5'-chloro-2'-hydroxybiphenyl-3-sulfonic acid;:

Follow the procedure of example 3d), except substituting from Example 113a) for the compound from example 3c), the title compound was prepared as a solid (0.87 g; 99 %). MS(ES) m/z 285 [M+H].

### c) 5'-chloro-2'-methoxy-3'-nitrobiphenyl-3-sulfonic acid;:

Following the procedure of Example 7c), except substituting the compound of 113b) for the compound of 7c), the title compound was prepared as a yellow solid. MS(ES) m/z 330 [M+H] used directly in the following step without further purification.

### d) 3'-amino-2'-hydroxybiphenyl-3-sulfonic acid;:

Following the procedure of example 3e) except substituting the compound from Example 113c) for 2'-hydroxy-3'-nitrobiphenyl-3-carboxylic acid;, the title compound was prepared as a solid MS(ES) m/z 266 (M+H) used directly in the following step without further purification.

### e) 3'-{N'-[1-(3,4-dimethylphenyl)-3-methyl-5-oxo-1,5-dihydropyrazol-4-ylidene]hydrazino}-2'-hydroxybiphenyl-3-sulfonic acid;:

Following the procedure of example 1e), except substituting the compound from Example 110d) for the compound from example 1c), the title compound was obtained as a red solid (0.01 g). MS(ES) m/z 479 (M+H).

### Example 114

### 5-(3'-{N'-[1-(3,4-Dimethylphenyl)-3-methyl-5-oxo-1,5-dihydropyrazol-4-ylidene]hydrazino}-2'-hydroxybiphenyl-3-ylmethylene)thiazolidine-2,4-dione;

### a) 3-Methoxy-4'-nitrobiphenyl-4-carbaldehyde

A solution of 1-bromo-2-methoxy-3-nitrobenzene (2.30 g; 9.9 mmol) in 1,4-dioxane (80.0 mL) was treated with tetrakis(triphenylphosphine)palladium(0) (0.15 g). After 2 min. 3-formylbenzeneboronic acid (1.48 g; 9.9 mmol) was added followed by of 2M aqu. sodium carbonate (9.9 mL; 19.8 mmol). The reaction mixture was stirred and heated under reflux for 18h. The reaction was cooled to room temperature and partitioned between ethyl acetate and 3M aqueous hydrochloric acid. The organic layer was separated and the aqueous layer was extracted with ethyl acetate (2x). The combined organic layers were washed with brine, dried and evaporated and the residue was purified by chromotography (silica gel, 5 % ethyl acetate/hexanes) to give the title compound (1.62g; 63%) as a yellow oil. MS(ES+) m/e 258 [M+H]⁺.

### b) 5-(2'-Methoxy-3'-nitrobiphenyl-3-ylmethylene)thiazolidine-2,4-dione

A solution of 3-methoxy-4'-nitrobiphenyl-4-carbaldehyde in (1.62 g; 6.3 mmol) in ethanol (35.0 mL) was treated with 2,4-thiazolidinedione (738 mg; 6.3 mmol) then with piperidine (100 uL). The mixture was stirred and heated under reflux for 12h. Additional 4-thiazolidinedione (370 mg; 3.1 mmol) was added and the reaction was stirred and heated under reflux for a further 1h with the formation of a yellow precipitate. The reaction was cooled to room temperature, filtered and the precipitate was washed with ethanol to give the title compound (880 mg; 40%) as a yellow solid.

### c) 5-(2'-Hydroxy-3'-nitro-biphenyl-3-ylmethylene)thiazolidine-2,4-dione

A solution of 5-(2'-methoxy-3'-nitrobiphenyl-3-ylmethylene)thiazolidine-2,4-dione (875 mg; 2.45 mmol) in glacial acetic acid (25.0 mL) was treated with 48% aqueous hydrobromic acid solution (25.0 mL). and the solution was stirred and heated under reflux for 12h. The reaction was cooled to room temperature and filtered to give 400 mg (48 %) of 5-(2'-hydroxy-3'-nitro-biphenyl-3-ylmethylene)-thiazolidine-2,4-dione as a bright yellow solid.

### d) 5-(3'-Amino-2'-hydroxybiphenyl-3-ylmethylene)thiazolidine-2,4-dione

A solution of 5-(2'-hydroxy-3'-nitrobiphenyl-3-ylmethylene)thiazolidine-2,4-dione in (400 mg; 1.17 mmol) in concentrated hydrochloric acid (20.0 mL) was added tin dichloride (640 mg; 3.36 mmol) and the mixture was stirred and heted under reflux for 12h. The reaction is cooled to room temperature, filtered and the precipitate is washed with water to give a solid which was purified by chromatography (ODS silica, gradient elution, [10-90% acetonitrile/water (0.1 % TFA)] to give the title compound (120 mg; 33%) as an orange solid. MS(ES+) m/e 313 [M+H]'.

### e) 5-(3'-{N'-[1-(3,4-Dimethylphenyl)-3-meihyl-5-oxo-1,5-dihydro-pyrazol-4-ylidene]hydrazino}-2'-hydroxybiphenyl-3-ylmethylene)thiazolidine-2,4-dione

Following the procedure of Example I (e) except substituting the compound from Example 114(d) for the compound from Example 1(c), the title compound was prepared (52 mg; 100%) as a brown solid. MS(ES+) m/e 526 [M+H]⁺.

### Example 115- Capsule Composition

An oral dosage form for administering a presently invented agonist of the TPO receptor is produced by filing a standard two piece hard gelatin capsule with the ingredients in the proportions shown in Table I, below.

**Table I**

| INGREDIENTS | AMOUNTS |
|---|---|
| 4'-{N'-[1-(3,4-Dimethylphenyl)-3-meihyl-5-oxo-1,5- | 25 mg |
| dihydropyrazol-4-ylidene]hydrazino}-3'-hydroxybiphenyl-4- | |
| carboxylic acid; (Compound of Example 1) | |
| Lactose | 55 mg |
| Talc | 16 mg |
| Magnesium Stearate | 4 mg |

### Example 116 - Injectable Parenteral Composition

An injectable form for administering a presently invented agonist of the TPO receptor is produced by stirring 1.5% by weight of 4'-{N'-[1-(3,4-dimethylphenyl)-3-methyl-5-oxo-1,5-dihydropyrazol-4-ylidene]hydrazino}-3'-hydroxybiphenyl-3-carboxylic acid; (Compound of Example 2) in 10% by volume propylene glycol in water.

### Example 117 - Tablet Composition

The sucrose, calcium sulfate dihydrate and a presently invented agonist of the TPO receptor, as shown in Table II below, are mixed and granulated in the proportions shown with a 10% gelatin solution. The wet granules are screened, dried, mixed with the starch, talc and stearic acid;, screened and compressed into a tablet.

**Table II**

| INGREDIENTS | AMOUNTS |
|---|---|
| 3'-{N'-[1-(3,4-dimethylphenyl)-3-methyl-5-oxo-1,5- | 20 mg |
| dihydropyrazol-4-ylidene]hydrazino}-2'-hydroxybiphenyl-3- | |
| carboxylic acid; | |
| (Compound of Example 3) | |
| calcium sulfate dehydrate | 30 mg |
| sucrose | 4 mg |
| starch | 2 mg |
| talc | 1 mg |
| stearic acid | 0.5 mg |

Preferred among the compounds of the present invention are the following;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-carboxylic acid;
3'-{N'-[1-(3,4-Dimethylphenyl)-3-methyl-5-oxo-1,5-dihydropyrazol-4-ylidene]hydrazino}-2'-hydroxybiphenyl-3-carboxylic acid;
3-{N'-[1-(3,4-dimethylphenyl)-3-methyl-5-oxo-1,5-dihydropyrazol-4-ylidene]hydrazino}-2-hydroxy-3'-tetrazol-5-ylbiphenyl;
3'-{N'-[1-(3-fluoro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-ethyl-5-oxo-1,5-dihydropyrazol-4-ylidene]hydrazino}-2'-hydroxybiphenyl-3-carboxylic acid;
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl;
3'-{N'-[1-(3-chloro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidenelhydrazino}-5'-fluoro-2'-hydroxybiphenyl-3-carboxylic acid;
3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;
3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid;
3'-{N'-[3-methyl-1-(4-methylphenyl)-5-oxo-1,5-dihydropyrazol-4-ylidene]hydrazino}-2'-hydroxybiphenyl-3-carboxylic acid;
[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[1-(3,5-dimethylphenyl)-3-methyl-5-oxo-1,5-dihydropyrazol-4-ylidene]hydrazino}-2'-hydroxybiphenyl-3-carboxylic acid;
(3-{N'-[1-(3,4-dimethylphenyl)-3-methyl-5-oxo-1,5-dihydropyrazol-4-ylidene]hydrazino}-2-hydroxy-3'-biphenyl)-1,1,1,-trifluoromethanesulfonamide; and
3'-{N'-[1-(3,4-dimethylphenyl)-5-oxo-1,5-dihydropyrazol-4-ylidene]hydrazino}-2'-hydroxybiphenyl-3-carboxylic acid.

Particularly preferred among the compounds of the invention are following;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
[1-(4-fluoro-3-methylphenyl)-3-methyl-5-oxo-1,5-dihydropyrazol-4-ylidene]hydrazino} -2'-hydroxybiphenyl-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-a-carboxylic acid;
3'-{N'-[1-(3,4-Dimethylphenyl)-3-methyl-5-oxo-1,5-dihydropyrazol-4-ylidene]hydrazino}-2'-hydroxybiphenyl-3-carboxylic acid;
3-{N'-[1-(3,4-dimethylphenyl)-3-methyl-5-oxo-1,5-dihydropyrazol-4-ylidene]hydrazino}-2-hydroxy-3'-tetrazol-5-ylbiphenyl;
3'-{N'-[1-(3-fluoro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-ethyl-5-oxo-1,5-dihydropyrazol-4-ylidene]hydrazino}-2'-hydroxybiphenyl-3-carboxylic acid;
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl;
3'-{N'-[1-(3-chloro-4-methylphenyl)-3-methyl-5-oxo-1,5-dihydropyrazol-4-ylidene]hydrazino)-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;
3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;
3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid; and
3'-{N'-[3-methyl-1-(4-methylphenyl)-5-oxo-1,5-dihydropyrazol-4-ylidene]hydrazino}-2'-hydroxybiphenyl-3-carboxylic acid.

Particularly preferred among the compounds of the invention are following;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-arboxylic acid;
3-{N'-[1-(3,4-dimethylphenyl)-3-methyl-5-oxo-1,5-dihydropyrazol-4-ylidene]hydrazino}-2-hydroxy-3'-tetrazol-5-ylbiphenyl;
3'-{N'-[1-(3,4-Dimethylphenyl)-3-methyl-5-oxo-1,5-dihydropyrazol-4-ylidene]hydrazino}-2'-hydroxybiphenyl-3-carboxylic acid;
3'-{N'-[1-(3-fluoro-4-methylphenyl)-3-methyl-5-oxo-1,5-dihydropyrazol-4-ylidene]hydrazino)-2'-hydroxybiphenyl-3-carboxylic acid;
3'-{N'-[1-(3,4-dimethylphenyl)-3-ethyl-5-oxo-1,5-dihydropyrazol-4-ylidene]hydrazino}-2'-hydroxybiphenyl-3-carboxylic acid; and
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl.

The most preferred among the compounds of the invention is,
3'-{N'-[1-(3,4-Dimethylphenyl)-3-methyl-5-oxo-1,5-dihydropyrazol-4-ylidene]hydrazino}-2'-hydroxybiphenyl-3-carboxylic acid.

The compound 3'- {N'-[1-(3,4-Dimethylphenyl)-3-methyl-5-oxo-1,5-dihydropyrazol-4-ylidene]hydrazino}-2'-hydroxybiphenyl-3-carboxylic acid demonstrated an activity of, EC50 = 0.03 uM, 100%TPO in the above proliferation assay.

While the preferred embodiments of the invention are illustrated by the above, it is to be understood that the invention is not limited to the precise instructions herein disclosed and that the right to all modifications coming within the scope of the following claims is reserved.

## Claims

1. A compound having Formula (VI): 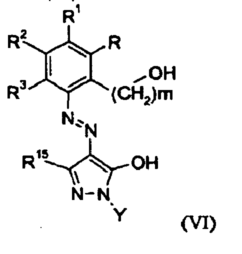 wherein:
R is a substituted aryl; and R¹ is hydrogen;
or:
R is hydrogen; and R¹ is a substituted aryl;
and in either case:
R² and R³ are each independently selected from hydrogen, C₁₋₆alkyl, C₁₋₆alkoxy, nitro, cyano, halogen, aryl, substituted aryl, substituted alkyl, cycloalkyl, phosphonic acid, phosphinic acid and sulfonic acid;
R¹⁵ is selected from the group consisting of alkyl, substituted alkyl, C₁-C₁₂aryl, alkoxy and halogen;
m is 0-4; and
Y is selected from:
phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and wherein:
by the term "aryl" as used herein, is meant a cyclic or polycyclic aromatic ring containing from 1 to 14 carbon atoms and optionally containing from one to five heteroatoms, provided that when the number of carbon atoms is 1 the aromatic ring contains at least four heteroatoms, when the number of carbon atoms is 2 the aromatic ring contains at least three heteroatoms, when the number of carbons is 3 the aromatic ring contains at least two heteroatoms and when the number of carbon atoms is 4 the aromatic ring contains at least one heteroatom;
by the term "C₁-C₁₂aryl" as used herein, is meant phenyl, naphthalene, 3,4-methylenedioxyphenyl, pyridine, biphenyl, quinoline, pyrimidine, quinazoline, thiophene, furan, pyrrole, pyrazole, imidazole or tetrazole; and
by the term "substituted" as used herein, is meant that the subject chemical moiety has one or more substituents selected from the group consisting of:
-CO₂R²⁰, aryl, -C(O)NHS(O)₂R²⁰, -NHS(O)₂R²⁰, hydroxyalkyl, alkoxy, -C(O)NR²¹R²², acyloxy, alkyl, amino, N-acylamino, hydroxy, -(CH₂)_{g}C(O)OR⁸, -S(O)ₙR⁸, nitro, tetrazole, cyano, oxo, halogen, and trifluoromethyl, where g is 0-6, R⁸ is hydrogen or alkyl, R²⁰ is selected from hydrogen, C₁-C₄alkyl, aryl and trifluoromethyl, and R²¹ and R²² are independently selected from hydrogen, C₁-C₄alkyl, aryl and trifluoromethyl, and n is 0-2;
and pharmaceutically acceptable salts, hydrates, and solvates thereof.

2. A compound of Formula VI as claimed in claim 1, wherein:
Ris a substituted C₁-C₁₂aryl;
and
R¹ is hydrogen;
R² and R³ are each independently selected from hydrogen, C₁₋₆alkyl, C₁₋₆alkoxy, nitro, cyano, halogen, substituted alkyl and cycloalkyl;
R¹⁵ is selected from the group consisting of alkyl, substituted alkyl, C₁-C₁₂aryl, alkoxy and halogen;
m is 0-2; and
Y is selected from,
phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, and solvates thereof.

3. A compound of Formula VI as claimed in claim 1, wherein:
R is a substituted phenyl or pyridinyl ring; and
R¹ is hydrogen;
R² and R³ are each independently selected from hydrogen, C₁₋₆alkyl, substituted alkyl and halogen;
R¹⁵ is selected from the group consisting of C₁₋₄alkyl, C₁₋₄alkoxy, C₁-C₁₂aryl and halogen;
m is 0; and
Y is selected from:
phenyl, pyridinyl and pyrimidinyl, where the phenyl, pyridinyl and pyrimidinyl is optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, and solvates thereof.

4. A compound of Formula VI as claimed in claim 1, 2 or 3, wherein:
by the term "substituted" as used herein, unless otherwise defined, is meant that the subject chemical moiety has one substituent selected from the group consisting of:
-CO₂R²⁰, aryl, -C(O)NHS(O)₂R²⁰, -NHS(O)₂R²⁰, hydroxyalkyl, alkoxy, -C(O)NR²¹R²², acyloxy, alkyl, amino, N-acylamino, hydroxy, -(CH₂)_{g}C(O)OR⁸, -S(O)ₙR⁸, nitro, tetrazole, cyano, oxo, halogen, and trifluoromethyl, where g is 0, R⁸ is hydrogen or alkyl, R²⁰ is selected from hydrogen, C₁-C₄alkyl, aryl and trifluoromethyl, and R²¹ and R²² are independently selected from hydrogen, C₁-C₄alkyl, aryl and trifluoromethyl, and n is 0-2.

5. A compound of Formula VI as claimed in claim 1, 2, 3 or 4, wherein:
by the term "alkoxy" as used herein is meant -OCH₃ or -OC(CH₃)₂CH₃;
by the term "cycloalkyl" or "substituted cycloalkyl" as used herein is meant:
cyclohexyl, 4-hydroxy-cyclohexyl, 2-ethylcyclohexyl, propyl 4-methoxycyclohexyl, 4-methoxycyclohexyl, 4-carboxycyclohexyl, cyclopropyl or cyclopentyl;
by the term "acyloxy" as used herein is meant -OC(O)CH₃, -OC(O)CH(CH₃)₂ or -OC(O)(CH₂)₃CH₃;
by the term "N-acylamino" as used herein is meant -N(H)C(O)CH₃, -N(H)C(O)CH(CH₃)₂ or -N(H)C(O)(CH₂)₃CH₃;
by the term "aryloxy" as used herein is meant phenoxy, 4-fluorophenyloxy or biphenyloxy; and
an alkyl substituent is: -CH₃, -CH₂-CH₃, -CH₂-CH₂-CH₃, -CH(CH₃)₂, -C(CH₃)₃, -(CH₂)₃-CH₃, -CH₂-CH(CH₃)₂, or -CH(CH₃)-CH₂-CH₃.

6. A compound as claimed in claim 1 which is selected from:
4'-{N'-[1-(3,4-Dimethylphenyl)-3-methyl-5-oxo-1,5-dihydropyrazol-4-ylidene]hydrazino}-3'-hydroxybiphenyl-4-carboxylic acid;
4'-{N'-[1-(3,4-Dimethylphenyl)-3-methyl-5-oxo-1,5-dihydropyrazol-4-ylidene]hydrazino}-3'-hydroxybiphenyl-3-carboxylic acid;
; 3'-{N'-[1-(4-tert-Butylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-5'-chloro-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxybiphenyl-3-carboxylic acid;
3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid;
2-Aza-5'-chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxy-5'-methylbiphenyl-3-carboxylic acid;
2-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-3-carboxylic acid;
3'-{N'-[1-(4-tert-Butylphenyl)-3-methyl-5-oxo-1,5-dihydropyrazol-4-ylidene]hydrazino}-2'-hydroxy-5'-methylbiphenyl-3-carboxylic acid;
3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;
7-({N'-[1-(3,4-dimethylphenyl)-3-methyl-5-oxo-1,5-dihydropyrazol-4-ylidene]hydrazino}-2-hydroxyphenyl)quinolin-4[1H]-one-3-carboxylic acid;
7-({N'-[1-(4-tert-butylphenyl)-3-methyl-5-oxo-1,5-dihydropyrazol-4-ylidene]hydrazino}-2-hydroxyphenyl)quinolin-4[1H]-one-3-carboxylic acid;
3-Aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3-Aza-3'-(N'-[1-{3-methyl-[4-(1-methylethyl)phenyl]-5-oxo-1,5-dihydropyrazol-4-ylidene}hydrazino)-2'-hydroxybiphenyl-5-carboxylic acid;
3-Aza-3'-{N'-[ 1-(4-tertbutylphenyl-3-methyl-5-oxo-1,5-dihydropyrazol-4-ylidene]hydrazino}-2'-hydroxybiphenyl-5-carboxylic acid;
5'-Chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-Dimethylphenyl)-3,5-dioxo-1,5-dihydropyrazol-4-ylidene]hydrazino}-2'-hydroxybiphenyl-3-carboxylic acid;
3'-{N'-[1-(2-Ethoxy-2-oxoethyl)-3-methyl-5-oxo-1,5-dihydropyrazol-4-ylidene]hydrazino}-2'-hydroxybiphenyl-3-carboxylic acid;
3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-(N'-{ 1-[2-(N-tert-butyl)amino-2-oxoethyl]-3-methyl-5-oxo-1,5-dihydropyrazol-4-ylidene}hydrazino)-2'-hydroxybiphenyl-3-carboxylic acid;
3'-{N'-[3-Chloro-1-(3,4-dimethylphenyl)-5-oxo-1,5-dihydropyrazol-4-ylidene]hydrazino}-2'-hydroxybiphenyl-3-carboxylic acid;
5-chloro-3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-{N'-[1-(3,4-Dimethylphenyl)-3-methyl-5-oxo-1,5-dihydropyrazol-4-ylidene]hydrazino}-2'-hydroxybiphenyl-3,5-dicarboxylic acid;
3-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-5-carboxylic acid;
3'-{N'-[1-(3,4-Dimethylphenyl)-3-methyl-5-oxo-1,5-dihydropyrazol-4-ylidene]hydrazino}-2'-hydroxybiphenyl-4-carboxylic acid;
3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;
3'-{N'-[1-(4-methoxyphenyl)-3-methyl-5-oxo-1,5-dihydropyrazol-4-ylidene]hydrazino}-2'-hydroxybiphenyl-3-carboxylic acid;
(3-{N'-[1-(3,4-dimethylphenyl)-3-methyl-5-oxo-1,5-dihydropyrazol-4-ylidene]hydrazino}-2-hydroxy-3'-biphenyl)-1,1,1,-trifluoromethanesulfonamide;
3'-{N'-[1-(3,4-Dichlorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(3-trifluoromethylphenyl)-1,5-dihydropyrazol-4-ylidene]hydrazino}-2'-hydroxybiphenyl-3-carboxylic acid;
8-{N'-[1-(3,4-dimethylphenyl)-3-methyl-5-oxo-1,5-dihydropyrazol-4-ylidene]hydrazino}quinolin-4[1H]-one-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-carboxylic acid;
3'-{N'-[3-methyl-5-oxo-1-(4-N-methylcarboxamidolphenyl)-1,5-dihydropyrazol-4-ylidene]hydrazino}-2'-hydroxybiphenyl-3-carboxylic acid;
N-[1-(3'-{N'-[1-(3,4-dimethylphenyl)-3-methyl-5-oxo-1,5-dihydropyrazol-4-ylidene]hydrazino}-2'-hydroxybiphenyl-3-yl)methanoyl]methanesulfonamide;
3'-{N'-[3-methyl-5-oxo-1-phenyl-1,5-dihydropyrazol-4-ylidene]hydrazino}-2'-hydroxybiphenyl-3-carboxylic acid;
3'-{N'-[3-methyl-1-(4-methylphenyl)-5-oxo-1,5-dihydropyrazol-4-ylidene]hydrazino}-2'-hydroxybiphenyl-3-carboxylic acid;
3'-{N'-[1-(4-chlorophenyl)-3-methyl-5-oxo-1,5-dihydropyrazol-4-ylidene]hydrazino}-2'-hydroxybiphenyl-3-carboxylic acid;
3'-{N'-[1-(4-fluorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethoxyphenyl)-1,5-dihydropyrazol-4-ylidene]hydrazino}-2'-hydroxybiphenyl-3-carboxylic acid;
3'-{N'-[1-(3,4-dimethylphenyl)-3-ethoxy-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethoxy)-5-oxo-1,5-dihydropyrazol-4-ylidene]hydrazino}-2'-hydroxybiphenyl-3-carboxylic acid;
3'-{N'-[3-tert-butyl-1-(3,4-dimethylphenyl)-5-oxo-1,5-dihydropyrazol-4-ylidene]hydrazino}-2'-hydroxybiphenyl-3-carboxylic acid;
3'-{N'-[3-methyl-1-(4-methyl-2,3,5,6-tetrafluorophenyl)-5-oxo-1,5-dihydropyrazol-4-ylidene]hydrazino}-2'-hydroxybiphenyl-3-carboxylic acid;
3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[1-(3.4-dimethylphenyl)-3-phenyl-5-oxo-1,5-dihydropyrazol-4-ylidene]hydrazino}-2'-hydroxybiphenyl-3-carboxylic acid;
3-{N'-[1-(3,4-dimethylphenyl)-5-oxo-3-phenyl-1,5-dihydropyrazol-4-ylidene]hydrazino}-2-hydroxy-3'-tetrazol-5-ylbiphenyl;
3-{N'-[1-(3,4-dimethylphenyl)-3-methoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-ethoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-(1-methylethoxy)-5-oxo-1,5-dihydropyrazol-4-ylidene]hydrazino}-2-hydroxy-3'-tetrazol-5-ylbiphenyl;
3-{N'-[1-(4-fluorophenyl)-3-methyl-5-oxo-1,5-dihydropyrazol-4-ylidene]hydrazino}-2-hydroxy-3'-tetrazol-5-ylbiphenyl;
3-{N'-[1-(4-fluoro-3-methylphenyl)-3-methyl-5-oxo-1,5-dihydropyrazol-4-ylidene]hydrazino}-2-hydroxy-3'-tetrazol-5-ylbiphenyl;
3-{N'-[3-methyl-5-oxo-1-(4-trifluoromethylphenyl)-1,5-dihydropyrazol-4-ylidene]hydrazino}-2-hydroxy-3'-tetrazol-5-ylbiphenyl;
3'-{N'-[1-(3.4-dimethylphenyl)-3-(pyridin-4-yl-5-oxo-1,5-dihydropyrazol-4-ylidene]hydrazino}-2'-hydroxybiphenyl-3-carboxylic acid;
3-{N'-[1-(3,4-dimethylphenyl)-3 -pyridin-4-yl-5-oxo-1,5-dihydropyrazol-4-ylidene]hydrazino}-2-hydroxy-3'-tetrazol-5-ylbiphenyl;
3-{N'-[1-(3,4-dimethylphenyl)-3-pyridin-2-yl-5-oxo-1,5-dihydropyrazol-4-ylidene]hydrazino }-2-hydroxy-3'-tetrazol-5-ylbiphenyl;
3'-{N'-[1-(3.4-dimethylphenyl)-3-(pyridin-2-yl-5-oxo-1,5-dihydropyrazol-4-ylidene]hydrazino}-2'-hydroxybiphenyl-3-carboxylic acid;
3-{N'-[1-(3-fluoro-4methylphenyl)-3-methyl-5-oxo-1,5-dihydropyrazol-4-ylidene]hydrazino}-2-hydroxy-3'-tetrazol-5-ylbiphenyl;
3'-{N'-[1-(3-fluoro-4-methylphenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethylpyrimidin-2-yl)-1,5-dihydropyrazol-4-ylidene]hydrazino}-2'-hydroxybiphenyl-3-carboxylic acid;
3'-N-tert-butoxycarbonylamino-3-{N'-[1-(3,4-Dimethylphenyl)-3-methyl-5-oxo-1,5-dihydropyrazol-4-ylidene]hydrazino}-2-hydroxybiphenyl;
3'-amino-3-{N'-[1-(3,4-dimethylphenyl)-3-methyl-5-oxo-1,5-dihydropyrazol-4-ylidene]hydrazino}-2-hydroxybiphenyl;
3-{N'-[1-(3-fluorophenyl)-3-methyl-5-oxo-1,5-dihydropyrazol-4-ylidene]hydrazino}-2-hydroxy-3'-tetrazol-5-ylbiphenyl;
3'-{N'-[1-(3-fluorophenyl)-3-methyl-5-oxo-1,5-dihydropyrazol-4-ylidene]hydrazino}-2'-hydroxybiphenyl-3-carboxylic acid;
3-{N'-[3-methyl-5-oxo-1-(2,3,4,5,6-pentafluorophenyl)-1,5-dihydropyrazol-4-ylidene]hydrazino}-2-hydroxy-3'-tetrazol-5-ylbiphenyl;
3'-{N'-[3-methyl-5-oxo-1-(2,3,4,5,6-pentafluorophenyl)-1,5-dihydropyrazol-4-ylidene]hydrazino}-2'-hydroxybiphenyl-3-carboxylic acid;
3'-{N'-[1-(3,4-difluorophenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methoxymethyl-5-oxo-1,5-dihydropyrazol-4-ylidene]hydrazino}-2'-hydroxybiphenyl-3-carboxylic acid;
3-{N'-[1-(3,4-dimethylphenyl)-3-methoxymethyl-5-oxo-1,5-dihydropyrazol-4-ylidene]hydrazino}-2-hydroxy-3'-tetrazol-5-ylbiphenyl;
3-{N'-[1-(3,4-difluorophenyl)-3-methyl-5-oxo-1,5-dihydropyrazol-4-ylidene]hydrazino }-2-hydroxy-3'-tetrazol-5-ylbiphenyl;
3'-{N'-[1-(3,4-dimethylphenyl)-5-oxo-3-trifluoromethyl-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-6-fluoro-2'-hydroxybiphenyl-3-carboxylic acid;
3'-{N'-[1-(3,4-dimethylphenyl)-5-oxo-3-propyl-1,5-dihydropyrazol-4-ylidene]hydrazino}-2'-hydroxybiphenyl-3-carboxylic acid;
3-{N'-[1-(3,4-dimethylphenyl)-5-oxo-3-propyl-1,5-dihydropyrazol-4-ylidene]hydrazino}-2-hydroxy-3'-tetrazol-5-ylbiphenyl;
3'-{N'-[1-(3,4-dimethylphenyl)-3-(1-methyl-1H-pyrrol-3-yl)-5-oxo-1,5-dihydropyrazol-4-ylidene]hydrazino}-2'-hydroxybiphenyl-3-carboxylic acid;
3-{N'-[1-(3,4-dimethylphenyl)-3-(1-methyl-1H-pyrrol-3-yl)-5-oxo-1,5-dihydropyrazol-4-ylidene]hydrazino}-2-hydroxy-3'-tetrazol-5-ylbiphenyl;
3'-{N'-[1-(3,4-dimethylphenyl)-3-furan-2-yl-5-oxo-1,5-dihydropyrazol-4-ylidene]hydrazino}-2'-hydroxybiphenyl-3-carboxylic acid;
3-{N'-[1-(3,4-dimethylphenyl)-3-furan-2-yl-5-oxo-1,5-dihydropyrazol-4-ylidene]hydrazino}-2-hydroxy-3'-tetrazol-5-ylbiphenyl;
N-(2'-hydroxy-3'-{N'-[3-methyl-5-oxo-1-(4-trifluoromethyl-phenyl)-1,5-dihydro-pyrazol-4-ylldene]hydrazino}biphenyl-3-yl)-1,1,1-trifluoromethanesulfonamide;
N-(2'-hydroxy-3'-{N'-[1-(3-fluoro-4-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'-{N'-[1-(4-fluoro-3-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'-{N'-[1-(3,4-difluorophenyl)-3-methyl-5-oxo-1,5-dihydro-pyrazol-4-ylidene]hydrazino}biphenyl-3-yl)-1,1,1-trifluoromethanesulfonamide;
N-(3'-{N'-[1-(3,4-dimethylphenyl)-3-methyl-5-oxo-1,5-dihydropyrazol-4-ylidene]hydrazino}-2'-hydroxybiphenyl-3-yl)guanidine;
3'-N'-[1-(3,4-dimethylphenyl)-3-ethyl-5-oxo-1,5-dihydropyrazol-4-ylidene]hydrazino}-2'-hydroxybiphenyl-3-carboxylic acid;
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-thien-2-yl-1,5-dihydropyrazol-4-ylidene]hydrazino}-2'-hydroxybiphenyl-3-carboxylic acid;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-5-oxo-3-thiazol-2-yl-1,5-dihydropyrazol-4-ylidene]hydrazino}-2'-hydroxybiphenyl-3-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethyl)-5-oxo-1,5-dihydropyrazol-4-ylidene]hydrazino}-2'-hydroxybiphenyl-3-carboxylic acid;
3'-{N'-[3-(benzyloxymethyl)-1-(3,4-dimethylphenyl)-5-oxo-1,5-dihydropyrazol-4-ylidene]hydrazino}-2'-hydroxybiphenyl-3-carboxylic acid;
3'-{N'-[3-ethyl-5-oxo-1-(4-trifluoromethylphenyl)-1,5-dihydropyrazol-4-ylidene]hydrazino}-2'-hydroxybiphenyl-3-carboxylic acid;
3'-{N'-[5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-hydroxymethyl-5-oxo-1,5-dihydropyrazol-4-ylidene]hydrazino}-2'-hydroxybiphenyl-3-carboxylic acid;
3'-{N'-[3-benzyloxymethyl-5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-methylsulfanylmethyl-5-oxo-1,5-dihydropyrazol-4-ylidene]hydrazino}-2'-hydroxybiphenyl-3-carboxylic acid;
3'-{N'-[-1-(3,4-dimethylphenyl)-5-oxo-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-methylsulfanylmethyl-1,5-dihydropyrazol-4-ylidene]hydrazino}-2'-hydroxybiphenyl-3-carboxylic acid;
N-(3'-{N'-[1-(3,4-dimethylphenyl)-3-methyl-5-oxo-1,5-dihydro-pyrazol-4-ylidene]hydrazinol-2'-hydroxybiphenyl-3-yl)methanesulfonamide;
3'-[N'-(1-benzo[1,3]dioxol-5-yl-3-methyl-5-oxo-1,5-dihydropyrazol-4-ylidene)hydrazino]-2'-hydroxybiphenyl-3-carboxylic acid;
3'-{N'-[1-(3,5-dimethylphenyl)-3-methyl-5-oxo-1,5-dihydropyrazol-4-ylidene]hydrazino}-2'-hydroxybiphenyl-3-carboxylic acid;
3'-{N'-[1-(3-chloro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-phosphonic acid;
3'-{N'-[1-(3,4-dimethylphenyl)-3-methyl-5-oxo-1,5-dihydropyrazol-4-ylidene]hydrazino}-2'-hydroxybiphenyl-3,4-dicarboxylic acid;
2',6-dihydroxy-3'-{N'-[1-(3,4-dimethylphenyl)-3-methyl-5-oxo-1,5-dihydropyrazol-4-ylidene]hydrazino}biphenyl-3-carboxylic acid;
4-aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-sulfonic acid; and
5-(3'-{N'-[1-(3,4-Dimethylphenyl)-3-methyl-5-oxo-1,5-dihydropyrazol-4-ylidene]hydrazino}-2'-hydroxybiphenyl-3-ylmethylene)thiazolidine-2,4-dione;
and pharmaceutically acceptable salts, hydrates, and solvates thereof.

7. A medicament for use in therapy, manufactured using and comprising a compound of Formula (VI) as defined in any one of claims 1 to 6.

8. A pharmaceutical composition comprising a compound of Formula (VI), as defined in any one of claims 1 to 6, and a pharmaceutically acceptable carrier.

9. A pharmaceutical composition for use in enhancing platelet production in a human in need thereof which comprises a compound of Formula (VI), as defined in any one of claims 1 to 6, and a pharmaceutically acceptable carrier,
and wherein the composition is for administration to the human of a therapeutically effective amount of the compound of Formula (VI).

10. A pharmaceutical composition for use in the treatment of thrombocytopenia which comprises a compound of Formula (VI), as defined in any one of claims 1 to 6, and a pharmaceutically acceptable carrier.

11. A pharmaceutical composition as claimed in claim 8, 9 or 10, which is for oral administration.

12. Use of a compound of Formula (VI), as defined in any one of claims 1 to 6, in the manufacture of a medicament for use in treating thrombocytopenia.

13. Use of a compound of Formula (VI), as defined in any one of claims 1 to 6, in the manufacture of a medicament for use in enhancing platelet production in a human in need thereof, and wherein the medicament is for administration to the human of a therapeutically effective amount of the compound of Formula (VI).

14. The use as claimed in claim 12 or 13, wherein the medicament is for oral administration.

15. A process for preparing a pharmaceutical composition containing a pharmaceutically acceptable carrier or diluent and an effective amount of a compound of the Formula (VI) as described in claim 1 and pharmaceutically acceptable salts, hydrates and solvates thereof, which process comprises bringing the compound of the Formula (VI) into association with the pharmaceutically acceptable carrier or diluent.

16. A process for preparing a compound of Formula (VI) as defined in any one of claims 1 to 6,
the process comprising reaction of a compound of Formula (VII) 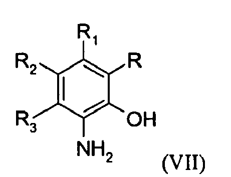 or a protected form thereof with a compound of Formula (VIII) or tautomeric equivalent (IX) 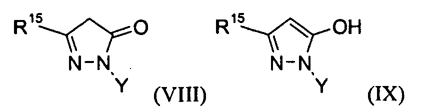 wherein
either:
R is a phenyl or pyridinyl ring substituted by one or more of the following:
-C(O)OH, tetrazole, sulfonic acid, hydroxy, methyl, fluoro, NHSO₂CF₃, and C(O)NHSO₂CH₃; and R¹ is hydrogen,
or:
R is hydrogen; and R¹ is a phenyl or pyridinyl ring substituted by one or more of the following: -C(O)OH, tetrazole, sulfonic acid, hydroxy, methyl, fluoro,
NHSO₂CF₃, and C(O)NHSO₂CH_{3;}
and in either case:
R² and R³ are independently selected from hydrogen, C₁₋₆alkyl and halogen;
R¹⁵ is selected from the group consisting of C₁₋₆alkyl, C₁₋₆alkoxy, trifluoromethyl and halogen; and
Y is selected from,
phenyl that is optionally substituted with from one to three substituents selected from the group consisting of: alkyl, substituted alkyl, trifluoromethyl and halogen; and
followed if necessary or desired by salt formation.

17. A pharmaceutical composition as claimed in Claim 9, for co-administration of a therapeutically effective amount of an agent selected from the group consisting of: a colony stimulating factor, cytokine, chemokine, interleukin or cytokine receptor agonist.

18. The composition as claimed in Claim 17 wherein the agent is selected from the group consisting of: G-CSF, GM-CSF, TPO, M-CSF, EPO, Gro-beta, IL-11, SCF, FLT3 Ligand, LIF, IL-3, IL-6, IL-1, or IL-5 or a biologically active derivative of any of said agents.

19. An in vitro method for enhancing stimulation of megakaryocyte maturation and/or platelet production which comprises adding an effective amount of a compound as defined in claim 1 to the culture medium of cells that express the TPO receptor.

20. An in vitro method for enhancing stimulation of megakaryocyte maturation and/or platelet production which comprises adding an effective amount of a compound as defined in claim 1 to the culture medium of stem cells, bone marrow cells, cord-blood cells or peripheral blood cells.

21. An in vitro method for enhancing the survival and/or proliferation of stem cells, bone marrow cells, cord-blood cells, peripheral blood cells or other types of cells expressing the TPO receptor in culture which comprises culturing said cell in a medium containing an effective amount of a compound as defined in claim 1.

22. A method as claimed in claim 21 further comprising co-administration of a therapeutically effective amount of a colony stimulating factor, cytokine, chemokine, interleukin or cytokine receptor agonist.

23. A compound of Claim 1, for use as an immunological adjuvant.

24. A compound according to claim 23, for use as an immunological adjuvant and for administration with a vaccine and/or immunomodulator.

25. A compound as claimed in Claim 6 which is selected from:
3'-{N'-[1-(4-tert-Butylphenyl)-3-methyl-5-oxo-1,5-dihydropyrazol-4-ylidene]hydrazino}-2'-hydroxybiphenyl-3-carboxylic acid
3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-carboxylic acid; and
3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid.

## Patentansprüche

1. Verbindung mit der Formel (VI): 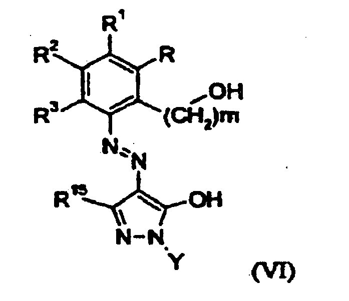 worin:
R ein substituiertes Aryl ist; und R¹ Wasserstoff ist;
oder:
R Wasserstoff ist; und R¹ ein substituiertes Aryl ist;
und in jedem Fall:
R² und R³ jeweils unabhängig ausgewählt sind aus Wasserstoff, C₁₋₆-Alkyl, C₁₋₆-Alkoxy, Nitro, Cyano, Halogen, Aryl, substituiertem Aryl, substituiertem Alkyl, Cycloalkyl, Phosphonsäure, Phosphinsäure und Sulfonsäure;
R¹⁵ ausgewählt ist aus der Gruppe, bestehend aus Alkyl, substituiertem Alkyl, C₁₋₁₂-Aryl, Alkoxy und Halogen;
m 0 bis 4 ist; und
Y ausgewählt ist aus:
Phenyl, Pyridinyl und Pyrimidinyl, wobei das Phenyl, Pyridinyl und Pyrimidinyl gegebenenfalls mit von einem bis drei Substituenten substituiert sind, die ausgewählt sind aus der Gruppe, bestehend aus: Alkyl, substituiertem Alkyl, C₁₋₁₂-Aryl, substituiertem C₁₋₁₂-Aryl, Alkoxy und Halogen;
und worin:
mit dem Begriff "Aryl", wie hier verwendet, ein cyclischer oder polycyclischer aromatischer Ring gemeint ist, der von 1 bis 14 Kohlenstoffatome enthält und gegebenenfalls von einem bis fünf Heteroatome enthält, vorausgesetzt, daß, wenn die Zahl der Kohlenstoffatome 1 ist, der aromatische Ring mindestens 4 Heteroatome enthält, wenn die Zahl der Kohlenstoffatome 2 ist, der aromatische Ring mindestens 3 Heteroatom enthält, wenn die Zahl der Kohlenstoffatome 3 ist, der aromatische Ring mindestens 2 Heteroatome enthält, und, wenn die Zahl der Kohlenstoffatome 4 ist, der aromatische Ring mindestens 1 Heteroatom enthält;
mit dem Begriff "C₁₋₁₂-Aryl", wie er hier verwendet, Phenyl, Naphthalin, 3,4-Methylendioxyphenyl, Pyridin, Biphenyl, Chinolin, Pyrimidin, Chinazolin, Thiophen, Furan, Pyrrol, Pyrazol, Imidazol oder Tetrazol gemeint ist; und
mit dem Begriff "substituiert", wie hier verwendet, gemeint ist, daß der betreffende chemische Rest einen oder mehrere Substituenten aufweist, ausgewählt aus der Gruppe, bestehend aus: -CO₂R²⁰, Aryl, -C(O) NHS(O)₂R²⁰, -NHS(O)₂R²⁰, Hydroxyalkyl, Alkoxy, -C(O)NR²¹R²², Acyloxy, Alkyl, Amino, N-Acylamino, Hydroxy, -(CH₂)_{g}C(O)OR⁸, -S(O)ₙR⁸, Nitro, Tetrazol, Cyano, Oxo, Halogen und Trifluormethyl, worin g 0 bis 6 ist, R⁸ Wasserstoff oder Alkyl ist, R²⁰ ausgewählt ist aus Wasserstoff, C₁₋₄-Alkyl, Aryl und Trifluormethyl, und R²¹ und R²² unabhängig ausgewählt sind aus Wasserstoff, C₁₋₄-Alkyl, Aryl und Trifluormethyl, und n 0 bis 2 ist;
und pharmazeutisch annehmbare Salze, Hydrate und Solvate davon.

2. Verbindung der Formel (VI) gemäß Anspruch 1, worin:
R ein substituiertes C₁₋₁₂-Aryl ist;
und
R¹ Wasserstoff ist;
R² und R³ jeweils unabhängig ausgewählt sind aus Wasserstoff, C₁₋₆-Alkyl, C₁₋₆-Alkoxy, Nitro, Cyano, Halogen, substituiertem Alkyl und Cycloalkyl;
R¹⁵ ausgewählt ist aus der Gruppe, bestehend aus Alkyl, substituiertem Alkyl, C₁₋₁₂-Aryl, Alkoxy und Halogen;
m 0 bis 2 ist; und
Y ausgewählt ist aus Phenyl, Pyridinyl und Pyrimidinyl, wobei das Phenyl, Pyridinyl und Pyrimidinyl gegebenenfalls mit von einem bis drei Substituenten substituiert sind, ausgewählt aus der Gruppe, bestehend aus: Alkyl, substituiertem Alkyl, C₁₋₁₂-Aryl, substituiertem C₁₋₁₂-Aryl, Alkoxy und Halogen;
und pharmazeutisch annehmbare Salze, Hydrate und Solvate davon.

3. Verbindung der Formel (VI) gemäß Anspruch 1, worin:
R ein substituierter Phenyl- oder Pyridinyl-Ring ist; und
R¹ Wasserstoff ist;
R² und R³ jeweils unabhängig ausgewählt sind aus Wasserstoff, C₁₋₆-Alkyl, substituiertem Alkyl und Halogen;
R¹⁵ ausgewählt ist aus der Gruppe, bestehend aus C₁₋₄-Alkyl, C₁₋₄-Alkoxy, C₁₋₁₂-Aryl und Halogen;;
m 0 ist; und
Y ausgewählt ist aus:
Phenyl, Pyridinyl und Pyrimidinyl, wobei das Phenyl, Pyridinyl und Pyrimidinyl gegebenenfalls mit von einem bis drei Substituenten substituiert sind, ausgewählt aus der Gruppe, bestehend aus: Alkyl, substituiertem Alkyl, C₁₋₁₂-Aryl, substituiertem C₁₋₁₂-Aryl, Alkoxy und Halogen;
und pharmazeutisch annehmbare Salze, Hydrate und Solvate davon.

4. Verbindung der Formel (VI) gemäß Anspruch 1, 2 oder 3, worin:
mit dem Begriff "substituiert", wie hier verwendet, sofern nicht anders festgelegt, gemeint ist, daß der betreffende chemische Rest einen Substituenten aufweist, der ausgewählt ist aus der Gruppe, bestehend aus:
-CO₂R²⁰, Aryl, -C(O)NHS(O)₂R²⁰, -NHS(O)₂R²⁰, Hydroxyalkyl, Alkoxy, -C(O)NR²¹R²², Acyloxy, Alkyl, Amino, N-Acylamino, Hydroxy, -(CH₂)_{g}C(O)OR⁸, -S(O)ₙR⁸, Nitro, Tetrazol, Cyano, Oxo, Halogen und Trifluormethyl, worin g 0 ist, R⁸ Wasserstoff oder Alkyl ist, R²⁰ ausgewählt ist aus Wasserstoff, C₁₋₄-Alkyl, Aryl und Trifluormethyl, und R²¹ und R²² unabhängig ausgewählt sind aus Wasserstoff, C₁₋₄-Alkyl, Aryl und Trifluormethyl, und n 0 bis 2 ist.

5. Verbindung der Formel (VI) gemäß Anspruch 1, 2, 3 oder 4, worin:
mit dem Begriff "Alkoxy", wie hier verwendet, -OCH₃ oder -OC(CH₃)₂CH₃ gemeint ist;
mit dem Begriff "Cycloalkyl" oder "substituiertem Cycloalkyl", wie hier verwendet, gemeint ist:
Cyclohexyl, 4-Hydroxy-cyclohexyl, 2-Ethylcyclohexyl, Propyl-4-methoxycyclohexyl, 4-Methoxycyclohexyl, 4-Carboxycyclohexyl, Cyclopropyl oder Cyclopentyl;
mit dem Begriff "Acyloxy" wie hier verwendet, -OC(O)CH₃, -OC(O)CH(CH₃)₂ oder -OC(O)(CH₂)₃CH₃ gemeint ist;
mit dem Begriff "N-Acylamino", wie hier verwendet, -N(H)C(O)CH₃, -N(H)C(O)CH(CH₃)₂ oder -N(H)C(O)(CH₂)₃CH₃ gemeint ist;
mit dem Begriff "Aryloxy", wie hier verwendet, Phenoxy, 4-Fluorphenyloxy oder Biphenyloxy gemeint ist; und
ein Alkyl-Substituent ist: -CH₃, -CH₂-CH₃, -CH₂-CH₂-CH₃, -CH(CH₃)₂, -C(CH₃)₃, -(CH₂)₃-CH₃, -CH₂-CH(CH₃)₂ oder -CH(CH₃)-CH₂-CH₃.

6. Verbindung gemäß Anspruch 1, die ausgewählt ist aus:
4'-{N'-[1-(3,4-Dimethylphenyl)-3-methyl-5-oxo-1,5-dihydropyrazol-4-yliden]hydrazino}-3'-hydroxybiphenyl-4-carbonsäure;
4'-{N'-[1-(3,4-Dimethylphenyl)-3-methyl-5-oxo-1,5-dihydropyrazol-4-yliden]hydrazino}-3'-hydroxybiphenyl-3-carbonsäure;
3'-{N'-[1-(4-tert-Butylphenyl)-3-methyl-5-oxo-1,5-dihydropyrazol-4-yliden]hydrazino}-2'-hydroxybiphenyl-3-carbonsäure;
2-Aza-3'-{N'-[1-(4-tert-butylphenyl)-3-methyl-5-oxo-1,5-dihydropyrazol-4-yliden]hydrazino}-5'-chlor-2'-hydroxybiphenyl-3-carbonsäure;
2-Aza-3'-{N'-[1-(4-tert-butylphenyl)-3-methyl-5-oxo-1,5-dihydropyrazol-4-yliden]hydrazino}-2'-hydroxybiphenyl-3-carbonsäure;
3-Aza-3'-{N'-[1-(4-tert-butylphenyl)-3-methyl-5-oxo-1,5-dihydropyrazol-4-yliden]hydrazino}-2'-hydroxybiphenyl-5-carbonsäure;
2-Aza-5'-chlor-3'-{N'-[1-(3,4-dimethylphenyl)-3-methyl-5-oxo-1,5-dihydropyrazol-4-yliden]hydrazino}-2'-hydroxybiphenyl-3-carbonsäure;
2-Aza-3'-{N'-[1-(4-tert-butylphenyl)-3-methyl-5-oxo-1,5-dihydropyrazol-4-yliden]hydrazino}-2'-hydroxy-5'-methylbiphenyl-3-carbonsäure;
2-Aza-3'-{N'-[1-(3,4-dimethylphenyl)-3-methyl-5-oxo-1,5-dihydropyrazol-4-yliden]hydrazino}-2'-hydroxy-5'-methylbiphenyl-3-carbonsäure;
3'-{N'-[1-(4-tert-Butylphenyl)-3-methyl-5-oxo-1,5-dihydropyrazol-4-yliden]hydrazino}-2'-hydroxy-5'-methylbiphenyl-3-carbonsäure;
3'-{N'-[1-(3,4-Dimethylphenyl)-3-methyl-5-oxo-1,5-dihydropyrazol-4-yliden]hydrazino}-5'-fluor-2'-hydroxybiphenyl-3-carbonsäure;
7'-({N'-[1-(3,4-Dimethylphenyl)-3-methyl-5-oxo-1,5-dihydropyrazol-4-yliden]hydrazino}-2-hydroxyphenyl)chinolin-4[1H]-on-3-carbonsäure;
7'-({N'-[1-(4-tert-Butylphenyl)-3-methyl-5-oxo-1,5-dihydropyrazol-4-yliden]hydrazino}-2-hydroxyphenyl)chinolin-4[1H]-on-3-carbonsäure;
3-Aza-3'-{N'-[1-(3,4-dimethylphenyl)-3-methyl-5-oxo-1,5-dihydropyrazol-4-yliden]hydrazino}-2'-hydroxybiphenyl-5-carbonsäure;
3-Aza-3'-(N'-[1-{3-methyl[4-(1-methylethyl)phenyl]-5-oxo-1,5-dihydropyrazol-4-yliden}hydrazino)-2'-hydroxybiphenyl-5-carbonsäure;
3-Aza-3'-[N'-[1-(4-tert-butylphenyl-3-methyl-5-oxo-1,5-dihydropyrazol-4-yliden]hydrazino}-2'-hydroxybiphenyl-5-carbonsäure;
5-Chlor-3'-{N'-[1-(3,4-dimethylphenyl)-3-methyl-5-oxo-1,5-dihydropyrazol-4-yliden]hydrazino}-2'-hydroxybiphenyl-3-carbonsäure;
3'-{N'-[1-(3,4-Dimethylphenyl)-3,5-dioxo-1,5-dihydropyrazol-4-yliden]hydrazino}-2'-hydroxybiphenyl-3-carbonsäure;
3'-{N'-[1-(2-Ethoxy-2-oxoethyl)-3-methyl-5-oxo-1,5-dihydropyrazol-4-yliden]hydrazino}-2'-hydroxybiphenyl-3-carbonsäure;
3-{N'-[1-(3,4-Dimethylphenyl)-3-methyl-5-oxo-1,5-dihydropyrazol-4-yliden]hydrazino}-2-hydroxy-4'-(tetrazol-5-yl)biphenyl;
3'-(N'-{1-[2-(N-tert-Butyl)amino-2-oxoethyl]-3-methyl-5-oxo-1,5-dihydropyrazol-4-yliden}hydrazino)-2'-hydroxybiphenyl-3-carbonsäure;
3'-{N'-[3-Chlor-1-(3,4-dimethylphenyl)-5-oxo-1,5-dihydropyrazol-4-yliden]hydrazino}-2'-hydroxybiphenyl-3-carbonsäure;
5-Chlor-3-{N'-[1-(3,4-dimethylphenyl)-3-methyl-5-oxo-1,5-dihydropyrazol-4-yliden]hydrazino}-2-hydroxy-4'-(tetrazol-5-yl)biphenyl;
3'-{N'-[1-(3,4-Dimethylphenyl)-3-methyl-5-oxo-1,5-dihydropyrazol-4-yliden]hydrazino}-2'-hydroxybiphenyl-3,5-dicarbonsäure;
3-Aza-3'-{N'-[1-(3,4-dimethylphenyl)-3-methyl-5-oxo-1,5-dihydropyrazol-4-yliden]hydrazino}-2'-hydroxy-5'-methylbiphenyl-5-carbonsäure;
3'-{N'-[1-(3,4-Dimethylphenyl)-3-methyl-5-oxo-1,5-dihydropyrazol-4-yliden]hydrazino}-2'-hydroxybiphenyl-4-carbonsäure;
3'-{N'-[1-(3,4-Dimethylphenyl)-3-methoxy-5-oxo-1,5-dihydropyrazol-4-yliden]hydrazino}-2'-hydroxybiphenyl-3-carbonsäure;
3'-{N'-[1-(4-Methoxyphenyl)-3-methyl-5-oxo-1,5-dihydropyrazol-4-yliden]hydrazino}-2'-hydroxybiphenyl-3-carbonsäure;
(3-{N'-[1-(3,4-Dimethylphenyl)-3-methyl-5-oxo-1,5-dihydropyrazol-4-yliden]hydrazino}-2-hydroxy-3'-biphenyl)-1,1,1-trifluormethansulfonamid;
3'-[N'-[1-(3,4-Dichlorphenyl)-3-methyl-5-oxo-1,5-dihydropyrazol-4-yliden]hydrazino}-2'-hydroxybiphenyl-3-carbonsäure;
3'-{N'-[3-Methyl-5-oxo-1-(3-trifluormethylphenyl)-1,5-dihydropyrazol-4-yliden]hydrazino}-2'-hydroxybiphenyl-3-carbonsäure;
8-{N'-[1-(3,4-Dimethylphenyl)-3-methyl-5-oxo-1,5-dihydropyrazol-4-yliden]hydrazino}chinolin-4[1H]-on-3-carbonsäure;
3'-{N'-[3-Methyl-5-oxo-1-(4-trifluormethylphenyl)-1,5-dihydropyrazol-4-yliden]hydrazino}-2'-hydroxybiphenyl-3-carbonsäure;
3'-{N'-[3-Methyl-5-oxo-1-(4-N-methylcarboxamidolphenyl)-1,5-dihydropyrazol-4-yliden]hydrazino}-2'-hydroxybiphenyl-3-carbonsäure;
N-[1-(3'-{N'-[1-(3,4-Dimethylphenyl)-3-methyl-5-oxo-1,5-dihydropyrazol-4-yliden]hydrazino}-2'-hydroxybiphenyl-3-yl)methanoyl]methansulfonamid;
3'-{N'-[3-Methyl-5-oxo-1-phenyl-1,5-dihydropyrazol-4-yliden]hydrazino}-2'-hydroxybiphenyl-3-carbonsäure;
3'-{N'-[3-Methyl-1-(4-methylphenyl)-5-oxo-1,5-dihydropyrazol-4-yliden]hydrazino}-2'-hydroxybiphenyl-3-carbonsäure;
3'-{N'-[1-(4-Chlorphenyl)-3-methyl-5-oxo-1,5-dihydropyrazol-4-yliden]hydrazino}-2'-hydroxybiphenyl-3-carbonsäure;
3'-{N'-[1-(4-Fluorphenyl)-3-methyl-5-oxo-1,5-dihydropyrazol-4-yliden]hydrazino}-2'-hydroxybiphenyl-3-carbonsäure;
3'-{N'-[3-Methyl-5-oxo-1-(4-trifluormethoxyphenyl)-1,5-dihydropyrazol-4-yliden]hydrazino}-2'-hydroxybiphenyl-3-carbonsäure;
3'-{N'-[1-(3,4-Dimethylphenyl)-3-ethoxy-5-oxo-1,5-dihydropyrazol-4-yliden]hydrazino}-2'-hydroxybiphenyl-3-carbonsäure;
3'-{N'-[1-(3,4-Dimethylphenyl)-3-(1-methylethoxy)-5-oxo-1,5-dihydropyrazol-4-yliden]hydrazino}-2'-hydroxybiphenyl-3-carbonsäure;
3'-{N'-[3-tert-Butyl-1-(3,4-dimethylphenyl)-5-oxo-1,5-dihydropyrazol-4-yliden]hydrazino}-2'-hydroxybiphenyl-3-carbonsäure;
3'-{N'-[3-Methyl-1-(4-methyl-2,3,5,6-tetrafluorphenyl)-5-oxo-1,5-dihydropyrazol-4-yliden]hydrazino}-2'-hydroxybiphenyl-3-carbonsäure;
3'-{N'-[1-(4-Fluor-3-methylphenyl)-3-methyl-5-oxo-1,5-dihydropyrazol-4-yliden]hydrazino}-2'-hydroxybiphenyl-3-carbonsäure;
3'-{N'-[1-(3,4-Dimethylphenyl)-3-phenyl-5-oxo-1,5-dihydropyrazol-4-yliden]hydrazino}-2'-hydroxybiphenyl-3-carbonsäure;
3-{N'-[1-(3,4-Dimethylphenyl)-5-oxo-3-phenyl-1,5-dihydropyrazol-4-yliden]hydrazino}-2-hydroxy-3'-tetrazol-5-ylbiphenyl;
3-{N'-[1-(3,4-Dimethylphenyl)-3-methoxy-5-oxo-1,5-dihydropyrazol-4-yliden]hydrazino}-2-hydroxy-3'-tetrazol-5-ylbiphenyl;
3-{N'-[1-(3,4-Dimethylphenyl)-3-ethoxy-5-oxo-1,5-dihydropyrazol-4-yliden]hydrazino}-2-hydroxy-3'-tetrazol-5-ylbiphenyl;
3-{N'-[1-(3,4-Dimethylphenyl)-3-(1-methylethoxy)-5-oxo-1,5-dihydropyrazol-4-yliden]hydrazino}-2-hydroxy-3'-tetrazol-5-ylbiphenyl;
3-{N'-[1-(4-Fluorphenyl)-3-methyl-5-oxo-1,5-dihydropyrazol-4-yliden]hydrazino}-2-hydroxy-3'-tetrazol-5-ylbiphenyl;
3-{N'-[1-(4-Fluor-3-methylphenyl)-3-methyl-5-oxo-1,5-dihydropyrazol-4-yliden]hydrazino}-2-hydroxy-3'-tetrazol-5-ylbiphenyl;
3-{N'-[3-Methyl-5-oxo-1-(4-trifluormethylphenyl)-1,5-dihydropyrazol-4-yliden]hydrazino}-2-hydroxy-3'-tetrazol-5-ylbiphenyl;
3'-{N'-[1-(3,4-Dimethylphenyl)-3-(pyridin-4-yl-5-oxo-1,5-dihydropyrazol-4-yliden]hydrazino}-2'-hydroxybiphenyl-3-carbonsäure;
3-{N'-[1-(3,4-Dimethylphenyl)-3-pyridin-4-yl-5-oxo-1,5-dihydropyrazol-4-yliden]hydrazino}-2-hydroxy-3'-tetrazol-5-ylbiphenyl;
3-{N'-[1-(3,4-Dimethylphenyl)-3-pyridin-2-yl-5-oxo-1,5-dihydropyrazol-4-yliden]hydrazino}-2-hydroxy-3'-tetrazol-5-ylbiphenyl;
3'-{N'-[1-(3,4-Dimethylphenyl)-3-(pyridin-2-yl-5-oxo-1,5-dihydropyrazol-4-yliden]hydrazino}-2'-hydroxybiphenyl-3-carbonsäure;
3-{N'-[1-(3-Fluor-4-methylphenyl)-3-methyl-5-oxo-1,5-dihydropyrazol-4-yliden]hydrazino}-2-hydroxy-3'-tetrazol-5-ylbiphenyl;
3'-{N'-[1-(3-Fluor-4-methylphenyl)-3-methyl-5-oxo-1,5-dihydropyrazol-4-yliden]hydrazino}-2'-hydroxybiphenyl-3-carbonsäure;
3'-{N'-[3-Methyl-5-oxo-1-(4-trifluormethylpyrimidin-2-yl)-1,5-dihydropyrazol-4-yliden]hydrazino}-2'-hydroxybiphenyl-3-carbonsäure;
3'-N-tert-Butoxycarbonylamino-3-{N'-[1-(3,4-dimethylphenyl)-3-methyl-5-oxo-1,5-dihydropyrazol-4-yliden]hydrazino}-2-hydroxybiphenyl;
3'-Amino-3-{N'-[1-(3,4-dimethylphenyl)-3-methyl-5-oxo-1,5-dihydropyrazol-4-yliden]hydrazino}-2-hydroxybiphenyl;
3-{N'-[1-(3-Fluorphenyl)-3-methyl-5-oxo-1,5-dihydropyrazol-4-yliden]hydrazino}-2-hydroxy-3'-tetrazol-5-ylbiphenyl;
3'-{N'-[1-(3-Fluorphenyl)-3-methyl-5-oxo-1,5-dihydropyrazol-4-yliden]hydrazino}-2'-hydroxybiphenyl-3-carbonsäure;
3-{N'-[3-Methyl-5-oxo-1-(2,3,4,5,6-pentafluorphenyl)-1,5-dihydropyrazol-4-yliden]hydrazino}-2-hydroxy-3'-tetrazol-5-ylbiphenyl;
3'-{N'-[3-Methyl-5-oxo-1-(2,3,4,5,6-pentafluorphenyl)-1,5-dihydropyrazol-4-yliden]hydrazino}-2'-hydroxybiphenyl-3-carbonsäure;
3'-{N'-[1-(3,4-Difluorphenyl)-3-methyl-5-oxo-1,5-dihydropyrazol-4-yliden]hydrazino}-2'-hydroxybiphenyl-3-carbonsäure;
3'-{N'-[1-(3,4-Dimethylphenyl)-3-methoxymethyl-5-oxo-1,5-dihydropyrazol-4-yliden]hydrazino}-2'-hydroxybiphenyl-3-carbonsäure;
3-{N'-[1-(3,4-Dimethylphenyl)-3-methoxymethyl-5-oxo-1,5-dihydropyrazol-4-yliden]hydrazino}-2-hydroxy-3'-tetrazol-5-ylbiphenyl;
3-{N'-[1-(3,4-Difluorphenyl)-3-methyl-5-oxo-1,5-dihydropyrazol-4-yliden]hydrazino}-2-hydroxy-3'-tetrazol-5-ylbiphenyl;
3'-{N'-[1-(3,4-Dimethylphenyl)-5-oxo-3-trifluormethyl-1,5-dihydropyrazol-4-yliden]hydrazino}-2'-hydroxybiphenyl-3-carbonsäure;
3'-{N'-[1-(3,4-Dimethylphenyl)-3-methyl-5-oxo-1,5-dihydropyrazol-4-yliden]hydrazino}-6-fluor-2'-hydroxybiphenyl-3-carbonsäure;
3'-{N'-[1-(3,4-Dimethylphenyl)-5-oxo-3-propyl-1,5-dihydropyrazol-4-yliden]hydrazino}-2'-hydroxybiphenyl-3-carbonsäure;
3-{N'-[1-(3,4-Dimethylphenyl)-5-oxo-3-propyl-1,5-dihydropyrazol-4-yliden]hydrazino}-2-hydroxy-3'-tetrazol-5-ylbiphenyl;
3'-{N'-[1-(3,4-Dimethylphenyl)-3-(1-methyl-1H-pyrrol-3-yl)-5-oxo-1,5-dihydropyrazol-4-yliden]hydrazino}-2'-hydroxybiphenyl-3-carbonsäure;
3-{N'-[1-(3,4-Dimethylphenyl)-3-(1-methyl-1H-pyrrol-3-yl)-5-oxo-1,5-dihydropyrazol-4-yliden]hydrazino}-2-hydroxy-3'-tetrazol-5-ylbiphenyl;
3'-{N'-[1-(3,4-Dimethylphenyl)-3-furan-2-yl-5-oxo-1,5-dihydropyrazol-4-yliden]hydrazino}-2'-hydroxybiphenyl-3-carbonsäure;
3-{N'-[1-(3,4-Dimethylphenyl)-3-furan-2-yl-5-oxo-1,5-dihydropyrazol-4-yliden]hydrazino}-2-hydroxy-3'-tetrazol-5-ylbiphenyl;
N-(2'-Hydroxy-3'-{N'-[3-methyl-5-oxo-1-(4-trifluormethyl-phenyl)-1,5-dihydro-pyrazol-4-yliden]hydrazino}biphenyl-3-yl)-1,1,1-trifluormethansulfonamid;
N-(2'-Hydroxy-3'-{N'-[1-(3-fluor-4-methylphenyl)-3-methyl-5-oxo-1,5-dihydro-pyrazol-4-yliden]hydrazino}biphenyl-3-yl)-1,1,1-trifluormethansulfonamid;
N-(2'-Hydroxy-3'-{N'-[1-(4-fluor-3-methylphenyl)-3-methyl-5-oxo-1,5-dihydro-pyrazol-4-yliden]hydrazino}biphenyl-3-yl)-1,1,1-trifluormethansulfonamid;
N-(2'-Hydroxy-3'-{N'-[1-(3,4-difluorphenyl)-3-methyl-5-oxo-1,5-dihydro-pyrazol-4-yliden]hydrazino}biphenyl-3-yl)-1,1,1-trifluormethansulfonamid;
N-(3'-{N'-[1-(3,4-Dimethylphenyl)-3-methyl-5-oxo-1,5-dihydropyrazol-4-yliden]hydrazino}-2'-hydroxybiphenyl-3-yl)guanidin;
3'-{N'-[1-(3,4-Dimethylphenyl)-3-ethyl-5-oxo-1,5-dihydropyrazol-4-yliden]hydrazino}-2'-hydroxybiphenyl-3-carbonsäure;
3-{N'-[1-(3,4-Dimethylphenyl)-3-ethyl-5-oxo-1,5-dihydropyrazol-4-yliden]hydrazino}-2-hydroxy-3'-tetrazol-5-ylbiphenyl;
3'-{N'-[1-(3,4-Dimethylphenyl)-5-oxo-3-thien-2-yl-1,5-dihydropyrazol-4-yliden]hydrazino}-2'-hydroxybiphenyl-3-carbonsäure;
3'-{N'-[3-Cyclopropyl-1-(3,4-dimethylphenyl)-5-oxo-1,5-dihydropyrazol-4-yliden]hydrazino}-2'-hydroxybiphenyl-3-carbonsäure;
3'-{N'-[1-(3,4-Dimethylphenyl)-5-oxo-3-thiazol-2-yl-1,5-dihydropyrazol-4-yliden]hydrazino}-2'-hydroxybiphenyl-3-carbonsäure;
3'-{N'-[1-(3,4-Dimethylphenyl)-5-oxo-1,5-dihydropyrazol-4-yliden]hydrazino}-2'-hydroxybiphenyl-3-carbonsäure;
3'-{N'-[1-(3,4-Dimethylphenyl)-3-(1-methylethyl)-5-oxo-1,5-dihydropyrazol-4-yliden]hydrazino}-2'-hydroxybiphenyl-3-carbonsäure;
3'-{N'-[3-(Benzyloxymethyl)-1-(3,4-dimethylphenyl)-5-oxo-1,5-dihydropyrazol-4-yliden]hydrazino}-2'-hydroxybiphenyl-3-carbonsäure;
3'-{N'-[3-Ethyl-5-oxo-1-(4-trifluormethylphenyl)-1,5-dihydropyrazol-4-yliden]hydrazino}-2'-hydroxybiphenyl-3-carbonsäure;
3'-{N'-[5-Oxo-1-(4-trifluormethylphenyl)-1,5-dihydropyrazol-4-yliden]hydrazino}-2'-hydroxybiphenyl-3-carbonsäure;
3'-{N'-[1-(3,4-Dimethylphenyl)-3-hydroxymethyl-5-oxo-1,5-dihydropyrazol-4-yliden]hydrazino}-2'-hydroxybiphenyl-3-carbonsäure;
3'-{N'-[3-Benzyloxymethyl-5-oxo-1-(4-trifluormethylphenyl)-1,5-dihydropyrazol-4-yliden]hydrazino}-2'-hydroxybiphenyl-3-carbonsäure;
3'-{N'-[1-(3,4-Dimethylphenyl)-3-methylsulfanylmethyl-5-oxo-1,5-dihydropyrazol-4-yliden]hydrazino}-2'-hydroxybiphenyl-3-carbonsäure;
3'-{N'-[1-(3,4-Dimethylphenyl)-5-oxo-3-thiophen-3-yl-1,5-dihydropyrazol-4-yliden]hydrazino}-2'-hydroxybiphenyl-3-carbonsäure;
3'-{N'-[5-Oxo-1-(4-trifluormethylphenyl)-3-thiophen-3-yl-1,5-dihydropyrazol-4-yliden]hydrazino}-2'-hydroxybiphenyl-3-carbonsäure;
3'-{N'-[5-Oxo-1-(4-trifluormethylphenyl)-3-methylsulfanylmethyl-1,5-dihydropyrazol-4-yliden]hydrazino}-2'-hydroxybiphenyl-3-carbonsäure;
N-(3'-{N'-[1-(3,4-Dimethylphenyl)-3-methyl-5-oxo-1,5-dihydro-pyrazol-4-yliden]hydrazino}-2'-hydroxybiphenyl-3-yl)methansulfonamid;
3'-[N'-(1-Benzo[1,3]dioxol-5-yl-3-methyl-5-oxo-1,5-dihydropyrazol-4-yliden)hydrazino]-2'-hydroxybiphenyl-3-carbonsäure;
3'-{N'-[1-(3,5-Dimethylphenyl)-3-methyl-5-oxo-1,5-dihydropyrazol-4-yliden]hydrazino}-2'-hydroxybiphenyl-3-carbonsäure;
3'-{N'-[1-(3-Chlor-4-methylphenyl)-3-methyl-5-oxo-1,5-dihydropyrazol-4-yliden]hydrazino}-2'-hydroxybiphenyl-3-carbonsäure;
3'-{N'-[1-(3,4-Dimethylphenyl)-3-methyl-5-oxo-1,5-dihydropyrazol-4-yliden]hydrazino}-2'-hydroxybiphenyl-3-phosphonsäure;
3'-{N'-[1-(3,4-Dimethylphenyl)-3-methyl-5-oxo-1,5-dihydropyrazol-4-yliden]hydrazino}-2'-hydroxybiphenyl-3,4-dicarbonsäure;
2',6-Dihydroxy-3'-[N'-[1-(3,4-Dimethylphenyl)-3-methyl-5-oxo-1,5-dihydropyrazol-4-yliden]hydrazino}-biphenyl-3-carbonsäure;
4-Aza-3'-{N'-[1-(3,4-dimethylphenyl)-3-methyl-5-oxo-1,5-dihydropyrazol-4-yliden]hydrazino}-2'-hydroxybiphenyl-5-carbonsäure;
3'-{N'-[1-(3,4-Dimethylphenyl)-5-oxo-1,5-dihydropyrazol-4-yliden]hydrazino}-2'-hydroxybiphenyl-3-carbonsäure;
3'-{N'-[1-(3,4-Dimethylphenyl)-3-methyl-5-oxo-1,5-dihydropyrazol-4-yliden]hydrazino}-2'-hydroxybiphenyl-3-sulfonsäure; und
5-(3'-{N'-[1-(3,4-Dimethylphenyl)-3-methyl-5-oxo-1,5-dihydropyrazol-4-yliden]hydrazino}-2'-hydroxybiphenyl-3-ylmethylen)thiazolidin-2,4-dion;
und pharmazeutisch annehmbare Salze, Hydrate und Solvate davon.

7. Medikament zur Verwendung in der Therapie, hergestellt unter Verwendung einer und umfassend eine Verbindung der Formel (VI), wie in irgendeinem der Ansprüche 1 bis 6 definiert.

8. Pharmazeutische Zusammensetzung, umfassend eine Verbindung der Formel (VI), wie in irgendeinem der Ansprüche 1 bis 6 definiert, und einen pharmazeutisch annehmbaren Träger.

9. Pharmazeutische Zusammensetzung zur Verwendung im Steigern der Plättchenproduktion in einem Menschen, der dieser bedarf, die eine Verbindung der Formel (VI), wie in irgendeinem der Ansprüche 1 bis 6 definiert, und einen pharmazeutisch annehmbaren Träger umfaßt,
und worin die Zusammensetzung für die Verabreichung einer therapeutisch wirksamen Menge der Verbindung der Formel (VI) an den Menschen hergerichtet ist.

10. Pharmazeutische Zusammensetzung zur Verwendung in der Behandlung von Thrombozytopenie, die eine Verbindung der Formel (VI), wie in irgendeinem der Ansprüche 1 bis 6 definiert, und einen pharmazeutisch annehmbaren Träger umfaßt.

11. Pharmazeutische Zusammensetzung gemäß Anspruch 8, 9 oder 10, die für die orale Verabreichung hergerichtet ist.

12. Verwendung einer Verbindung der Formel (VI), wie in irgendeinem der Ansprüche 1 bis 6 definiert, in der Herstellung eines Medikaments zur Verwendung in der Behandlung von Thrombozytopenie.

13. Verwendung einer Verbindung der Formel (VI), wie in irgendeinem der Ansprüche 1 bis 6 definiert, in der Herstellung eines Medikaments zur Verwendung im Steigern der Plättchenproduktion in einem Menschen, der dieser bedarf, und worin das Medikament für die Verabreichung einer therapeutisch wirksamen Menge der Verbindung der Formel (VI) an den Menschen hergerichtet ist.

14. Verwendung gemäß Anspruch 12 oder 13, worin das Medikament für die orale Verabreichung hergerichtet ist.

15. Verfahren zur Herstellung einer pharmazeutischen Zusammensetzung, die einen pharmazeutisch annehmbaren Träger oder Verdünnungsstoff und eine wirksame Menge einer Verbindung der Formel (VI), wie in Anspruch 1 definiert, und pharmazeutisch annehmbarer Salze, Hydrate und Solvate davon enthält, worin das Verfahren das In-Verbindung-Bringen der Verbindung der Formel (VI) mit dem pharmazeutisch annehmbaren Träger oder Verdünnungsstoff umfaßt.

16. Verfahren zur Herstellung einer Verbindung der Formel (VI), wie in irgendeinem der Ansprüche 1 bis 6 definiert,
das Verfahren umfaßt das Umsetzen einer Verbindung der Formel (VII) 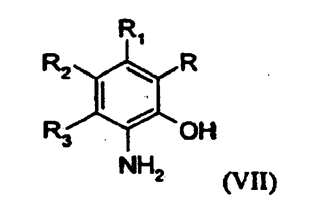 oder einer geschützten Form davon mit einer Verbindung der Formel (VIII) oder einem tautomeren Äquivalent (IX) 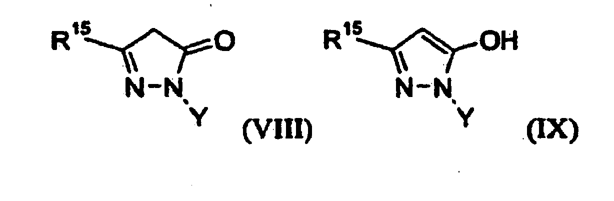 worin
entweder:
R ein Phenyl- oder Pyridinyl-Ring ist, substituiert mit einem oder mehreren der folgenden: -C(O)OH, Tetrazol, Sulfonsäure, Hydroxy, Methyl, Fluor, NHSO₂CF₃ und C(O)NHSO₂CH₃; und R¹ Wasserstoff ist,
oder:
R Wasserstoff ist; und R¹ ein Phenyl- oder Pyridinyl-Ring ist, substituiert mit einem oder mehreren der folgenden: -C(O)OH, Tetrazol, Sulfonsäure, Hydroxy, Methyl, Fluor, NHSO₂CF₃ und C(O)NHSO₂CH₃;
und in jedem Fall:
R² und R³ unabhängig ausgewählt sind aus Wasserstoff, C₁₋₆-Alkyl und Halogen;
R¹⁵ ausgewählt ist aus der Gruppe, bestehend aus C₁₋₆-Alkyl, C₁₋₆-Alkoxy, Trifluormethyl und Halogen; und
Y ausgewählt ist aus Phenyl, das gegebenenfalls mit von einem bis drei Substituenten substituiert ist, ausgewählt aus der Gruppe, bestehend aus: Alkyl, substituiertem Alkyl, Trifluormethyl und Halogen; und
gefolgt, falls nötig oder erwünscht, von Salzbildung.

17. Pharmazeutische Zusammensetzung gemäß Anspruch 9 für die Co-Verabreichung mit einer therapeutisch wirksamen Menge eines Mittels, ausgewählt aus der Gruppe, bestehend aus: einem Kolonie-stimulierenden Faktor, einem Cytokin, einem Chemokin, einem Interleukin oder einem Cytokin-Rezeptoragonisten.

18. Zusammensetzung gemäß Anspruch 17, worin das Mittel ausgewählt ist aus der Gruppe, bestehend aus: G-CSF, GM-CSF, TPO, M-CSF, EPO, Gro-beta, IL-11, SCF, FLT3-Ligand, LIF, IL-3, IL-6, IL-1 oder IL-5 oder einem biologisch aktiven Derivat eines dieser Mittel.

19. In-vitro-Verfahren zur Steigerung der Stimulation der Megakaryozyten-Reifung und/oder der Plättchenproduktion, das die Zugabe einer wirksamen Menge einer Verbindung, wie in Anspruch 1 definiert, zu dem Kulturmedium von Zellen umfaßt, die den TPO-Rezeptor exprimieren.

20. In-vitro-Verfahren zur Steigerung der Stimulation der Megakaryozyten-Reifung und/oder der Plättchenproduktion, das die Zugabe einer wirksamen Menge einer Verbindung, wie in Anspruch 1 definiert, zu dem Kulturmedium von Stammzellen, Knochenmarkzellen, Nabelschnurblutzellen oder peripheren Blutzellen umfaßt.

21. In-vitro-Verfahren zur Steigerung des Überlebens und/oder der Proliferation von Stammzellen, Knochenmarkzellen, Nabelschnurblutzellen, peripheren Blutzellen und weiteren Zelltypen, die den TPO-Rezeptor exprimieren, in Kultur, das das Kultivieren dieser Zellen in einem Medium umfaßt, das eine wirksame Menge einer wie in Anspruch 1 definierten Verbindung enthält.

22. Verfahren gemäß Anspruch 21, ferner umfassend die Co-Verabreichung einer therapeutisch wirksamen Menge eines Kolonie-stimulierenden Faktors, eines Cytokins, eines Chemokins, eines Interleukins oder eines Cytokin-Rezeptoragonisten.

23. Verbindung gemäß Anspruch 1 für die Verwendung als immunologisches Adjuvans.

24. Verbindung gemäß Anspruch 23 für die Verwendung als immunologisches Adjuvans und für die Verabreichung mit einem Impfstoff und/oder Immunmodulator.

25. Verbindung gemäß Anspruch 6, die ausgewählt ist aus:
3'-{N'-[1-(4-tert-Butylphenyl)-3-methyl-5-oxo-1,5-dihydropyrazol-4-yliden]hydrazino}-2'-hydroxybiphenyl-3-carbonsäure;
3'{N'-[1-(3,4-Dimethylphenyl)-3-methyl-5-oxo-1,5-dihydropyrazol-4-yliden]hydrazino}-5'-fluor-2'-hydroxybiphenyl-3-carbonsäure;
3'-{N'-[3-Methyl-5-oxo-1-(4-trifluormethylphenyl)-1,5-dihydropyrazol-4-yliden]hydrazino}-2'-hydroxybiphenyl-3-carbonsäure; und
3'-[N'-[1-(4-Fluor-3-methylphenyl)-3-methyl-5-oxo-1,5-dihydropyrazol-4-yliden]hydrazino}-2'-hydroxybiphenyl-3-carbonsäure.

## Revendications

1. Composé de formule (VI) : 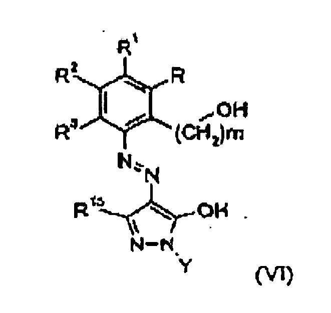 dans laquelle :
R est un groupe aryle substitué ; et R¹ est un atome d'hydrogène ;
ou
R est un atome d'hydrogène et R¹ est un groupe aryle substitué ;
et dans l'un ou l'autre cas :
R² et R³ sont respectivement choisis indépendamment parmi un atome d'hydrogène, un groupe alkyle en C₁ à C₆, alcoxy en C₁ à C₆, nitro, cyano, un atome d'halogène, un groupe aryle, aryle substitué, alkyle substitué, cycloalkyle, acide phosphonique, acide phosphinique et acide sulfonique ;
R¹⁵ est choisi dans le groupe comprenant les groupes alkyle, alkyle substitué, aryle en C₁ à C₁₂, alcoxy et un atome d'halogène ;
m vaut de 0 à 4 ; et
Y est choisi parmi les groupes :
phényle, pyridinyle et pyrimidinyle, les groupes phényle, pyridinyle et pyrimidinyle étant éventuellement substitués par un à trois substituants choisis dans le groupe comprenant les groupes alkyle, alkyle substitué, aryle en C₁ à C₁₂, aryle en C₁ à C₁₂ substitué, alcoxy et un atome d'halogène ;
et dans lequel :
le terme "aryle" tel qu'il est utilisé ici désigne un cycle aromatique cyclique ou polycyclique contenant de 1 à 14 atomes de carbone et éventuellement, contenant de un à cinq atomes de hétéroatomes à condition que si le nombre d'atomes de carbone est de 1, le cycle aromatique contient au moins quatre hétéroatomes, si le nombre d'atomes de carbone est de 2, le cycle aromatique contient au moins trois hétéroatomes, si le nombre d'atomes de carbone est de 3, le cycle aromatique contient au moins deux hétéroatomes et si le nombre d'atomes de carbone est de 4, le cycle aromatique contient au moins un hétéroatome ;
l'expression "aryle en C₁ à C₁₂" telle qu'elle est utilisée ici, désigne un groupe phényle, naphtalène, 3,4-méthylènedioxyphényle, pyridine, biphényme, quinoline, pyrimidine, quinazoline, thiophène, furane, pyrrole, pyrazole, imidazole ou tétrazole ; et
le terme "substitué" tel qu'il est utilisé ici, signifie que le fragment chimique en question a un ou plusieurs substituants choisis dans le groupe comprenant : -CO₂R²⁰, un groupe aryle, -C(O)NHS(O)₂R²⁰, -NHS(O)₂R²⁰, hydroxyalkyle, alcoxy, -C(O)NR²¹R²², un groupe alcoxy, alkyle, amino, N-acylamino, hydroxy, -(CH₂)_{g}C(O)OR⁸, -S(O)ₙR⁸, nitro, tétrazole, cyano, oxo, un atome d'halogène et un groupe trifluorométhyle, où g vaut de 0 à 6, R⁸ est un atome d'hydrogène ou un groupe alkyle, R²⁰ est choisi parmi un atome d'hydrogène, un groupe alkyle en C₁ à C₄, aryle et trifluorométhyle et R²¹ et R²² sont choisis indépendamment parmi un atome d'hydrogène, un groupe alkyle en C₁ à C₄, aryle et trifluorométhyle et n vaut de 0 à 2 ;
et leurs sels, hydrates et solvates pharmaceutiquement acceptables.

2. Composé de formule VI selon la revendication 1, dans lequel :
R¹ est un aryle en C₁ à C₁₂ substitué ;
et
R¹ est un atome d'hydrogène ;
R² et R³ sont respectivement choisis indépendamment parmi un atome d'hydrogène, un groupe alkyle en C₁ à C₆, alcoxy en C₁ à C₆, nitro, cyano, un atome d'halogène, un groupe alkyle substitué et cycloalkyle ;
R¹⁵ est choisi dans le groupe comprenant les groupes alkyle, alkyle substitué, aryle en C₁ à C₁₂, alcoxy et un atome d'halogène ;
m vaut de 0 à 2 ; et
Y est choisi parmi les groupes :
phényle, pyridinyle et pyrimidinyle, si les groupes phényle, pyridinyle et pyrimidinyle sont éventuellement substitués par un à trois substituants choisis dans le groupe comprenant les groupes alkyle, alkyle substitué, aryle en C₁ à C₁₂, aryle en C₁ à C₁₂ substitué, alcoxy et un atome d'halogène ;
et leurs sels, hydrates et solvates pharmaceutiquement acceptables.

3. Composé de formule VI selon la revendication 1, dans laquelle :
R est un groupe phényle ou pyridinyle substitué ; et
R¹ est un atome d'hydrogène ;
R² et R³ sont respectivement choisis indépendamment parmi un atome d'hydrogène, un groupe alkyle en C₁ à C₆, alkyle substitué et un atome d'halogène ;
R¹⁵ est choisi dans le groupe comprenant un groupe alkyle en C₁ à C₄, alcoxy en C₁ à C₄, aryle en C₁ à C₁₂ et atome d'halogène ;
m vaut 0 ; et
Y est choisi parmi les groupes :
phényle, pyridinyle et pyrimidinyle, les groupes phényle, pyridinyle et pyrimidinyle étant éventuellement substitués par un à trois substituants choisis dans le groupe comprenant les groupes alkyle, alkyle substitué, aryle en C₁ à C₁₂, aryle en C₁ à C₁₂ substitué, alcoxy et un atome d'halogène ;
et leurs sels, hydrates et solvates pharmaceutiquement acceptables.

4. Composé de formule VI selon la revendication 1, 2 ou 3, dans lequel :
le terme "substitué" tel qu'il est utilisé ici, sauf indication contraire, signifie que le fragment chimique en question a un substituant choisi dans le groupe comprenant : -CO₂R²⁰, un groupe aryle, -C(O)NHS(O)₂R²⁰, -NHS (O)₂R²⁰, hydroxyalkyle, alcoxy, -C(O)NR²¹R²², un groupe acyloxy, alkyle, amino, N-acylamino, hydroxy, -(CH2)_{g}C(O)OR⁸, -S(O)ₙR⁸, nitro, tétrazole, cyano, oxo, un atome d'halogène et un groupe trifluorométhyle, où g vaut 0, R⁸ est un atome d'hydrogène ou un groupe alkyle, R²⁰ est choisi parmi un atome d'hydrogène, un groupe alkyle en C₁ à C₄, aryle et trifluorométhyle et R²¹ et R²² sont choisis indépendamment parmi un atome d'hydrogène, un groupe alkyle en C₁ à C₄, aryle et trifluorométhyle et n vaut de 0 à 2.

5. Composé de formule VI selon la revendication 1, 2, 3 ou 4, dans laquelle :
le terme "alcoxy" tel qu'il est utilisé ici désigne -OCH₃ ou -OC(CH₃)₂CH₃ ;
le terme "cycloalkyle" ou "cycloalkyle substitué" tel qu'il est utilisé ici désigne un groupe cyclohexyle, 4-hydroxy-cyclohexyle, 2-éthylcyclohexyle, propyl-4-méthoxycyclohexyle, 4-méthoxycyclohexyle, 4-carboxycyclohexyle, cyclopropyle ou cyclopentyle ;
le terme "acyloxy" tel qu'il est utilisé ici désigne -OC(O)CH₃, -OC(O)CH(CH₃)₂ ou -OC(O)(CH₂)₃CH₃ ;
le terme "N-acylamino" tel qu'il est utilisé ici désigne -N(H)C(O)CH₃, -N(H)C(O)CH(CH₃)₂ ou -N(H)C(O)(CH₂)₃CH₃;
le terme "aryloxy" tel qu'il est utilisé ici désigne un groupe phénoxy, 4-fluorophényloxy ou biphényloxy ; et
un substituant alkyle est -CH₃, -CH₂-CH₃, -CH₂-CH₂-CH₃, -CH(CH₃)₂, -C(CH₃)₃, -(CH₂)₃-CH₃, -CH₂-CH(CH₃)₂ ou - CH(CH₃)-CH₂-CH₃.

6. Composé selon la revendication 1, qui est choisi parmi :
l'acide 4'-{N'-[1-(3,4-diméthylphényl)-3-méthyl-5-oxo-1,5-dihydropyrazol-4-ylidène]hydrazino}-3'-hydroxybiphényl-4-carboxylique ;
l'acide 4'-{N'-[1-(3,4-diméthylphényl)-3-méthyl-5-oxo-1,5-dihydropyrazol-4-ylidène]hydrazino}-3'-hydroxybiphényl-3-carboxylique ;
l'acide 3'-{N'-[1-(4-tert-butylphényl)-3-méthyl-5-oxo-1,5-dihydropyrazol-4-ylidène]hydrazino}-2'-hydroxybiphényl-3-carboxylique ;
l'acide 2-aza-3'-{N'-[1-(4-tert-butylphényl)-3-méthyl-5-oxo-1,5-dihydropyrazol-4-ylidène]hydrazino}-5'-chloro-2'-hydroxybiphényl-3-carboxylique ;
l'acide 2-aza-3'-{N'-[1-(4-tert-butylphényl)-3-méthyl-5-oxo-1,5-dihydropyrazol-4-ylidène]hydrazino}-2'-hydroxybiphényl-3-carboxylique ;
l'acide 3-aza-3'-{N'-[1-(4-tert-butylphényl)-3-méthyl-5-oxo-1,5-dihydropyrazol-4-ylidène]hydrazino}-2'-hydroxybiphényl-5-carboxylique ;
l'acide 2-aza-5'-chloro-3'-{N'-[1-(3,4-diméthylphényl)-3-méthyl-5-oxo-1,5-dihydropyrazol-4-ylidène]hydrazino}-2'-hydroxybiphényl-3-carboxylique ;
l'acide 2-aza-3'-{N'-[1-(4-tert-butylphényl)-3-méthyl-5-oxo-1,5-dihydropyrazol-4-ylidène]hydrazino}-2'-hydroxy-5'-méthylbiphényl-3-carboxylique ;
l'acide 2-aza-3'-{N'-[1-(3,4-diméthylphényl)-3-méthyl-5-oxo-1,5-dihydropyrazol-4-ylidène]hydrazino}-2'-hydroxy-5'-méthylbiphényl-3-carboxylique ;
l'acide 3'-{N'-[1-(4-tert-butylphényl)-3-méthyl-5-oxo-1,5-dihydropyrazol-4-ylidène]hydrazino}-2'-hydroxy-5'-méthylbiphényl-3-carboxylique ;
l'acide 3'-{N'-[1-(3,4-diméthylphényl)-3-méthyl-5-oxo-1,5-dihydropyrazol-4-ylidène]hydrazino)-5'-fluoro-2'-hydroxybiphényl-3-carboxylique ;
l'acide 7-({N'-[1-(3,4-diméthylphényl)-3-méthyl-5-oxo-1,5-dihydropyrazol-4-ylidène]hydrazino}-2-hydroxyphényl)quinolin-4[1H]-one-3-carboxylique ;
l'acide 7-({N'-[1-(4-tert-butylphényl)-3-méthyl-5-oxo-1,5-dihydropyrazol-4-ylidène]hydrazino}-2-hydroxyphényl)quinolin-4[1H]-one-3-carboxylique ;
l'acide 3-aza-3'-{N'-[1-(3,4-diméthylphényl)-3-méthyl-5-oxo-1,5-dihydropyrazol-4-ylidène]hydrazino}-2'-hydroxybiphényl-5-carboxylique ;
l'acide 3-aza-3'-(N'-[1-{3-méthyl[4-(1-méthyléthyl)phényl]-5-oxo-1,5-dihydropyrazol-4-ylidène]hydrazino}-2'-hydroxybiphényl)-5-carboxylique ;
l'acide 3-aza-3'-{N'-[1-(4-tétrabutylphényl)-3-méthyl-5-oxo-1,5-dihydropyrazol-4-ylidène]hydrazino}-2'-hydroxybiphényl-5-carboxylique ;
l'acide 5'-chloro-3'-{N'-[1-(3,4-diméthylphényl)-3-méthyl-5-oxo-1,5-dihydropyrazol-4-ylidène]-2'-hydroxybiphényl-3-carboxylique ;
l'acide 3'-{N'-[1-(3,4-diméthylphényl)-3,5-dioxo-1,5-dihydropyrazol-4-ylidène]hydrazino}-2'-hydroxybiphényl-3-carboxylique ;
l'acide 3'-{N'-[1-(2-éthoxy-2-oxoéthyl)-3-méthyl-5-oxo-1,5-dihydropyrazol-4-ylidène]hydrazino}-2'-hydroxybiphényl-3-carboxylique ;
le 3'-{N'-[1-(3,4-diméthylphényl)-3-méthyl-5-oxo-1,5-dihydropyrazol-4-ylidène]hydrazino}-2-hydroxy-4'-(tétrazol-5-yl)biphényle ;
l'acide 3'-(N'-{1-(2-(N-tert-butyl)amino-2-oxoéthyl]-3-méthyl-5-oxo-1,5-dihydropyrazol-4-ylidène}hydrazino)-2'-hydroxybiphényl-3-carboxylique ;
l'acide 3'-{N'-[3-chloro-1-(3,4-diméthylphényl)-5-oxo-1,5-dihydropyrazol-4-ylidène]hydrazino}-2'-hydroxybiphényl)-3-carboxylique ;
le 5-chloro-3-{N'-[1-(3,4-diméthylphényl)-3-méthyl-5-oxo-1,5-dihydropyrazol-4-ylidène]hydrazino}-2-hydroxy-4'-(tétrazol-5-yl)biphényle ;
l'acide 3'-{N'-[1-(3,4-diméthylphényl)-3-méthyl-5-oxo-1,5-dihydropyrazol-4-ylidène]hydrazino}-2'-hydroxybiphényl-3,5-dicarboxylique ;
l'acide 3-aza-3'-{N'-[1-(3,4-diméthylphényl)-3-méthyl-5-oxo-1,5-dihydropyrazol-4-ylidène]hydrazino}-2'-hydroxy-5'-méthylbiphényl-5-carboxylique ;
l'acide 3'-{N'-[1-(3,4-diméthylphényl)-3-méthyl-5-oxo-1,5-dihydropyrazol-4-ylidène]hydrazino}-2'-hydroxybiphényl-4-carboxylique ;
l'acide 3'-{N'-[1-(3,4-diméthylphényl)-3-méthoxy-5-oxo-1,5-dihydropyrazol-4-ylidène]hydrazino}-2'-hydroxybiphényl-3-carboxylique ;
l'acide 3'-{N'-[1-(4-méthoxyphényl)-3-méthyl-5-oxo-1,5-dihydropyrazol-4-ylidène]hydrazino}-2'-hydroxybiphényl-3-carboxylique ;
le (3-{N'-[1-(3,4-diméthylphényl)-3-méthyl-5-oxo-1,5-dihydropyrazol-4-ylidène]hydrazino}-2-hydroxy-3'-biphényl)-1,1,1-trifmuorométhane-sulfonamide ;
l'acide 3'-{N'-[1-(3,4-dichlorophényl)-3-méthyl-5-oxo-1,5-dihydropyrazol-4-ylidène]hydrazino}-2'-hydroxybiphényl-3-carboxylique ;
l'acide 3'-{N'-[3-méthyl-5-oxo-1-(3-trifluorométhylphényl)-1,5-dihydropyrazol-4-ylidène]hydrazino}-2'-hydroxybiphényl-3-carboxylique ;
l'acide 8-{N'-[1-(3,4-diméthylphényl)-3-méthyl-5-oxo-1,5-dihydropyrazol-4-ylidène]hydrazino}quinolin-4[1H]-one-3-carboxylique ;
l'acide 3'-{N'-[3-méthyl-5-oxo-1-(4-trifluorométhylphényl)-1,5-dihydropyrazol-4-ylidène]hydrazino}-2'-hydroxybiphényl-3-carboxylique ;
l'acide 3'-{N'-[3-méthyl-5-oxo-1-(4-N-méthylcarboxamidophényl)-1,5-dihydropyrazol-4-ylidène]hydrazino}-2'-hydroxybiphényl-3-carboxylique ;
le N-[1-(3'-{N'-[1-(3,4-diméthylphényl)-3-méthyl-5-oxo-1,5-dihydropyrazol-4-ylidène]hydrazino}-2'-hydroxybiphényl-3-yl)méthanoyl]méthanesulfonamide ;
l'acide 3'-{N'-[3-méthyl-5-oxo-1-phényl-1,5-dihydropyrazol-4-ylidène]hydrazino}-2'-hydroxybiphényl-3-carboxylique ;
l'acide 3'-{N'-[3-méthyl-1-(4-méthylphényl)-5-oxo-1,5-dihydropyrazol-4-ylidène]hydrazino}-2'-hydroxybiphényl-3-carboxylique ;
l'acide 3'-{N'-[1-(4-chlorophényl)-3-méthyl-5-oxo-1,5-dihydropyrazol-4-ylidène]hydrazino}-2'-hydroxybiphényl-3-carboxylique ;
l'acide 3'-{N'-[1-(4-fluorophényl)-3-méthyl-5-oxo-1,5-dihydropyrazol-4-ylidène]hydrazino}-2'-hydroxybiphényl-3-carboxylique ;
l'acide 3'-{N'-[3-méthyl-5-oxo-1-(4-trifluorométhoxyphényl)-1,5-dihydropyrazol-4-ylidène]hydrazino}-2'-hydroxybiphényl-3-carboxylique ;
l'acide 3'-{N'-[1-(3,4-diméthylphényl)-3-éthoxy-5-oxo-1,5-dihydropyrazol-4-ylidène]hydrazino}-2'-hydroxybiphényl-3-carboxylique ;
l'acide 3'-{N'-[1-(3,4-diméthylphényl)-3-(1-méthyléthoxy)-5-oxo-1,5-dihydropyrazol-4-ylidène]hydrazino}-2'-hydroxybiphényl-3-carboxylique ;
l'acide 3'-{N'-[3-tert-butyl-1-(3,4-diméthylphényl)-5-oxo-1,5-dihydropyrazol-4-ylidène]hydrazino}-2'-hydroxybiphényl-3-carboxylique ;
l'acide 3'-{N'-[3-méthyl-1-(4-méthyl-2,3,5,6-tétrafluorophényl)-5-oxo-1,5-dihydropyrazol-4-ylidène]hydrazino}-2'-hydroxybiphényl-3-carboxylique ;
l'acide 3'-{N'-[1-(4-fluoro-3-méthylphényl)-3-méthyl-5-oxo-1,5-dihydropyrazol-4-ylidène]hydrazino}-2'-hydroxybiphényl-3-carboxylique ;
l'acide 3'-{N'-[1-(3,4-diméthylphényl)-3-phényl-5-oxo-1,5-dihydropyrazol-4-ylidène]hydrazino}-2'-hydroxybiphényl-3-carboxylique ;
le 3-{N'-[1-(3,4-diméthylphényl)-5-oxo-3-phényl-1,5-dihydropyrazol-4-ylidène]hydrazino}-2-hydroxy-3'-tétrazol-5-ylbiphényle ;
le 3-{N'-[1-(3,4-diméthylphényl)-3-méthoxy-5-oxo-1,5-dihydropyrazol-4-ylidène]hydrazino}-2-hydroxy-3'-tétrazol-5-ylbiphényle ;
le 3-{N'-[1-(3,4-diméthylphényl)-3-éthoxy-5-oxo-1,5-dihydropyrazol-4-ylidène]hydrazino}-2-hydroxy-3'-tétrazol-5-ylbiphényle ;
le 3-{N'-[1-(3,4-diméthylphényl)-3-(1-méthyléthoxy)-5-oxo-1,5-dihydropyrazol-4-ylidène]hydrazino}-2-hydroxy-3'-tétrazol-5-ylbiphényle ;
le 3-{N'-[1-(4-fluorophényl)-3-méthyl-5-oxo-1,5-dihydropyrazol-4-ylidène]hydrazino}-2-hydroxy-3'-tétrazol-5-ylbiphényle ;
le 3-{N'-[1-(4-fluoro-3-méthylphényl)-3-méthyl-5-oxo-1,5-dihydropyrazol-4-ylidène]hydrazino}-2-hydroxy-3'-tétrazol-5-ylbiphényle ;
le 3-{N'-[3-méthyl-5-oxo-1-(4-trifluorométhylphényl)-1,5-dihydroxypyrazol-4-ylidène]hydrazino}-2-hydroxy-3'-tétrazol-5-ylbiphényle ;
l'acide 3'-{N'-[1-(3,4-diméthylphényl)-3-(pyridin-4-yl-5-oxo-1,5-dihydropyrazol-4-ylidène]hydrazino}-2'-hydroxybiphényl-3-carboxylique ;
le 3-{N'-[1-(3,4-diméthylphényl)-3-pyridin-4-yl-5-oxo-1,5-dihydropyrazol-4-ylidène]hydrazino}-2-hydroxy-3'-tétrazol-5-ylbiphényle;
le 3-{N'-[1-(3,4-diméthylphényl)-3-pyridin-2-yl-5-oxo-1,5-dihydropyrazol-4-ylidènelhydrazino}-2-hydroxy-3'-tétrazol-5-ylbiphényle ;
l'acide 3-{N'-[1-(3,4-diméthylphényl)-3-(pyridin-2-yl-5-oxo-1,5-dihydropyrazol-4-ylidène]hydrazino}-2'-hydroxybiphényl-3-carboxylique ;
le 3-{N'-[1-(3-fluoro-4-méthylphényl)-3-méthyl-5-oxo-1,5-dihydropyrazol-4-ylidène]hydrazino}-2-hydroxy-3'-tétrazol-5-ylbiphényle ;
l'acide 3'-{N'-[1-(3-fluoro-4-méthylphényl)-3-méthyl-5-oxo-1,5-dihydropyrazol-4-ylidène]hydrazino}-2'-hydroxybiphényl-3-carboxylique ;
l'acide 3'-{N'-[3-méthyl-5-oxo-1-(4-trifluorométhylpyrimidin-2-yl)-1,5-dihydropyrazol-4-ylidène]hydrazino}-2'-hydroxybiphényl-3-carboxylique ;
le 3'-N-tert-butoxycarbonylamino-3-{N'-[1-(3,4-diméthylphényl)-3-méthyl-5-oxo-1,5-dihydropyrazol-4-ylidène]hydrazino}-2-hydroxybiphényle ;
le 3'-amino-3-{N'-[1-(3,4-diméthylphényl)-3-méthyl-5-oxo-1,5-dihydropyrazol-4-ylidène]hydrazino}-2-hydroxybiphényle ;
le 3-{N'-[1-(3-fluorophényl)-3-méthyl-5-oxo-1,5-dihydropyrazol-4-ylidène]hydrazino}-2-hydroxy-3'-tétrazol-5-yl-biphényle ;
l'acide 3'-{N'-[1-(3-fluorophényl)-3-méthyl-5-oxo-1,5-dihydropyrazol-4-ylidène]hydrazino}-2'-hydroxybiphényl-3-carboxylique ;
le 3-{N'-[3-méthyl-5-oxo-1-(2,3,4,5,6-pentafluorophényl)-1,5-dihydropyrazol-4-ylidène]hydrazino}-2-hydroxy-3'-tétrazol-5-ylbiphényle ;
l'acide 3'-{N'-[3-méthyl-5-oxo-1-(2,3,4,5,6-pentafluorophényl)-1,5-dihydropyrazol-4-ylidène]hydrazino}-2'-hydroxybiphényl-3-carboxylique ;
l'acide 3'-{N'-[1-(3,4-difluorophényl)-3-méthyl-5-oxo-1,5-dihydropyrazol-4-ylidène]hydrazino}-2'-hydroxybiphényl-3-carboxylique ;
l'acide 3'-{N'-[1-(3,4-diméthylphényl)-3-méthoxyméthyl-5-oxo-1,5-dihydropyrazol-4-ylidène]hydrazino}-2'-hydroxybiphényl-3-carboxylique ;
le 3-{N'-[1-(3,4-diméthylphényl)-3-méthoxyméthyl-5-oxo-1,5-dihydropyrazol-4-ylidène]hydrazino}-2-hydroxy-3'-tétrazol-5-ylbiphényle ;
le 3-{N'-[1-(3,4-diméthylphényl)-3-méthyl-5-oxo-1,5-dihydropyrazol-4-ylidène]hydrazino}-2-hydroxy-3'-tétrazol-5-ylbiphényle ;
l'acide 3'-{N'-[1-(3,4-diméthylphényl)-5-oxo-3-trifluorométhyl-1,5-dihydropyrazol-4-ylidène]hydrazino}-2'-hydroxybiphényl-3-carboxylique ;
l'acide 3'-{N'-[1-(3,4-diméthylphényl)-3-méthyl-5-oxo-1,5-dihydropyrazol-4-ylidène]hydrazinol-6-fluoro-2'-hydroxybiphényl-3-carboxylique ;
l'acide 3'-{N'-[1-(3,4-diméthylphényl)-5-oxo-3-propyl-1,5-dihydropyrazol-4-ylidène]hydrazino}-2'-hydroxybiphényl-3-carboxylique ;
le 3-{N'-[1-(3,4-diméthylphényl)-5-oxo-3-propyl-1,5-dihydropyrazol-4-ylidène]hydrazino}-2-hydroxy-3'-tétrazol-5-ylbiphényle ;
l'acide 3'-{N'-[1-(3,4-diméthylphényl)-3-(1-méthyl-1H-pyrrol-3-yl)-5-oxo-1,5-dihydropyrazol-4-ylidène]hydrazino}-2_{'}-hydroxybiphényl-3-carboxylique ;
le 3-{N'-[1-(3,4-diméthylphényl)-3-(1-méthyl-1H-pyrrol-3-yl)-5-oxo-1,5-dihydropyrazol-4-ylidène]hydrazino}-2-hydroxy-3'-tétrazol-5-ylbiphényle ;
l'acide 3'-{N'-[1-(3,4-diméthylphényl)-3-furan-2-yl-5-oxo-1,5-dihydropyrazol-4-ylidène]hydrazino}-2'-hydroxybiphényl-3-carboxylique ;
le 3-{N'-[1-(3,4-diméthylphényl)-3-furan-2-yl-5-oxo-1,5-dihydropyrazol-4-ylidène]hydrazino}-2-hydroxy-3'-tétrazol-5-ylbiphényle ;
le N-(2'-hydroxy-3'-{N'-[3-méthyl-5-oxo-1-(4-trifluorométhyl-phényl)-1,5-dihydropyrazol-4-ylidène]hydrazino}-biphényl-3-yl)-1,1,1-trifluorométhanesulfonamide ;
le N-(2'-hydroxy-3'-{N'-[1-(3-fluoro-4-méthylphényl)-3-méthyl-5-oxo-1,5-dihydropyrazol-4-ylidène]hydrazino}-biphényl-3-yl)-1,1,1-trifluorométhanesulfonamide ;
le N-(2'-hydroxy-3'-{N'-[1-(4-fluoro-3-méthylphényl)-3-méthyl-5-oxo-1,5-dihydropyrazol-4-ylidène]hydrazino}-biphényl-3-yl)-1,1,1-trifluorométhanesulfonamide ;
le N-(2'-hydroxy-3'-{N'-[1-(3,4-difluorophényl)-3-méthyl-5-oxo-1,5-dihydropyrazol-4-ylidène]hydrazino}-biphényl-3-yl)-1,1,1-trifluorométhanesulfonamide ;
la N-(3'-{N'-[1-(3,4-diméthylphényl)-3-méthyl-5-oxo-1,5-dihydropyrazol-4-ylidène]hydrazino}-2'-hydroxybiphényl-3-yl)-guanidine ;
l'acide 3'-{N'-[1-(3,4-diméthylphényl)-3-éthyl-5-oxo-1,5-dihydropyrazol-4-ylidène]hydrazino}-2'-hydroxybiphényl-3-carboxylique ;
le 3-{N'-[1-(3,4-diméthylphényl)-3-éthyl-5-oxo-1,5-dihydropyrazol-4-ylidène]hydrazino}-2-hydroxy-3'-tétrazol-5-ylbiphényle ;
l'acide 3'-{N'-[1-(3,4-diméthylphényl)-5-oxo-3-thién-2-yl-1,5-dihydropyrazol-4-ylidène]hydrazino}-2'-hydroxybiphényl-3-carboxylique ;
l'acide 3'-{N'-[3-cyclopropyl-1-(3,4-diméthylphényl)-5-oxo-1,5-dihydropyrazol-4-ylidène]hydrazino}-2'-hydroxybiphényl-3-carboxylique ;
l'acide 3'-{N'-[1-(3,4-diméthylphényl)-5-oxo-3-thiazol-2-yl-1,5-dihydropyrazol-4-ylidène]hydrazino}-2'-hydroxybiphényl-3-carboxylique ;
l'acide 3'-{N'-[1-(3,4-diméthylphényl)-5-oxo-1,5-dihydropyrazol-4-ylidène]hydrazino}-2'-hydroxybiphényl-3-carboxylique ;
l'acide 3'-{N'-[1-(3,4-diméthylphényl)-3-(1-méthyléthyl)-5-oxo-1,5-dihydropyrazol-4-ylidène]hydrazino}-2'-hydroxybiphényl-3-carboxylique ;
l'acide 3'-{N'-[3-(benzyloxyméthyl)-1-(3,4-diméthylphényl)-5-oxo-1,5-dihydropyrazol-4-ylidène]hydrazino}-2'-hydroxybiphényl-3-carboxylique ;
l'acide 3'-{N'-[3-éthyl-5-oxo-1-(4-trifluorométhylphényl)-1,5-dihydropyrazol-4-ylidène]hydrazino}-2'-hydroxybiphényl-3-carboxylique ;
l'acide 3'-{N'-[5-oxo-1-(4-trifluorométhylphényl)-1,5-dihydropyrazol-4-ylidène]hydrazino}-2'-hydroxybiphényl-3-carboxylique ;
l'acide 3'-{N'-[1-(3,4-diméthylphényl)-3-hydroxyméthyl-5-oxo-1,5-dihydropyrazol-4-ylidène]hydrazino}-2'-hydroxybiphényl-3-carboxylique ;
l'acide 3'-{N'-[3-benzyloxyméthyl-5-oxo-1-(4-trifluorométhylphényl)-1,5-dihydropyrazol-4-ylidène]hydrazino}-2'-hydroxybiphényl-3-carboxylique ;
l'acide 3'-{N'-[1-(3,4-diméthylphényl)-3-méthylsulfanylméthyl-5-oxo-1,5-dihydropyrazol-4-ylidène]hydrazino}-2'-hydroxybiphényl-3-carboxylique ;
l'acide 3'-{N'-[1-(3,4-diméthylphényl)-5-oxo-3-thiophén-3-yl-1,5-dihydropyrazol-4-ylidène]hydrazino}-2'-hydroxybiphényl-3-carboxylique ;
l'acide 3'-{N'-[5-oxo-1-(4-trifluorométhylphényl)-3-thiophén-3-yl-1,5-dihydropyrazol-4-ylidène]hydrazino}-2'-hydroxybiphényl-3-carboxylique ;
l'acide 3'-{N'-[5-oxo-1-(4-trifluorométhylphényl)-3-méthylsulfanylméthyl-1,5-dihydropyrazol-4-ylidène]hydrazino}-2'-hydroxybiphényl-3-carboxylique ;
le N-(3'-{N'-[1-(3,4-diméthylphényl)-3-méthyl-5-oxo-1,5-dihydro-pyrazol-4-ylidène]hydrazino}-2'-hydroxybiphényl-3-yl)méthanesulfonamide ;
l'acide 3'-[N'-(1-benzo[1,3]dioxol-5-yl-3-méthyl-5-oxo-1,5-dihydropyrazol-4-ylidène)hydrazino]-2'-hydroxybiphényl-3-carboxylique ;
l'acide 3'-{N'-[1-(3,5-diméthylphényl)-3-méthyl-5-oxo-1,5-dihydropyrazol-4-ylidène]hydrazino}-2'-hydroxybiphényl-3-carboxylique ;
l'acide 3'-{N'-[1-(3-chloro-4-méthylphényl)-3-méthyl-5-oxo-1,5-dihydropyrazol-4-ylidène]hydrazino}-2'-hydroxybiphényl-3-carboxylique ;
l'acide 3'-{N'-[1-(3,4-diméthylphényl)-3-méthyl-5-oxo-1,5-dihydropyrazol-4-ylidène]hydrazino}-2'-hydroxybiphényl-3-phosphonique ;
l'acide 3'-{N'-[1-(3,4-diméthylphényl)-3-méthyl-5-oxo-1,5-dihydropyrazol-4-ylidène]hydrazino}-2'-hydroxybiphényl-3,4-dicarboxylique ;
l'acide 2',6-dihydro-3'-{N'-[1-(3,4-diméthylphényl)-3-méthyl-5-oxo-1,5-dihydropyrazol-4-ylidène]hydrazino}-biphényl-3-carboxylique ;
l'acide 4-aza-3'-{N'-[1-(3,4-diméthylphényl)-3-méthyl-5-oxo-1,5-dihydropyrazol-4-ylidène]hydrazino}-2'-hydroxybiphényl-5-carboxylique ;
l'acide 3'-{N'-[1-(3,4-diméthylphényl)-5-oxo-1,5-dihydropyrazol-4-ylidène]hydrazino}-2'-hydroxybiphényl-3-carboxylique ;
l'acide 3'-{N'-[1-(3,4-diméthylphényl)-3-méthyl-5-oxo-1,5-dihydropyrazol-4-ylidène]hydrazino}-2'-hydroxybiphényl-3-sulfonique ; et
le 5-(3'-{N'-[1-(3,4-diméthylphényl)-3-méthyl-5-oxo-1,5-dihydropyrazol-4-ylidène]hydrazino}-2'-hydroxybiphényl-3-ylméthylène)thiazolidine-2,4-dione ;
et leurs sels, hydrates et solvates pharmaceutiquement acceptables.

7. Médicament à utiliser en traitement, fabriqué en utilisant et comprenant un composant de formule (VI) tel que défini dans l'une quelconque des revendications 1 à 6.

8. Composition pharmaceutique comprenant un composé de formule (VI) tel que défini dans l'une quelconque des revendications 1 à 6, et véhicule pharmaceutiquement acceptable.

9. Composition pharmaceutique à utiliser dans le renforcement de la production de plaquettes chez un être humain qui en a besoin, qui comprend un composé de formule (VI) tel que défini dans l'une quelconque des revendications 1 à 6, et un véhicule pharmaceutiquement acceptable,
et la composition étant destinée à l'administration à l'être humain d'une quantité thérapeutiquement efficace du composé de formule (VI).

10. Composition pharmaceutique à utiliser dans le traitement de la thrombopénie, comprenant un composé de formule (VI) tel que défini dans l'une quelconque des revendications 1 à 6, et un véhicule pharmaceutiquement acceptable.

11. Composition pharmaceutique selon la revendication 8, 9 ou 10, qui est destinée à l'administration orale.

12. Utilisation d'un composé de formule (VI) tel que défini dans l'une quelconque des revendications 1 à 6, dans la production d'un médicament pour l'utilisation dans le traitement de la thrombopénie.

13. Utilisation d'un composé de formule (VI) tel que défini dans l'une quelconque des revendications 1 à 6, dans la production d'un médicament destiné à être utilisé pour renforcer la production de plaquettes chez un être humain qui en a besoin et le médicament étant destiné à l'administration à l'être humain d'une quantité thérapeutiquement efficace du composé de formule (VI).

14. Utilisation selon la revendication 12 ou 13, dans laquelle le médicament est destiné à l'administration orale.

15. Procédé de préparation d'une composition pharmaceutique contenant un véhicule ou diluant pharmaceutiquement acceptable d'un composé de formule (VI) tel que décrit dans la revendication 1 et ses sels, hydrates et solvates pharmaceutiquement acceptables, ledit procédé comprenant l'association du composé de formule (VI) avec le véhicule ou diluant pharmaceutiquement acceptable.

16. Procédé de préparation d'un composé de formule (VI) tel que défini dans l'une quelconque des revendications 1 à 6,
le procédé comprenant la réaction d'un composé de formule (VII) 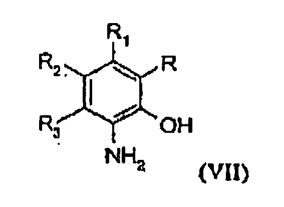 ou une forme protégée de celui-ci avec un composé de formule (VIII) ou un équivalent tautomère (IX) 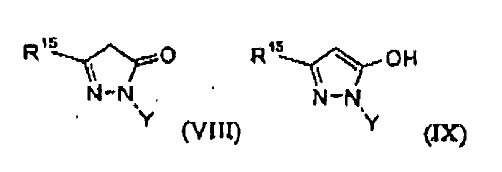 dans laquelle :
soit :
R est un cycle phényle ou pyridinyle substitué par un ou plusieurs des groupes suivants : -C(O)OH, tétrazole, acide sulfonique, hydroxy, méthyle, fluoro, NHSO₂CF₃ et C(O)NHSO₂CH₃ ; et R¹ est un atome d'hydrogène,
soit :
R est un atome d'hydrogène ; et R¹ est un cycle phényle ou pyridinyle substitué par un ou plusieurs des groupes suivants : -C(O)OH, tétrazole, acide sulfonique, hydroxy, méthyle, fluoro, NHSO₂CF₃ et C(O)NHSO₂CH₃ ;
et dans l'un ou l'autre cas :
R² et R³ sont choisis indépendamment parmi un atome d'hydrogène, un groupe alkyle en C₁ à C₆ et un atome d'halogène ;
R¹⁵ est choisi dans le groupe comprenant un groupe alkyle en C₁ à C₆, alcoxy en C₁ à C₆, trifluorométhyle et un atome d'halogène ; et
Y est choisi parmi
un groupe phényle qui est éventuellement substitué par un à trois substituants choisis dans le groupe comprenant un groupe alkyle, alkyle substitué, trifluorométhyle et un atome d'halogène ; et
suivi si nécessaire ou si on le souhaite, par la formation de sel.

17. Composition pharmaceutique selon la revendication 9, pour la co-administration d'une quantité thérapeutiquement efficace d'un agent choisi dans le groupe comprenant un facteur de stimulation des colonies, une cytokine, une chimiokine, une interleukine ou un agoniste du récepteur des cytokines.

18. Composition selon la revendication 17, dans laquelle l'agent est choisi dans le groupe comprenant G-CSF, GM-CSF, TPO, M-CSF, EPO, Gro-bêta, IL-11, SCF, FLT3, un ligand, LIF, IL-3, IL-6, IL-1 ou IL-5 ou un dérivé biologiquement actif de l'un quelconque desdits agents.

19. Procédé *in vitro* de renforcement de la stimulation de la maturation des mégakaryocytes et/ou de la production plaquettaire qui comprend l'addition d'une quantité efficace d'un composé tel que défini à la revendication 1 au milieu de culture de cellules qui expriment le récepteur de TPO.

20. Procédé *in vitro* de renforcement de la stimulation de la maturation des mégakaryocytes et/ou de la production plaquettaire qui comprend l'addition d'une quantité efficace d'un composé tel que défini à la revendication 1 au milieu de culture de cellules souches, de cellules de moelle osseuse et de cellules de sang de cordon ou de cellules de sang périphérique.

21. Procédé *in vitro* de renforcement de la survie et/ou de la prolifération de cellules souches, de cellules de moelle osseuse, de cellules de sang de cordon, de cellules de sang périphérique ou autres types de cellules exprimant le récepteur de TPO en culture, qui comprend la culture desdites cellules dans un milieu contenant une quantité efficace d'un composé tel que défini à la revendication 1.

22. Procédé selon la revendication 21, comprenant en outre la co-administration d'une quantité thérapeutiquement efficace d'un facteur de stimulation des colonies, de cytokine, chimiokine, interleukine ou d'un agoniste du récepteur des cytokines.

23. Composé selon la revendication 1, à utiliser comme adjuvant immunologique.

24. Composé selon la revendication 23, à utiliser comme adjuvant immunologique et pour l'administration avec un vaccin et/ou un immunomodulateur.

25. Composé selon la revendication 6, qui est choisi parmi
l'acide 3'-{N'-[1-(4-tert-butylphényl)-3-méthyl-5-oxo-1,5-dihydropyrazol-4-ylidène]hydrazino}-2'-hydroxybiphényl-3-carboxylique ;
l'acide 3'-{N'-[1-(3,4-diméthylphényl)-3-méthyl-5-oxo-1,5-dihydropyrazol-4-ylidène]hydrazino}-5'-fluoro-2'-hydroxybiphényl-3-carboxylique ;
l'acide 3'-{N'-[3-méthyl-5-oxo-1-(4-trifluorométhylphényl)-1,5-dihydropyrazol-4-ylidène]hydrazino}-2'-hydroxybiphényl-3-carboxylique ; et
l'acide 3'-{N'-[1-(4-fluoro-3-méthylphényl)-3-méthyl-5-oxo-1,5-dihydropyrazol-4-ylidène]hydrazino}-2'-hydroxybiphényl-3-carboxylique.
